# EUROPEAN PATENT SPECIFICATION

(11) **EP 3 746 225 B1**
(45) Date of publication and mention of the grant of the patent: **30.10.2024**
(21) Application number: 19705026.3
(22) Date of filing: 29.01.2019
(51) Int. Cl.: B01L 7/00, C12Q 1/686

(54) **ANALYTICAL SYSTEMS AND METHODS**
ANALYTISCHE SYSTEME UND VERFAHREN
SYSTÈMES ET PROCÉDÉS ANALYTIQUES

(30) Priority: 29.01.2018 US 201862623327 P; 05.02.2018 US 201862626552 P; 09.02.2018 US 201862628919 P; 12.02.2018 US 201862629571 P; 10.07.2018 US 201862696147 P; 17.08.2018 US 201862764946 P
(43) Date of publication of application: 09.12.2020
(73) Proprietor: Gen-Probe Incorporated, San Diego, CA 92121 (US)
(72) Inventor: TIDD, Jennifer L., Vista, California 92084 (US); SHAPIRO, Anne-Laure, La Jolla, California 92037 (US); RHUBOTTOM, Jason F., San Diego, California 92129 (US); LIO, Alberto A., San Diego, California 92126 (US); SCHEER, Timothy J., San Diego, California 92126 (US); TUGGLE, James T., Oceanside, California 92054 (US); SHAH, Ankur H., San Diego, California 92131 (US); OPALSKY, David, San Diego, California 92130 (US)
(74) Representative: Script Intellectual Property LLP
(86) International application number: PCT/US2019/015589
(87) International publication number: WO 2019/148169

(56) References cited:
- EP-A1- 2 907 575
- WO-A1-2013/130910
- WO-A1-2019/014239
- WO-A2-2014/153193

## Description

### TECHNICAL FIELD

The present disclosure relates to methods, an automated analyzer, and a computer program product as set forth in the claims.

### BACKGROUND

Molecular assays are nucleic acid-based tests that are used in clinical diagnosis, screening, monitoring, industrial and environmental testing, health science research, and other applications, to detect the presence or amount of an analyte of interest in a sample, such as a microbe or virus, or to detect genetic abnormalities or mutations in an organism. Molecular assays may permit practitioners to determine the extent of an infection or to monitor the effectiveness of a therapy. As known to people skilled in the art, molecular assays generally include multiple steps leading to the detection or quantification of a target nucleic acid belonging to an organism or virus of interest in a sample. Most molecular assays include a detection step where the sample is exposed to a detection probe or amplification primer that exhibits specificity for the target nucleic acid. To increase the sensitivity of an assay, the target nucleic acid may be amplified by a nucleic acid amplification reaction, such as, for example, Polymerase Chain Reaction ("PCR"), which amplifies the nucleic acid by several orders of magnitude ("amplicon"). PCR employs thermal cycling, which consists of repeated cycles of heating and cooling of a reaction mixture. The reaction is generally initiated with amplification primers (e.g., short DNA fragments containing sequences complementary to the target nucleic acid region), along with enzymes and additional reaction materials. The growth of amplicon over time may be monitored in "real-time" (i.e., while the amplification reaction in progress), or at the conclusion of the reaction (i.e., "end-point" monitoring). The growth of the amplicon may be detected using signal detecting devices (e.g., fluorescence detection devices) that measure signal emissions (e.g., level of fluorescence at a predetermined wavelength or range of wavelengths, etc.) indicative of the amplicon.

Molecular assays may generally be classified as in vitro diagnostic ("IVD") assays and lab developed assays (referred to herein as "Lab Developed Tests" or "LDTs") that are developed, validated and used by a customer or other third party. In a world of newly emerging pathogens and variants, customers or other third parties may wish to develop LDTs for detecting a targeted analyte for which no IVD is commercially available, or the customer or third party may wish to develop an LDT by incorporating an analyte specific reagent ("ASR") with an IVD to supplement the IVD.

Molecular LDTs require amplification oligomers, detection probes, etc. that are usually specific to the particular LDT. Known analytical systems capable of performing LDTs are designed to perform IVD assays and LDTs in batch mode or without the use of shared modules or resources. When performed in batch mode, a first assay type (e.g., IVD or LDT) is completed on a first collection of samples before initiating a second assay type on a second collection of samples. Often, reagents and consumables for performing the second assay type are not introduced into the system until after completion of the first assay type.

A molecular assay, such as a nucleic acid amplification assay, is performed by a computer controlled, automated molecular system in accordance with different parameters that define a protocol for performing the assay. In general, these parameters define the steps performed by system during the assay (e.g., the types and quantities of reagents to be used, incubation conditions, temperature cycling parameters (e.g., cycle times, temperatures, including denaturation, annealing and extension temperatures, selection of an RNA or DNA target, etc.), etc.). These parameters also define data processing, data reduction, and result interpretation for the data generated by the protocols.

Often the protocols (i.e., parameters) for IVD assays that are performed on a molecular system are preinstalled/preloaded on the system. Since IVD assays are known standardized (and regulated) assays, their parameters are typically known and/or fixed and cannot be changed by a user. Because LDTs are developed or established by a user or a third party, custom protocols may be required as at least some of the parameters that define LDT protocols are provided by the user/third party.

EP2907575 describes a laboratory device with user input function and a method for user input in a laboratory device.

There is a need, therefore, to improve the flexibility of molecular systems so as to be able to perform assay protocols that are not are preinstalled/preloaded on the system and to facilitate the configurability by the user in the operation of the molecular system.

### SUMMARY

The aspects and embodiments of the present invention are set forth in the claims.

Methods and systems are disclosed that enable a user to define an LDT by selecting user-defined assay parameters associated with the assay.

A software tool is capable of generating assay protocols for molecular systems. Each assay may be defined in an Assay Definition File (ADF), which may include information that describes how to process results, what process steps are executed, the order they are executed, interpretations generated, etc. The software tool enables a user to develop and define an LDT via one or more windows, screens, or graphical user interfaces ("GUIs") that include interactive buttons, menus, and/or icons that provide access to different functions and information.

As will be described in more detail later, after an LDT is run or performed by the molecular system and a data set is obtained, a controller may enable the user to process the data and review the results of the assay. The controller may also enable the user to modify at least some of the user-defined assay parameters, rerun the data set using the modified user-defined assay parameters, and re-review the results to study the effect of the selected user-defined assay parameters on the assay results. Thus, the controller may enable a user to determine an optimized set of user-defined assay parameters (e.g., a set of user-defined assay parameters that produces the results approved by the user) for performing the LDT. The controller may then allow a user to associate the optimized user-defined assay parameters with the created (or established) LDT protocol and finalize and lock the parameters (e.g., so that they are not inadvertently changed) for the developed LDT.

### DESCRIPTION OF THE DRAWINGS

The accompanying drawings, which are incorporated herein and form part of the specification, illustrate various, non-limiting embodiments of the present disclosure. Where appropriate, reference numerals illustrating like structures, components, materials and/or elements in different drawings are labeled similarly. It should be understood that various combinations of the structures, components, and/or elements, other than those specifically shown in these drawings, are contemplated and are within the scope of the present disclosure.

For simplicity and clarity of illustration, the drawings depict the general structure and/or manner of construction of the described embodiments, as well as associated methods of manufacture. Well-known features (e.g., fasteners, electrical connections, control systems, etc.) are not shown in these drawings (and not described in the corresponding description for brevity) to avoid obscuring other features, since these features are well known to those of ordinary skill in the art. The features in the drawings are not necessarily drawn to scale. The dimensions of some features may be exaggerated relative to other features to improve understanding of the exemplary embodiments. Cross-sectional views are provided to help illustrate the relative positioning of various features. One skilled in the art would appreciate that the cross-sectional views are not necessarily drawn to scale and should not be viewed as representing proportional relationships between different features. It should be noted that, even if it is not specifically mentioned, aspects and features described with reference to one embodiment may also be applicable to, and may be used with, other embodiments.
FIGs. 1A-1B are perspective views of an analytical system.
FIGs. 2A-2E are top plan views of different regions of exemplary first modules of the analytical system of FIG. 1A.
FIG. 2F is a perspective view of an exemplary magnetic wash station of the analytical system of FIG. 1A.
FIG. 2G is a perspective view of an exemplary magnetic moving apparatus of the magnetic wash station of FIG. 2F.
FIGs. 3A-3C are perspective views of an exemplary sample bay of the analytical system of FIG. 1A.
FIG. 4A-4B are perspective views of an exemplary sample holding rack that may be used in the sample bay of FIG. 3A.
FIGs. 5A-5F are top plan views of different regions of exemplary second modules of the analytical system of FIG. 1A.
FIGs. 6A-6D are different views of an exemplary reagent container carrier of the analytical system of FIG. 1A.
FIGs. 7A-7C are different views of another exemplary reagent container carrier of the analytical system of FIG. 1A.
FIG. 8 is a perspective view of an exemplary reagent container transport mechanism of the analytical system of FIG. 1A.
FIGs. 9A-9C are different views of an exemplary reagent container carrier of the analytical system of FIG. 1A.
FIGs. 10A-10C are different views of an exemplary reagent container of the analytical system of FIG. 1A.
FIGs. 11A-11B are different views of another exemplary reagent container of the analytical system of FIG. 1A.
FIGs. 12A-12B are exemplary graphical user interfaces (GUIs) displayed in a display device of the analytical system of FIG. 1A.
FIGs. 13A-13D are different views of an exemplary reagent pack of the analytical system of FIG. 1A.
FIG. 14A is a perspective view of an exemplary fluid transfer and handling system of the analytical system of FIG. 1A.
FIGs. 14B-14C are perspective views of a bottom portion of an exemplary pipettor of the fluid transfer and handling system of FIG. 14A
FIGs. 15A-15B are different views of an exemplary cap/vial assembly of the analytical system of FIG. 1A.
FIGs. 16A-16I are different views of a thermal cycler of the analytical system of FIG. 1A.
FIGs. 17A-17B are different views of an exemplary signal detector of the analytical system of FIG. 1A.
FIGs. 18A-18C are different views of an exemplary centrifuge of the analytical system of FIG. 1A.
FIG. 19 is a perspective view of an exemplary multi-receptacle unit (MRU) of the analytical system of FIG. 1A.
FIGs. 20A-20B are perspective views of an exemplary receptacle distribution system of the analytical system of FIG. 1A.
FIGs. 21A-21D illustrate different views of exemplary receptacle distributor of the receptacle distribution system of FIG. 20A.
FIGs. 22A-22B are different views of an exemplary receptacle handoff device of the analytical system of FIG. 1A.
FIGs. 23A-23B are different views of an exemplary reagent pack loading station of the analytical system of FIG. 1A.
FIG. 24 is a perspective view of an exemplary reagent pack carousel of the analytical system of FIG. 1A.
FIG. 25 illustrates an exemplary fluid transfer device of the analytical system of FIG. 1A.
FIG. 26 is a flow chart of an exemplary extraction process using the analytical system of FIG. 1A.
FIG. 27 is a flow chart of an exemplary reaction setup process using the analytical system of FIG. 1A.
FIG. 28 is a flow chart of an exemplary thermal cycling process using the analytical system of FIG. 1A.
FIG. 29 is a flow chart of an exemplary sample preparation process using the analytical system of FIG. 1A.
FIG. 30 is a flowchart of an exemplary reaction mixture preparation process using the analytical system of FIG. 1A.
FIG. 31 is a flowchart of an exemplary nucleic acid amplification reaction process (such as, for example, PCR) using the analytical system of FIG. 1A.
FIG. 32 is a flowchart of a method of performing multiple assays using the analytical system of FIG. 1A.
FIG. 33 is a schematic illustration of an exemplary control system of the analytical system of FIG. 1A.
FIGs. 34A-34M are exemplary GUIs used to develop an LDT protocol for the analytical system of FIG. 1A.
FIGs. 35A-35C are flowcharts of exemplary method for performing data analysis on the data produced by the analytical system of FIG. 1A.
FIGs. 36A-36F are exemplary plots illustrating the effect of different data analysis operations on the data produced by the analytical system of FIG. 1A.
FIGs. 37A-37C are exemplary GUIs used to install an LDT protocol on the analytical system of FIG. 1A.
FIG. 38 is an exemplary GUI that illustrates the association of assays with samples in the analytical system of FIG. 1A.
FIG. 39 is a schematic view of a workflow for protocol optimization.

The features and advantages of the present disclosure will become more apparent from the detailed description set forth below when taken in conjunction with the drawings. There are many embodiments described and illustrated herein. Each of the aspects/features described with reference to one embodiment may be employed in combination with aspects/features disclosed with reference to another embodiment. For the sake of brevity, many of these combinations and permutations are not discussed separately herein.

### DETAILED DESCRIPTION

Unless defined otherwise, all terms of art, notations and other scientific terms or terminology used herein have the same meaning as is commonly understood by one of ordinary skill in the art to which this disclosure belongs. Many of the techniques and procedures described or referenced herein are well understood and commonly employed using conventional methodology by those skilled in the art. As appropriate, procedures involving the use of commercially available kits and reagents are generally carried out in accordance with manufacturer defined protocols and/or parameters unless otherwise noted. None of the references described or referenced herein is admitted to be prior art to the current disclosure.

References in the specification to "one embodiment," "an embodiment," a "further embodiment," "an example embodiment," "some aspects," "a further aspect," "aspects," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, such feature, structure, or characteristic is also a description in connection with other embodiments whether or not explicitly described. As used herein, "a" or "an" means "at least one" or "one or more."

As used herein, "sample" refers to any substance suspected of containing an organism, virus or cell of interest or, alternatively, an analyte derived from an organism, virus or cell of interest, or any substance suspected of containing an analyte of interest. The substance may be, for example, an unprocessed clinical specimen, such as a blood or genitourinary tract specimen, a buffered medium containing the specimen, a medium containing the specimen and lytic agents for releasing an analyte belonging to an organism, virus or cell, or a medium containing an analyte derived from an organism, virus or cell which has been isolated and/or purified ("extracted") in a receptacle or on a material or device. For this reason, the term "sample" will be understood to mean a specimen in its raw form or to any stage of processing to release, isolate and purify ("extract") an analyte derived from the organism, virus or cell. Thus, references to a "sample" may refer to a substance suspected of containing an analyte derived from an organism, virus or cell at different stages of processing and is not limited to the initial form of the substance.

With reference to nucleic acids, the term "extraction" refers to the recovery of a nucleic acid molecule (e.g., DNA or RNA of any form) from a sample comprising non-nucleic acid components, such as the native environment of the nucleic acid molecule, a partially purified sample, or a crude sample (i.e., a sample that is in substantially the same form as it was upon being obtained from its source). Extraction can result in substantially purified nucleic acid molecules or nucleic acid molecules that are in a more pure form than in the pre-extraction sample and can be used to obtain such molecules for use in analytical procedures from samples comprising biological material, such as cells (including cells isolated directly from a source or cultured), blood, urine, mucus, semen, saliva, or tissue (e.g., a biopsy). Many extraction methods are available. In various embodiments, extraction may comprise one or more of cell lysis, removal of insoluble material such as by centrifugation or filtration, chromatography, precipitation of nucleic acids, or capture of nucleic acids with capture probes.

An "analyte" refers to a molecule present or suspected of being present in a sample and which is targeted for detection in an assay. Exemplary types of analytes include biological macromolecules such as nucleic acids, polypeptides, and prions.

"Nucleic acid" and "polynucleotide" refer to a multimeric compound comprising nucleosides or nucleoside analogs which have nitrogenous heterocyclic bases or base analogs linked together to form a polynucleotide, including conventional RNA, DNA, mixed RNA-DNA, and polymers that are analogs thereof. A nucleic acid "backbone" can be made up of a variety of linkages, including one or more of sugar-phosphodiester linkages, peptide-nucleic acid bonds ("peptide nucleic acids" or PNA; International Publication No. WO 95/32305), phosphorothioate linkages, methylphosphonate linkages, or combinations thereof. Sugar moieties of a nucleic acid can be ribose, deoxyribose, or similar compounds with substitutions, e.g., 2' methoxy or 2' halide substitutions. Nitrogenous bases can be conventional bases (A, G, C, T, U), analogs thereof (e.g., inosine or others; see The Biochemistry of the Nucleic Acids 5-36, Adams et al., ed., 11th ed., 1992), derivatives of purines or pyrimidines (e.g., N⁴-methyl guanine, N⁶-methyladenine, deaza- or aza-purines, deaza- or aza-pyrimidines, pyrimidine bases with substituent groups at the 5 or 6 position (e.g., 5-methylcytosine), purine bases with a substituent at the 2, 6, or 8 positions, 2-amino-6-methylaminopurine, O⁶-methylguanine, 4-thio-pyrimidines, 4-amino-pyrimidines, 4-dimethylhydrazine-pyrimidines, and O⁴-alkyl-pyrimidines; U.S. Patent No. 5,378,825 and International Publication No. WO 93/13121). Nucleic acids can include one or more "abasic" residues where the backbone includes no nitrogenous base for position(s) of the polymer (U.S. Patent No. 5,585,481). A nucleic acid can comprise only conventional RNA or DNA sugars, bases and linkages, or can include both conventional components and substitutions (e.g., conventional bases with 2' methoxy linkages, or polymers containing both conventional bases and one or more base analogs). Nucleic acid includes "locked nucleic acid" (LNA), an analogue containing one or more LNA nucleotide monomers with a bicyclic furanose unit locked in an RNA mimicking sugar conformation, which enhance hybridization affinity toward complementary RNA and DNA sequences (Vester and Wengel, 2004, Biochemistry 43(42): 13233-41). Embodiments of oligomers that can affect stability of a hybridization complex include PNA oligomers, oligomers that include 2'-methoxy or 2'-fluoro substituted RNA, or oligomers that affect the overall charge, charge density, or steric associations of a hybridization complex, including oligomers that contain charged linkages (e.g., phosphorothioates) or neutral groups (e.g., methylphosphonates). Methylated cytosines such as 5-methylcytosines can be used in conjunction with any of the foregoing backbones/sugars/linkages including RNA or DNA backbones (or mixtures thereof) unless otherwise indicated. RNA and DNA equivalents have different sugar moieties (i.e., ribose versus deoxyribose) and can differ by the presence of uracil in RNA and thymine in DNA. The differences between RNA and DNA equivalents do not contribute to differences in homology because the equivalents have the same degree of complementarity to a particular sequence. It is understood that when referring to ranges for the length of an oligonucleotide, amplicon, or other nucleic acid, that the range is inclusive of all whole numbers (e.g., 19-25 contiguous nucleotides in length includes 19, 20, 21, 22, 23, 24, and 25).

"Nucleic acid amplification" or simply "amplification" refers to any *in vitro* procedure that produces multiple copies of a target nucleic acid sequence, or its complementary sequence, or fragments thereof (i.e., an amplified sequence containing less than the complete target nucleic acid). Amplification methods include, for example, replicase-mediated amplification, polymerase chain reaction (PCR), ligase chain reaction (LCR), strand-displacement amplification (SDA), helicase-dependent amplification (HDA), transcription-mediated amplification (TMA), and nucleic acid sequence-based amplification (NASBA). TMA and NASBA are both forms of transcription-based amplification. Replicase-mediated amplification uses self-replicating RNA molecules, and a replicase such as QB-replicase (see, e.g., U.S. Patent No. 4,786,600). PCR uses a DNA polymerase, pairs of primers, and thermal cycling to synthesize multiple copies of two complementary strands of dsDNA or from a cDNA (see, e.g., U.S. Patent Nos. 4,683,195, 4,683,202, and 4,800,159). LCR uses four or more different oligonucleotides to amplify a target and its complementary strand by using multiple cycles of hybridization, ligation, and denaturation (see, e.g., U.S. Patent Nos. 5,427,930 and 5,516,663). SDA uses a primer that contains a recognition site for a restriction endonuclease and an endonuclease that nicks one strand of a hemimodified DNA duplex that includes the target sequence, whereby amplification occurs in a series of primer extension and strand displacement steps (see, e.g., U.S. Patent Nos. 5,422,252, 5,547,861, and 5,648,211). HDA uses a helicase to separate the two strands of a DNA duplex generating single-stranded templates, followed by hybridization of sequence-specific primers hybridize to the templates and extension by DNA polymerase to amplify the target sequence (see, e.g., U.S. Patent No. 7,282,328). Transcription-based amplification uses a DNA polymerase, an RNA polymerase, deoxyribonucleoside triphosphates, ribonucleoside triphosphates, a promoter-containing oligonucleotide, and optionally can include other oligonucleotides, to ultimately produce multiple RNA transcripts from a nucleic acid template. Examples of transcription-based amplification are described in U.S. Patent Nos. 4,868,105, 5,124,990, 5,130,238, 5,399,491, 5,409,818, and 5,554,516; and in International Publication Nos. WO 88/01302, WO 88/10315 and WO 95/03430. Amplification may be either linear or exponential.

In cyclic amplification methods that detect amplicons in real-time, the term "threshold cycle" (Ct) is a measure of the emergence time of a signal associated with amplification of target, and may, for example, be approximately 10x standard deviation of the normalized reporter signal. Once an amplification reaches the "threshold cycle," generally there is considered to be a positive amplification product of a sequence to which the probe binds. Binding of the probe generally provides substantial information about the identity of the product (e.g., that it is an amplicon from a particular target sequence or a member of a certain class of alleles of a gene in the case of one or more allele-specific probe(s)). The amplification product can additionally be further characterized through methods known to one of skill in the art, such as gel electrophoresis, nucleic acid sequencing, and other such analytical procedures.

An "oligomer" or "oligonucleotide" refers to a nucleic acid of generally less than 1,000 nucleotides (nt), including those in a size range having a lower limit of about 2 to 5 nt and an upper limit of about 500 to 900 nt. Some particular embodiments are oligomers in a size range with a lower limit of about 5 to 15, 16, 17, 18, 19, or 20 nt and an upper limit of about 50 to 600 nt, and other particular embodiments are in a size range with a lower limit of about 10 to 20 nt and an upper limit of about 22 to 100 nt. Oligomers can be purified from naturally occurring sources, but can be synthesized by using any well-known enzymatic or chemical method. Oligomers can be referred to by a functional name (e.g., capture probe, primer or promoter primer) but those skilled in the art will understand that such terms refer to oligomers. Oligomers can form secondary and tertiary structures by self-hybridizing or by hybridizing to other polynucleotides. Such structures can include, but are not limited to, duplexes, hairpins, cruciforms, bends, and triplexes. Oligomers may be generated in any manner, including chemical synthesis, DNA replication, reverse transcription, PCR, or a combination thereof. In some embodiments, oligomers that form invasive cleavage structures are generated in a reaction (e.g., by extension of a primer in an enzymatic extension reaction).

By "amplicon" or "amplification product" is meant a nucleic acid molecule generated in a nucleic acid amplification reaction and which is derived from a target nucleic acid. An amplicon or amplification product contains a target nucleic acid sequence that can be of the same or opposite sense as the target nucleic acid. In some embodiments, an amplicon has a length of about 100-2000 nucleotides, about 100-1500 nucleotides, about 100-1000 nucleotides, about 100-800 nucleotides, about 100-700 nucleotides, about 100-600 nucleotides, or about 100-500 nucleotides.

An "amplification oligonucleotide" or "amplification oligomer" refers to an oligonucleotide that hybridizes to a target nucleic acid, or its complement, and participates in a nucleic acid amplification reaction, e.g., serving as a primer and/or promoter-primer. Particular amplification oligomers contain at least 10 contiguous bases, and optionally at least 11, 12, 13, 14, 15, 16, 17, 18, 19, or 20 contiguous bases, that are complementary to a region of the target nucleic acid sequence or its complementary strand. The contiguous bases can be at least 80%, at least 90%, or completely complementary to the target sequence to which the amplification oligomer binds. In some embodiments, an amplification oligomer comprises an intervening linker or non-complementary sequence between two segments of complementary sequence, e.g., wherein the two complementary segments of the oligomer collectively comprise at least 10 complementary bases, and optionally at least 11, 12, 13, 14, 15, 16, 17, 18, 19, or 20 complementary bases. One skilled in the art will understand that the recited ranges include all whole and rational numbers within the range (e.g., 92% or 98.377%). Particular amplification oligomers are 10 to 60 bases long and optionally can include modified nucleotides.

A "primer" refers to an oligomer that hybridizes to a template nucleic acid and has a 3' end that is extended by polymerization. A primer can be optionally modified, e.g., by including a 5' region that is non-complementary to the target sequence. Such modification can include functional additions, such as tags, promoters, or other sequences that may be used or useful for manipulating or amplifying the primer or target oligonucleotide. Examples of primers incorporating tags, or tags and promoter sequences, are described in U.S. Patent No. 9,284,549. A primer modified with a 5' promoter sequence can be referred to as a "promoter-primer." A person of ordinary skill in the art of molecular biology or biochemistry will understand that an oligomer that can function as a primer can be modified to include a 5' promoter sequence and then function as a promoter-primer, and, similarly, any promoter-primer can serve as a primer with or without its 5' promoter sequence.

A "forward amplification oligomer" (e.g., forward primer) is configured to hybridize to the (-) strand of a target nucleic acid and can have a sequence partially or completely identical to the sequence of the (+) strand of the target nucleic acid. A "reverse amplification oligomer" (e.g., reverse primer) is configured to hybridize to the (+) strand of a target nucleic acid and can have a sequence partially or completely identical to the sequence of the (-) strand of the target nucleic acid. Unless otherwise indicated, the (+) strand refers to the coding strand of a protein-coding nucleic acid and the transcribed strand of non-coding sequences such as ribosomal and transfer RNAs and their corresponding DNAs, and the (-) strand refers to the reverse complement of the (+) strand.

"Detection oligomer" or "detection probe" as used herein refers to an oligomer that interacts with a target nucleic acid to form a detectable complex. A probe's target sequence generally refers to the specific sequence within a larger sequence (e.g., gene, amplicon, locus, etc.) to which the probe specifically hybridizes. A detection oligomer can include target-specific sequences and a non-target-complementary sequence. Such non-target-complementary sequences can include sequences which will confer a desired secondary or tertiary structure, such as a flap or hairpin structure, which can be used to facilitate detection and/or amplification (e.g., U.S. Patent Nos. 5,118,801, 5,312,728, 6,835,542, 6,849,412, 5,846,717, 5,985,557, 5,994,069, 6,001,567, 6,913,881, 6,090,543, and 7,482,127; International Publication Nos. WO 97/27214 and WO 98/42873; Lyamichev et al., Nat. Biotech., 17:292 (1999); and Hall et al., PNAS, USA, 97:8272 (2000)). Probes of a defined sequence can be produced by techniques known to those of ordinary skill in the art, such as by chemical synthesis, and by in vitro or in vivo expression from recombinant nucleic acid molecules.

"Label" or "detectable label" as used herein refers to a moiety or compound that is detected or leads to a detectable signal. The label may be joined directly or indirectly to a probe or it may be, for example, an intercalating dye (e.g., SYBR^{®} Green). Direct joining can use covalent bonds or non-covalent interactions (e.g., hydrogen bonding, hydrophobic or ionic interactions, and chelate or coordination complex formation), whereas indirect joining can use a bridging moiety or linker (e.g., via an antibody or additional oligonucleotide(s). Any detectable moiety can be used, e.g., radionuclide, ligand such as biotin or avidin, enzyme, enzyme substrate, reactive group, chromophore such as a dye or particle (e.g., latex or metal bead) that imparts a detectable color, luminescent compound (e.g. bioluminescent, phosphorescent, or chemiluminescent compound), and fluorescent compound (i.e., fluorophore). Embodiments of fluorophores include those that absorb light (e.g., have a peak absorption wavelength) in the range of 495 to 690 nm and emit light (e.g., have a peak emission wavelength) in the range of 520 to 710 nm, which include those known as FAM^{®}, TET^{®}, HEX^{®}, CAL FLUOR^{®} (Orange or Red), CY^{®}, and QUASAR^{®} compounds. Fluorophores can be used in combination with a quencher molecule that absorbs light when in close proximity to the fluorophore to diminish background fluorescence. Such quenchers are well known in the art and include, e.g., BLACK HOLE QUENCHER^{®} (or BHQ^{®}), Blackberry Quencher^{®} (or BBQ-650^{®}), Eclipse^{®}, or TAMRA^{™} compounds. Particular embodiments include a "homogeneous detectable label" that is detectable in a homogeneous system in which bound labeled probe in a mixture exhibits a detectable change compared to unbound labeled probe, which allows the label to be detected without physically removing hybridized from unhybridized labeled probe (e.g., U.S. Patent Nos. 5,283,174, 5,656,207, and 5,658,737). Exemplary homogeneous detectable labels include chemiluminescent compounds, including acridinium ester ("AE") compounds, such as standard AE or AE derivatives which are well known (U.S. Patent Nos. 5,656,207, 5,658,737, and 5,639,604). Methods of synthesizing labels, attaching labels to nucleic acid, and detecting signals from labels are known (e.g., Sambrook et al., Molecular Cloning, A Laboratory Manual, 2nd ed. (Cold Spring Harbor Laboratory Press, Cold Spring Harbor, NY, 1989) at Chapt. 10, and U.S. Patent Nos. 5,658,737, 5,656,207, 5,547,842, 5,283,174, 5,585,481, 5,639,604, and 4,581,333, and European Patent No. 0 747 706). Other detectably labeled probes include FRET cassettes, TaqMan^{®} probes, and probes that undergo a conformational change in the presence of a targeted nucleic acid, such as molecular torches and molecular beacons. FRET cassettes are described in U.S. Patent Application Publication No. 2005/0186588 and U.S. Patent No. 9,096,893. TaqMan^{®} probes include a donor and acceptor label wherein fluorescence is detected upon enzymatically degrading the probe during amplification in order to release the fluorophore from the presence of the quencher. Chemistries for performing TaqMan assays are described in PCT Application No. PCT/US2018/024021, filed March 23, 2018, and U.S. Patent No. 5,723,591. Molecular torches and beacons exist in open and closed configurations wherein the closed configuration quenches the fluorophore and the open position separates the fluorophore from the quencher to allow a change in detectable fluorescent signal. Hybridization to target opens the otherwise closed probes. Molecular torches are described in U.S. Patent No. 6,361,945; and molecular beacons are described in U.S. Patent No. 6,150,097.

"Target capture" or "a target capture procedure" as used herein refers to a procedure for immobilizing a target analyte on a solid support and purifying the analyte by removing potential inhibitors of an amplification reaction (e.g., heparin, proteins, and heme).

"Capture probe," "target capture probe," "capture oligonucleotide," "capture oligomer," "target capture oligomer," and "capture probe oligomer" are used interchangeably herein to refer to a nucleic acid oligomer that hybridizes to a target sequence in a target nucleic acid by standard base pairing and joins to a binding partner on an immobilized probe to capture the target nucleic acid to a support. In one embodiment, "target capture" refers to a process in which a target nucleic acid is purified or isolated by hybridization to a capture probe. In another embodiment, "target capture" refers to direct immobilization of a target nucleic acid on a solid support. One example of a capture probe includes two binding regions: a sequence-binding region (e.g., target-specific portion) and an immobilized probe-binding region, usually on the same oligomer, although the two regions may be present on two different oligomers joined together by one or more linkers. Another embodiment of a capture probe uses a target-sequence binding region that includes random or non-random poly-GU, poly-GT, or poly U sequences to bind non-specifically to a target nucleic acid and link it to an immobilized probe on a support.

An "internal control" refers to a molecule detected in order to validate an assay result, such as a negative assay result in which no analyte was detected. An internal control can be supplied in an assay kit or composition, or it can be an endogenous molecule present in essentially all samples tested in an assay (e.g., a housekeeping gene or mRNA for assays that test samples comprising cells). In assays in which the analyte is a nucleic acid, an internal control typically has a sequence different from the analyte at least in part, but it can have properties that result in similar amplification and detection characteristics (e.g., similar GC content). A nucleic acid internal control can be amplified with dedicated amplification oligomers or with the same amplification oligomers as an analyte. An internal control nucleic acid can lack the sequence targeted by probe oligomers for the analyte and contain a sequence targeted by a probe oligomer specific for the internal control.

The term "buffer" as used herein refers to any solution with a controlled pH that may serve to dissolve a solid (e.g., lyophilized) substance (e.g., reagent, sample, or combination thereof) or as a diluent to dilute a liquid (e.g., a liquid reagent, liquid sample, or combination thereof; or a solution of a reagent, sample, or combination thereof).

An "elution buffer" is a buffer for releasing a nucleic acid from a solid support, including from a capture probe associated with a solid support. An elution buffer can destabilize at least one interaction that contributes to the association of the nucleic acid with the solid support. For example: where the nucleic acid is ionically associated, elution buffer can contain sufficient salt to destabilize the association; where the nucleic acid is hydrophobically associated, elution buffer can contain sufficient organic solvent or cosolvent to destabilize the association; where the nucleic acid is associated through base pairing (hybridization), elution buffer can contain sufficient denaturing agent to destabilize the association; and where the nucleic acid is associated through specific binding (e.g., a capture probe labeled with a tag, which is bound to a binding partner for the tag), the elution buffer can contain sufficient free tag to destabilize the association.

A "reconstitution solution" as used herein refers to a solvent (including water, organic solvents, and mixtures thereof) or buffer that can be used to dissolve another substance, such as a dried substance (e.g., lyophilizate). As used herein the terms "reconstitution solution" and "solvent" may be used interchangeably, as may the terms "reconstitute" and "dissolve."

An "assay" as used herein is a procedure for detecting and/or quantifying an analyte in a sample. A sample comprising or suspected of comprising the analyte is contacted with one or more reagents and subjected to conditions permissive for generating a detectable signal informative of whether the analyte is present or the amount (e.g., mass or concentration) of analyte in the sample.

A "unit-dose reagent" as used herein refers to a reagent provided in an amount or concentration sufficient for use in performing one or more steps of a single assay or test.

A "molecular assay" as used herein is a procedure for specifically detecting and/or quantifying a target molecule, such as a target nucleic acid. A sample comprising or suspected of comprising the target molecule is contacted with one or more reagents, including at least one reagent specific for the target molecule, and subjected to conditions permissive for generating a detectable signal informative of whether the target molecule is present. For example, where the molecular assay is PCR, the reagents include primers specific for the target and the generation of a detectable signal can be accomplished at least in part by providing a labeled probe that hybridizes to the amplicon produced by the primers in the presence of the target. Alternatively, the reagents can include an intercalating dye for detecting the formation of double-stranded nucleic acids.

"Analyte-specific reagents" or "ASRs" refer to reagents that interact specifically with a single analyte or substance generated in the presence of an analyte. For example, in a PCR assay, primers and probes for a single analyte would be considered ASRs. In an ELISA assay, a primary antibody that recognizes a single analyte would be considered an ASR.

An "in vitro diagnostic" or "IVD" is a product used to perform an assay on a biological sample in isolation from the source of the sample. Where the source is a multicellular organism, a sample is generally obtained from the organism and then subjected to analytical procedures (e.g., amplification and/or binding reactions) in an artificial environment, e.g., a reaction vessel. An IVD is a regulated product, such as one requiring CE marking or approval by a governmental agency, such as the Food and Drug Administration.

A "lab developed test" or "LDT" is an assay designed, validated and used by a laboratory, where kits or devices for performing the assay are not commercially marketed or sold as a product for use by other laboratories.

A "reagent" as used herein refers to any substance or combination thereof that participates in a molecular assay, other than sample material and products of the assay. Exemplary reagents include nucleotides, enzymes, amplification oligomers, probes, and salts.

As used herein, a "PCR master mix" refers to a composition comprising a buffer, salt, and a polymerase enzyme for use in DNA amplification by PCR. A PCR master mix generally does not include a sample or primers and probes that may be necessary for carrying out PCR amplification or detection of particular products, although of course a sample and reagents such as primers and probes can be combined with a PCR master mix to form a complete reaction mixture.

The terms "lyophilization," "lyophilized," and "freeze-dried" as used herein refer to a process by which the material to be dried is first frozen and then the ice or frozen solvent is removed by sublimation in a vacuum environment. "Lyophilisate" refers to lyophilized material. A "lyophilized reagent" is a lyophilisate comprising at least one reagent.

As used herein, "time-dependent" monitoring of nucleic acid amplification, or monitoring of nucleic acid amplification in "real-time" refers to a process wherein the amount of amplicon present in a nucleic acid amplification reaction is measured as a function of reaction time or cycle number, and then used to determine a starting amount of template that was present in the reaction mixture at the time the amplification reaction was initiated. For example, the amount of amplicon can be measured prior to commencing each complete cycle of an amplification reaction that comprises thermal cycling, such as PCR. Alternatively, isothermal amplification reactions that do not require physical intervention to initiate the transitions between amplification cycles can be monitored continuously, or at regular time intervals to obtain information regarding the amount of amplicon present as a function of time.

"Real-time amplification" as used herein refers to an amplification reaction in which time-dependent monitoring of amplification is performed.

"End-point amplification" refers to an amplification reaction in which the presence or amount of product (amplicon) is determined near or at completion of the reaction, as opposed to continuously or at regular intervals.

As used herein, a "random access" capability refers to a capability of a system to perform two or more different assays on a plurality of samples in an arbitrary order independent of the order in which the samples are grouped or loaded into the system. For example, if samples are loaded in sequential order as samples 1, 2, 3, 4, 5 (or simultaneously loaded as a group), then a system with random access capability could run assays on the samples in an arbitrary order such as 4, 3, 2, 5, 1, and the assays can vary in their reagents and conditions from sample to sample. This includes the capability of running the same assay on samples not necessarily grouped together. For example, assay A could be run on samples 4 and 2, assay B on sample 3, and assay C on samples 5 and 1. In some embodiments, a random access system runs or can run an IVD assay on one or more samples at the same time as an LDT and/or an assay using an ASR(s) on other sample(s).

As used herein, "target nucleic acid analyte-dependent fluorescence" refers to fluorescence emitted from a fluorophore that directly or indirectly results from an interaction of a probe with a target nucleic acid analyte. This includes (but is not limited to) fluorescence generated by: (i) self-hybridizing probes, such as molecular torches or molecular beacons, e.g., in assays in which the torch or beacon hybridizes with the target and thereby undergoes a conformational change that increases the distance between a fluorophore and a quencher or FRET acceptor, thus increasing observable emission by the fluorophore; (ii) TaqMan^{®} probes, e.g., in assays in which the probe hybridizes with the target, leading to 5'-3' exonucleolysis of the probe and an increase in the distance between a fluorophore and a quencher or FRET acceptor, thus increasing observable emission by the fluorophore; and (iii) secondary Invader probes, e.g., in assays in which a primary probe hybridizes with the target and undergoes cleavage to release a fragment that hybridizes with the secondary Invader probe, which then itself undergoes cleavage to release a fragment comprising a fluorophore, thus increasing the distance of the fluorophore from a quencher or FRET acceptor and increasing observable emission by the fluorophore.

A nucleic acid amplification assay is performed by system 1000 in accordance with parameters that define the steps that are to be performed in the assay. These parameters may include, among others, the type/quantity of extraction, amplification and detection reagents to be used, process conditions (e.g., incubation conditions, mixing rates and times, temperature cycling parameters, etc.), analytes, etc. As used herein, "assay parameters" refer to the parameters that define an assay (e.g., an IVD assay, LDT, or assay requiring ASR reagents).

As used herein, "graphical user interface" or "GUI" refers to a graphics-based user interface that allows a user to interact visually with the computer system. A user can select files, programs, and commands or enter data and text by pointing to interactive pictorial representations, such as windows, icons, and buttons, by pointing to interactive and selectable menus, or by entering text into text fields positioned among such windows, icons, buttons, and menus.

For known, standardized assays, the assay parameters are fixed and unalterable by the user (e.g., IVD assays). Therefore, assay parameters associated with known, standardized assays are referred to herein as "system-defined assay parameters." In contrast, for assays developed by a user or a third party (e.g., LDTs, including assays that use ASRs), at least some of the assay parameters that define the assay are developed/determined/provided by the user/third party. In this disclosure, the term "user-defined assay parameters" is used to refer to assay parameters that are defined by a user.

This description may use relative spatial and/or orientation terms in describing the position and/or orientation of a component, apparatus, location, feature, or a portion thereof. Unless specifically stated, or otherwise dictated by the context of the description, such terms, including, without limitation, top, bottom, above, below, under, on top of, upper, lower, left of, right of, inside, outside, inner, outer, proximal, distal, in front of, behind, next to, adjacent, between, horizontal, vertical, diagonal, longitudinal, transverse, etc., are used for convenience in referring to such component, apparatus, location, feature, or a portion thereof in the drawings and are not intended to be limiting. Further, relative terms such as "about," "substantially," "approximately," etc. are used to indicate a possible variation of ±10% in a stated numeric value or range. The section headings used in the present application are merely intended to orient the reader to various aspects of the disclosed system and are not intended to limit the disclosure. Similarly, the section headings are not intended to suggest that materials, features, aspects, methods, or procedures described in one section do not apply in another section.

Aspects of the present disclosure involve analytical systems and methods that can be used in conjunction with nucleic acid analytical assays, including "real-time" amplification assays and "end-point" amplification assays. The assays performed in accordance with the description herein may include capturing, amplifying, and detecting nucleic acids from cells or target organisms or viruses in patient samples employing conventional technologies. Such conventional technologies include target capture on a solid support, such as a glass bead or magnetic particle, to isolate and purify a targeted nucleic acid, a nucleic acid amplification reaction to increase the copy number of a targeted nucleic acid sequence (or its complement), and a detection modality for determining the presence or amount of the targeted nucleic acid.

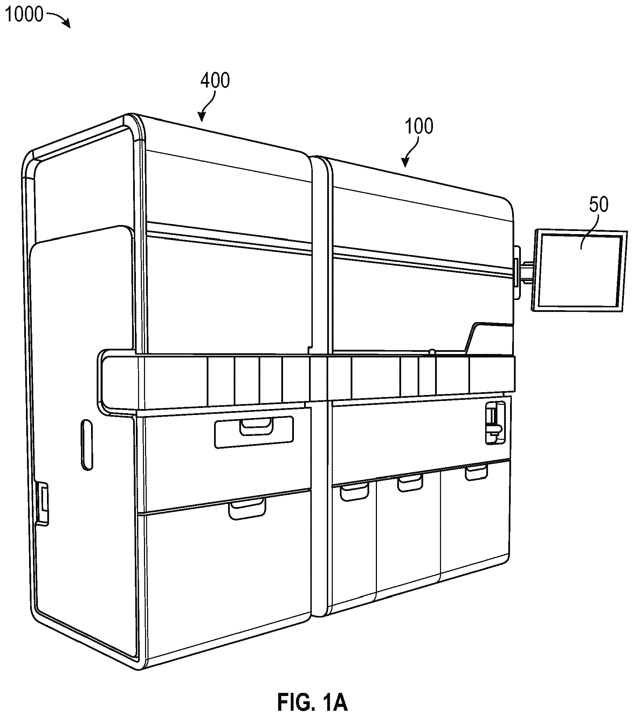

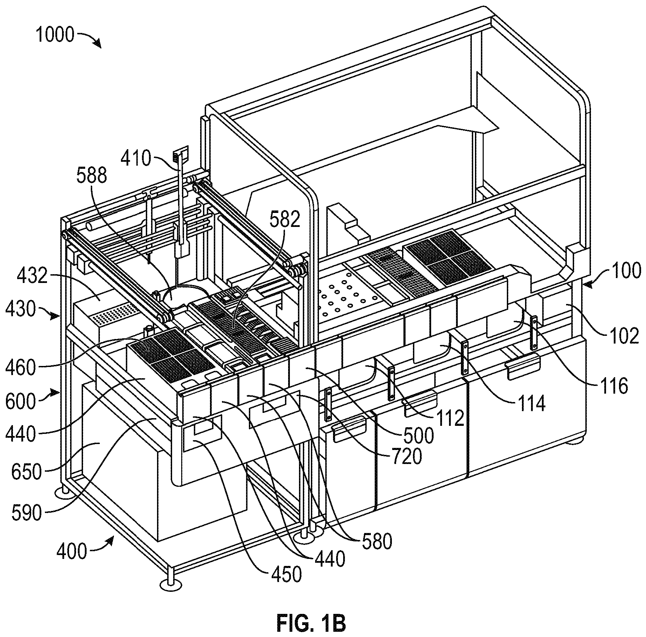

FIGs. 1A and 1B illustrate an exemplary analytical system 1000 that may be used to simultaneously analyze a plurality of samples. FIG. 1A is a perspective view of system 1000 and FIG. 1B is view of system 1000 with its canopy removed to show features within. In the discussion below, reference will be made to both FIGs. 1A and 1B. System 1000 is configured to isolate and purify nucleic acid obtained from a plurality of samples introduced into the system and to amplify and detect targeted nucleic acid contained in any of the samples using differently configured assay reagents. In some embodiments, as will be explained in more detail later, system 1000 may be a random access system that allows IVD assays and LDTs to be performed in an interleaved manner. System 1000 may be configured to perform any type of molecular assay. In some embodiments, system 1000 may be configured to perform a plurality of different (e.g., differently configured) molecular assays on a plurality of samples. For example, a plurality of samples may be loaded in system 1000, processed to specifically or non-specifically isolate and purify targeted nucleic acids (or other macromolecules, such as polypeptides or prions), subject a first subset of the samples to a first set of conditions for performing a first nucleic acid amplification, and, simultaneously, subject a second subset of the samples to a second set of conditions for performing a second nucleic acid amplification, where the reagents for performing the first and second nucleic acid amplifications are differently configured as will be described in more detail later.

In some embodiments, system 1000 may have a modular structure and may be comprised of multiple modules operatively coupled together. However, it should be noted that the modular structure of system 1000 is only exemplary, and in some embodiments, system 1000 may be an integrated system having multiple regions or zones, with each region or zone, for example, performing specific steps of an assay which may be unique to that region. System 1000 includes a first module 100 and a second module 400 operatively coupled together. First module 100 and second module 400 may each be configured to perform one or more steps of an assay. In some embodiments, first and second modules 100, 400 may be separate modules selectively coupled together. That is, first module 100 can be selectively and operatively coupled to second module 400, and first module 100 can be selectively decoupled from second module 400 and coupled to a different second module 400. First and second modules 100, 400 may be coupled together by any method. For example, fasteners (e.g., bolts or screws), clamps, belts, straps, or any combination of fastening/attachment devices may be used to couple these modules together. As explained above, the modular structure of system 1000 is only exemplary, and in some embodiments, system 1000 may be an integral, self-contained structure (with, for example, the first module 100 forming a first region and the second module 200 forming a second region within the integrated structure). It should be noted that in this disclosure, the term "module" is used to refer to a region (zone, location, etc.) of the analytical system. In some embodiments, each such region may be configured to perform specific steps of an assay which may be unique to that region of the system.

In some embodiments, power, data, and/or utility lines or conduits (air, water, vacuum, etc.) may extend between first and second modules 100, 400. In some embodiments, first module 100 may be a system that was previously purchased by a customer, and second module 400 may be a later acquired module that expands the analytical capabilities of the combined system. For example, in one embodiment the first module 100 may be a Panther^{®} system (Hologic Inc., Marlborough, MA) configured to perform sample processing and isothermal, transcription-based amplification assays (e.g., TMA or NASBA) on samples provided to the system, and module 400 may be a bolt-on that is configured to extend the functionality of the Panther^{®} system by, inter alia, adding thermal cycling capabilities to enable, for example, real-time PCR reactions. An exemplary system 1000 with exemplary first and second modules 100, 400 is the Panther Fusion^{®} system (Hologic Inc., Marlborough, MA), which is described in U.S. Patent Nos. 9,732,374, 9,465,161, and 9,604,185, and U.S. Patent Publication No. 2016/0032358. Exemplary systems, functions, devices or components, and capabilities of first and second modules 100, 400 are described in the above-referenced publications (and in the publications identified below) and are therefore not described in detail herein for the sake of brevity.

### First Module

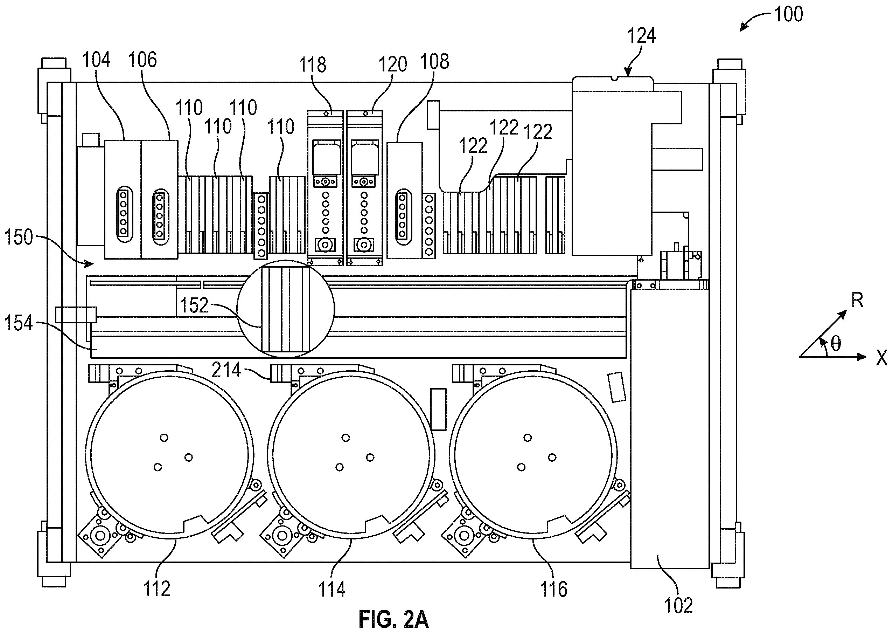

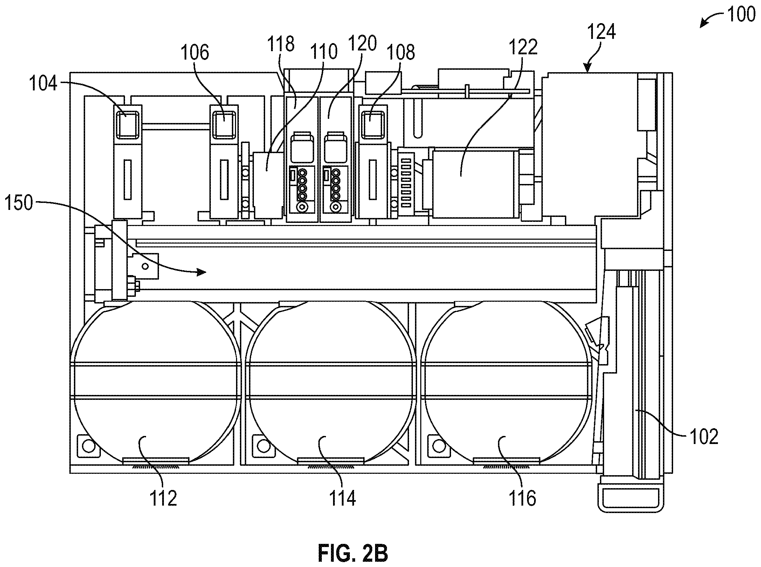

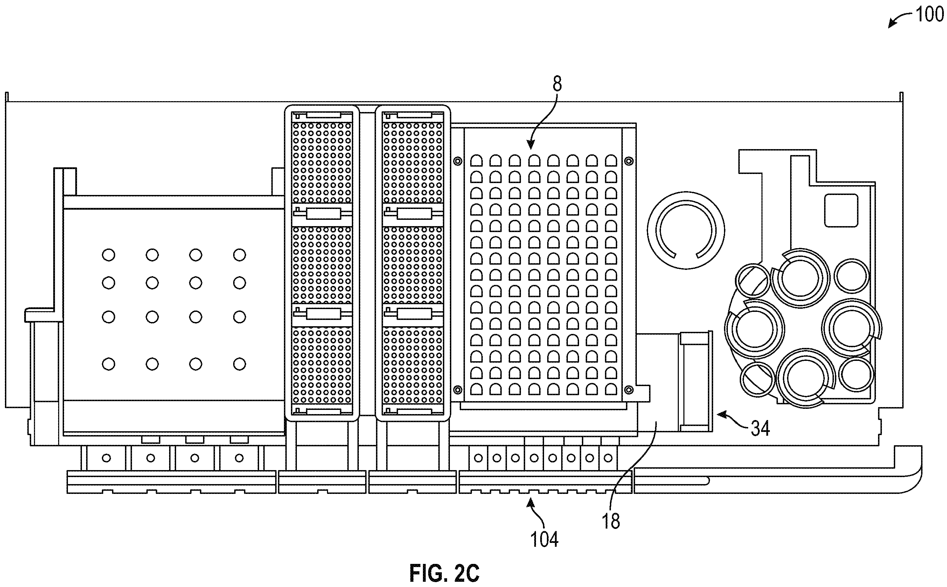

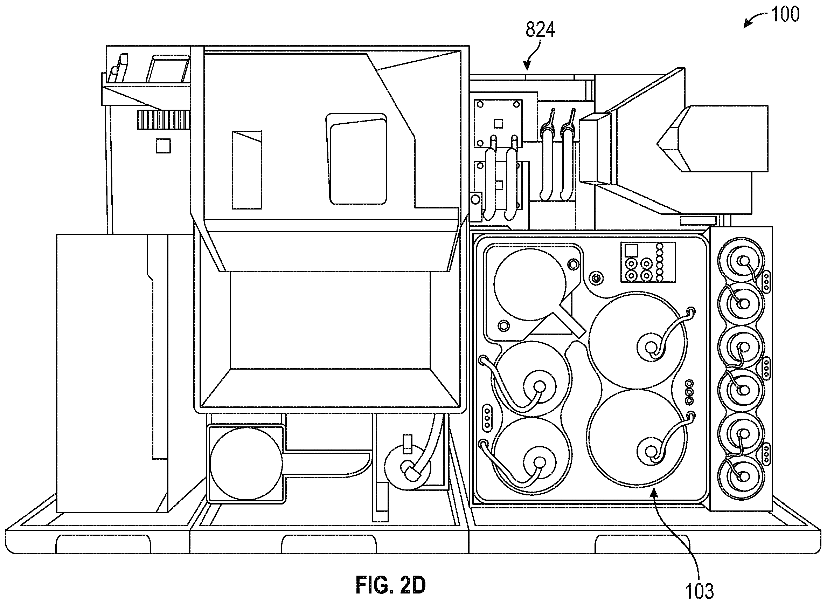

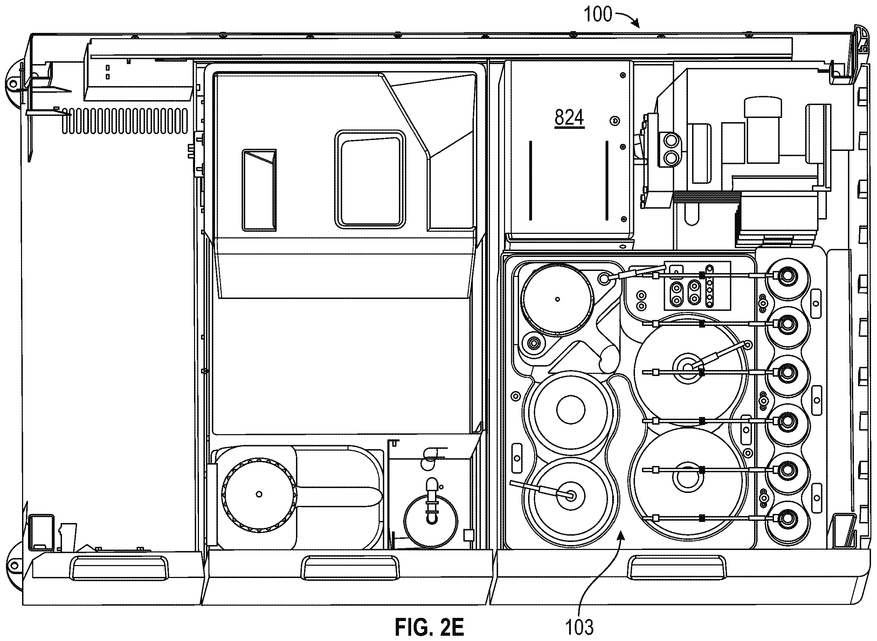

In some embodiments, first module 100 may include multiple vertically stacked decks. FIGs. 2A and 2B illustrate top plan views of exemplary embodiments of the middle deck of first module 100, FIG. 2C illustrates a top plan view of the top deck of first module 100 in an exemplary embodiment, and FIGs. 2D and 2E illustrate top plan views of exemplary embodiments of the bottom deck of first module 100. In the description below, reference will be made to FIGs. 2A-2E. It should be noted that some of FIGs. 2A-2E illustrate top views of different embodiments of system 1000. Therefore, some of the components described with reference to one figure may not be visible or may be positioned at different locations on another figure. As illustrated, first module 100 may be configured to perform one or more steps of a multi-step molecular assay designed to detect at least one analyte (e.g., targeted nucleic acid). First module 100 may include receptacle-receiving components configured to receive and hold the reaction receptacles and, in some instances, to perform process steps on the contents of the receptacles. Exemplary process steps may include: dispensing sample and/or reagents into reaction receptacles, including, for example, target capture reagents, buffers, oils, primers and/or other amplification oligomers, probes, polymerases, etc.; aspirating material from the reaction receptacles, including, for example, non-immobilized components of a sample or wash solutions; mixing the contents of the reaction receptacles; maintaining and/or altering the temperature of the contents of reaction receptacles; heating or chilling the contents of the reaction receptacles or reagent containers; altering the concentration of one or more components of the contents of the reaction receptacles; separating or isolating constituent components of the contents of the reaction receptacles; detecting a signal, such as electromagnetic radiation (e.g., visible light) from the contents of the reaction receptacles; and/or deactivating nucleic acid or halting on-going reactions.

In some embodiments, first module 100 may include a receptacle drawer or compartment 102 adapted to receive and support a plurality of empty reaction receptacles. Compartment 102 may include a cover or door for accessing and loading the compartment with the reaction receptacles. Compartment 102 may further include a receptacle feeding device for moving the reaction receptacles into a receptacle pick-up position (e.g., a registered or known position) to facilitate removal of the reaction receptacles by a receptacle distributor. First module 100 may further include one or more compartments (e.g., compartment 103 of FIGs. 2D and 2E) configured to store containers that hold bulk reagents (i.e., reagent volumes sufficient to perform multiple assays) or are configured to receive and hold waste material. The bulk reagents may include fluids such as, for example, water, buffer solutions, target capture reagents, and nucleic acid amplification and detection reagents. In some embodiments, the bulk reagent container compartments may be configured to maintain the containers at a desired temperature (e.g., at a prescribed storage temperature), and include holding structures that hold and/or agitate the containers to maintain their contents in solution or suspension. An exemplary holding structure for supporting and agitating fluid containers is described in U.S. Patent No. 9,604,185.

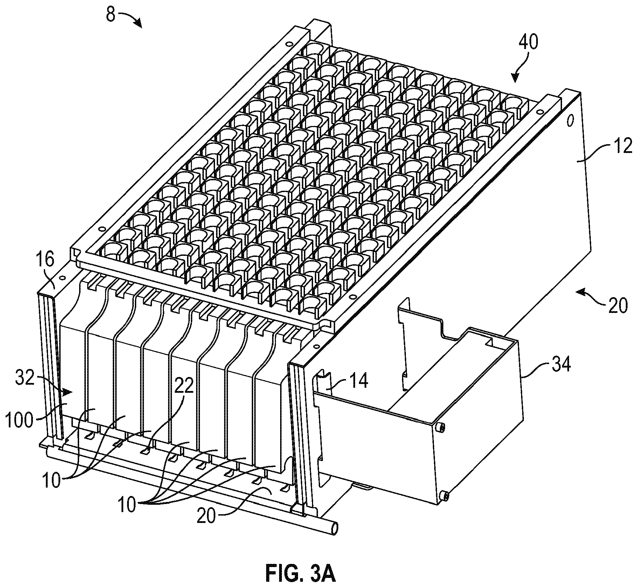

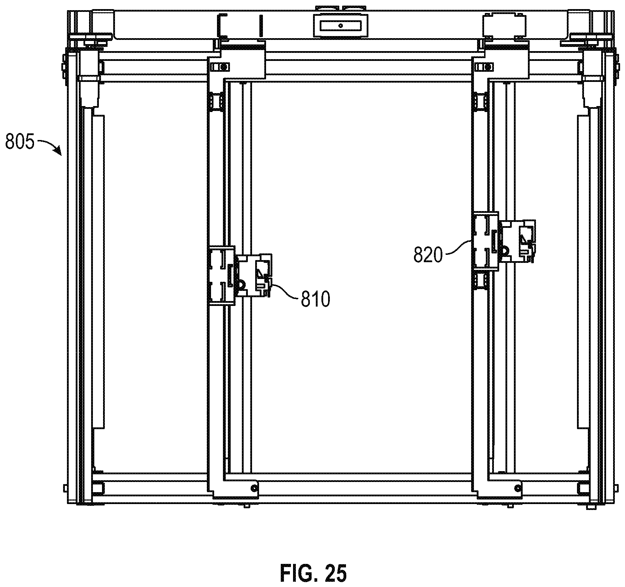

First module 100 may further include a sample bay 8 supporting one or more sample holding racks 10 with sample-containing receptacles (see FIGs. 2C, 3A-3C). First module 100 may also include one or more fluid transfer devices (see fluid transfer device 805 of FIG. 25) for transferring fluids, for example, sample fluids, reagents, bulk fluids, waste fluids, etc., to and from reaction receptacles and/or other containers. In some embodiments, the fluid transfer devices may comprise one or more robotic pipettors (e.g., pipettors 810, 820 of FIG. 25) configured for controlled, automated movement and access to the reaction receptacles, bulk containers holding reagents, and containers holding samples. In some embodiments, the fluid transfer devices may also include fluid dispensers, for example, nozzles, disposed within other devices and connected by suitable fluid conduits to containers, for example, bulk containers holding reagents, and to pumps or other devices for causing fluid movement from the containers to the dispensers. First module 100 may further include a plurality of load stations (e.g., heated load stations), such as load stations 104, 106, 108 configured to receive sample receptacles (see FIGs. 2A and 2B) and other forms of holders for supporting sample receptacles and reagent containers. An exemplary load station and receptacle holder is described in U.S. Patent No. 8,309,036.

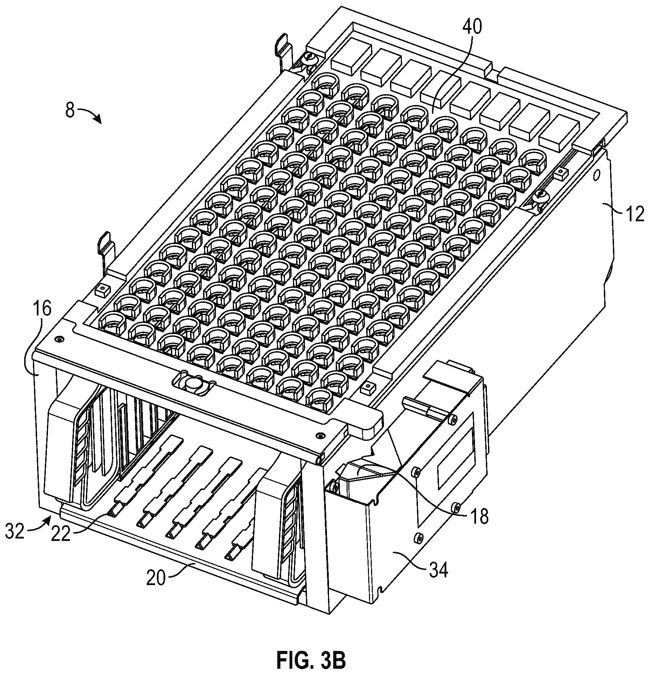

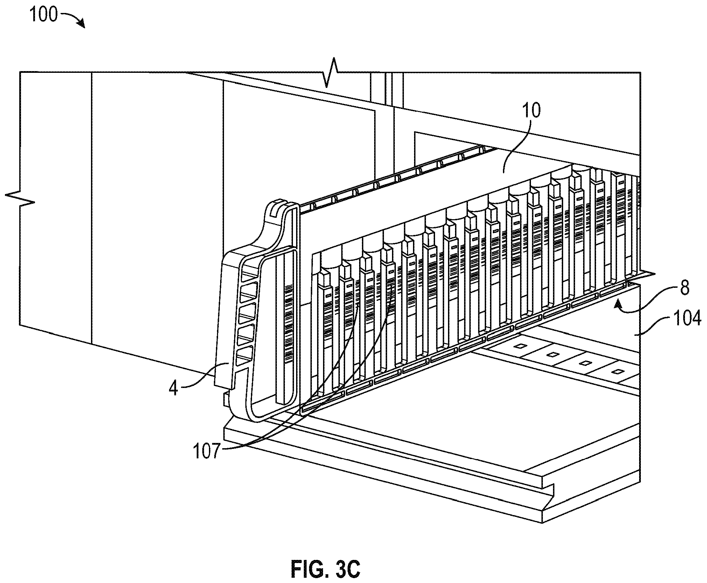

In some embodiments, sample bay 8 is a box-like structure having side walls 12, 16 and a floor plate 20. FIGs. 3A and 3B depict different embodiments of sample bay 8 that may be used with system 1000. In the discussion below, reference is made to both FIGs. 3A and 3B. Walls 12, 16 may be thermally insulated. Sample bay 8 further includes a sample bay cover 40 carried at its edges by the walls 12, 16. A front end 32 of sample bay 8 is open (see FIG. 3B) to permit sample-holding racks 10 with receptacles 107 containing samples to be inserted into and removed from the sample bay 8. FIG. 3C illustrates a sample-holding rack 10 with receptacles 107 containing samples being inserted into sample bay 8. As can be seen in FIG. 3B, floor plate 20 may further include sample rack guides 22 (see FIG. 3B) which engage mating guides formed in the bottom of each sample-holding rack 10 for accurately and repeatably positioning each rack. Sample bay 8 further includes a barcode bracket 34 mounted to side wall 12 and configured to carry a barcode reader 18 (see FIGs. 2C and 3B) in an operative position with respect to a barcode window 14 (visible in FIG. 3A) formed in side wall 12. The barcode reader 18 is configured to read barcodes on individual sample receptacles 107 (see FIG. 3C) carried in each of sample-holding racks 10 as well as barcodes on sample-holding racks 10 themselves. The barcodes may be read through barcode window 14 as sample-holding racks 10 are pushed into or removed from sample bay 8.

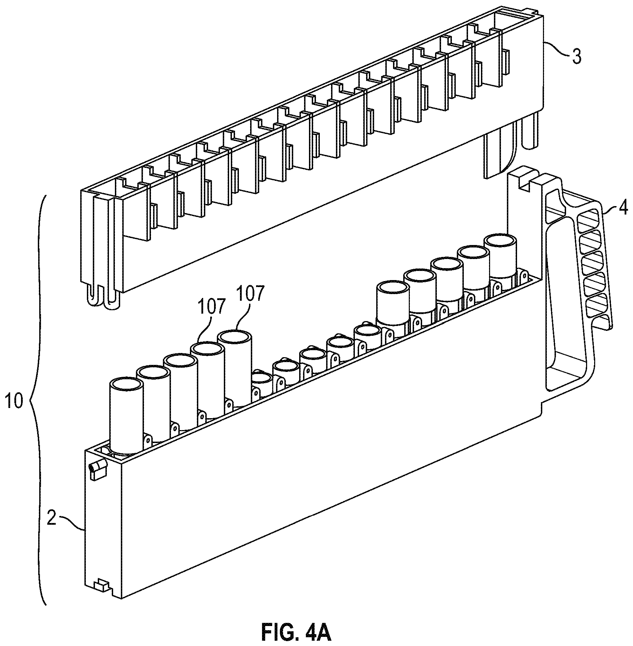

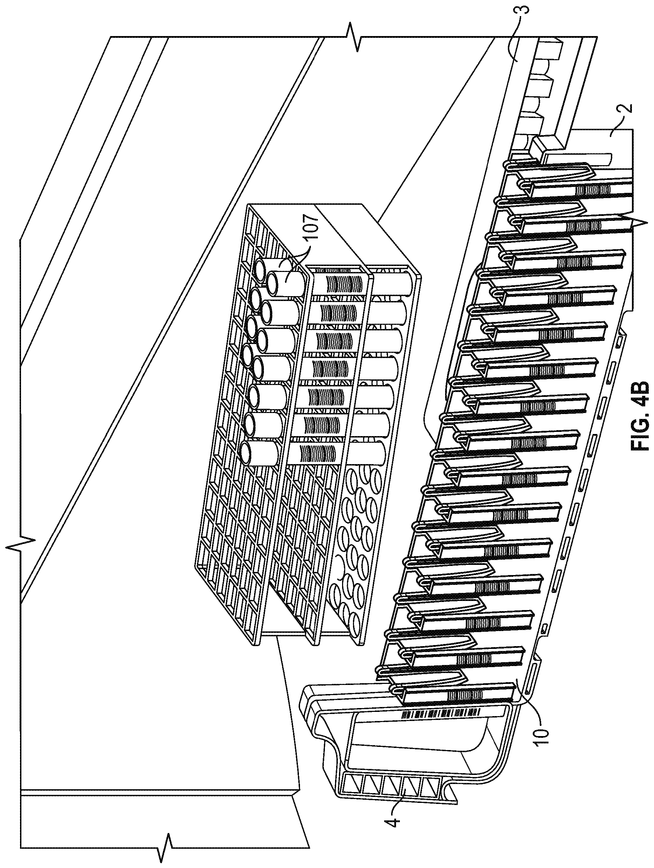

FIGs. 4A and 4B illustrate different embodiments of sample-holding racks 10 that may be used with sample bay 8. In the discussion below, reference will be made to both FIGs. 4A and 4B. Sample-holding rack 10 is adapted to receive and hold a plurality of receptacles 107 containing samples. In some embodiments, receptacles 107 may be, or may include, tubular containers, such as test tubes. Sample-holding rack 10 includes a receptacle holder 2 and a cover 3. Receptacle holder 2 includes a handle 4 for grasping and inserting sample-holding rack 10 into sample bay 8. As illustrated in FIGs. 3C and 4B, receptacles 107 containing samples may be loaded on rack 10, and rack 10 inserted into sample bay 8 of load station 104. In some embodiments, load station 104 is configured such that receptacles 107 containing samples can be loaded into sample bay 8 in any order and at any time (e.g., while system 1000 is performing an assay on some samples). For example, a rack 10 with different, new, or recently arrived samples may be loaded onto a rack 10, and the loaded rack 10 inserted into sample bay 8 of a load station while system 1000 is in the process of performing assay on other samples. In one embodiment, a machine-readable label, such as a barcode, is provided on receptacle holder 2 near handle 4 (see FIG. 3C).

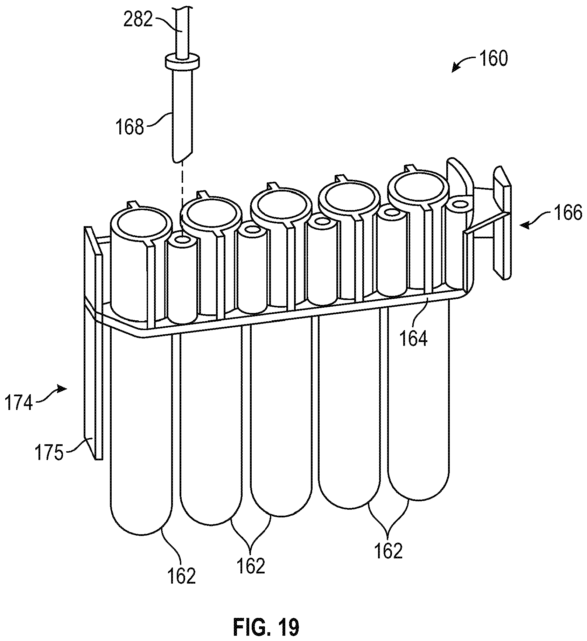

With reference to FIGs. 2A and 2B, in some embodiments, first module 100 may include one or more magnetic parking stations 110 and heated incubators 112, 114, 116 configured to heat (and/or maintain) the contents of reaction receptacles at a temperature higher than ambient temperature, and one or more chilling modules 122 configured to cool (and/or maintain) the contents of reaction receptacles at a temperature lower than ambient temperature. Chilling module 122 may be used to aid in oligo hybridization and to cool a receptacle (such as, for example, MRU 160 discussed below with reference to FIG. 19) before performing luminescence measurements. In some embodiments, incubator 112 (which may be referred to as a transition incubator) may be set at a temperature of about 43.7°C and may be used for process steps such as, for example, lysis, target capture, and hybridization. Incubator 114 may be a high temperature incubator which, in some embodiments, may be set at a temperature of about 64°C and used for process steps such as, for example, lysis, target capture, and hybridization. And, incubator 116 (referred to as an amplification incubator) may be set at a temperature of about 42°C and may be incubator used for amplification during an assay. Incubator 116 may include real time fluorometers for the detection of fluorescence during amplification. Exemplary temperature ramping stations are described in U.S. Patent No. 8,192,992, and exemplary incubators are described in U.S. Patent Nos. 7,964,413 and 8,718,948. First module 100 may include sample-processing devices, such as magnetic wash stations 118, 120, adapted to separate or isolate a target nucleic acid or other analyte (e.g., immobilized on a magnetically-responsive solid support) from the remaining contents of the receptacle.

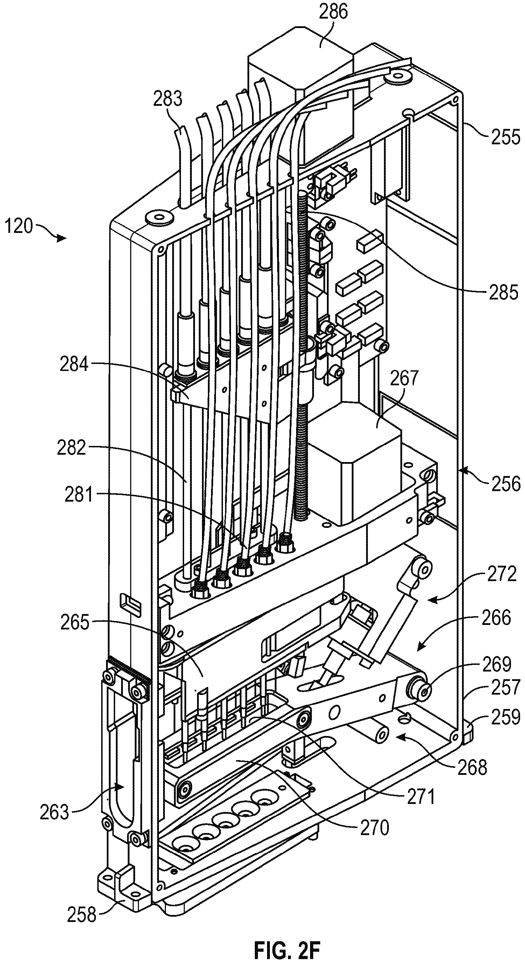

FIG. 2F illustrates an exemplary magnetic wash station 120 of first module 100 with its side plate removed (to show internal details). In some assays, samples are treated to release materials capable of interfering with the detection of an analyte (e.g., a targeted nucleic acid) in a magnetic wash station 118, 120. To remove these interfering materials, samples may be treated with a target capture reagent that includes a magnetically-responsive solid support for immobilizing the analyte. Suitable solid supports may include paramagnetic particles (0.7-1.05 micron particles, Sera-Mag^{™} MG-CM (available from Seradyn, Inc., Indianapolis, Indiana). When the solid supports are brought into close proximity to a magnetic force, the solid supports are drawn out of suspension and aggregate adjacent a surface of a sample holding container, thereby isolating any immobilized analyte within the container. Non-immobilized components of the sample may then be aspirated or otherwise separated from immobilized analyte. Magnetic wash station 120 includes a module housing 256 having an upper section 255 and a lower section 257. Mounting flanges 258, 259 extend from lower section 257 to attach wash station 120 to a support surface of first module 100. A loading slot 263 extends through a front wall of lower section 257 to allow receptacle distributor 150 of first module 100 (see FIG. 2A) to place an MRU 160 (described with reference to FIG. 19) (or another receptacle) into housing 256 of magnetic wash station 120 (and to remove MRU 160 from housing 256). A receptacle carrier unit 265 is disposed adjacent to loading slot 263 for supporting MRU 160 within magnetic wash station 120. In some embodiments, receptacle carrier unit 265 may include a spring clip (or another retention mechanism) to releasably hold MRU 160 in receptacle carrier unit 265. An orbital mixer assembly 266 is coupled to carrier unit 265 for orbitally mixing the contents of MRU 160 held by receptacle carrier unit 265. Orbital mixer assembly 266 includes a stepper motor 267 that is coupled to receptacle carrier unit 265 (by a drive mechanism) such that, when motor 267 turns, carrier unit 265 is moved in a horizontal orbital path to mix the contents of MRU 160.

Magnetic wash station 120 includes a magnet moving apparatus 268 configured to move one or more magnets towards and away from MRU 160 in receptacle carrier unit 265. In the embodiment illustrated in FIG. 2F, magnet moving apparatus 268 is a pivotable structure configured to be pivotable about a pivot point 269. Magnet moving apparatus 268 carries permanent magnets 270, which are positioned on either side of a slot 271 formed in the magnet moving apparatus 268. In some embodiments, magnet moving apparatus includes five magnets 270 to correspond to each individual receptacle 162 of an MRU 160 carried in receptacle carrier unit 265. In some embodiments, magnets 270 may be made of neodymium-iron-boron (NdFeB). An electric actuator, generally represented at 272, pivots magnet moving apparatus 268 up and down, thereby moving magnets 270 between an operational position and a non-operational position with respect to an MRU 160 supported in receptacle carrier unit 265. In the operational position, magnets 270 are disposed proximate to each receptacle 162 of MRU 160, such that the magnetically-responsive solid supports mixed with the contents of each receptacle 162 are drawn out of suspension by the attraction of the magnetic fields of magnets 270. In the non-operational position, magnets 270 are disposed at a sufficient distance from receptacles 162 so as to have no substantial effect on the contents of receptacles 162. In the present context, "no substantial effect" means that the magnetically-responsive solid supports are not drawn out of suspension by the attraction of the magnetic fields of magnets 270.

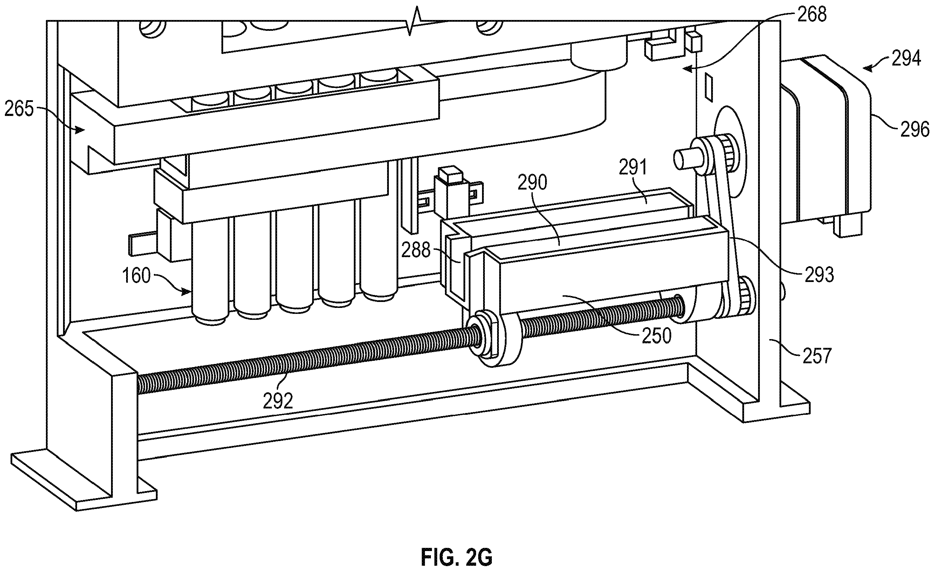

FIG. 2G illustrates another embodiment of magnet moving apparatus 268 of magnetic wash station 120 (of FIG. 2F). Magnet moving apparatus 268 of FIG. 2G includes a magnet sled 250 positioned within lower section 257 (of module housing 256) and a drive system 294 which moves magnet sled 250 between a non-operational position (as shown in FIG. 2G) and an operational position with respect to MRU 160 supported in receptacle carrier unit 265. Magnet sled 250 includes an elongate opening 288 (in some embodiments, having a substantially rectangular shape) extending longitudinally therethrough. A first magnet 290 is disposed on one side of opening 288 and a second magnet 291 disposed on the opposite side of opening 288. In some embodiments, instead of single magnets 290 and 291, five individual magnets (in some embodiments, having a size of approximately 12 mm×12 mm×8 mm and made from NdFeB, grade n-40) may be provided on opposite sides of sled 250. Drive system 294 includes a threaded drive screw 292 that is journaled at its opposite ends to the walls of lower section 257 so as to be rotatable about its longitudinal axis. A drive motor 296 is coupled to drive screw 292 via a drive belt 293. Rotation of drive motor 296 causes linear translation of magnet sled 250 in a longitudinal direction with respect to drive screw 292. Rotation of drive screw 292 in one direction causes translation of magnet sled 250 towards MRU 160 and moves magnets 290 and 291 to their operational position. And, rotation of drive screw 292 in the opposite direction causes translation of magnet sled 250 in the opposite direction and moves magnets 290 and 291 to their non-operational position (the position illustrated in FIG. 2G). When magnet sled 250 is moved from the non-operational position to the operational position, MRU 160 passes through the longitudinal opening 288 of magnet sled 250 and is disposed between first magnet 290 and second magnet 291.

With continued reference to FIG. 2F, magnetic wash station 120 includes wash solution delivery tubes 281 that extend through module housing 256 to form a wash solution delivery network. Nozzles connected to delivery tubes 281 are located above each receptacle 162 of MRU 160 supported in receptacle carrier unit 265. In some embodiments, these nozzles may be positioned in an off-centered manner with respect to each receptacle 162 to direct a wash solution down the sides of each receptacle 162 of MRU 160 to rinse away materials clinging to the sides. Suitable wash solutions are known to those skilled in the art, an example of which contains 10 mM Trizma base, 0.15 M LiCl, 1 mM EDTA, and 3.67 mM lithium lauryl sulfate (LLS), at pH 7.5. Aspirator tubes 282, coupled to a tube holder 284, also extend through housing 256 of magnetic wash station 120. Aspirator hoses 283 coupled to aspirator tubes 282 extend to a vacuum pump 824 (see FIG. 2D). Tube holder 824 is attached to a drive screw 285 actuated by a lift motor 286. Tube holder 284 and aspirator tubes 282 are lowered by lift motor 286 and drive screw 285 such that each aspirator tube 282 frictionally engages with a disposable tip (e.g., tiplet 168 of MRU 160 discussed below with reference to FIG. 19).

After successful engagement of aspirator tubes 282 with tiplet 168 (see FIG. 19), orbital mixer assembly 266 moves receptacle carrier unit 265 to a fluid transfer position. Magnet moving apparatus 268 then moves magnets 270 (or magnets 290 and 291 of FIG. 2G) to their operational position adjacent opposite sides of receptacles 162 of MRU 160. With the contents of receptacles 162 subjected to the magnetic fields of magnets 270 (or magnets 290, 291 of FIG. 2G), the magnetically-responsive solid supports having targeted nucleic acids immobilized thereon will be drawn to the sides of the individual receptacles 162 adjacent the magnets 270 (or magnets 290, 291 of FIG. 2G). Magnet moving apparatus 268 will remain in the operational position for an appropriate dwell time, as defined by the assay protocol to cause the magnetic solid supports to adhere to the sides of the respective receptacles 162. Aspirator tubes 282 are then lowered into receptacles 162 of the MRU 160 to aspirate the fluid contents of the individual receptacles 162, while the magnetic solid supports remain in receptacles 162, aggregated along the sides thereof, adjacent magnets 270. The attached tiplet 168 at the ends of aspirator tubes 282 ensure that the contents of each receptacle 162 do not come into contact with the sides of aspirator tubes 282 during the aspirating procedure. Tiplet 168 will be discarded before a subsequent MRU 160 is processed in magnetic wash station 120 to reduce the chance of cross-contamination by aspirator tubes 282.

Following aspiration, aspirator tubes 282 are raised and magnet moving apparatus 268 moves magnets 270 (or magnets 290, 291 of FIG. 2G) to their non-operational position. Receptacle carrier unit 265 is then moved to a fluid dispense position and a prescribed volume of wash solution is dispensed into each receptacle 162 of the MRU 160 through nozzles connected to wash solution delivery tubes 281. Orbital mixer assembly 266 then moves receptacle carrier 265 in a horizontal orbital path at high frequency (in one embodiment, 14 HZ, accelerating from 0 to 14 HZ in 1 second) to mix the contents of MRU 160. Following mixing, orbital mixer assembly 266 stops receptacle carrier unit 265 at a fluid transfer position. In some embodiments, magnet moving apparatus 268 is again moved to the operational position and maintained in the operational position for a prescribed dwell period. After magnetic dwell, aspirator tubes 282 with their engaged tiplets 168 are lowered into receptacles 162 to aspirate the test specimen fluid and wash solution as described above. In some embodiments, multiple wash cycles (each comprising a dispense, mix, magnetic dwell, and aspirate sequence) may be performed as defined by the assay protocol. Exemplary magnetic wash stations are described in U.S. Patent Nos. 6,605,213 and 9,011,771.

With continued reference to FIGs. 2A and 2B, first module 100 may include a detector 124 configured to receive a reaction receptacle and detect a signal (e.g., an optical signal) emitted by the contents of the reaction receptacle. In one implementation, detector 124 may comprise a luminometer for detecting luminescent signals emitted by the contents of a reaction receptacle and/or a fluorometer for detecting fluorescent emissions from the contents of the reaction receptacle. First module 100 may also include one or more signal detecting devices, such as, for example, fluorometers (e.g., coupled to one or more of incubators 112, 114, 116) configured to detect (e.g., at periodic intervals) signals emitted by the contents of receptacles contained in the incubators while a process, such as nucleic acid amplification, is occurring within the reaction receptacles. Exemplary luminometers and fluorometers are described in U.S. Patent Nos. 7,396,509 and 8,008,066.

First module 100 may further include a receptacle transfer device, which, in the illustrated embodiment, includes a receptacle distributor 150 configured to move receptacles between various devices of first module 100 (e.g., sample bay 8, incubators 112, 114, 116, load stations 104, 106, 108, magnetic parking stations 110, wash stations 118, 120, and chilling modules 122). These devices may include a receptacle transfer portal (e.g., a port covered by an openable door) through which receptacles may be inserted into or removed from the devices. Receptacle distributor 150 may include a receptacle distribution head 152 configured to move in an X direction along a transport track assembly 154, rotate in a theta (θ) direction, and move in an R direction, to move receptacles into and out of the devices of first module 100. An exemplary receptacle distributor, exemplary receptacle transfer portal doors, and mechanisms for opening the doors are described in U.S. Patent No. 8,731,712.

### Second Module

In an exemplary embodiment, second module 400 is configured to perform nucleic acid amplification reactions (such as, for example, PCR), and to measure fluorescence in real-time. System 1000 may include a controller (discussed in more detail later) that directs system 1000 to perform the different steps of a desired assay. The controller may accommodate LIS ("laboratory information system") connectivity and remote user access. In some embodiments, second module 400 houses component modules that enable additional functionalities, such as melt analyses. An example of a melt station that could be adapted for use in the second module is described in U.S. Patent No. 9,588,069. Other devices may include a printer and an optional uninterruptible power supply.

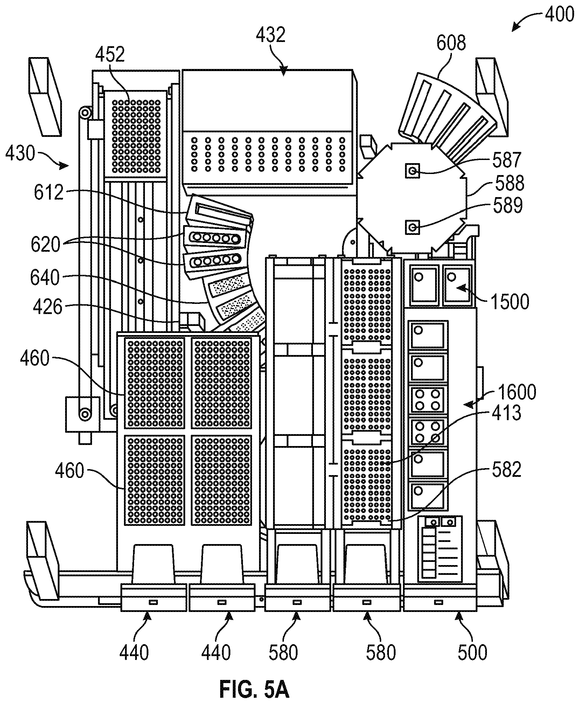

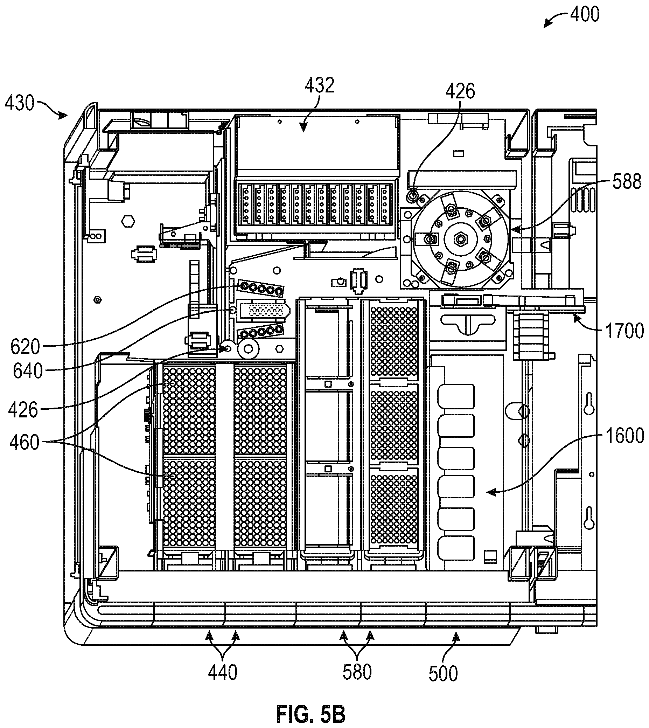

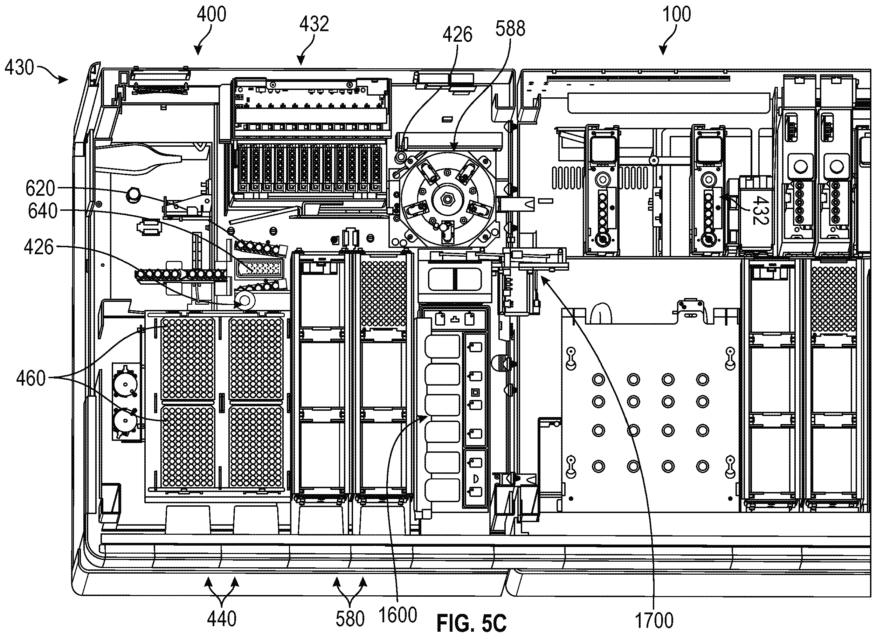

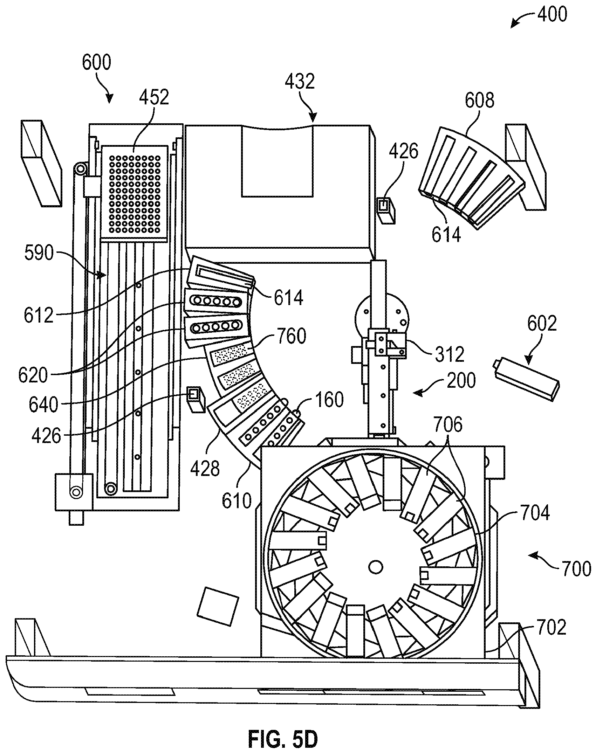

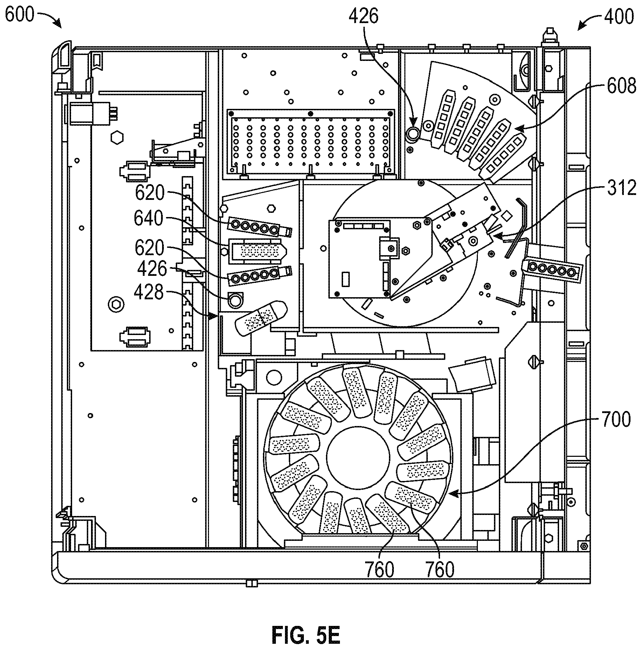

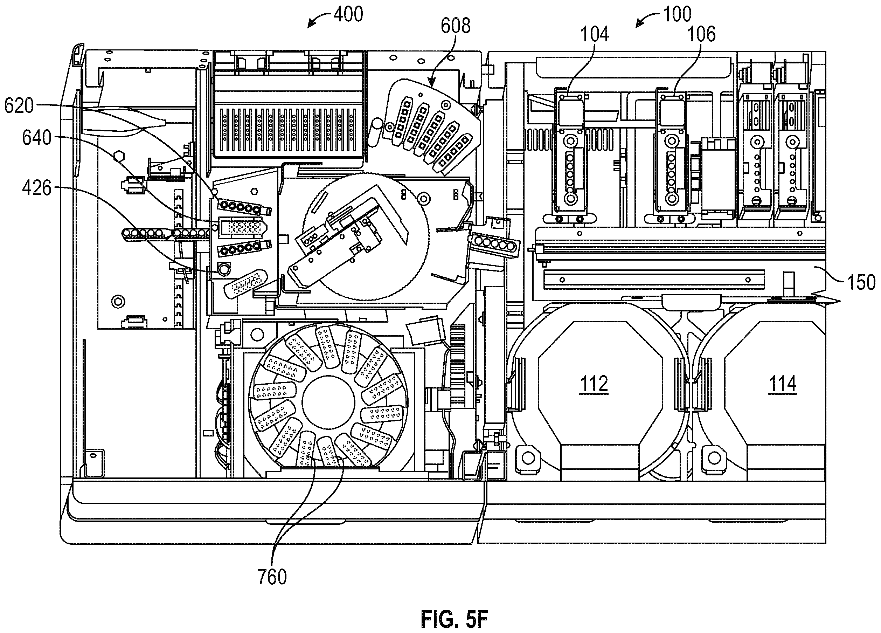

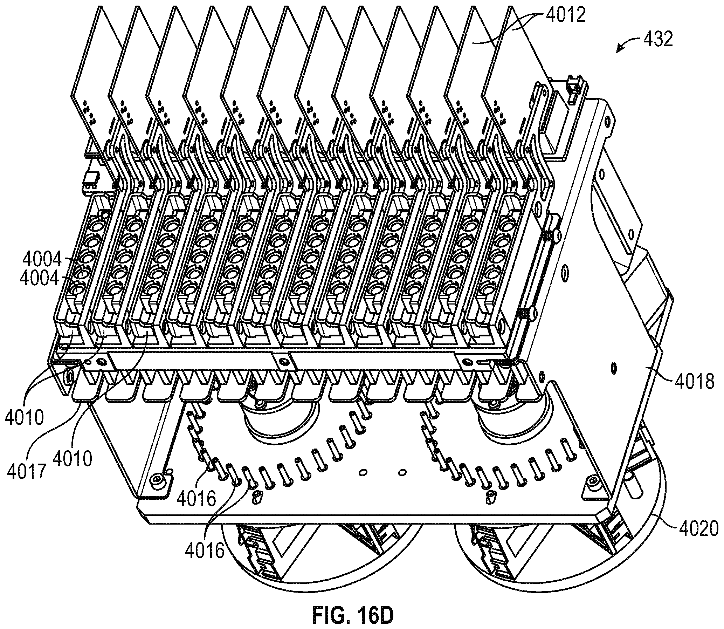

With reference to FIG. 1B, in some embodiments, second module 400 includes multiple vertically stacked levels (or decks) including devices configured for different functions. These levels include an amplification processing deck 430 and a receptacle processing deck 600. In the illustrated embodiment, receptacle processing deck 600 is positioned below amplification processing deck 430. However, this is not a requirement, and the vertical order of the decks (and their devices) may vary according to the intended use of analytical system 1000. Schematic plan views of different embodiments of exemplary amplification processing decks 430 are illustrated in FIGs. 5A, 5B, and 5C. Schematic plan view of different embodiments of exemplary receptacle processing decks 600 are illustrated in FIGs. 5D, 5E, and 5F. In the description that follows, reference will be made to FIGs. 5A-5F. However, it should be noted that some of the features and components described below may not be visible in all these figures. Second module 400 may include devices positioned at different levels. These devices include, among others, a fluid transfer device in the form of one or more robotic pipettor(s) 410 (see FIG. 1B), a thermal cycler 432 with a signal detector 4020 (see FIG. 16D), tip compartments 580 configured to store trays of disposable tips for pipettor(s) 410, cap/vial compartments 440 configured to store trays 460 of disposable processing vials and associated caps, a bulk reagent container compartment 500, a bulk reagent container transport 1700, a receptacle distribution system including a receptacle handoff device 602 and a receptacle distribution system 200 including a receptacle distributor 312 (which, in the exemplary embodiment shown, comprises a rotary distributor), receptacle storage units 608, 610, 612 configured to store receptacles and/or multi-receptacle units (MRUs) (that, for example, includes multiple receptacles joined together as a single piece, integral unit), magnetic slots 620, a waste bin coupled to one or more trash chutes, a centrifuge 588, a reagent pack changer 700, reagent pack loading stations 640, and one or more compartments 450 (see FIG. 1B) configured to store accessories, such as, for example, consumables and/or storage trays 452 for post-cap/vial assemblies. Exemplary embodiments of trays 460 for disposable processing vials and caps are disclosed in U.S. Patent Publication No. US 2017/0297027 A1. Several devices and features of system 1000 are described in U.S. Patent No. 9,732,374 and other references that are identified herein. Therefore, for the sake of brevity, these devices and features are not described in detail herein.

In the illustrated embodiment, robotic pipettor 410 is disposed near the top of second module 400. Below robotic pipettor 410, amplification processing deck 430 includes bulk reagent container compartment 500, centrifuge 588, the top of thermal cycler 432, tip compartments 580, and cap/vial compartments 440. Below amplification processing deck 430, receptacle processing deck 600 includes receptacle handoff device 602, receptacle distributor 312, receptacle storage units 608, 610, 612, magnetic slots 620, reagent pack changer 700, and reagent pack loading stations 640. As can be seen in FIG. 4D, magnetic slots 620 and reagent pack loading stations 640 on receptacle processing deck 600 are accessible by robotic pipettor 410 through a gap between the devices of amplification processing deck 430.

The receptacles in receptacle storage units 608, 610, 612 may include individual receptacles (e.g., a container configured to store a fluid) having an open end and an opposite closed end, or multiple receptacles (e.g., five) coupled together as a unit (MRU). These MRUs may include a manipulating structure that is configured to be engaged by an engagement member (e.g., a hook) of a robotically controlled receptacle distribution system for moving the receptacle between different devices of system 1000. Exemplary receptacles are described in U.S. Patent Nos. 6,086,827 and 9,732,374. As will be described in more detail infra, receptacle distribution system 200, including receptacle handoff device 602 and receptacle distributor 312, is configured to receive a receptacle or an MRU from receptacle distributor 150 of first module 100 and transfer the receptacle to second module 400, and move the receptacle into different positions in second module 400.

### Reagent Container Compartment

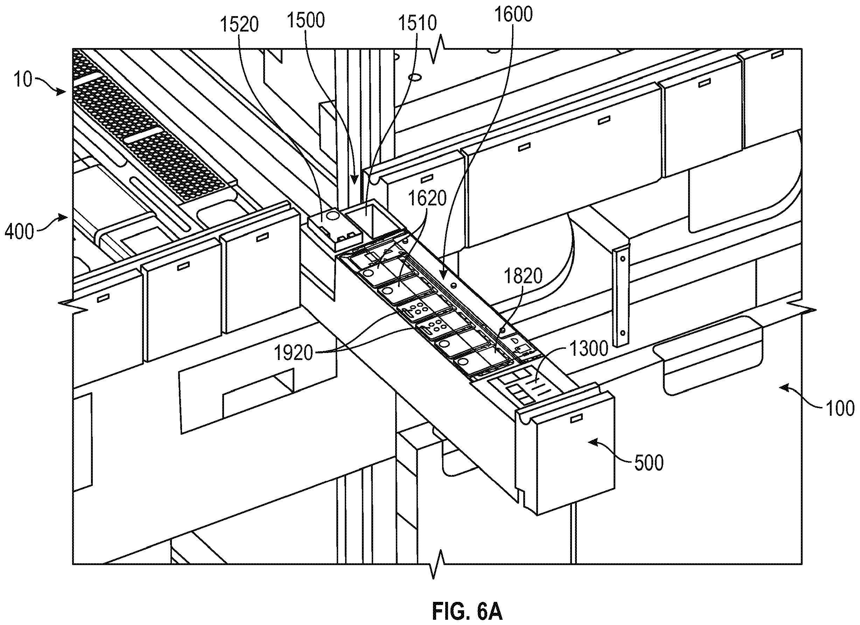

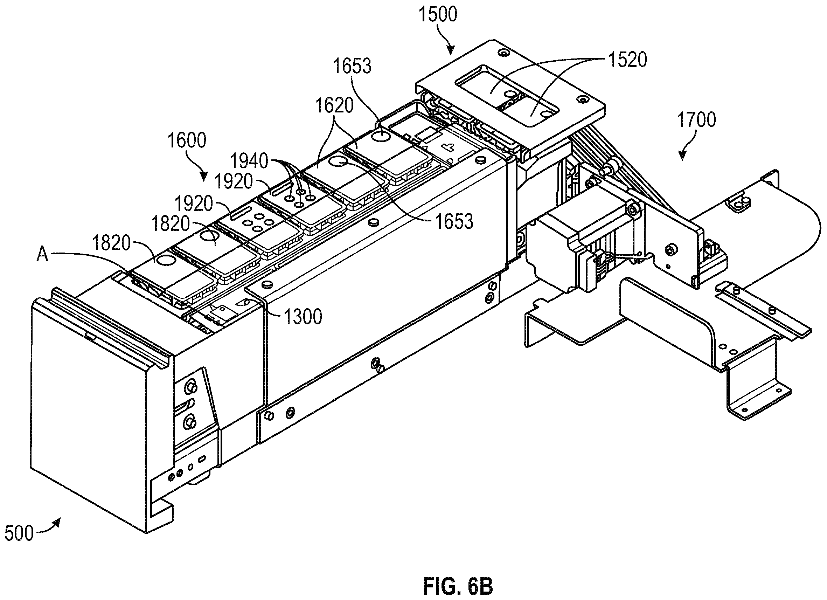

With reference to FIG. 1B, bulk reagent container compartment 500 of second module 400 is configured to hold a plurality of reagent containers. A door or cover panel of second module 400 may be opened to access the contents of reagent container compartment 500. In some embodiments, automated locks (e.g., activated by a controller of system 1000) may prevent reagent container compartment 500 from being pulled open when second module 400 is operating. In some embodiments, visible and/or audible warning signals may be provided to indicate that reagent container compartment 500 is not closed properly. FIG. 6A is a perspective view of a portion of system 1000 with reagent container compartment 500 in an open state. FIG. 6B is a perspective view of an exemplary reagent container compartment 500 separated from second module 400. In the discussion below, reference will be made to both FIGS. 6A and 6B. As illustrated in FIG. 6A, reagent container compartment 500 may be a cabinet that slides out from the main body of second module 400 to load containers carrying reagents for use in performing an analytical procedure on system 1000. Reagent container compartment 500 may include one or more trays or container carriers configured to hold containers carrying the same or different types of reagents. In general, a container-carrier may be a component that includes one or more pockets or cavities formed to receive fluid filled containers therein. In some embodiments, a container-carrier may be a component molded using a non-conductive plastic or polymeric material. As seen in FIG. 6B, in some exemplary embodiments, reagent container compartment 500 includes two reagent container carriers - a first reagent container-carrier 1500 and a second reagent container-carrier 1600. It should be noted that, in some embodiments, second module 400 may include multiple bulk reagent container compartments (in some embodiments, similar to compartment 500) that each support one or more reagent containers. Some of these multiple compartments may be configured to maintain reagent containers at different temperatures (heated, cooled, etc.).

### First Reagent Container-Carrier

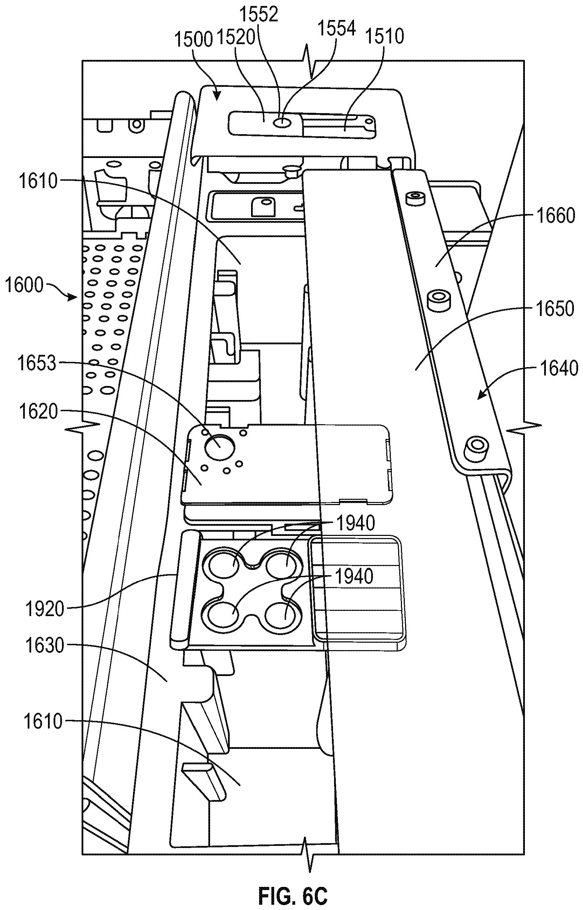

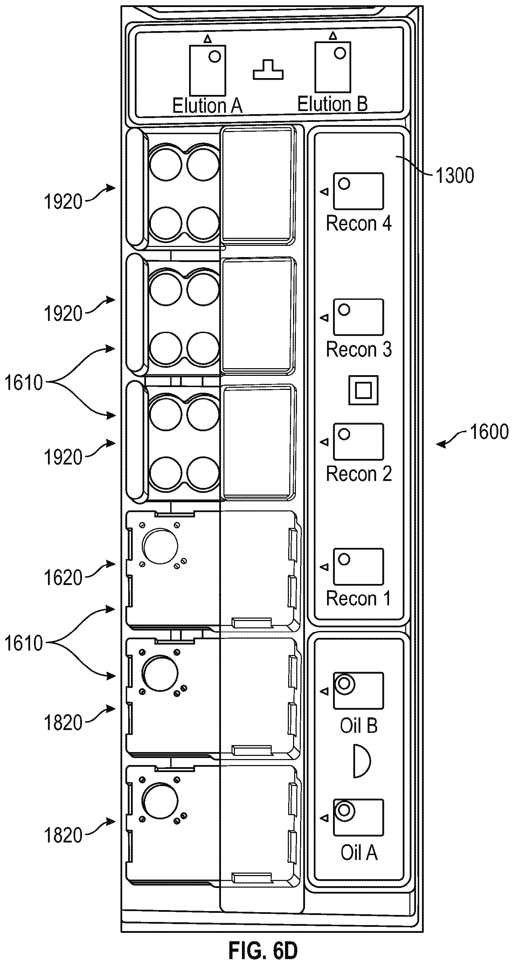

Although not a requirement, in some embodiments, first reagent container-carrier 1500 may be a component that includes two pockets 1510, each configured to receive a reagent container 1520 containing a reagent, such as an elution buffer, therein. And, second reagent container-carrier 1600 may be a component with multiple pockets 1610 (e.g., six pockets) configured to receive reagent carrying containers therein. FIG. 6C illustrates an exemplary reagent container compartment 500 with a first reagent container-carrier 1500 and a second reagent container-carrier 1600. In the embodiment illustrated in FIG. 6C, first reagent container-carrier 1500 is shown with one reagent container 1520 positioned in one of its two pockets 1510, and second reagent container-carrier 1600 is shown with two solvent containers (e.g., an IVD solvent container 1620 and an LDT solvent container 1920) in two of its six pockets 1610. In some embodiments, second reagent container-carrier 1600 may include six pockets 1610, and as illustrated in FIG. 6B, these six pockets 1610 may be configured to receive, for example, two oil containers 1820 and four solvent containers (e.g., two IVD solvent containers 1620 and two LDT solvent containers 1920, etc.). In general, the six pockets 1610 may include any container 1620, 1820, 1920. FIG. 6D is the top view of an exemplary second reagent container-carrier 1600 with two oil containers 1820, one IVD solvent container 1620, and three LDT solvent containers 1920 in its pockets 1610. As illustrated in FIG. 6D, system 1000 may identify the oil containers 1820 and solvent containers (1620 or 1920) positioned in the different pockets 1610 of container-carrier 1600 as "Oil A," "Oil B," and "Recon 1," "Recon 2," etc. In some embodiments, as depicted in FIG. 6B, the oil containers 1820 may be structurally similar to an IVD solvent container 1620. However, this is not a requirement, and in general, the oil containers 1820 may be any shape and configuration. Although not a requirement, in some embodiments, first reagent container-carrier 1500 and second reagent container-carrier 1600 may be separate components that are placed adjacent to, or spaced apart from, each other. In general, reagent container compartment 500 may include any number of container carriers, each having any number of pockets. For instance, in some embodiments, instead of a single second reagent container-carrier 1600 with six pockets 1610, multiple single reagent container carriers (e.g., two) with pockets (e.g., three pockets each) may be provided in reagent container compartment 500. The number and size of the pockets in a container-carrier may be dictated by, among other things, considerations of intended throughput and desired time period between required re-stocking of supplies. In some embodiments, the size and geometry of pockets 1610 in second reagent container-carrier 1600 may be identical or substantially the same. In such embodiments, IVD solvent containers 1620 and LDT solvent containers 1920 having the same or substantially the same external dimensions may be positioned in pockets 1610. Containers in reagent container compartment 500 may be identified by machine-readable code, such as RFID. An indicator panel 1300 having visible signals (e.g., red and green LEDs) and/or other indicators (textual, audible, etc.) may be provided in reagent container compartment 500 (and/or on the container carriers) to provide feedback to the user regarding container status. Indicator panel 1300 may be positioned at any location in reagent container compartment 500 or the container carriers (note different exemplary locations of indicator panels 1300 in FIGs. 6A and 6B). Reagent container compartment 500 may include a reagent container transport 1700 (see FIG. 6B) that is configured to move first reagent container-carrier 1500 from reagent container compartment 500 in second module 400 to a location within first module 100.

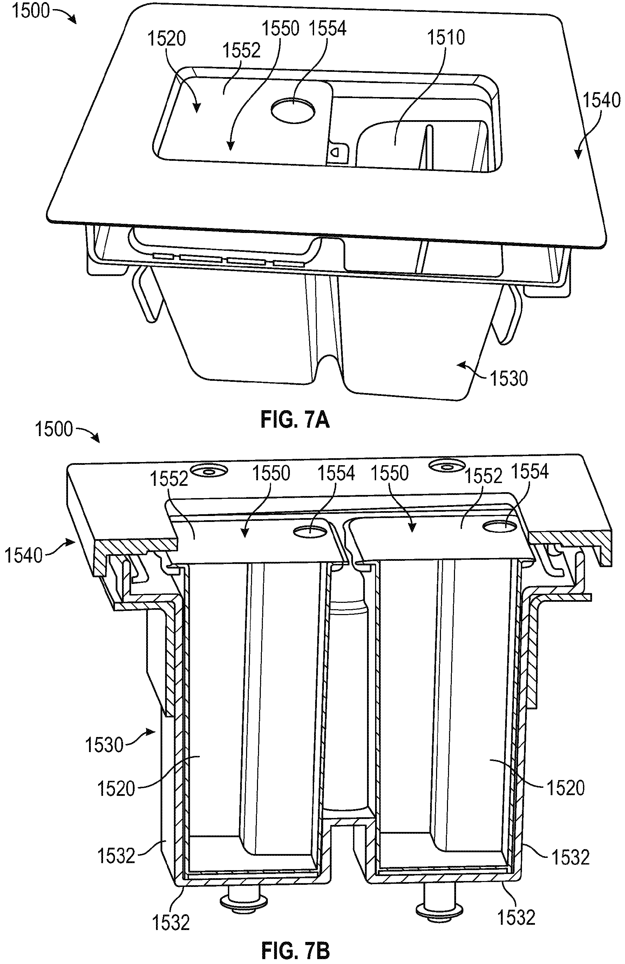

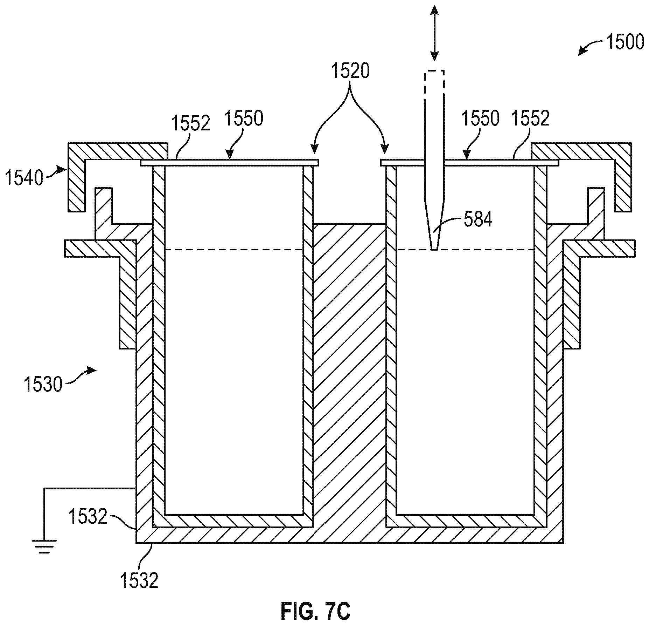

FIG. 7A illustrates an exemplary first reagent container-carrier 1500 with an exemplary reagent container 1520 in one of its two pockets 1510. FIG. 7B is a cross-sectional perspective view, and FIG. 7C is a cross-sectional schematic view of an exemplary first reagent container-carrier 1500 with a reagent container 1520 in each of its two pockets 1510. First reagent container-carrier 1500 may include a base or a tub portion 1530 that forms two pockets 1510 for receiving reagent containers 1520 therein, and a frame 1540 attached to tub portion 1530 to retain reagent containers 1520 in pockets 1510. In general, the shape and size of pockets 1510 of tub portion 1530 may correspond to the shape and size of reagent containers 1520 that will be received in these pockets. In some embodiments, pockets 1510 may be sized to snugly receive reagent containers 1520 therein. When a container 1520 is placed in a pocket 1510, and frame 1540 is attached to tub portion 1530, a portion of frame 1540 extends over a portion of container 1520 and prevents the withdrawal of container 1520 from pocket 1510. As illustrated in FIGS. 7A and 7B, frame 1540 may have a window-frame shape with an opening that exposes the top of container 1520 therethrough. In some embodiments, some or all of outer surfaces 1532 of tub portion 1530 may be metallized and grounded to support capacitive sensing of the fluid level in reagent containers 1520.

### Reagent Container

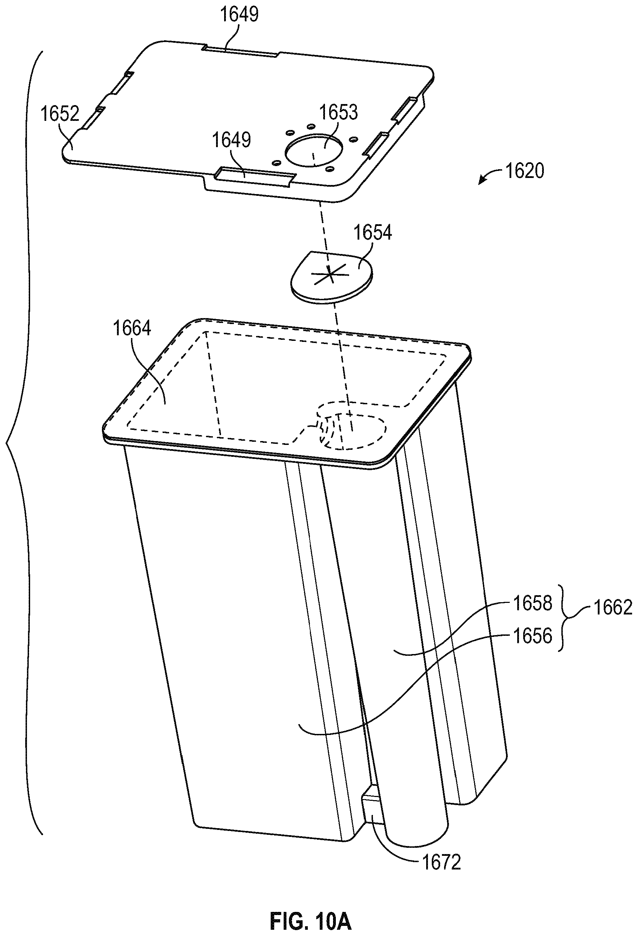

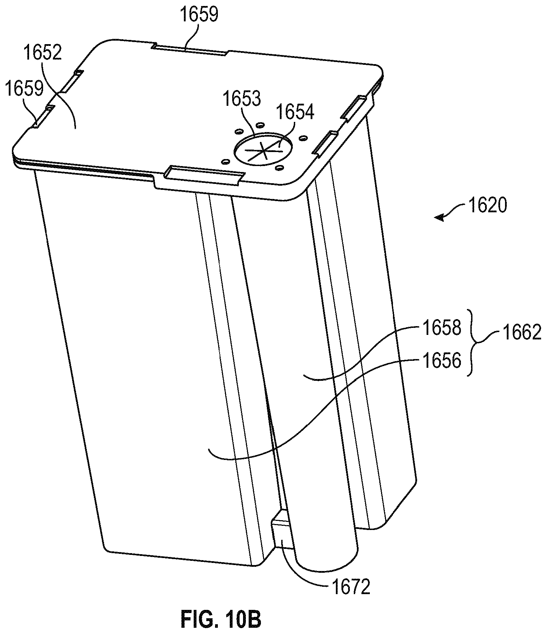

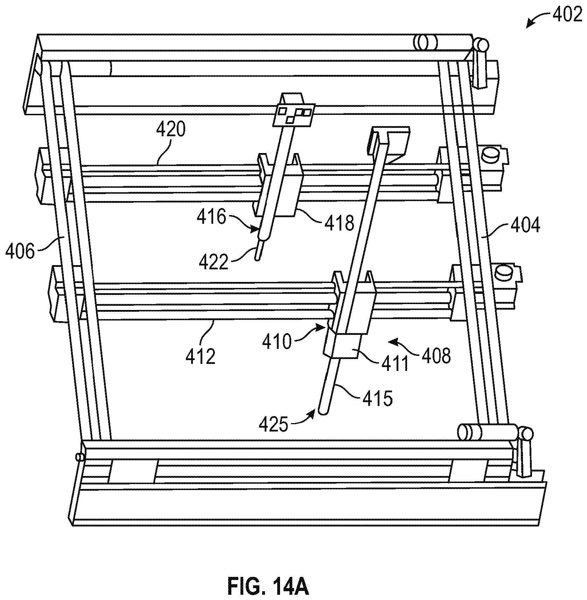

Reagent container 1520 may include a cup-like reservoir that contains a fluid reagent with a pipettor-pierceable cover 1550 that covers the mouth of the reservoir (see FIGs. 7A-7C). In some embodiments, the fluid reagent in reagent container 1520 may be an elution buffer. In some embodiments, cover 1550 may include one or more frangible materials (e.g., foil, elastomer, etc.) adapted to be pierced by an aspirator probe 415, or a disposable pipette tip 584 affixed to a mounting end 425 of aspirator probe 415, of a robotic pipettor (e.g., robotic pipettor 410, see FIGs. 14A-14C). During use, aspirator probe 425 or pipette tip 425 (attached to aspirator probe 415) may penetrate through the pipettor-pierceable cover 1550 and access the fluid stored in container 1520. FIG. 7C illustrates a schematic view of a pipette tip 584 (affixed to mounting end 425 of aspirator probe 415 of pipettor 410 of second module 400) accessing the fluid reagent stored in reagent container 1520 by piercing through cover 1550. In some embodiments, as illustrated in FIG. 7A (and in FIGs. 10A and 10B in more detail), a plastic (or another rigid material) lid 1552 with an opening may be attached over the pipettor-pierceable cover 1550 and a septum 1554 positioned between frangible cover 1550 and rigid lid 1552 to cover the opening. Septum 1554 may be made of a pipettor-pierceable material or include features (e.g., slits, etc.) that allow aspirator probe 415 or pipette tip 584 affixed to a mounting end 425 of pipettor 410 to access container 1520 therethrough. In such embodiments, aspirator probe 425 or pipette tip 584 may contact and pierce the frangible cover 1550 through septum 1554. When withdrawing pipette tip 584 from container 1520, the portion of frame 1540 above container 1520 may block removal of container 1520 from first reagent container-carrier 1500.

In some embodiments, reagent container 1520 may be structurally similar to IVD solvent container 1620 discussed infra with reference to FIGs. 10A and 10B. Some exemplary configurations of reagent containers 1520 are described in U.S. Patent Application Publication No. 2018/0290141 and titled "Fluid Receptacles."

In some embodiments, as pipettor 410 contacts the fluid in reagent container 1520, the level of the fluid in container 1520 may be detected using capacitive level sensing. To enable capacitive level sensing, the metallized outer surfaces 1532 of tub portion 1530 (of first reagent container-carrier 1500) may be coupled to the system ground (e.g., a ground surface of system 1000), and aspirator probe 415 or pipette tip 584 affixed to mounting end 425 of pipettor 410 may be connected to a voltage source (e.g., an alternating voltage source). In such a configuration, pipettor 410 (and, optionally, pipette tip 584 having conductive properties) serves as one conductor of a capacitor and the grounded outer surfaces 1532 serve as the other conductor. A capacitance signal (a signal related to the capacitance) measured between these two conductors may be used to detect the level of the fluid in reagent container 1520. In use, as aspirator probe 415 (or pipette tip 584 affixed to mounting end 425 of pipettor 410) moves downward into container 1520, the position (height) of aspirator probe 415 (or pipette tip 584) is monitored simultaneously along with the capacitance signal. When the capacitance signal increases rapidly (e.g., a spike caused by aspirator probe 415 or pipette tip 584 contacting the fluid), the height of aspirator probe 415 (or pipette tip 584) is recorded, thereby establishing the height of the fluid surface in container 1520. Although aspiration of the fluid in container 1520 using pipettor 410 of second module 400 is described above, fluid may also be extracted from container 1520 using other fluid transfer devices (such as, for example, pipettor 810 of first module 100).

### Reagent Container Transport

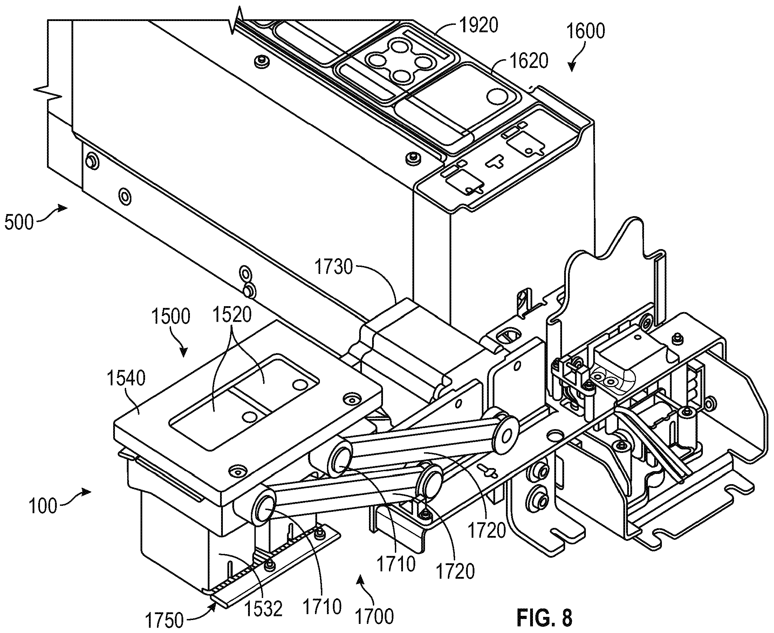

When reagent container compartment 500 is closed (see FIG. 1B), reagent container transport 1700 of second module 400 may engage with the ledges on frame 1540 of first reagent container-carrier 1500 to move first reagent container-carrier 1500 from second module 400 to a location in first module 100. FIG. 8 illustrates an exemplary reagent container transport 1700 engaged with first reagent container-carrier 1500. Reagent container transport 1700 includes links 1720, operatively coupled to an electric motor 1730, and pivotably coupled to structural members of second module 400 connected to the system ground (i.e., links 1720 are electrically grounded). Upon activation of reagent container transport 1700, links 1720 engage with frame 1540 via bearings 1710, and rotate about respective pivots, to move first reagent container-carrier 1500 from compartment 500 of second module 400 to a location within first module 100. When links 1720 are thus engaged with frame 1540, the metallized portions of first reagent container-carrier 1500 are electrically connected to the system ground (or is grounded) via links 1720. When first reagent container-carrier 1500 is positioned in first module 100, a grounded electrically conductive brush 1750 makes electrical contact with the metallized portions (e.g., metallized outer surfaces 1532 of tub portion 1530) of the first reagent container-carrier 1500. When positioned in first module 100, a fluid transfer device (e.g., pipette tip 584 of pipettor 810, see FIG. 7C) of first module 100 may access and aspirate a desired quantity of a reagent, such as an elution buffer, from reagent container 1520. The aspirated reagent is transported and discharged into a receptacle or a vial during an analytical procedure. In an exemplary embodiment, the reagent fluid is an elution buffer useful for eluting a targeted nucleic acid from a solid support, such as a magnetic particle or silica bead.

### Reagent Container-Carrier

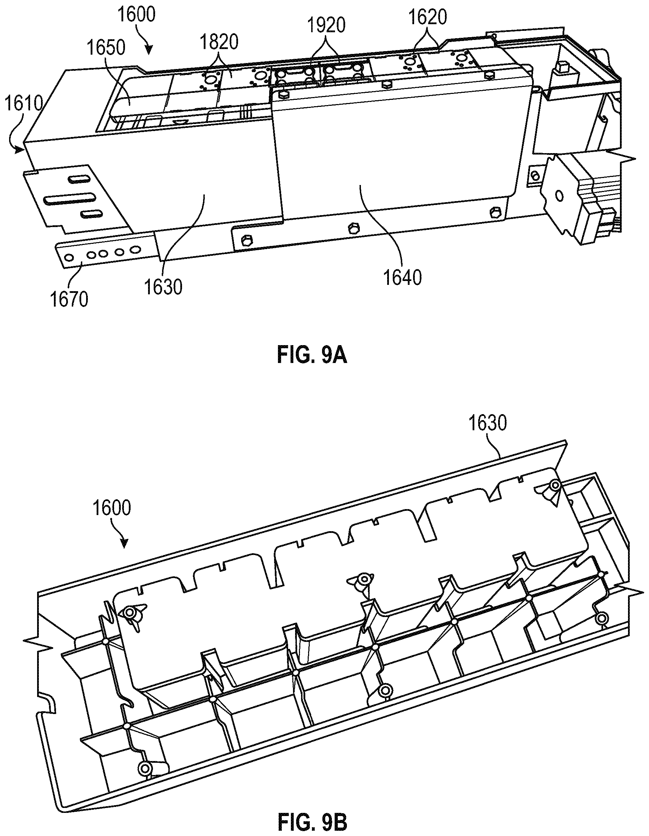

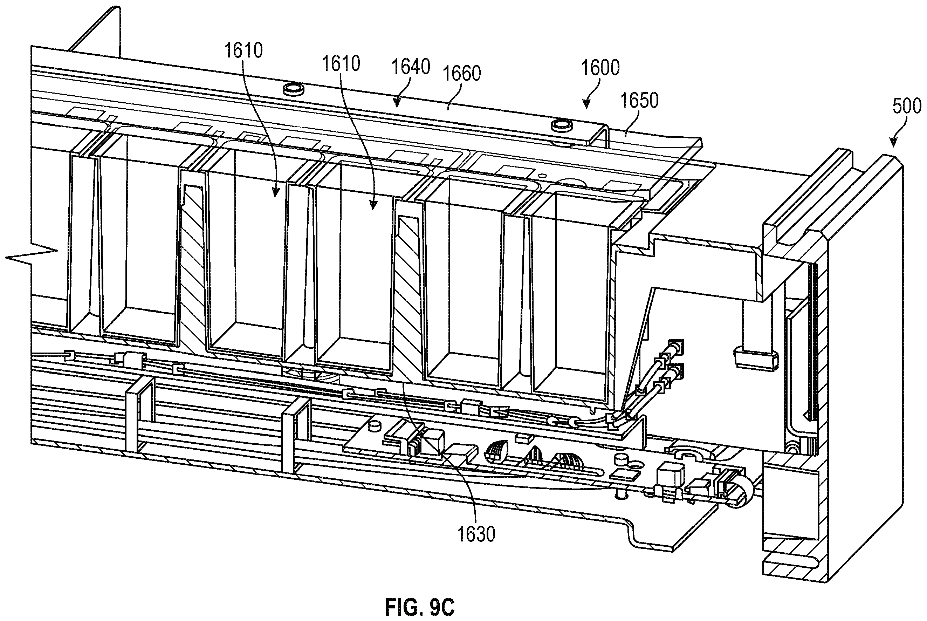

As explained previously with reference to FIGs. 6A-6C, the multiple pockets 1610 of second reagent container-carrier 1600 may include solvent containers (e.g., IVD solvent containers 1620 and/or LDT solvent containers 1920) containing a solvent (e.g., a solvent), and oil containers 1820 containing an oil (e.g., silicone oil). As known to those skilled in the art, the solvent and the oil may be reagents used in a molecular assay performed by analytical system 1000. Similar to first reagent container-carrier 1500 described above, as best seen in FIG. 6C, second reagent container-carrier 1600 may also include a base or a tub portion 1630 that includes pockets 1610 (that support the solvent containers and the oil containers therein), and a lid 1640 that retains these containers in their respective pockets 1610. FIGs. 9A, 9B, and 9C are perspective side, bottom, and cross-sectional views, respectively, of an exemplary second reagent container-carrier 1600. In the description below, reference will be made to FIGs. 6A-6C and FIGs. 9A-9C. In general, the shape and size of pockets 1610 (of tub portion 1630) may correspond to the shape and size of the containers (e.g., IVD and LDT solvent containers 1620, 1920 and oil containers 1820) that will be received in pockets 1610. In some embodiments, as illustrated in FIG. 9B, opposing side surfaces of tub portion 1630 may include crevices that separate individual pockets 1610. Typically, the shape and size of a pocket 1610 may match the shape and size of the fluid filled container that will be received in that pocket 1610. For example, the size and shape of a pocket 1610 may correspond to the shape and size of a solvent container that it supports, thereby providing a close fit in some embodiments. In some embodiments, pockets 1610 may all have the same or substantially the same shape and dimensions. However, it is also contemplated that pockets 1610 may have different shapes and/or sizes (e.g., to receive differently shaped and/or sized containers therein).

As best seen in FIG. 6C, lid 1640 of second reagent container-carrier 1600 may include a top portion 1650 and a bracket portion 1660. Although not a requirement, in some embodiments, top portion 1650 may be formed of an electrically nonconductive material and bracket portion 1660 may be formed of an electrically conductive material. In some embodiments, top portion 1650 may be a transparent or a translucent plate-like member. Top portion 1650 and bracket portion 1660 may be two parts that are attached together to form lid 1640 or may be two regions of a single-piece lid 1640. When lid 1640 is positioned on tub portion 1630, top portion 1650 of lid 1640 may extend over a portion of the top surface of tub portion 1630. In this configuration, top portion 1650 may extend over (and overlie) a portion of a solvent container 1620, 1920 placed in a pocket 1610 and prevent that container 1620, 1920 from being accidentally removed from pocket 1610. Although not a requirement, in some embodiments, the overlying region of top portion 1650 may press down on the underlying region of container to constrain the container in pocket 1610. The portion of IVD solvent container 1620 and/or LDT solvent container 1920 (in pocket 1610) that is not covered by top portion 1650 of lid 1640 provides access to aspirator probe 415 or pipette tip 584 affixed to mounting end 425 of pipettor 410 to extract solvents from container 1620, 1920.

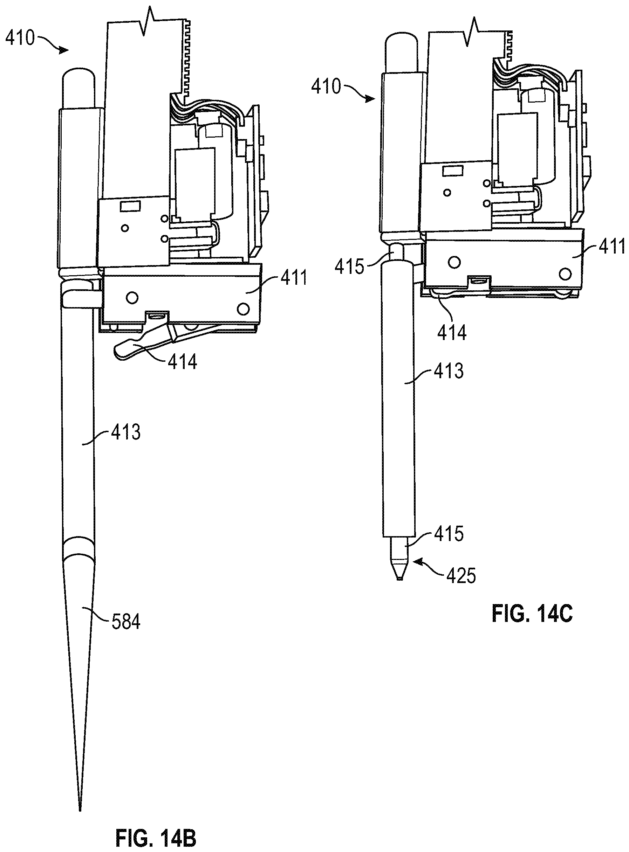

As best seen in FIG. 9A, lid 1640 of second reagent container-carrier 1600 may be attached to a frame/chassis 1670 of second module 400 such that, when reagent container compartment 500 is closed (see FIG. 1A), top portion 1650 of lid 1640 extends over containers 1620, 1820, 1920 positioned in pockets 1610 of second reagent container-carrier 1600. When in this configuration, aspirator probe 415 or pipette tip 584 (affixed to mounting end 425 of aspirator probe 415) of robotic pipettor 410 (see FIGs. 14B-14C) may extract a solvent from a solvent container 1620, 1920 (and oil from an oil container 1820) positioned in second reagent container-carrier 1600 as will be described in more detail infra (with reference to FIGs. 10A-10C). When aspirator probe 415 (or pipette tip 584 affixed to mounting end 425) of pipettor 410 withdraws from a container (1620, 1820, 1920) after aspirating fluid, the container may have a tendency to come out of its respective pocket 1610. Top portion 1650 extends over a portion of the top of the containers 1620, 1820, 1920 and prevents the accidental removal of the container from its pocket. When reagent container compartment 500 is opened (see FIG. 6A), tub portion 1630 of second reagent container-carrier 1600 slides out from under lid 1640, so that the user can load (and unload) IVD solvent containers 1620, LDT solvent containers 1920, and oil containers 1820 into pockets 1610. In some embodiments, similar to that described with reference to first reagent container-carrier 1500, some surfaces of tub portion 1630 may be metallized, such that, when second reagent container-carrier 1600 is placed in reagent container compartment 500, these metallized portions will be electrically connected to the system ground (e.g., a housing of system 1000) and serve as a ground plane to enable capacitive fluid level sensing using aspirator probe 415 or pipette tip 584 (affixed to mounting end 425 of pipettor 410). U.S. Patent Application Publication No. 2018/0282788 and titled "Systems and Methods for Capacitive Fluid Level Detection, and Handling Containers," describes exemplary first and second reagent container carriers 1500, 1600 that may be used in system 1000.

### IVD Solvent Containers

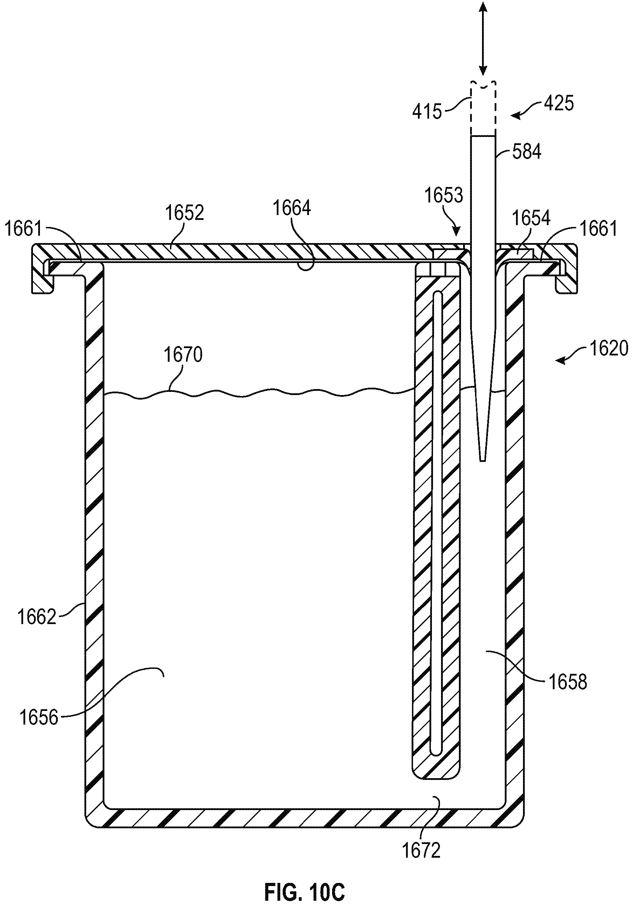

In some embodiments, an IVD solvent container 1620 may be similar in structure to reagent container 1520 described previously. FIG. 10A illustrates an exploded perspective view of an exemplary IVD solvent container 1620, FIG. 10B illustrates a perspective view of IVD solvent container 1620, and FIG. 10C is a cross-sectional view of IVD solvent container 1620 containing a solvent 1670 therein. In the description below, reference will be made to FIGs. 10A-10C. In some embodiments, IVD solvent container 1620 may be a heat sealed pack (e.g., foil pack) that includes a reconstitution buffer suitable for known (e.g., FDA approved or CE marked) IVD assays. That is, solvent 1670 in IVD solvent container 1620 may be a reconstitution buffer (i.e., a universal reagent adapted for reconstituting dried reagents that include amplification oligomers and/or detection probes). Exemplary reconstitution buffers that may be used as solvent 1670 and exemplary dried reagents for use with the reconstitution buffers are described in International Publication No. WO 2017/136782. For some assays (e.g., PCR), multiple amplification oligomers (forward amplification oligomer or primer, reverse amplification oligomer or primer, etc.) and/or probes may be used. During an exemplary molecular assay, solvent 1670 (i.e., reconstitution buffer) in IVD solvent container 1620 may be used to reconstitute dried or lyophilized reagents (or a reagent in another form, e.g., a gel, etc.) that include different types of amplification oligomers and probes for amplifying different target nucleic acids.

Similar to reagent container 1520, IVD solvent container 1620 may include a cup-like reservoir 1662 (containing reconstitution fluid 1670) sealed with a pipettor-pierceable (e.g., foil, elastomer, etc.) frangible cover 1664. In some embodiments, reservoir 1662 may be configured to contain an amount of fluid 1670 sufficient to perform about 50 to about 2,000 assays. However, it is also contemplated that the amount of fluid 1670 may be sufficient to perform less than 50 assays or more that 2000 assays. In some embodiments, pipettor-pierceable cover 1664 of reservoir 1662 may be covered by a lid 1652 (e.g., made of a relatively rigid material, such as, for example, plastic, etc.) having an opening 1653. A septum 1654 may be positioned between cover 1664 and lid 1652, such that the septum covers opening 1653 on lid 1652.

As best seen in FIG. 10C, reservoir 1662 of solvent container 1620 may define multiple fluidly connected chambers that are configured to hold reconstitution fluid 1670 therein. These chambers may include a first chamber 1656 and a second chamber 1658 fluidly coupled together at the bottom of chambers 1656, 1658 by a conduit 1672. First chamber 1656 may have a greater volume than second chamber 1658 and may consequently be configured to carry a larger volume of fluid 1670 than second chamber 1658. After the chambers are filled with a desired quantity of fluid 1670, the pipettor-pierceable frangible cover 1664 is attached to a top surface 1661 of reservoir 1662 to hermetically seal chambers 1656 and 1658. Cover 1664 may be attached to reservoir 1662 by any suitable method (adhesive, heat welding, ultrasonic welding, etc.). As illustrated in FIG. 10A, lid 1652 is then attached to reservoir 1662 over cover 1664 with septum 1654 covering the opening on lid 1652. As can be seen in FIGs. 10A-10C, lid 1652 includes features that engage with corresponding features on reservoir 1662 to secure lid 1652 to reservoir 1662. These features may include lips or protrusions 1659 on reservoir 1662 (or lid 1652) that engage with corresponding cutouts or recesses 1649 on lid 1652 (or reservoir 1662). When lid 1652 is attached to reservoir 1662, septum 1654 is positioned over second chamber 1658 of reservoir 1662. Thus, second chamber 1658 is an "access-chamber" for receiving a fluid transfer device, such as aspirator probe 415, or a pipette tip 584 affixed to mounting end 425 of aspirator probe 415, of robotic pipettor 410. During use, the pipettor (i.e., aspirator probe 415 or pipette tip 584) enters second chamber 1658 (or access-chamber) through septum 1654 (after piercing through frangible cover 1664 over second chamber 1658) to extract fluid 1670 (e.g., aspirate fluid 1670) from reservoir 1662. In some embodiments, septum 1654 may include a structure that enables the pipettor to enter second chamber 1658 through septum 1654. In some embodiments, septum 1654 may include a starburst pattern of slits that form flexible flaps that bend and allow aspirator probe 415 or pipette tip 584 (affixed to mounting end 425) of pipettor 410 to pass through. These slits may be pre-formed (e.g., flaps precut) or may be formed after aspirator probe 415 (or pipette tip 584) of pipettor 410 penetrates through a scored pattern provided on septum 1654. When the pipettor withdraws from second chamber 1658 (of reservoir 1662 after aspirating fluid 1670), the flaps of the septum 1654 cover the opening on frangible cover 1664 (formed by aspirator probe 415 or pipette tip 584) and reduces evaporation of the fluid 1670 from the reservoir 1662. Since the surface area of fluid in second chamber 1658 is lower than that in first chamber 1656, extracting fluid 1670 from second chamber 1658 (as opposed to first chamber 1656) further helps in reducing fluid loss from reservoir 1662 through evaporation. As fluid 1670 is extracted from second chamber 1658, fluid from first chamber 1656 enters second chamber 1658 through conduit 1672 to equalize the fluid level in both the chambers.

U.S. Patent Application Publication No. 2018/0290141 describes an embodiment of IVD solvent container 1620. As explained previously, in some embodiments, reagent container 1520 and oil container 1820 may also have a structure similar to that of IVD solvent container 1620. In a manner similar to that described with reference to reagent container 1520, when fluid 1670 is extracted from IVD solvent container 1620, pipettor 410 may detect the fluid level in container 1620 by capacitive fluid level sensing. During capacitive fluid level sensing, the metallized portions of second reagent container-carrier 1600 (that is connected to the system ground) positioned close to the base of fluid 1670 in IVD solvent container 1620 improves the accuracy and sensitivity of the fluid level measurement.

### LDT Solvent Containers

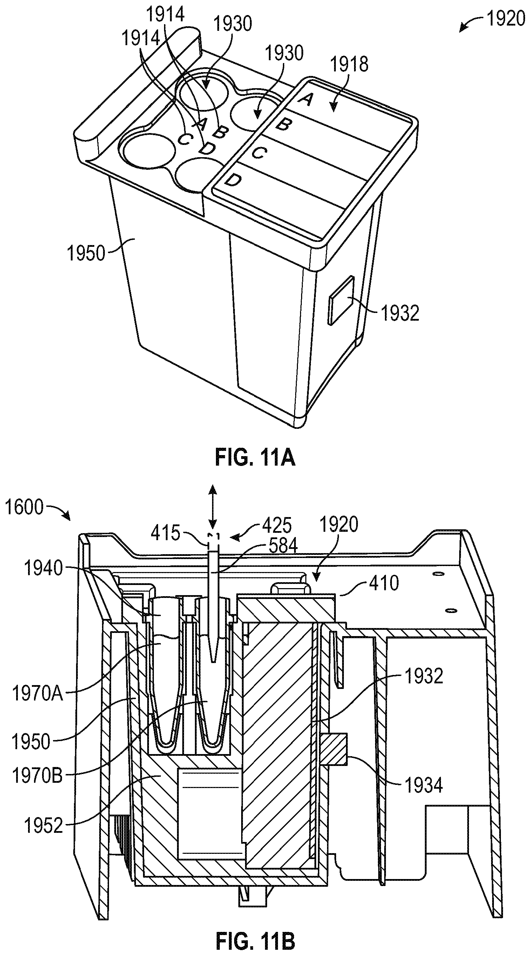

In some embodiments, an LDT solvent container 1920 used in system 1000 may have a different configuration than the IVD solvent container 1620 described above. FIGs. 11A and 11B illustrate an exemplary LDT solvent container 1920 that may be used in system 1000. FIG. 11A illustrates a perspective view of container 1920 and FIG. 11B illustrates a schematic cross-sectional view of container 1920 positioned in second reagent container-carrier 1600. In the description below, reference will be made to both FIGs. 11A and 11B. LDT solvent container 1920 includes a body 1950 having multiple recesses 1930 (e.g., cavities formed in a solid portion of the body) that are each configured to support a fluid-containing receptacle 1940 (such as, for example, a tube or a vial containing reconstitution fluid) therein. For example, in some embodiments, four substantially cylindrically shaped recesses 1930 may be arranged in a rectangular configuration (e.g., in a 2 × 2 grid) in body 1950. However, in general, LDT solvent container 1920 may define more or less than four recesses 1930, and recesses 1930 may have any shape (e.g., conical, frusto-conical, rectangular, etc.) and may be arranged in any suitable configuration (e.g., circular, linear, etc.). Although not a requirement, in some embodiments each recess 1930 of container 1920 may be sized to receive therein a similarly dimensioned receptacle 1940. In some embodiments, some or all of recesses 1930 may have different dimensions to receive correspondingly sized receptacles 1940 therein.

Receptacles 1940 containing reconstitution fluids 1970A, 1970B, etc. are placed in each recess 1930 of LDT solvent container 1920. In general, the different receptacles 1940 of container 1920 may contain the same reconstitution fluid or different reconstitution fluids (i.e., reconstitution fluid to be used for the same assay or for different assays). For example, in some embodiments, reconstitution fluid 1970A may be a reagent that includes one type of amplification oligomer(s) and/or probe(s), and reconstitution fluid 1970B may be a reagent that includes a different type of amplification oligomer(s) and/or probe(s). In some embodiments, each set of amplification oligomers and probes in a reconstitution fluid 1970A, 1970B may be designed to detect a different analyte, which may be different nucleic acids or different regions of the same nucleic acid. In some embodiments, one or more of reconstitution fluids 1970A, 1970B may include at least one forward amplification oligomer and at least one reverse amplification oligomer. In some embodiments, one or more of reconstitution fluids 1970A, 1970B may include a probe having a detectable label (or signaling moiety) or which can be detected when hybridized to a target nucleic acid using an intercalating dye, such as SYBR^{®} Green. Body 1950 of container 1920 may include one or more indicators 1914 (e.g., a unique indicator) to identify each recess 1930. Indicators 1914 may include alphanumeric text as shown in FIG. 11A, a symbol, a color, or any other suitable indicator that will assist in distinguishing between the fluids supported in recesses 1930. For example, indicators 1914 may identify the type of reconstitution fluid (e.g., amplification oligomer(s), probe(s), etc.) included in the reconstitution fluid contained in a receptacle 1940. Indicators 1914 may be labels affixed to body 1950 (e.g., proximate each recess 1930) or may be marks integrally formed on body 1950. In some embodiments, body 1950 may also include a surface adapted to receive one or more user-provided indicators 1918. Indicators 1918 may, for example, describe the process (for example, an assay) to be performed using the fluid in a receptacle 1940 received in a recess 1930. User-provided indicators 1918 may include alphanumeric text, symbols, colors, or any other indicator that has a known association with the fluid (e.g., indicative of the fluid, a particular process to be performed using the fluid, etc.) in a recess 1930. In some embodiments, a user-provided indicator 1918 may identify the target analyte for a test. For example, a solvent for amplifying and detecting nucleic acid derived from Mycoplasma genitalium may be identified as "M. gen." in user-provided indicators 1918. In some embodiments, indicator 1918 may include the name of a test to be performed using a fluid in a recess 1930. In some embodiments, user-provided indicator 1918 may be a user-applied mark (e.g., from a writing instrument) or a user-affixed label (e.g., a sticker).

Solvent container 1920 may also include an RFID transponder 1932 attached thereto. RFID transponder 1932 may be attached to an electrically nonconductive portion of solvent container 1920 or may be positioned such that it is isolated from the electrically conductive portions of container 1920. RFID transponder 1932 may be configured to wirelessly transmit information related to container 1920 (e.g., receptacle identifiers that identify each receptacle 1940, a holder identifier that identifies container 1920, process identifiers that identify the processes to be performed using the fluids contained in receptacles 1940, etc.) to an RFID reader 1934 of system 1000. Although FIG. 11B illustrates RFID reader 1934 as being attached to second reagent container-carrier 1600, this is only exemplary. In general, RFID reader 1934 may be attached to any part of system 1000 such that it receives the information transmitted by RFID transponder 1932. Any type of RFID transponder 1932 and reader 1934 may be used in system 1000. Since suitable RFID transponders 1932 and readers 1934 are known in the art, they are not described in detail herein. U.S. Provisional Application No. 62/530,743, filed on July 10, 2017 and titled "Receptacle Holders, Systems, and Methods for Capacitive Fluid Level Detection," describes exemplary solvent containers 1920 that may be used in system 1000.

In the description above, two types of solvent containers (i.e., IVD solvent container 1620 and LDT solvent container 1920) are described. And, in some embodiments, both of these containers 1620 and 1920 may be sized to be positioned in a pocket 1610 of second reagent container-carrier 1600 (see FIGs. 6A-6C). Any type of solvent container (e.g., container 1620 or 1920) may be used in system 1000. Typically, for IVD assays, suitable reconstitution buffers may be obtained (e.g., commercially obtained) in sealed (e.g., heat-sealed) IVD solvent containers 1620. Thus, when system 1000 is used to perform an IVD assay, sealed IVD solvent containers 1620 that include reconstitution buffers may be procured and loaded on second reagent container-carrier 1600 and used in a nucleic acid amplification assay. During the assay, the reconstitution buffer may be used to reconstitute a reagent (e.g., a dried reagent) for amplification. Typically, the dried reagent used in IVD assays includes the required constituents (such as, for example, amplification oligomers, probes, polymerases, etc.) for an amplification reaction, and therefore, the reconstitution buffers provided in sealed IVD solvent containers 1620 may not include these constituents. In contrast, for an assay developed or evaluated by a customer or other third party (i.e., an LDT), at least some of the constituents needed for the amplification reaction (e.g., some or all of the amplification oligomers, probes, etc.) are typically designed, developed and validated by the customer or third party. Therefore, these constituents are not included in the reagent (e.g., dried reagent) used for such LDTs. Instead, the customer or other third party may prepare reconstitution fluid(s) (e.g., 1970A, 1970B, etc.) that includes one or more of amplification oligomers, probes, etc., and provide them in receptacles 1940 of LDT solvent container 1920. For example, reconstitution fluids 1970A and 1970B may contain different amplification oligomers and probes that target different nucleic acids or different regions of the same nucleic acid. Further, reconstitution fluids that include amplification oligomers (and/or probes) may be used to reconstitute dried amplification reagents that do not include any amplification oligomers and/or probes.

In some embodiments, only a single type of solvent container (e.g., container 1620 or 1920) may be used in system 1000 during an analysis. For example, if all the samples will be analyzed by system 1000 using one or more IVD assays, system 1000 may use only IVD solvent containers 1620 with a reconstitution buffer therein. Similarly, if all the samples are planned to be analyzed by system 1000 using one or more LDTs, only LDT solvent containers 1920 may be used. In some embodiments, system 1000 may be an open channel system that permits a user to perform both IVD assays and LDTs on the same or different samples without replacing or reloading solvent containers (and/or samples). In such embodiments, both IVD solvent containers 1620 and LDT solvent containers 1920 may be used at the same time in system 1000. For example, when one or more samples will be analyzed using an IVD assay(s) and one or more samples will be analyzed using an LDT(s) during an analysis run, both IVD and LDT solvent containers 1620 and 1920 may be loaded in system 1000. In such cases, as illustrated in FIGs. 6A-6C, one or more IVD solvent containers 1620 with a reconstitution buffer (that does not include constituents such as, for example, amplification oligomers, probes, etc.) and one or more LDT solvent containers 1920 with a reconstitution solution or a solvent (that includes constituents such as, for example, amplification oligomers, probes, etc.) may both be loaded on second reagent container-carrier 1600 provided in reagent container compartment 500 of system 1000. The IVD assays may then be conducted using reconstitution buffer in IVD solvent container(s) 1620 and the LDTs may be conducted using one or more of reconstitution fluids 1970A, 1970B (as needed by the particular assay) in LDT solvent container(s) 1920. In some embodiments, the IVD assays and the LDTs may be performed by system 1000 in an interleaved or random access manner. That is, the IVD assays and the LDTs may be alternately performed by system 1000, without having to pause system 1000 to replace reagents or consumables between IVD assays and LDTs. For example, an IVD assay(s) may first be initiated (e.g., one or more IVD assays initiated with one or more samples), followed by LDT(s) (e.g., one or more LDTs initiated with one or more of the same or different samples), which may then followed by an IVD assay(s), etc. without swapping, loading, or replenishing reconstitution fluids, reagents, and/or other consumables between the different assays. While the IVD assays and LDTs may be initiated at different times, these two assay types may be performed simultaneously by system 1000 (i.e., processing of a sample by one assay type is initiated before processing is completed on a sample by the other assay type). Any number of IVD solvent containers 1620 and LDT solvent containers 1920 may be loaded in second reagent container-carrier 1600 (e.g., based on need). For example, if during a run it is expected that more of reconstitution buffer 1656 (e.g., used in IVD assays) will be required than reconstitution fluids 1970A, 1970B, then a greater number of IVD solvent containers 1620 may be provided to system 1000 than LDT solvent containers 1920 (or vice versa). The number of each type of solvent container 1620, 1920 required will also be driven by the volume capacity of the different containers 1620, 1920.

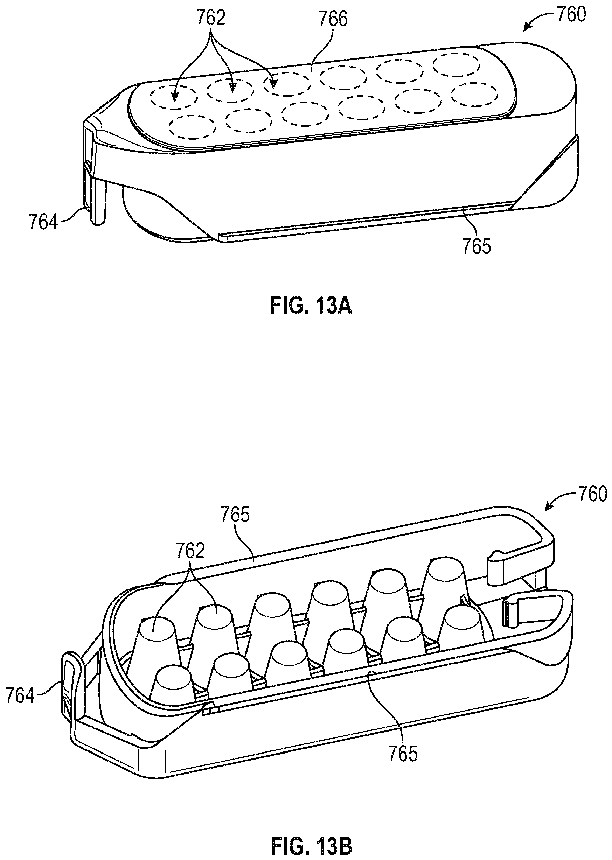

As explained previously, system 1000 can perform both IVD assays and LDTs in an interleaved manner. In embodiments where an IVD assay and an LDT performed by system 1000 both incorporate PCR amplification reaction, the amplification reactions for both assays (i.e., IVD and LDT) occur in second module 400 (e.g., in thermal cycler 432). However, in embodiments where one assay (e.g., an IVD assay) is not subjected to PCR conditions and another assay (e.g., an LDT) is subjected to PCR conditions, amplification of the IVD assay occurs in first module 100 (e.g., in amplification incubator 114) and the amplification of the LDT occurs in second module 400 (e.g., in thermal cycler 432). When first module 100 is used for amplification, a reagent 768 in a reagent pack 760 (described below with reference to FIGs. 13A-13D) may not be used. Instead, liquid reagents stored in first module 100 may be used.

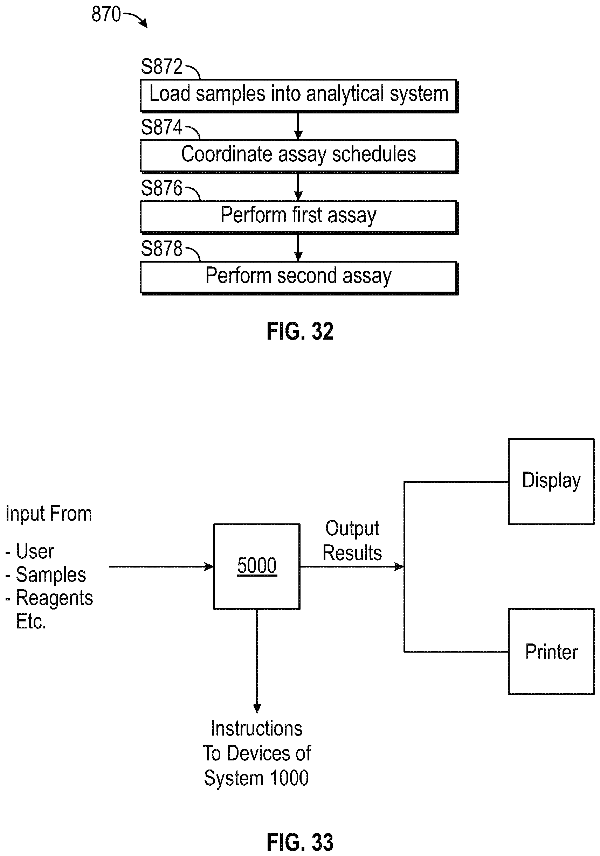

With reference to FIGs. 11A and 11B, during use, receptacles 1940 containing reconstitution fluids 1970A, 1970B, etc. are positioned in respective recesses 1930 of LDT solvent container 1920, and container 1920 is inserted into a pocket 1610 of second reagent container-carrier 1600 positioned in reagent container compartment 500 (see FIGs. 6A-6C). In some embodiments, all four recesses 1930 of a container 1920 may be loaded with a reconstitution fluid containing receptacle 1940, while in other embodiments, less than all recesses 1930 of container 1920 may include a receptacle. As explained previously, the reconstitution fluids (e.g., fluids 1970A, 1970B) in receptacles 1940 of LDT solvent container 1920 may be the same fluid or different fluids. After loading a desired number and types of containers (e.g., containers 1620, 1820, and 1920) in second reagent container-carrier 1600, the user closes compartment 500. When an LDT solvent container 1920 is seated within pocket 1610 of container-carrier 1600, RFID transponder 1932 on container 1920 (see FIGs. 11A and 11B) is positioned within the operational field of RFID reader 1934. While in this position, RFID reader 1934 transmits information about container 1920 to a controller (e.g., controller 5000 of FIG. 33). This information may include, among other information, one or more of the following: (1) a receptacle identifier that identifies each receptacle 1940 supported in container 1920; (2) a holder identifier that identifies container 1920; and (3) a process identifier that identifies the processes (e.g., assays) to be performed using reconstitution fluids 1970A, 1970B, etc. in receptacles 1940 of container 1920. Additionally, RFID reader 1934 may determine the presence of a container 1920 in a pocket 1610 of second reagent container-carrier 1600. For example, if RFID reader 1934 does not receive any transmitted information that would typically be transmitted by RFID transponder 1932, this may indicate that there is no LDT solvent container 1920 present in a pocket 1610.

Based on the information received from RFID reader 1934, the controller may determine the process to be performed using reconstitution fluids 1970A and 1970B contained in receptacles 1940 of container 1920 based on a known association of the received information with a particular process (e.g., saved on system 1000). For example, the received information may indicate that a type of LDT, the user-defined assay parameters of which are known to system 1000 (e.g., parameters previously saved on a storage device of system 1000), is to be performed using the fluids in container 1920. In some cases, the information received from RFID reader 1934 does not have a known association with a process known to system 1000. For example, reconstitution fluids 1970A and 1970B in LDT solvent container 1920 are intended to perform one or more assays that have not been previously performed (or saved) on system 1000. In some embodiments, if there is a known association with a protocol to be performed using reconstitution fluids 1970A and 1970B, system 1000 processes one or more samples by performing the associated protocol using these fluids without further user input based on protocols saved on system 1000. But if there is no known association, additional user input may be required from the user. In some such embodiments, system 1000 (e.g., controller 5000 of FIG. 33) may prompt the user for information using, for example, a graphical user interface (GUI) displayed on a display device 50 of system 1000 (see FIG. 1A) or another display associated with system 1000 (e.g., a remote computer running a software tool to develop an LDT protocol, discussed infra), defining one or more parameters of an assay protocol that can be saved and later associated with the LDT reconstitution fluids 1970A, 1970B. In this context, a first computer is "remote" from a second (and, possibly, one or more additional computers) if the first and second computers are separate computers having independent logic and computing functionality and independent data input and output components. The first computer and the remote second computer may or may not be in communication - e.g., wired or wirelessly - with each other and may or may not be networked with one another.

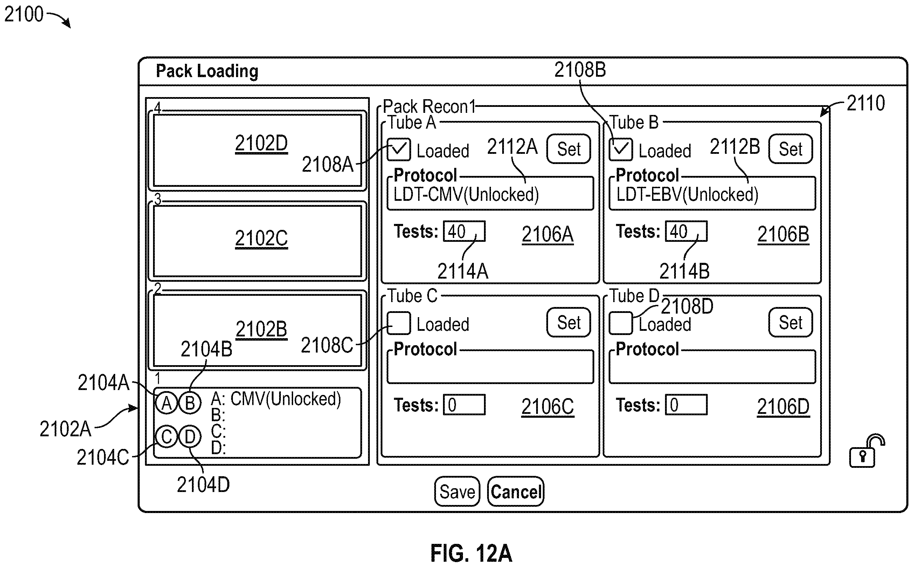

To load an LDT solvent container 1920 into system 1000, reagent container compartment 500 of second module 400 is first opened. In some embodiments, compartment 500 may be opened by selecting an icon (e.g., pressing the icon) on display 50. An LDT solvent container 1920 is placed into any one of the pockets 1610 of second reagent container-carrier 1600 (for example, in the pocket labelled "Recon 4" in FIG. 6D). A pack loading screen or GUI 2100 is displayed on display device 50. FIG. 12A illustrates an exemplary pack loading GUI 2100 displayed on display device. GUI 2100 includes regions 2102A-2102D that represent/correspond to each reconstitution container pocket (e.g., "Recon 1," "Recon 2," "Recon 3," and "Recon 4" of FIG. 6D) of container-carrier 1600. Controller 5000 (discussed infra) of system 1000 is configured to change a characteristic of regions 2102A-2102D to indicate the presence or absence of a container 1920 in a pocket 1610 of container-carrier 1600 based on signals from, for example, RFID reader 1934 and/or other sensors indicating the presence or absence of the container 1920.

When LDT solvent container 1920 is loaded in the "Recon 1" position of container-carrier 1600, as illustrated in FIG. 12A, the appearance of region 2102A changes to indicate the presence of container 1920 in this position. Window 2110 of GUI 2100 also changes to correspond to four regions 2106A-2106D. Each region 2106A-2106D corresponds to one of the four recesses 1930 of container 1920 (marked A-D in FIG. 12A). If a receptacle 1940 is present in a recess 1930 (e.g., recess A) of container 1920, the user may select box 2108A (e.g., click on box 2108A) of region 2106A to indicate that a receptacle 1940 is "Loaded" in recess A. The "Set" button in region 2106A is then clicked to select an LDT protocol from a menu. Clicking on "Set" may present the user with a menu (e.g., a drop-down menu) of available LDT protocols saved in system 1000. To associate the reconstitution fluid in receptacle 1940 of recess A with an LDT protocol, the user may then select from the menu presented a desired assay to be performed using the reconstitution fluid in receptacle 1940 of recess A. GUI 2100 may then display the selected assay in sub-area 2112A. For example, the user selects "LDT-CMV," which is then displayed in sub-area 2112A. Sub-area 2112A also indicates whether the selected assay is an unlocked assay or a locked assay. A sub-area 2114A indicates the maximum number of times the selected assay can be performed using the fluid contained in the receptacle 1940 in recess A. In some embodiments, a default value (e.g., 40) may be presented in sub-area 2114A which may be changed by the user, if desired. Assigning or associating the reconstitution fluid in recess A to an LDT is now complete.

If another receptacle 1940 is present in another recess (e.g., one of recesses B-D) of container 1920, the above-described steps are completed for the corresponding region 2106B-2106D of window 2110. Indicators 2104A-2104D of region 2102A indicate when all the receptacles have been assigned or associated. After the information for a recess A-D is entered in the corresponding region 2106A-2106D, the corresponding indicator 2104A-2104D in region 2102A changes color to indicate the status of the assignation. For example, if a recess A-D is loaded with a receptacle 1940 and all the information in the corresponding region 2106A-2106D has been entered, the corresponding indicator 2104A-2104D displays a green light, if a receptacle 1940 has been loaded but the required information has not been entered, the indicator displays a red light. And, if a recess A-D has not been loaded with a receptacle 1940, the corresponding indicator 2104A-2104D appears black.

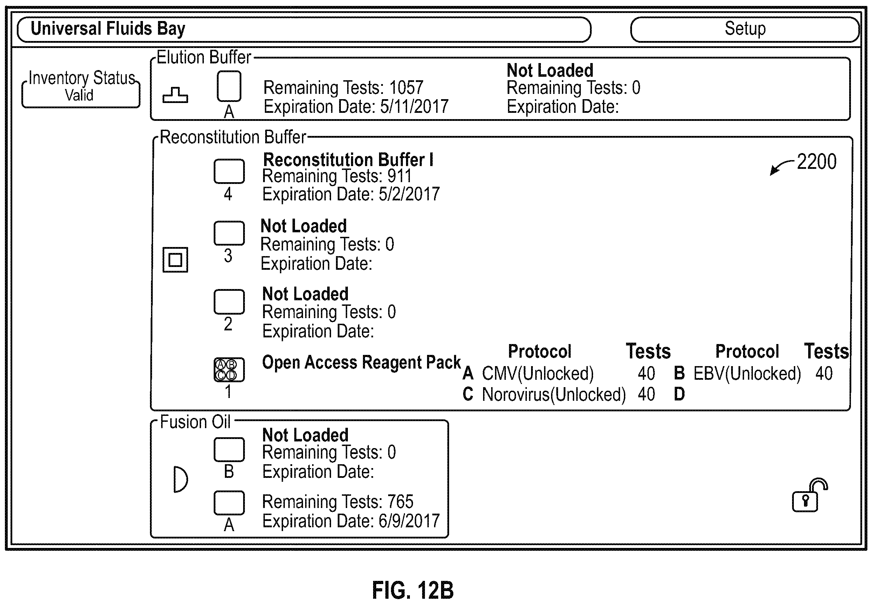

Once all the receptacles 1940 of container 1920 have been assigned or associated with an LDT, the user selects "Save" on GUI 2100 and closes reagent container compartment 500. After all the desired containers (oil container 1820, reconstitution fluid containers 1620, 1920, and reagent containers 1520) have been loaded in bulk reagent container compartment 500, display device 50 displays a universal fluids bay GUI 2200. FIG. 12B illustrates an exemplary universal fluids bay GUI 2200. As illustrated in FIG. 12B, GUI 2200 displays the status (e.g., loaded or not loaded) of all the containers, type of container, and other information (number or remaining tests, expiration date, etc.) associated with each container in reagent container compartment 500.

Using the user input received using GUI 2100 (FIG. 12A), the controller of system 1000 may associate reconstitution fluids 1970A and 1970B in container 1920 to user-selected assays, and when one of these assays is scheduled to be performed on a sample, system 1000 uses the corresponding reconstitution fluid for performing the assay. When a step of the assay is scheduled to be performed, a robotic pipettor 410 may move to align itself with a receptacle 1940 (of container 1920) that contains the required reconstitution fluid (e.g., fluid 1970A, 1970B, etc.), and aspirator probe 415 or pipette tip 584 on mounting end 425 of pipettor 410 may enter receptacle 1940 and aspirate a portion of the fluid from receptacle 1940. The level of fluids 1970A and 1970B in receptacle 1940 may be determined by pipettor 410 using capacitive level sensing during aspiration (in a manner similar to that described previously). To enable capacitive level sensing, body 1950 of solvent container 1920 may include electrically conductive regions 1952 that are coupled to a ground plane of system 1000 (e.g., via the base of second reagent container-carrier 1600). In some embodiments, receptacles 1940 may be uncovered (i.e., not be covered by a frangible cover or a lid) and aspirator probe 415 or pipettor tip 584 (affixed to mounting end 425 of pipettor 410) may enter the receptacles to extract fluid without having to penetrate through a cover. However, it is also contemplated that, in some embodiments, receptacles 1940 may be covered with a pipettor-penetrable cover and/or a lid, and aspirator probe 415 or pipettor tip 584 affixed to mounting end 425 of pipettor 410 may enter receptacle 1940 by piercing through the cover.

In the discussion above, both the IVD and LDT solvent containers 1620 and 1920 are described as being retained by the same support of system 1000. That is, IVD solvent containers 1620 with the reconstitution buffer for the IVD assays, and LDT solvent containers 1920 with the reconstitution fluids 1970A and 1970B for the LDTs, are both supported on a single second reagent container-carrier 1600 located in reagent container compartment 500 of second module 400. However, this is not a requirement. In some embodiments, solvent containers 1620 may be provided on one reagent container-carrier and solvent containers 1920 may be provided on another reagent container-carrier. These two container carriers may have the same (or different) configuration as second reagent container-carrier 1600. Positioning the IVD and LDT solvents on different container carriers may allow system 1000 to support a greater number of (and/or a greater volume of) solvents and/or solvent containers of differing shapes and/or sizes. In some embodiments, second reagent container-carrier 1600 supporting multiple (e.g., four) IVD solvent containers 1620 (with a reconstitution buffer for IVD assays) may be provided in reagent container compartment 500 of second module 400, and one or more LDT solvent containers 1920 (with a reconstitution fluid for LDTs) may be provided to a different reagent compartment of module 400 (in some embodiments, supported in a different container-carrier). Providing the IVD and LDT solvents in different reagent compartments also may enable the solutions to be maintained at different ambient conditions (e.g., temperature, humidity, etc.). For example, in some embodiments, LDT solvent containers 1920 with the solvent for LDTs may be provided in a chilled (or heated) reagent compartment of second module 400, while containers 1620 with the reconstitution buffer for IVD assays may remain at ambient temperature (or at a different temperature), or vice versa.

### Reagent Packs

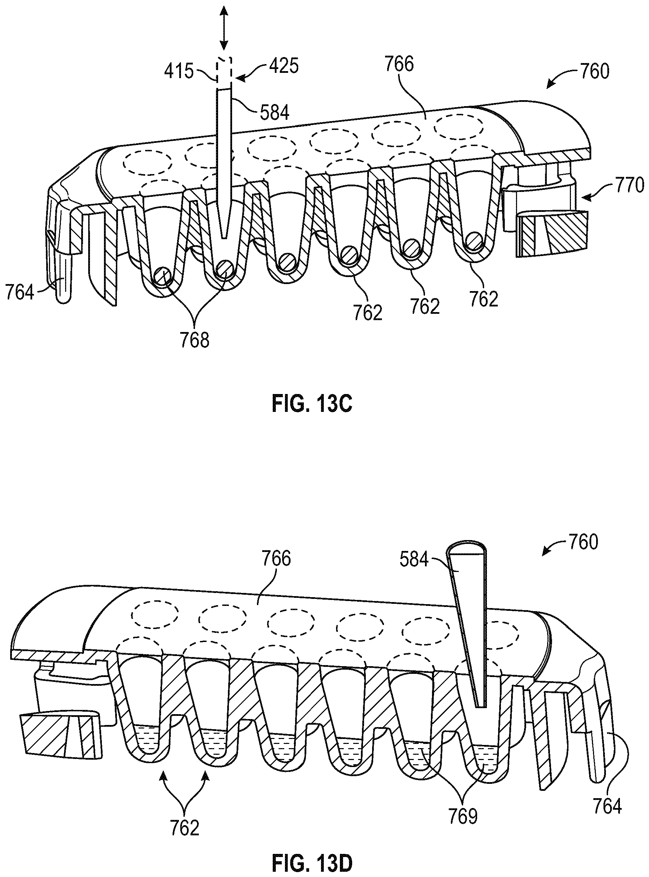

Although not a requirement, in some embodiments, amplification reagents and other reagents may be provided in second module 400 in a reagent pack. As described in more detail below, reagent pack may include a cartridge with wells within which the reagent is provided. FIGs. 13A-13D illustrate different views of an exemplary reagent pack 760 that may be used in system 1000. FIGs. 13A and 13B illustrate top and bottom views of an exemplary reagent pack 760, and FIGS. 13C and 13D illustrate cross-sectional views of an exemplary reagent pack 760 to show the contents of its wells 762. In the discussion below, reference will be made to FIGs. 13A-13D. Reagent pack 760 may include a plurality of mixing wells 762, each of which contains a reagent 768. In some embodiments, reagent 768 is a unit-dose reagent. Although, in general, reagent 768 may be in any state (solid, liquid, etc.), in some embodiments, reagent 768 may be a non-liquid reagent. In some preferred embodiments, reagent 768 may be a solid or a dried reagent (such as a lyophilizate). In some embodiments, reagent pack 760 includes twelve foil-covered mixing wells 762 that each contains a dried, unit-dose reagent 768 (see FIG. 13C). An exemplary unit-dose reagent that may be provided in reagent pack 760 is described in International Published Application No. WO 2017/136782. Reagent pack 760 may include a bar code (or other machine-readable indicator) that identifies the contents of the pack (e.g., type of reagent 768, etc.). The unit-dose reagent 768 in each mixing well 762 may be configured to perform an amplification reaction corresponding to an IVD assay or an LDT. Typically, reagents 768 configured for IVD assays are assay-specific reagents, while reagents 768 configured for LDTs are not assay-specific and may include, amongst other possible constituents, a polymerase(s), nucleoside triphosphates, and magnesium chloride. In some embodiments, each reagent 768 is held at the bottom of the associated mixing well 762 with an electrostatic charge imparted to reagent 768 and/or mixing well 762. In some embodiments, each reagent 768 is maintained at or near the bottom of the associated mixing well 762 with one or more physical features present in mixing well 762, for example, those described in U.S. Patent No. 9,162,228.

In some embodiments, mixing wells 762 are covered by a pierceable foil 766 adhered to the top of reagent pack 760. During use, as aspirator probe 415 or pipette tip 584 affixed to mounting end 425 of a pipettor 410 (see FIGs. 14B-14C) carrying the previously described solvent (e.g., from containers 1620, 1920, etc.) may pierce foil 766 and dispense the solvent into mixing well 762 to reconstitute reagent 768 and form a liquid reagent 769 (see FIG. 13D). Reconstitution refers to the act of returning a solid (e.g., dried or lyophilized) reagent 768 to a liquid form. Pipettor 410 may then aspirate the reconstituted liquid reagent 769 from mixing well 762. As explained previously, reagents 768 configured for IVD assays may include constituents such as, for example, amplification oligomers, probes, while reagents 768 configured for LDTs may not include such constituents (because the solvent used for LDTs may include these constituents). In some embodiments, reagents 768 for IVD assays and/or reagents 768 for the LDTs may include one or more of a polymerase and nucleoside triphosphates. In some embodiments, reagents 768 for IVD assays may include at least one forward amplification oligomer and at least one reverse amplification oligomer. In some embodiments, reagents 768 used for IVD assays may include a probe for performing a real-time amplification reaction. Exemplary probes for real-time amplification reactions are described in "Holland, P.M., et al., "Detection of specific polymerase chain reaction product by utilizing the 5' ----3' exonuclease activity of Thermus aquaticus DNA polymerse," PNAS, 88(16):7276-7280 (1991)." Other exemplary probes for performing real-time amplification reactions are disclosed in U.S. Patent Nos. 6,361,945 and 5,925,517. In some embodiments, reagents 768 for IVD assays and reagents 768 for LDTs may be provided in different reagent packs 760. However, this is not a requirement, and in some embodiments reagents 768 for IVD assays and reagents 768 for LDTs may be provided in different wells 762 of a same reagent pack 760.

In the illustrated embodiment in FIGs. 13A-13D, reagent pack 760 includes twelve mixing wells 762 in a 2 × 6 pattern. But in some embodiments, reagent pack 760 may include more or fewer than twelve mixing wells in any suitable pattern (linear pattern, square grid, circular pattern, etc.). Each mixing well 762 of a single reagent pack 760 may hold the same reagent, or each well 762 may hold a different reagent, or some wells 762 may hold the same reagent and some may hold different reagents. In some embodiments, unit-dose reagents 768 used to perform IVD assays include the components required for performing a nucleic acid amplification reaction in accordance with a particular assay. These components may include a polymerase, nucleoside triphosphates, or any other suitable component(s). Such reagents may be specific for one target nucleic acid or a plurality of different target nucleic acids. Unit-dose reagents 768 configured for LDTs may not include some or all of the above described components. Instead, in some embodiments, these missing components may be included in the reconstitution fluid used to reconstitute that reagent 768.

In some embodiments, reagent pack 760 further includes a manipulating structure 764 (e.g., in the shape of a hook) configured to be engageable by a corresponding structure of receptacle distribution system 200 (e.g., a correspondingly shaped hook of receptacle distributor 312 described later). Reagent pack 760 may be configured to be stored in compartment 702 of second module 400 and, in some embodiments, to be moved within second module 400 by distributor 312, and inserted and removed from reagent pack changer 700 (see FIG. 5D). Reagent pack 760 may include a structure 770 configured to align the reagent pack within a reagent pack carrier. Exemplary reagent packs that may be used in system 1000 are described in U.S. Patent No. 9,162,228. It should be noted that, although a dried (e.g., lyophilized) reagent is described above, this is not a requirement. That is, in general, as would be recognized by a person of ordinary skill in the art, reagents may also be provided in other forms (e.g., gel, etc.).

### Fluid Transfer and Handling System

Second module 400 includes a fluid transfer and handling system, which includes robotic pipettor 410 (see FIG. 1B). FIG. 14A illustrates an exemplary fluid transfer and handling system 402 of second module 400. Fluid transfer and handling system 402 may be configured to transfer (e.g., dispense and/or aspirate) fluids between different receptacles (containers, wells, vials, etc.) of second module 400. As illustrated in FIG. 14A, system 402 may include a front arm 408 that comprises robotic pipettor 410 and a back arm 416 that includes a vial transfer arm 418. The vial transfer arm 418 may be, for example, a pick-and-place mechanism having no pipetting capabilities, or it may be another pipettor (e.g., similar to pipettor 410). In the illustrated embodiment, fluid transfer and handling system 402 includes a gantry assembly with multiple tracks 404, 406, 412, 420 oriented in orthogonal directions (e.g., transverse, longitudinal, etc.). Pipettor 410 and vial transfer arm 418 may be driven back and forth in the transverse and longitudinal directions along tracks 404, 406, 412, 420, and in the vertical direction using motors coupled to these components.

Pipettor 410 is configured to aspirate and dispense fluid. As can be seen in FIG. 14A, pipettor 410 includes an aspirator probe 415 at its bottom end. As previously described with reference to FIGs. 7C, 10C, 11B, 13C, etc., aspirator probe 415 may be inserted (in some cases, by piercing through a pipettor-pierceable cover) into a receptacle and used to aspirate fluid from (and/or discharge fluid into) the receptacle. The bottom end of aspirator probe 415 forms a mounting end 425 in some embodiments that may be inserted into the receptacle. FIGs. 14B and 14C illustrate enlarged views of a bottom portion of pipettor 410 in an exemplary embodiment. In the discussion below, reference will be made to FIGs. 14A-14C. In some embodiments, aspirator probe 415 may be directly inserted into a receptacle to aspirate a fluid therefrom (or discharge a fluid thereinto). In some embodiments, to reduce cross-contamination, a disposable pipette tip 584 may be affixed to mounting end 425 of aspirator probe 415 before pipettor 410 is used to aspirate a fluid from a receptacle (and/or discharge a fluid into a receptacle). As illustrated in FIG. 1B, second module 400 includes tip compartments 580 with trays 582 (see FIG. 5A) of disposable pipette tips 584 that may be accessed by pipettor 410. In some embodiments, pipette tip 584 may be affixed to mounting end 425 of aspirator probe 415 by a frictional fit. That is, in some embodiments, an outer cylindrical surface of aspirator probe 415 may frictionally engage with an inner cylindrical surface of a pipette tip 584 to retain pipette tip 584 on aspirator probe 415. As described previously, pipettor 410 may be configured to detect the level of fluids in receptacles (e.g., containers 1620, 1820, 1920) by capacitive fluid level testing. Pipette tips 584 may be made of a conductive material (e.g., carbon-based material) to enable capacitive fluid level testing by pipettor 410.

In some embodiments, pipettor 410 may have an ejection mechanism that enables a pipette tip 584 that is coupled (or affixed) to mounting end 425 to be separated therefrom. In the embodiment illustrated in FIGs. 14B and 14C, the ejection mechanism includes a hollow sleeve 413 slidably disposed around aspirator probe 415 and a mounting member 411 operatively coupled to sleeve 413 by a linkage assembly. Sleeve 413 may be mounted on aspirator probe 415 such that mounting end 425 of aspirator probe 415 is exposed below sleeve 413. Pipette tip 584 may be affixed to aspirator probe 415 on the portion of mounting end 425 exposed below sleeve 413. FIG. 14B illustrates a view of sleeve 413 with a pipette tip 584 attached thereto. Mounting member 411 includes an actuator arm 414 pivotably coupled thereto. Actuator arm 414 is coupled to sleeve 413 by a linkage assembly such that when the free end of actuator arm 414 is forced towards mounting member 411, sleeve 413 slides downward on aspirator probe 415 (see FIG. 14C), thereby ejecting pipette tip 584 from mounting end 425 of aspirator probe 415. That is, when actuator arm 414 is actuated (moved towards mounting member 411), sleeve 413 slides down aspirator probe 415 and pushes pipette tip 584 off aspirator probe 415. During use, after a pipette tip 584 has aspirated and dispensed a fluid, it may be separated from (or ejected from) pipettor 410 and discarded. Pipettor 410 may also include a sensor configured to detect the presence (or absence) of a pipette tip 584 affixed thereon, and a pump to aspirate and dispense fluid.

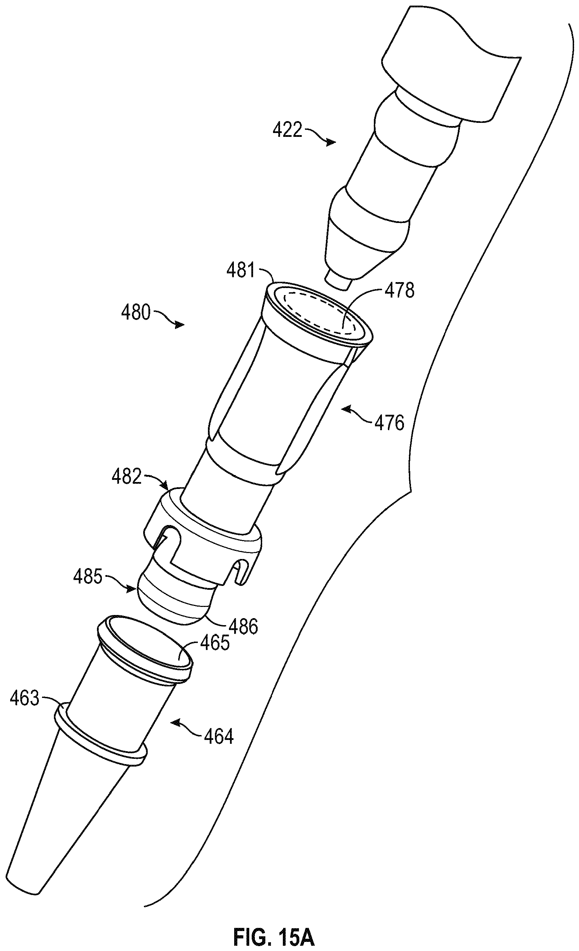

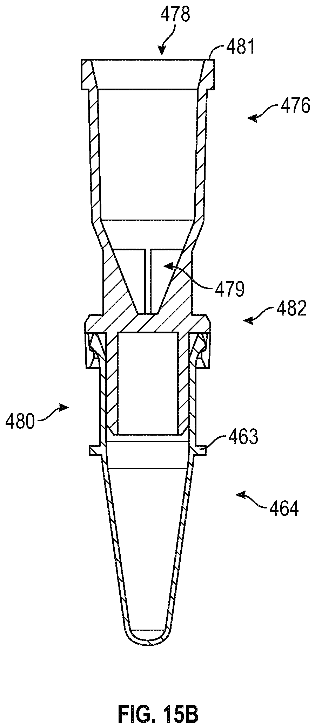

Aspirator probe 415 of pipettor 410 may also configured to engage with receptacles (e.g., cap/vial assembly 480) in a similar manner. For example, mounting end 425 of aspirator probe 415 may engage with the open top end 478 of a cap/vial assembly 480 (see FIGs. 15A, 15B) to couple pipettor 410 with cap/vial assembly 480. Once coupled, pipettor 410 may be used to move the coupled cap/vial assembly 480 from one location to another of module 400. A cap/vial assembly 480 coupled to pipettor 410 (i.e., probe 415 of pipettor 410) may be decoupled, separated, or ejected from pipettor 410 in a manner similar to that described above. For example, to eject a coupled cap/vial assembly 480 from pipettor 410, the actuator arm 414 may be pushed up towards mounting member 411. Actuating the actuator arm 414 causes sleeve 413 to slide down aspirator probe 415 and push against a rim surrounding top end 478 of cap 476 to separate cap/vial assembly 480 from pipettor 410.

As described in detail below, vial transfer arm 418 may be a "pick and place" device configured to pick up a cap/vial assembly 480 by inserting a mounting end 422 of vial transfer arm 418 into a cap that is coupled to a vial of the cap/vial assembly 480 (e.g., to cause a frictional fit between the cap and mounting end 422). In some embodiments, mounting end 422 of vial transfer arm 418 and mounting end 425 of pipettor 410 may have similar or identical configurations for engaging tips and caps. In some embodiments, vial transfer arm 418 may also include an eject mechanism similar to that described above with reference to pipettor 410.

### Cap/Vial Assembly

Cap/vial assembly includes a processing vial 464 that serves as a receptacle for containing a reaction fluid (for performing an amplification reaction or other process steps related to an assay) and a processing vial cap 476 that closes vial 464. Processing vials 464 can also be used to store reaction fluids, such as aliquots of eluate, for later use. FIGs. 15A and 15B illustrate a perspective view and a schematic cross-sectional view of an exemplary cap/vial assembly 480. Cap 476 and vial 464 may initially be held in a cap well and a vial well respectively of a cap/vial tray 460 (see FIG. 5A) of second module 400. Cap 476 has an open top end 478, a closed lower end 479, and an annular collar 482 that extends about cap 476. Open top end 478 of cap 476 is sized to receive mounting end 422 of vial transfer arm 418 in an interference fit. During use, fluids may be dispensed into processing vial 464 via a disposable pipette tip 584 of robotic pipettor 410. After dispensing a fluid(s) into processing vial 464, pipettor 410 may pick up cap 476 from tray 460 and place cap 476 on vial 464 in an automated manner to close vial 464. A lower portion of cap 476 beneath collar 482 defines a plug 485 with seal rings 486 that fits into open top end 465 of processing vial 464 in a friction fit. Cap 476 includes locking features (e.g., locking collar, etc.) that form an interference fit with a lip formed around the open top end 465 of vial 464.

Cap 476 and vial 464 are configured to lock together so that, once plug 485 of cap 476 is inserted into open top end 465 of processing vial 464, the cap and the vial are interlocked to form a closed cap/vial assembly 480 that inhibits or prevents evaporation of a fluid from vial 464. Mounting end 422 of vial transfer arm 418 may then be inserted into open top end 478 of cap 476 to pick up the closed cap/vial assembly 480 and transfer it from one location to another in second module 400. In some embodiments, pipettor 410 transfers the closed cap/vial assembly 480 to a desired location in second module 400. In general, both pipettor 410 and vial transfer arm 418 may be used to move cap/vial assembly 480 between components of system 1000. Typically, if pipettor 410 is engaged with (e.g., coupled to) a cap/vial assembly 480 (e.g., to move it to a location in system 1000), cap/vial assembly 480 must be ejected or otherwise disengaged from pipettor 410 before it can be engaged by vial transfer arm 418. In a preferred embodiment, pipettor 410 moves a closed cap/vial assembly 480 to centrifuge 588 (e.g., to remove air bubbles and concentrate the contents at the bottom of vial 464) and vial transfer arm 418 moves the cap/vial assembly 480 from centrifuge 588 to thermal cycler 432. As described previously, a coupled cap/vial assembly 480 can be separated or ejected from pipettor 410 (or mounting end 422 of vial transfer arm by an eject mechanism that engages a rim 481 surrounding top end 478 of cap 476 to eject cap/vial assembly 480 from pipettor 410 (or mounting end 422).

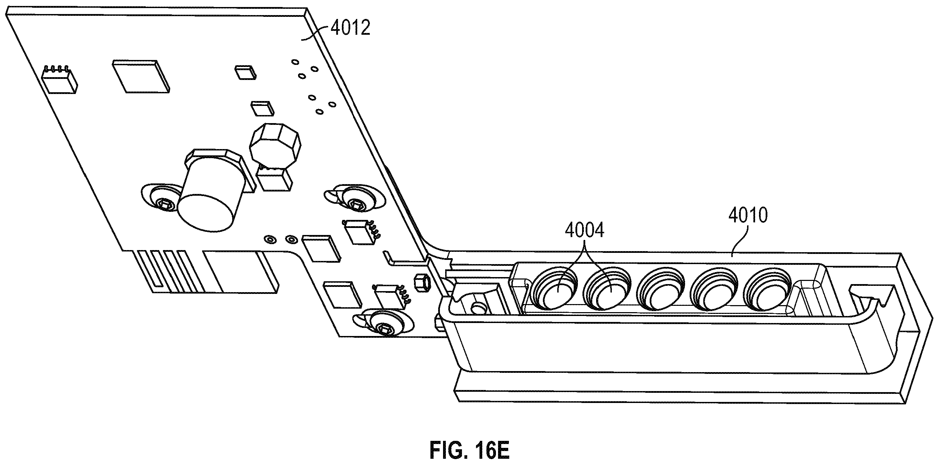

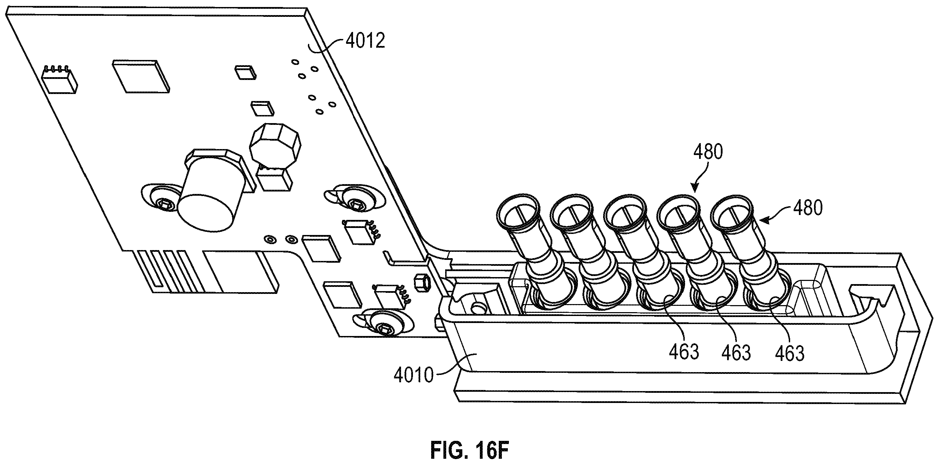

It should be noted that two different devices (e.g., pipettor 410 and vial transfer arm 418) to move a cap/vial assembly 480 between components is not a requirement. In some embodiments, the same device (e.g., a vial transfer arm or pipettor) may move cap/vial assembly 480 between components. As will be described below, in thermal cycler 432, a closed cap/vial assembly 480 will be placed with its vial 464 inserted into a receptacle well 4004 of a receptacle holder 4010 of thermal cycler 432 (see FIGs. 16E and 16F). Vial 464 includes an annular ring 463 (extending around its body) that rests on top of receptacle well 4004, and an external surface of the vial maintains close contact with the inner wall of well 4004 when cap/vial assembly 480 is placed on receptacle holder 4010. Exemplary caps and processing vials, and methods of moving a closed cap/vial assembly are described in U.S. Patent Nos. 9,732,374. Exemplary caps and processing vials are also described in U.S. Patent No. 9,162,228. And, exemplary cap/vial trays are described in U.S. Patent Publication No. US 2017/0297027 A1.

### Thermal Cycler

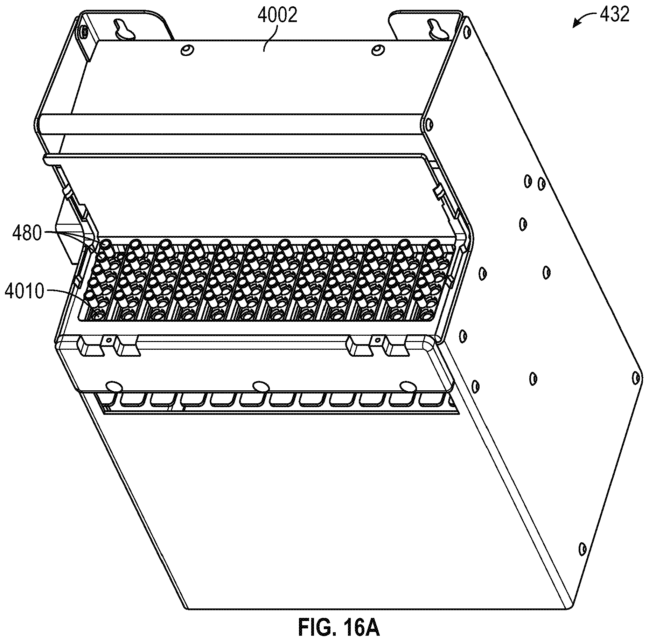

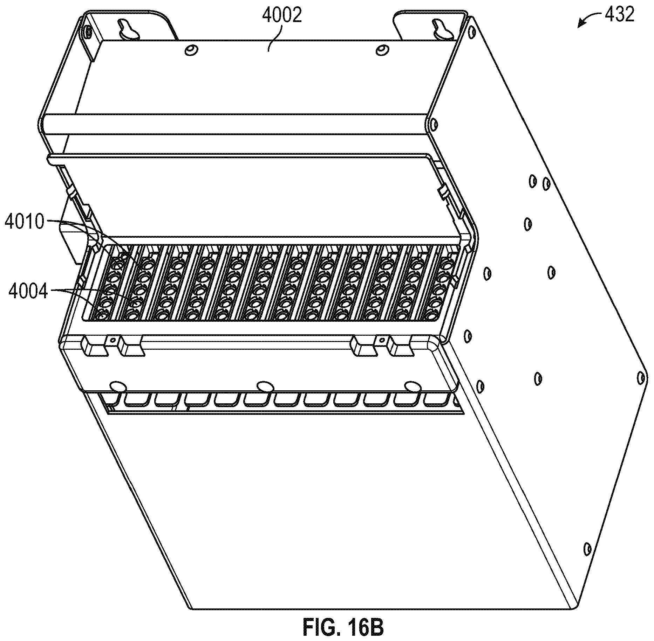

Second module 400 includes thermal cycler 432 (see FIGs. 5A-5D). Thermal cycler 432 is typically used in nucleic acid amplification reactions. The conditions of a nucleic acid amplification reaction may be substantially isothermal, or they may require periodic temperature changes, as with PCR thermal cycling. Thermal cycler 432 may be used to heat and maintain a nucleic acid containing sample to a constant or ambient temperature or it may be used to fluctuate the temperature thereof. FIGs. 16A-16I illustrate different views of an exemplary thermal cycler 432 that may be used in system 1000. In the discussion below, reference will be made to FIGs. 16A-16I. Thermal cycler 432 includes multiple receptacle holders 4010 supported on the upper end of an upright frame 4018 (see FIG. 16D). Each receptacle holder 4010 may be configured to support multiple receptacles (e.g., a cap/vial assembly 480 of FIG. 15B) containing, for example, a reaction mixture. FIG. 16 A illustrates a perspective view of thermal cycler 432 with cap/vial assemblies 480 positioned in receptacle holders 4010, and FIG. 16B is an illustration of thermal cycler 432 without cap/vial assemblies 480. Receptacle holder 4010 includes multiple receptacle wells 4004 with each well 4004 configured to receive a receptacle, such as, a cap/vial assembly 480 therein (i.e., vial 464 of cap/vial assembly 480). Receptacle holders 4010 are positioned within a housing 4002 (e.g., made of metal, plastic, etc.) of thermal cycler 432.

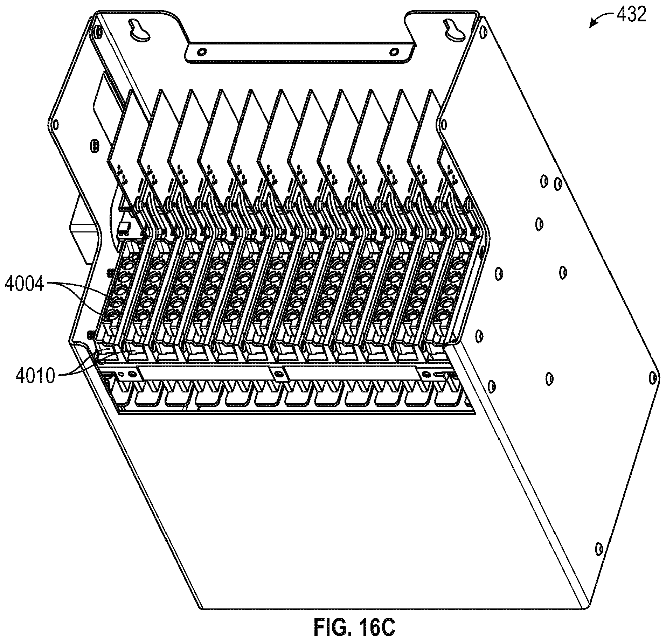

FIGs. 16C and 16D illustrate perspective views of thermal cycler 432 with portions of housing 4002 removed to show the structure within. In general, thermal cycler 432 may include any number of receptacle holders 4010, and each receptacle holder 4010 may include any number of receptacle wells 4004. Typically, the multiple receptacle holders 4010 (and the multiple wells 4004) are disposed in alignment with one another to facilitate the automated processing steps involved in nucleic acid amplification assays. In some embodiments, as illustrated in FIGs. 16C-16D, thermal cycler 432 may include twelve receptacle holders 4010 with each receptacle holder 4010 including five wells 4004. In such embodiments, thermal cycler 432 can support a maximum of 60 cap/vial assemblies 480 (or other receptacles) with each receptacle holder 4010 supporting five cap/vial assemblies 480. Each receptacle well 4004 of receptacle holder 4010 may be configured to maximize thermal contact between the surface of the receptacle well 4004 and the surface of the receptacle received therein. For example, in some embodiments, each receptacle well 4004 may have internal dimensions substantially corresponding to the external dimensions of a receptacle (e.g., via, 464) received therein, such that vial 464 fits snugly within well 4004.

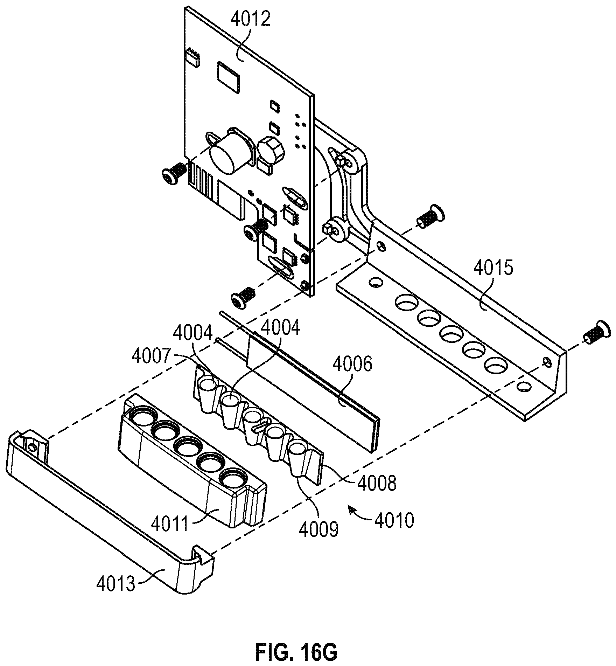

FIG. 16E illustrates a receptacle holder 4010 separated from thermal cycler 432, and FIG. 16F illustrates the receptacle holder 4010 (of FIG. 16E) with vials 464 of cap/vial assemblies 480 positioned in its wells 4004. When vial 464 of a cap/vial assembly 480 is inserted into a well 4004 of receptacle holder 4010, annular ring 463 of vial 464 rests on top of the receptacle well 4004. When in this configuration, external surface of vial 464 is in close thermal contact with the inner wall of well 4004. FIG. 16G illustrates an exploded view of receptacle holder 4010 showing its constituent parts. As best seen in FIG. 16G, each receptacle holder 4010 includes a receptacle supporting member 4008 that includes the multiple receptacle wells 4004 of receptacle holder 4010. Receptacle wells 4004 may be through-holes that extend from a top surface 4007 to a bottom surface 4009 of receptacle supporting member 4008. In general, the size or diameter of the opening that forms well 4004 at top surface 4007 may be larger than the size of the opening of well 4004 at bottom surface 4009. The shape of receptacle well 4004 between top and bottom surfaces 4007 and 4009 may be configured to maximize contact between the surface of vial 464 placed in well 4004. Receptacle supporting member 4008 may be formed of any thermally conductive material and may be independently thermally coupled to a thermal element 4006. Any type of suitable heating and/or cooling device (e.g., resistance heating elements, Peltier devices, etc.) known in the art may be used as thermal element 4006. In some embodiments, as illustrated in FIG. 16G, thermal element 4006 may be placed in contact with a length of receptacle supporting member 4008 such that the receptacle wells 4004 formed in member 4008 are substantially equidistant from thermal element 4006. Thus, thermal element 4006 may heat and cool each receptacle supported in receptacle holder 4010 to a substantially equal temperature. A block 4011, made of a thermally insulating material, covers receptacle supporting member 4008 and serves to reduce heat loss from member 4008 (and cap/vial assemblies 408 in its wells 4004) during thermal cycling. Block 4011 may be made of any thermally insulating material to reduce the amount of heat transferred to block 4011 from receptacle supporting member 4008. In some embodiments, block 4011 may be made of Ultem or another thermoplastic material.

As illustrated in FIG. 16G, a spring element 4013 attaches block 4011, receptacle supporting member 4008, and thermal element 4006 to a heat sink interface 4015. Spring element 4013 may be made of any suitable material. In some embodiments, spring element 4013 may be made of a stainless steel material. Spring element 4013 may be configured to bend and conform to the outer shape of block 4011, and press the components tightly together, when it attaches these components to heat sink interface 4015. Thus, spring element 4013 serves to maximize the thermal contact between thermal element 4006 and receptacle supporting member 4008. Heat sink interface 4015 thermally couples receptacle supporting member 4008 to a heat sink 4017 (see FIG. 16D). Heat sink interface 4015 and heat sink 4017 may be made of any thermally conductive material. In some embodiments, each receptacle supporting member 4008 is provided in thermal communication with a single heat sink 4017. Each heat sink 4017 may further include a plurality of through-holes (not visible) positioned in direct alignment with the through-holes (i.e., receptacle wells 4004) of receptacle supporting member 4008. Optical fibers 4016 and/or associated components may extend through these through-holes to provide optical communication between each receptacle well 4004 and an emission signal detector (signal detector assemblies 4020), as discussed below.

Thermal element 4006 of each receptacle holder 4010 is electrically connected to a controllable power source 4012 to independently control (i.e., heat and cool) thermal element 4006 such that cap/vial assemblies 480 supported by each receptacle holder 4010 can be independently heated and cooled (i.e., independently thermally cycled). That is, the five cap/vial assemblies 480 supported by each receptacle holder 4010 may be (if desired) subjected to a temperature cycle different from cap/vial assemblies 480 supported by another receptacle holder 4010.

As explained above, thermal cycler 432 is configured such that each receptacle holder 4010 forms an independently controlled thermal zone. Thus, thermal cycler 432 includes twelve independently controlled thermal zones, with each thermal zone configured to support five individual receptacles. However, this configuration is only exemplary, and in general, thermal cycler 432 may include any number of independently controlled thermal zones, and each thermal zone may be configured to support any number of receptacles. For example, in some embodiments, some of the adjacent receptacle holders 4010 of thermal cycler 432 may be thermally coupled together to form a common temperature zone. The selection of thermal cycler 432 depends on the nature of the amplification reaction intended to be run on second module 400. In some embodiments, the different thermal zones of thermal cycler 432 may be adapted to run separate amplification reactions (e.g., simultaneously) under different conditions. For example, one or more thermal zones of thermal cycler 432 may run one or more amplification reactions associated with IVD assays, while other thermal zones are running one or more amplification reactions associated with LDTs.

An exemplary thermal cycler 432 that may be used in system 1000 and exemplary methods of thermal cycling are described in U.S. Patent Application Publication No. 2014/0038192. It should be noted that, in some embodiments of system 1000, a heating device that does not include thermal cycling capabilities may be used to heat cap/vial assembly 480 (e.g., if the amplification reaction is to be performed under isothermal conditions). Therefore, any reference to thermal cycler in this application also covers a heating device for maintaining an essentially constant temperature.

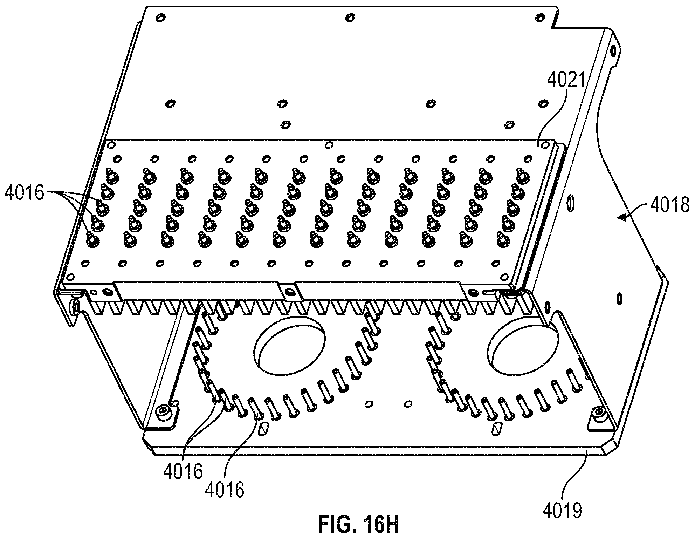

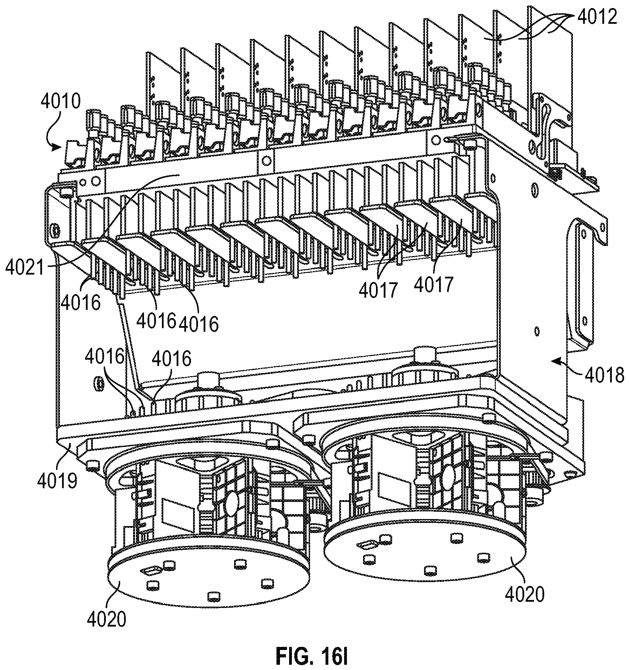

An optical fiber 4016 (see FIG. 16D) may be in optical communication with each receptacle well 4004 of thermal cycler 432 through the opening of well 4004 on bottom surface 4009 of receptacle supporting member 4008. Although not a requirement, in some embodiments, optical fiber 4016 (or an associated component, such as, for example, a fixed or moveable ferrule coupled to optical fiber 4016) may extend into well 4004 through bottom surface 4009. When a receptacle (e.g., cap/vial assembly 480) is positioned in receptacle well 4004, optical fiber 4016 may provide optical communication between the receptacle and one or more signal detector assemblies 4020 (see FIGs. 16D, 16I) coupled to a lower end of frame 4018. In some embodiments, a separate optical fiber 4016 may provide optical communication between each receptacle well 4004 of thermal cycler 432 and a signal detector assembly 4020. It should be noted that, a portion of optical fibers 4016 between receptacle holders 4010 and signal detector assembly 4020 is not shown in FIGs. 16D, 16H, and 16I for clarity.

With reference to FIG. 16H, frame 2018 includes an interface plate 4021 at its upper end and a base plate 4019 at its lower end. Interface plate 4021 includes fiber-positioning holes in a rectangular pattern and base plate 4019 includes fiber-positioning holes in a circular pattern. The fiber-positioning holes in interface plate 4021 may be arranged in the same pattern as receptacle wells 4004 (of receptacle holders 4010) are arranged in thermal cycler 432. Receptacle holders 4010 are coupled to the top surface of the interface plate, and as illustrated in FIG. 16I, signal detector assemblies 4020 are coupled to the back side of base plate 4019. In some embodiments, as illustrated in FIG. 16I, two signal detector assemblies 4020 may be used. Optical fibers 4016 operatively coupled to half of the receptacle wells 4004 of thermal cycler 432 may be coupled to one signal detector assembly 4020 and optical fibers 4016 coupled to the other half of receptacle wells 4004 may be coupled to the other signal detector assembly 4020. Optical fibers 4016 extend between signal detector assemblies 4020 and receptacle holders 4010 through the fiber-positioning holes in base plate 4019 and interface plate 4021. The shape and structure of frame 4018 may be suitable to arrange the plurality of optical fibers 4016 that extend between signal detector assemblies 4020 and receptacle holders 4010 in an optimal optical pathway.

### Signal Detector

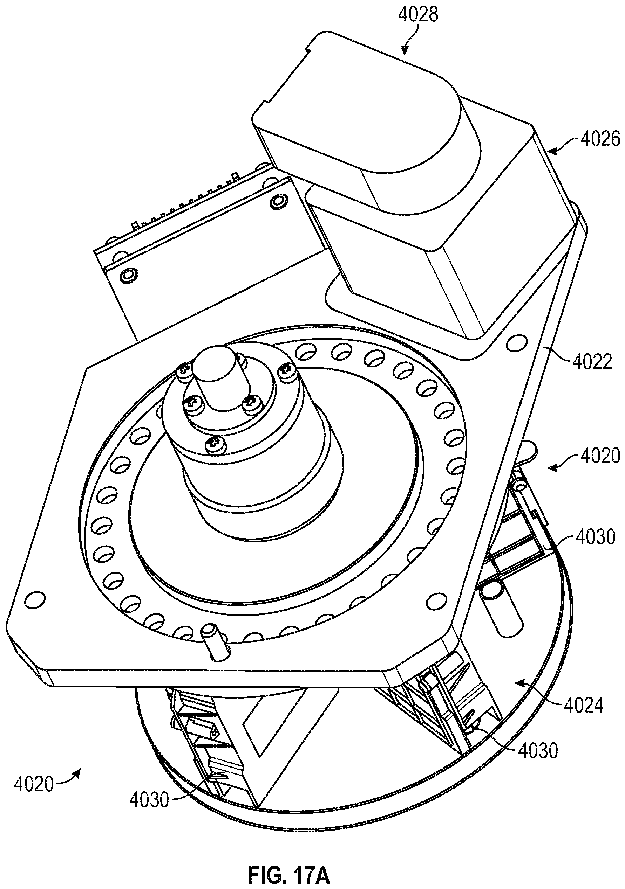

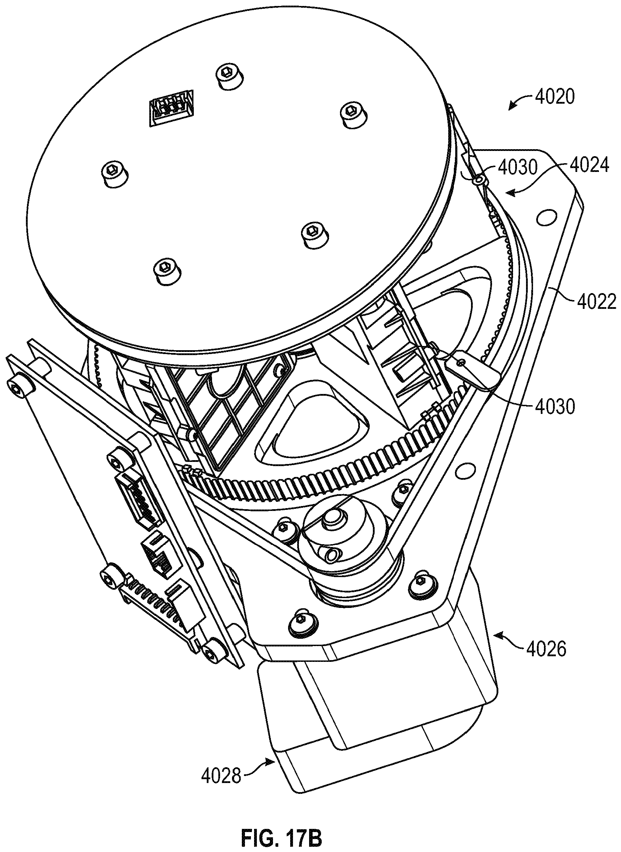

FIGs. 17A and 17B illustrate perspective top and bottom views of a signal detector assembly 4020 that may be used with thermal cycler 432. Signal detector assembly 4020 includes a base plate 4022 configured to be attached to the base plate 4019 of frame 4018 (see FIG. 16I). Base plate 4022 includes a plurality of fiber-positioning holes arranged in a configuration corresponding to the spatial arrangement of the fiber-positioning holes in base plate 4019 of frame 4018. Signal detector assembly 4020 further includes a detector carrier 4024, which in the illustrated embodiment comprises a carousel that supports a plurality of signal detectors 4030 in a circular pattern. In general, signal detector assembly 4020 is configured to rotate signal detectors 4030 to sequentially align each signal detector 4030 with each optical fiber 4016 to detect a signal transmitted through the fiber. In general, signal detector assembly 4020 may include any number (3, 4, 6, 8, etc.) of signal detectors 4020. In the illustrated embodiment, signal detector assembly 4020 includes five individual signal detectors 4030. Each signal detector 4030 may be configured to excite and detect a different emission signal or an emission signal having different characteristics (e.g., wavelength), and thus, in the context of the present disclosure, each signal detector 4030 constitutes a different channel for detecting a different signal.

Detector carrier 4024 is configured so as to be rotatable with respect to the base plate 4022. A detector drive system 4026 includes a drive motor 4028 configured to rotate detector carrier 4024 via a belt drive system (see FIG. 17B). As would be appreciated by persons of ordinary skill in the art, other mechanisms and arrangements (e.g., gear mechanism, etc.) may be employed to rotate detector carrier 4024. Motor 4028 is preferably a stepper motor and may include a rotary encoder or other position feedback sensors. Signal detectors 4030 include, among other optical components (objective lens, etc.), an excitation source (e.g., an LED) and emission detector (e.g., photodiode). Detector carrier 4024 is rotatable with respect to the base plate 4220 so that an objective lens associated with each signal detector 4030 can be selectively aligned with an optical fiber 4016 disposed in base plate 4019. Thus, in the illustrated embodiment, six optical fibers 4016 are optically aligned with a signal detector 4030 at any given time.

Signal detector 4030 may be fluorometer that is configured to generate an excitation signal of a particular predetermined wavelength. The generated excitation signal is directed to the contents of a receptacle (e.g., cap/vial assembly 480, see FIG. 16A) positioned in a receptacle well 4004 of a receptacle holder 4010 (see FIG. 16A), to determine if a probe or marker having a corresponding emission signal of a known wavelength is present in the contents of the receptacle. Each signal detector 4030 of signal detector assembly 4020 is configured to excite and detect an emission signal having a different wavelength to detect a different label associated with a different probe hybridized to a different target analyte. A label that is present in the receptacle, and is responsive to the excitation signal, will emit an emission signal (e.g., light). At least a portion of the emission signal (from the contents of the receptacle) enters the optical fiber 4016 (coupled to the receptacle well 4004 that the receptacle is positioned in) and passes back to signal detector 4030. Signal detector 4030 includes components (lens, filters, photodiode, etc.) that is configured to generate a voltage signal corresponding to the intensity of the emission light that impinges on signal detector 4030.

As detector carrier 4024 rotates, each signal detector 4030 is sequentially aligned with an optical fiber 4016 to interrogate (i.e., measure a signal from) an emission signal directed through optical fiber 4016. The detector carrier 4024 may pause momentarily at each optical fiber 4016 to permit signal detector 4030 to detect fluorescence of a specified wavelength emitted by the contents of a receptacle. Each optical fiber 4016 is interrogated once by each signal detector 4030 for every revolution of detector carrier 4024. Since signal detector assembly 4020 includes multiple signal detectors 4030 configured to detect different signals, each receptacle in receptacle holder 4010 is interrogated once for each different signal for every revolution of the detector carrier 4024. An exemplary signal detector that may be used in system 1000 is described in U.S. Patent No. 9,465,161.

### Centrifuge

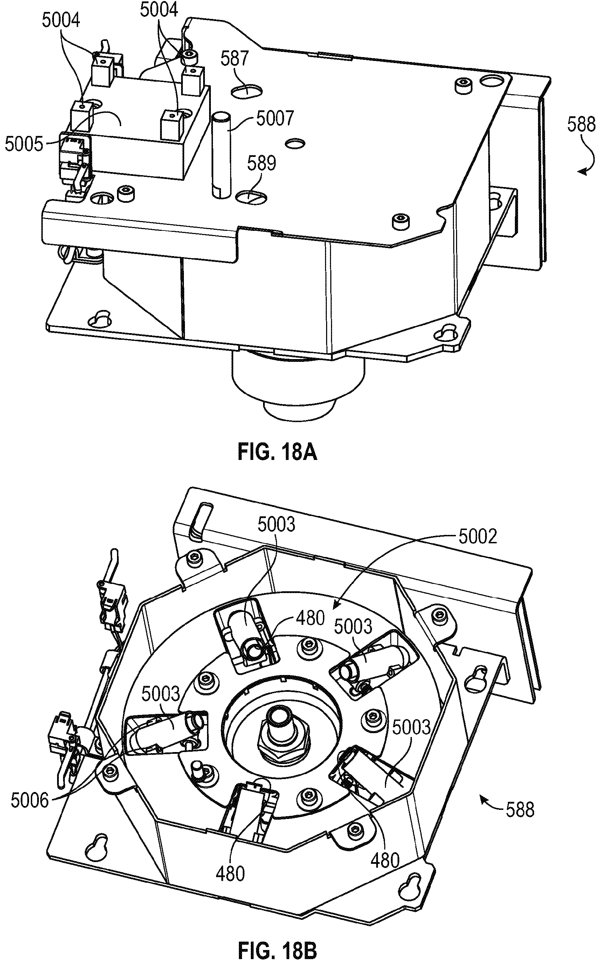

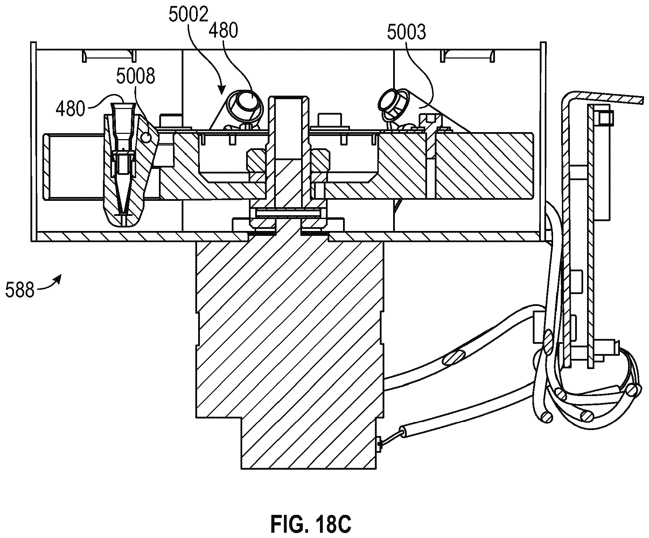

Second module 400 includes a centrifuge 588 located on amplification processing deck 430 (see FIGs. 1B and 5A-5C). FIGs. 18A, 18B, and 18C illustrate different views of a centrifuge 588 in an exemplary embodiment. Centrifuge 588 is configured to centrifuge one or more (up to five in one embodiment) cap/vial assemblies 480 at a time. In some embodiments, assemblies 480 may be centrifuged before an amplification reaction (e.g., to remove air bubbles from the contents of vial 464 and to cause the sample material to be concentrated primarily at the bottom of vial 464) to improve heat transfer and optical transmission quality. As seen in FIG. 18A, a top cover of centrifuge 588 includes first and second access ports 589, 587. During use, pipettor 410 of fluid transfer and handling system 402 (see FIG. 14A) places a cap/vial assembly 480 (see FIGs. 15A, 15B) into centrifuge 588 through first access port 589. As explained previously with reference to FIGs. 14B and 14C, pipettor 410 includes an actuator arm 414 that, when forced towards mounting member 411, enables a cap/vial assembly 480 coupled to pipettor 410 to be released therefrom. When a cap/vial assembly 480 engaged with pipettor 410 is inserted into centrifuge 588 through first access port 589, a strip bar 5007 of centrifuge 588 forces actuator arm 414 of pipettor 410 (see FIGs. 14B and 14C) towards mounting member 411. Forcing actuator arm 414 towards mounting member 411 pushes sleeve 413 (that is mounted on aspirator probe 415 of pipettor 410) in a downward direction towards mounting end 525 of aspirator probe 415 (see FIG. 14C). As sleeve 413 moves downwards, the bottom end of the sleeve pushes on rim 481 of cap/vial assembly and separates the cap/vial assembly 480 from pipettor 410. An example of a pipettor-based system for transferring cap/vial assemblies is described in U.S. Patent Application Publication No. 2016/0032358.

Centrifuge 588 includes multiple teach points 5004 that assist pipettor 410 in determining the positions of access ports 587, 589. In some embodiments, as illustrated in FIG. 18A, four teach points 5004 may be provided on a teach block 5005 located on a top cover of centrifuge 588. During system setup, these teach points 5004 may be utilized to "teach" pipettor 410 the locations of access ports 587, 589. In some embodiments, pipettor 410 may determine the locations of the access ports, by, for example, triangulation, based on the location of teach points 5004. It should be noted that, although FIG. 18A illustrates four teach points 5004, this is not a requirement. In some embodiments, centrifuge 588 may include a different number (e.g., 1, 2, 3, 5, etc.) of teach points 5004. Typically, multiple teach points (instead of a single teach point) are used so that pipettor 410 can reliably determine the positions of access ports 587, 589 even when centrifuge 588 is slightly misaligned (e.g., not level, etc. after assembly).

As seen in FIG. 18B, centrifuge 588 includes multiple buckets 5003 (five in the illustrated embodiment) arranged around a turntable 5002. Each bucket 5003 includes a pocket or an opening into which pipettor 410 places a cap/vial assembly 480 (as best seen in FIG. 18C). Buckets 5003 are rotatably coupled to turntable 5002 via a pin 5008 (see FIG. 18C), such that when turntable 5002 rotates, the resulting centrifugal force causes buckets 5002 (and cap/vial assemblies 480 positioned therein) to rotate about pin 5008. The centrifugal force acting on cap/vial assemblies 480 serve to retain them in buckets 5003 when turntable 5002 rotates. Stops 5006 positioned on either side of each bucket 5003 may prevent over-rotation of buckets 5003 when turntable 5002 rotates. In some embodiments, a stepper motor may rotate turntable 5002 to centrifuge cap/vial assemblies 480. The stepper motor also serves to move cap/vial assembles 480 from first access port 589 to second access port 587. Centrifuge 588 may also include encoders and/or other position indicators to track the movement of cap/assemblies 480 in centrifuge 588.

Although not a requirement, in some embodiments, centrifuge 588 may have a maximum revolution speed of about 3000 revolutions per minute. However, other revolution speeds are also contemplated based on, inter alia, the composition of the solution being centrifuged and the time period required for adequate centrifuging. After centrifuging is complete, vial transfer arm 418 (of fluid transfer and handling system 402) removes the centrifuged cap/vial assembly 480 through second access port 587 and places it in thermal cycler 432. A centrifuge 588 with separate first and second access ports 589, 587 allows pipettor 410 and vial transfer arm 418 to simultaneously load and unload cap/vial assemblies 480 from different locations of centrifuge 588 without colliding with each other.

### Multiple Receptacle Units

System 1000 includes one or more reaction receptacles (or test tubes) that serve as containers for performing one or more processes of the different types of assays. In general, the reaction receptacles may be any container suitable for holding a fluid (e.g., cuvette, beaker, well formed in a plate, test tube, pipette tip, etc.). These reaction receptacles may be configured as individual receptacles (e.g., test tubes) or may be configured as a device that comprises a plurality or receptacles connected together (referred to herein as multiple receptacle units (MRUs)). FIG. 19 illustrates a perspective view of an exemplary MRU 160 that may be used in system 1000. In the illustrated embodiment, MRU 160 comprises five individual receptacles 162. It should be noted that, in general, any number of receptacles 162 may be connected together to form an MRU 160. In the illustrated embodiment, each receptacle 162 is configured as a substantially cylindrical tube with an open top end and a closed bottom end, and multiple receptacles 162 are connected together by a connecting rib structure 164 that forms a shoulder extending longitudinally along either side of MRU 160. MRU 160 includes manipulating structure 166 that extends from one side, and a label-receiving structure 174 having a flat label-receiving surface 175 that extends from the opposite side. Label-receiving surface 175 is adapted to receive human and/or machine-readable labels (e.g., bar codes) to provide identifying and instructional information regarding MRU 160. Manipulating structure 166 is configured to be engaged by the receptacle hook of receptacle distribution system 200 (see FIG. 5D, described in more detail below), or another transport mechanism, for moving MRU 160 between different components of system 1000.

Fluids can be dispensed into or removed from receptacles 162 through their open top ends by means of a fluid transfer device, such as a pipettor 410 or another suitable mechanism (e.g., aspirator tubes 282 of magnetic wash stations 118, 120, see FIG. 2F). In some embodiments, as explained with reference to FIG. 2F, an aspirator tube 282 of magnetic wash station 120 (and/or 118) may aspirate fluid contained in receptacle 162. During operation of system 1000, a single aspirator tube 282 may be used to aspirate fluids from multiple individual receptacles 162. Accordingly, to reduce the likelihood of cross-contamination between these receptacles 162, when aspirating fluid from a receptacle 162, it is desirable to limit the amount of the aspirator tube 282 that comes into contact with the fluid or walls of any receptacle 162. Therefore, a contact-limiting element, in the form of a protective disposable tip, or tiplet 168, may be used to cover the end of aspirator tube 282 when it is used to aspirate fluid from a receptacle 162. Before the same aspirator tube 282 moves to another receptacle 162 to aspirate fluid, the used tiplet 168 is discarded and a fresh tiplet 168 coupled to the end of aspirator tube 282. In some embodiments, another tubular component (e.g., aspirator probe 415 with or without a pipette tip 584 coupled to its end) may be used to aspirate fluid from receptacle. In some embodiments, to reduce cross-contamination, the tip of aspirator probe 415 may be covered with a disposable cover (e.g., pipette tip 584) when it is used to aspirate fluid from receptacle. In some embodiments, the fluid transfer device may include multiple tubular elements (e.g., five tubular elements, one for each receptacle). In such embodiments, the fluid transfer device may not move between different receptacles 162. Instead, a different tubular element with a tiplet 168 may be used to aspirate fluid from each receptacle 162 of MRU 160. For example, magnetic wash station 120 (discussed previously with reference to FIG. 2F) includes five aspirator tubes 282 that may each be used to aspirate fluid from a different receptacle 162 of MRU 160 (with a tiplet 168 attached to each aspirator tube 282). In some embodiments, a tubular element with or without a tiplet 168 may also be used when dispensing fluid into a receptacle 162.

As illustrated in FIG. 19, in some embodiments, tiplet 168 comprises a tubular body with a radially extending peripheral flange. An axial bore extends through the length of tiplet 168. The diameter of the bore is sized to provide a frictional fit with the outer diameter of aspirator tube 282 to frictionally secure tiplet 168 onto the free end of aspirator tube 282 when it is forced into the bore of tiplet 168. An exemplary MRU 160 and an exemplary transport mechanism compatible with MRU 160 are described in U.S. Patent Nos. 6,086,827 and 6,335,166 respectively. An exemplary fluid transfer device or pipettor is also described in U.S. Patent No. 6,335,166.

### Receptacle Distribution System and Receptacle Distributor

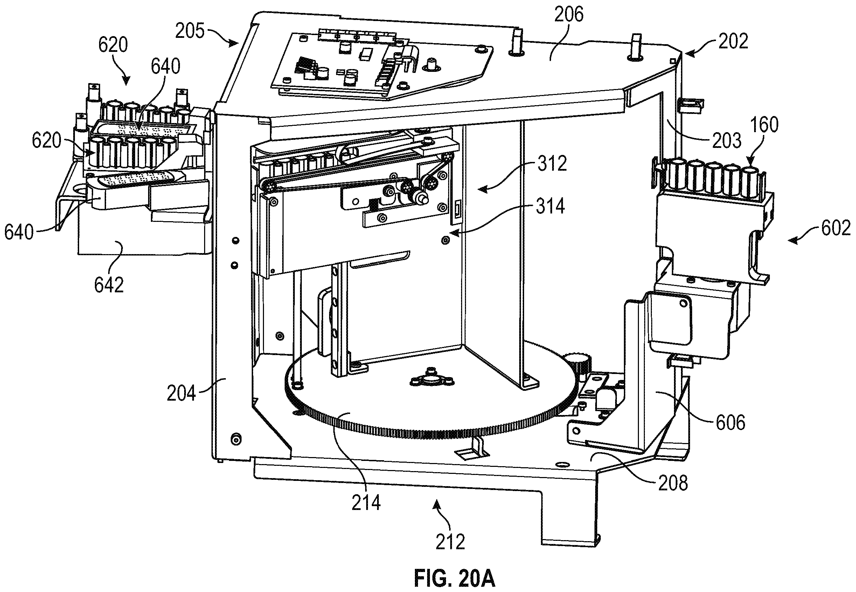

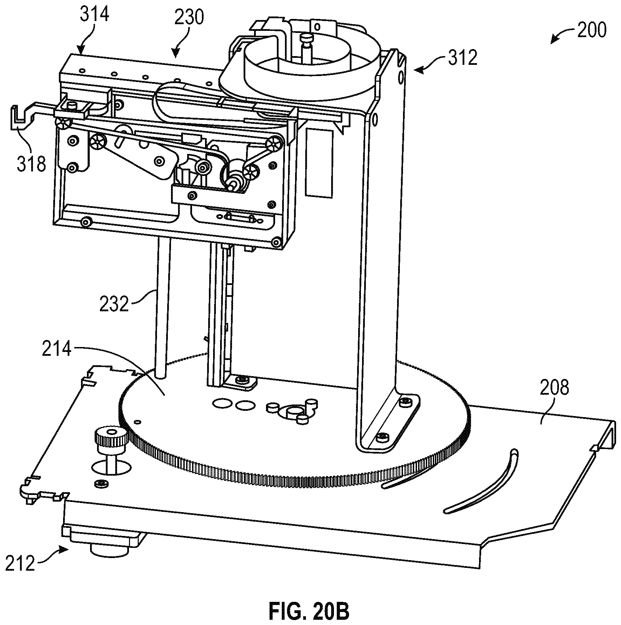

FIGs. 20A and 20B illustrate an exemplary receptacle distribution system 200 of system 1000 (see also FIG. 5D). In the embodiment of FIG. 20B, some components of system 200 have been removed to show some hidden features. In the description below, reference will be made to both FIGs. 20A and 20B. In the illustrated embodiment of FIG. 20A, receptacle distribution system 200 includes a frame 202 comprising multiple vertically oriented legs 203, 204, 205 extending between a bottom panel 208 and a top panel 206. A receptacle handoff station 602 is mounted on a handoff station bracket 606 attached to bottom panel 208 of frame 202 and will be discussed further below. Magnetic slots 620 and reagent pack loading stations 640 are supported on a bracket 642 attached to legs 204 and 205 of frame 202 and will be discussed further below. A receptacle distributor 312 is supported on frame 202. Receptacle distributor 312 is configured to transport MRUs 160 (and/or other receptacles) and reagent packs 760 between different locations of second module 400. Receptacle distributor 312 includes a distributor head 314 defining a partial enclosure for holding an MRU 160 and reagent pack 760, and a manipulating hook 318 configured to engage with manipulating structure 166 of MRU 160 and manipulating structure 764 of reagent pack 760. Receptacle distribution system 200 includes a rotary drive system 212 configured to move receptacle distributor 312 in a circular path. In the illustrated embodiment, the rotary drive system includes a turntable 214 upon which the receptacle distributor 312 is supported. Turntable 214 is mounted for rotation about its central axis on the bottom panel 208 of the frame 202. A motor (not visible) attached to the bottom panel 208 rotates turntable 214 and receptacle distributor 312. Rotary drive system 212 may also include a rotary encoder (or another position feedback device) that provides rotational position feedback to a control system of system 1000. Other methods for rotationally coupling receptacle distributor 312 to frame 202 (e.g., using belts, pulleys, gear trains, etc.) are also contemplated. Receptacle distribution system 200 also includes an elevation system 230 configured to move receptacle distributor 312 in a vertical direction to transport MRUs 160 and reagent packs 760 between the different components and decks of second module 400. In an exemplary embodiment, elevation system 230 includes a threaded rod 232 extending upwardly from the turntable 214 through a motor and an internal thread drive (not shown) mounted to the distributor head 314. Rotation of the internal thread drive by the motor causes the distributor head 314 to translate up or down the threaded rod 232. It should be noted that other elevation systems (e.g., rack and pinion, belt drive system, etc.) are also contemplated and are within the scope of this disclosure.

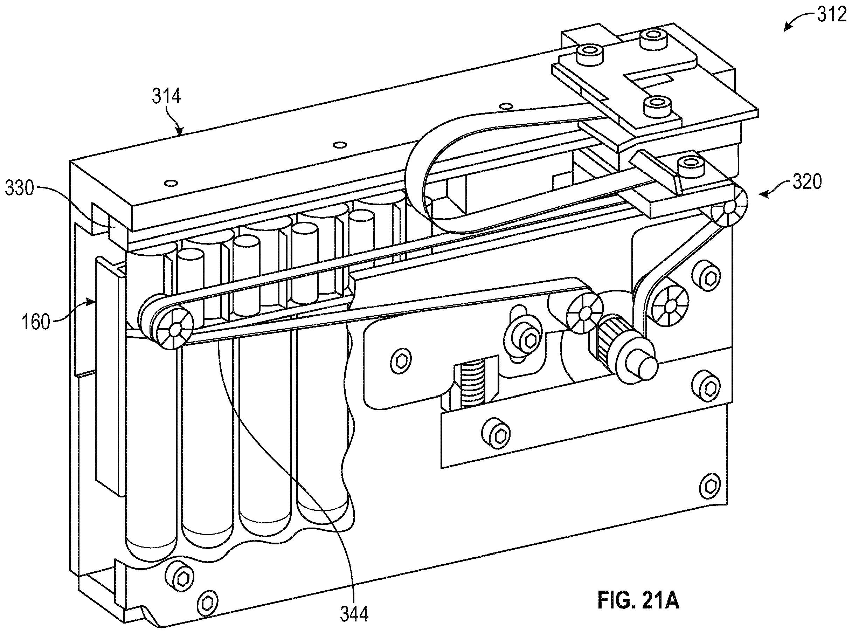

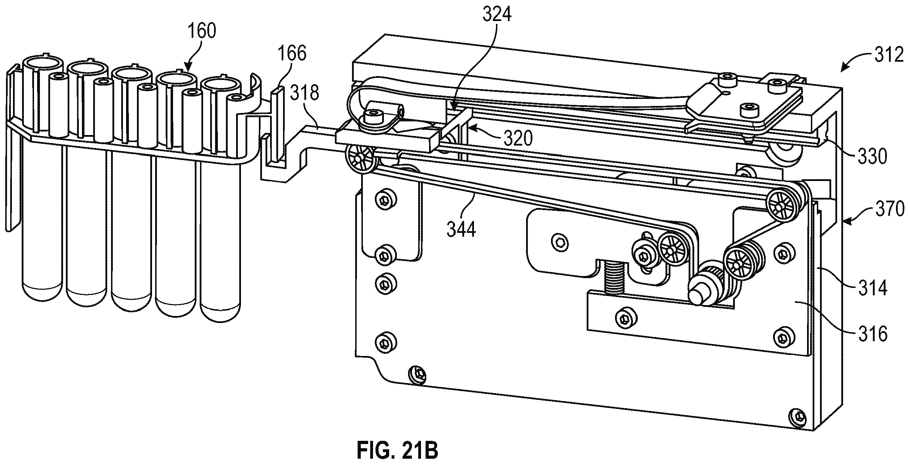

FIGs. 21A and 21B illustrate perspective views of an exemplary receptacle distributor 312 engaged with an MRU 160. A hook actuator system 316 linearly translates manipulating hook 318 with respect to distributor head 314 between an extended position (see FIG. 21B) and a retracted position (see FIG. 21A). Hook actuator system 316 includes a hook carriage 320 to which manipulating hook 318 is attached, and a drive belt 344 attached to hook carriage 320. Hook carriage 320 includes a rail channel 324 that translates along a hook carriage guide rail 330 formed on (or attached to) an upper portion of distributor head 314. A drive motor 370, attached to distributor head 314, drives belt 344 to extend and retract hook carriage 320 with respect to distributor head 314. It should be noted that although a belt drive system is illustrated in FIGs. 21A and 21B, any type of drive system (e.g., screw-drive system, linear piston actuators, etc.) may be used to drive hook carriage 320. To transfer an MRU 160 (or a reagent pack 760), distributor head 314 is rotated a few degrees by rotary drive system 212, hook 318 is extended by hook actuator system 316, and head 314 is rotated in an opposite direction to engage manipulating structure 166 of MRU 160 (or manipulating structure 764 of reagent pack 760). Hook 318 along with MRU 160 (or reagent pack 760) is then retracted into distributor head 314. Distributor head 314 is then be rotated and/or translated and MRU 160 (or reagent pack 760) deposited at a desired location.

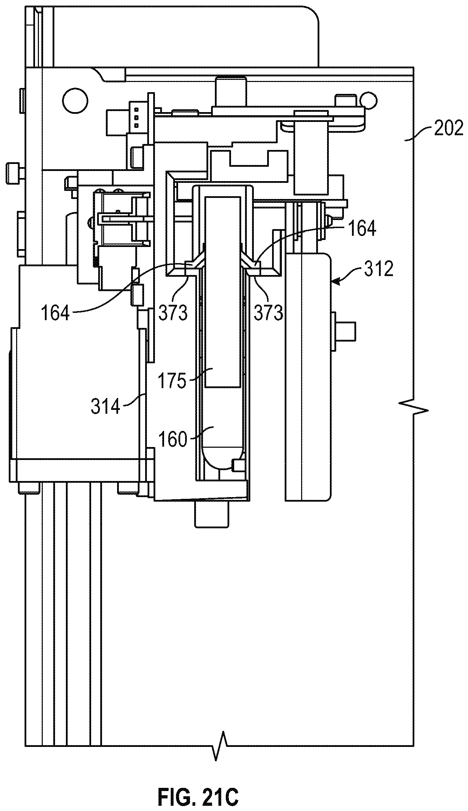

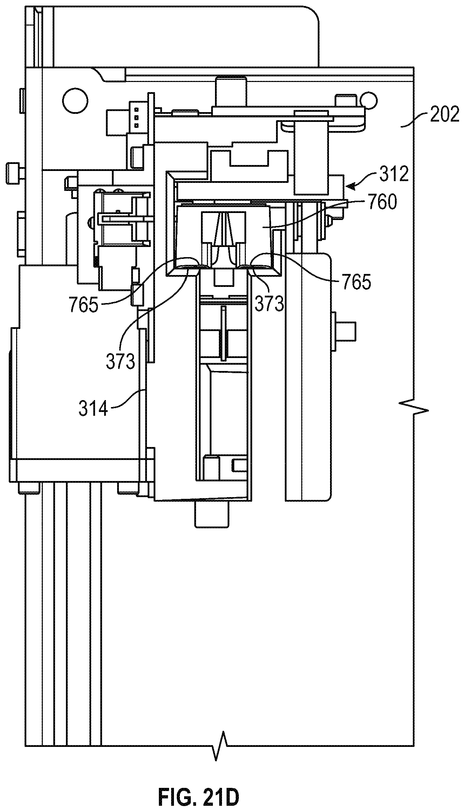

FIG. 21C illustrates an MRU 160 positioned within distributor head 314 of an exemplary receptacle distributor 312 in one embodiment. As shown in FIG. 21C, the receptacle distributor 312 is sized to receive and hold an MRU 160 that is pulled into distributor head 314 by manipulating hook 318. While positioned in distributor head 314, the connecting rib structure 164 of MRU 160 is supported on a ledge or a rail 373 formed on the inner walls of the distributor head 314. FIG. 21D illustrates a reagent pack 760 positioned within distributor head 314 of an exemplary receptacle distributor 312 in one embodiment. As shown in FIG. 21D, receptacle distributor 312 is also configured to receive and hold reagent pack 760 with a bottom edge 765 of pack 760 supported on rail 373.

### Receptacle Handoff Device

Receptacle handoff device 602 of receptacle distribution system 200 is configured to transfer MRU 160 (or another receptacle) between receptacle distributor 150 (see FIGs. 2A, 2B) of first module 100 and receptacle distributor 312 of second module 400. Both receptacle distributor 150 and receptacle distributor 312 transport an MRU 160 by engaging with manipulating structure 166 of MRU 160. To enable quick transfer of MRU 160 from receptacle distributor 150 to receptacle distributor 312, when an MRU 160 is transferred from first module 100 to second module 400, MRU 160 should be oriented such that receptacle distributor 312 (of second module 400) can engage with manipulating structure 166. Receptacle handoff device 602 is configured to receive an MRU 160 from receptacle distributor 150 and rotate MRU 160 such that its manipulating structure 166 is presented to receptacle distributor 312.

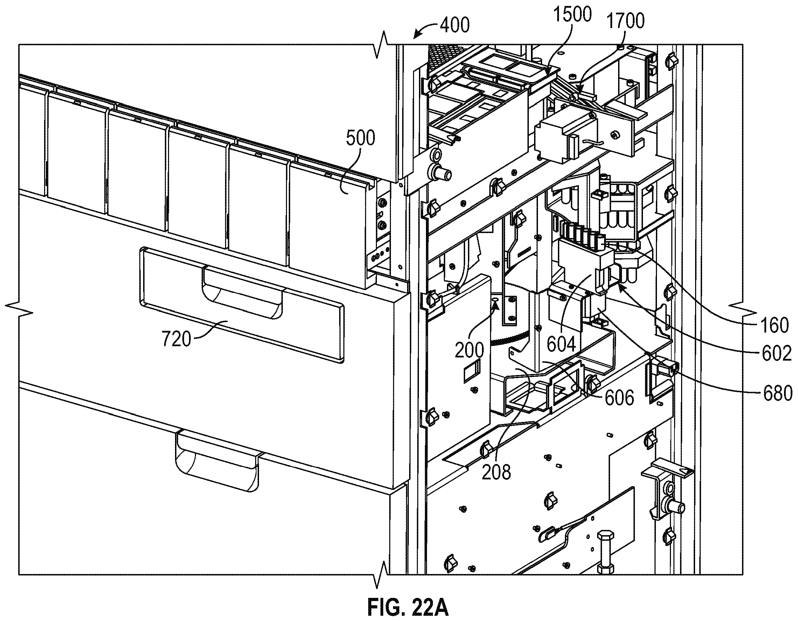

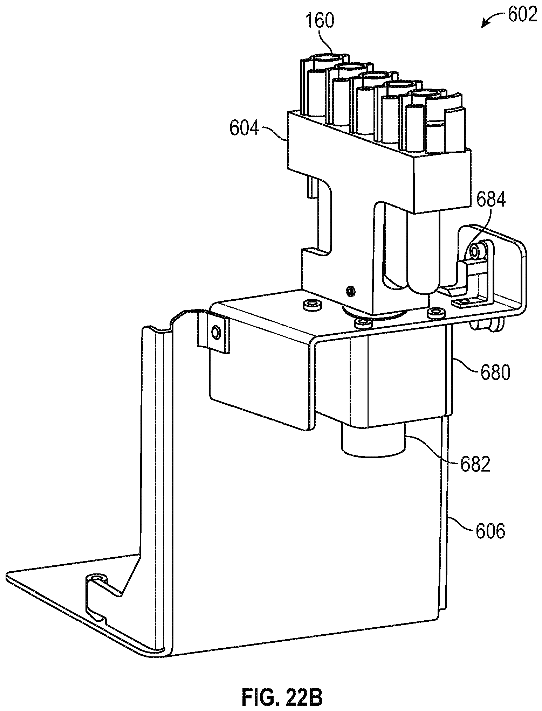

FIGs. 22A and 22B illustrate an exemplary receptacle handoff device 602 in one embodiment. In FIG. 22A, receptacle handoff device 602 is shown attached to second module 400, and in FIG. 22B, receptacle handoff device 602 is shown separated from second module 400 to show details of the device. Receptacle handoff device 602 includes a receptacle yoke 604 configured to receive and hold an MRU 160 placed into yoke 604 by receptacle distributor 150 (of first module 100). Yoke 604 is mounted on handoff device bracket 606 (which is attached to bottom panel 208 of receptacle distribution system 200) such that it is rotatable about a vertical axis of rotation. In the illustrated embodiment, yoke 604 is coupled to a handoff device motor 680 attached to bracket 606. Motor 680 may be a stepper motor for precise motion control and may include a rotary encoder 682 configured to provide rotational position feedback of yoke 604 to a controller. A sensor 684 (e.g., optical sensor, proximity sensor, magnetic sensor, capacitive sensor, etc.) may also be mounted on bracket 606 to provide feedback (e.g., orientation of yoke, etc.) to the controller. After MRU 160 is placed in yoke 604 by receptacle distributor 150 of first module 100, motor 680 rotates yoke 604 such that manipulating structure 166 of the MRU 160 faces receptacle distributor 312 of second module 400.

### MRU Storage Stations, Magnetic Slots, and Reagent Pack Loading Stations

With reference to FIGs. 5D and 5E, receptacle processing deck 600 of second module 400 incudes MRU storage stations 608, 610, 612, magnetic slots 620, and reagent pack loading stations 640 arranged in an arc to accommodate the rotational path of motion of receptacle distributor 312. MRU storage stations 608, 610, 612 serve as temporary storage locations for MRUs 160 and include slots 614 configured to receive an MRU 160. Providing additional storage for MRUs within second module 400 provides the advantage of enhancing workflow by permitting flexibility in the timing that any particular MRU(s) is/are utilized within second module 400. This permits MRUs that may arrive in second module 400 later to be processed out of order, for example, to address urgent needs.

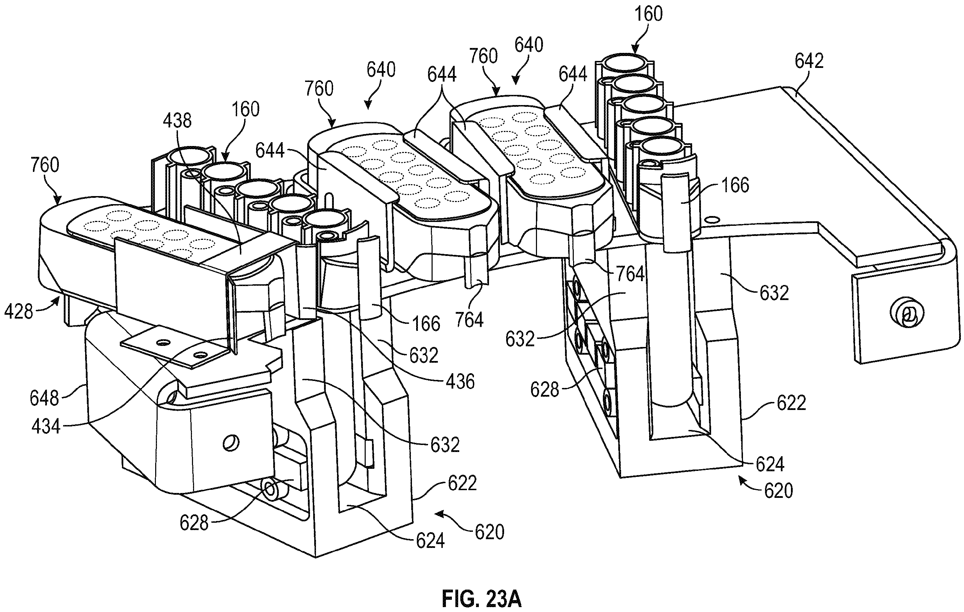

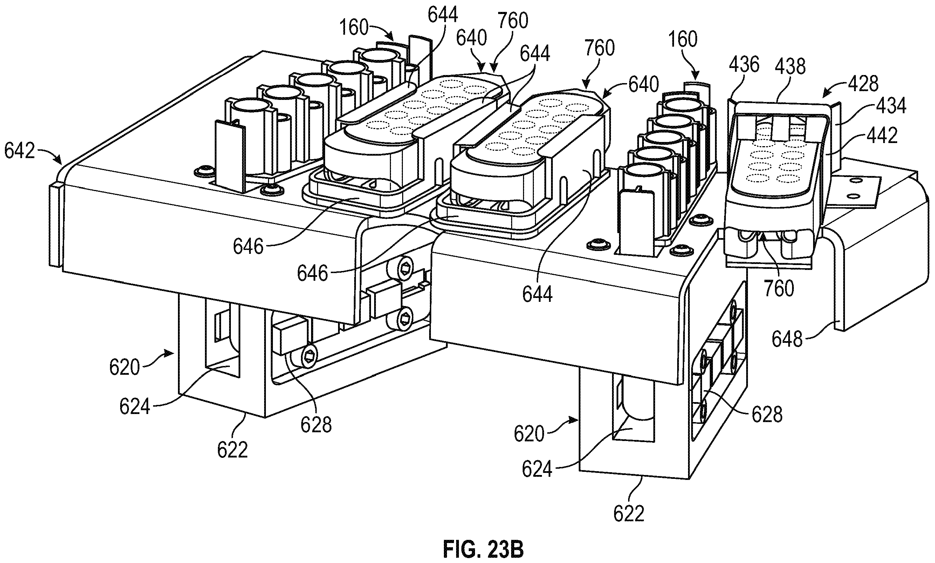

Magnetic slots 620 support MRUs 160 while the contents of the individual receptacles 162 are exposed to a magnetic force, and reagent pack loading stations 640 support reagent packs 760. Details of magnetic slots 620 and reagent pack loading stations 640 in an exemplary embodiment are illustrated in FIGs. 23A and 23B. With reference to these figures, magnetic slots 620 and reagent pack loading stations 640 (two of each are shown in the illustrated embodiment) are supported on a bracket 642 attached to frame 202 of receptacle distribution system 200. The purpose of each magnetic slot 620 is to hold an MRU 160 and apply a magnetic force to the contents of the receptacles 162 to pull the magnetically-responsive solid supports (e.g., magnetic beads) in the contents to the side walls of each receptacle 162 while pipettor 410 aspirates eluate fluid from receptacles 162 of MRU 160. Each magnetic slot 620 includes a block 622 within which is formed a slotted opening 624. An MRU 160 placed within the slotted opening 624 is supported within opening 624 by connecting rib structure 164 (see FIG. 19) of MRU 160 resting on the top of bracket 642. Manipulating structure 166 of MRU 160 extends out of opening 624, and a cutout 632 on each side wall of block 622 enables manipulating hook 318 of receptacle distributor 312 to engage with manipulating structure 166 of an MRU 160 positioned in the slotted opening 624. The top of the MRU is uncovered, thus enabling pipettor 410 access to receptacles 162 of an MRU 160 held in elution slot 620. Magnets 628 are attached to, or embedded within, one or both walls defining the slotted opening 624. Individual magnets 628 may be provided for each receptacle 162 of the MRU, as shown in FIGS. 23A and 23B, or a single magnet may be provided for MRU 160. Examples of covered magnetic slots that can be adapted for use in the embodiments of the present disclosure are described in U.S. Patent No. 8,276,762.

Reagent pack loading stations 640 are defined by spaced-apart, hold-down features 644 extending above bracket 642 and a backstop 646 defining a back end of each reagent pack loading station 640. A reagent pack 760 is inserted between hold-down features 644, under a lateral flange, and is pushed into loading station 640 until the back end of reagent pack 760 contacts backstop 646. A reagent pack trash chute 428 is supported on bracket 642. In the embodiment illustrated, trash chute 428 includes an entrance structure, defined by side walls 434, 436 and a top panel 438, through which a reagent pack 760 is inserted into trash chute 428. Sidewalls 434, 436 are attached to the top of bracket 642 and are bent or flared outwardly at their forward edges to provide a funneling entrance to trash chute 428. One or more resilient tabs 442 may extend down from top panel 438. To discard a reagent pack 760, the receptacle distributor 312 inserts the pack 760 into trash chute 428 between side walls 434, 436. When reagent pack 760 is inserted into trash chute 428, there is a clearance between top panel 438 and the top of the reagent pack 760. The resilient tabs 442 bear against the top of reagent pack 760 and hold the reagent pack down within the trash chute 428. When a subsequent reagent pack 760 is inserted into trash chute 428, it pushes against the previously inserted reagent pack, thereby pushing the previously-inserted pack further into trash chute 428. A cut-out 648 is formed in bracket 642 to enable the previously-inserted pack to eventually falls from trash chute 428 into trash bin 650 located below trash chute 428. Although FIGs. 5D and 5E (and FIGs. 23A and 23B) illustrate a particular number and arrangement (i.e., in an arc) of MRU storage stations 608, 610, 612, magnetic slots 620, and reagent pack loading stations 640, this is only exemplary. In general, second module 400 may include any number of these features and they may be arranged in any pattern.

### Reagent Pack Changer

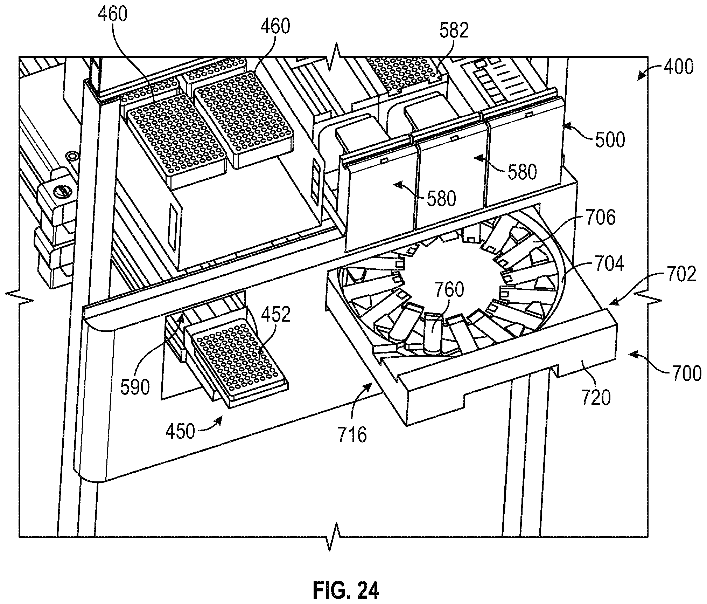

With continuing reference to FIGs. 5D and 5E, second module 400 includes a reagent pack changer 700. Reagent pack changer 700 may provide fully independent reagent pack loading and test execution, whereby an operator may place reagent packs in a reagent pack input device and/or remove reagent packs 760 from the reagent pack input device. In some embodiments, the reagent pack input device comprises a compartment 702 which may be pulled open from second module 400 and which contains a rotatable reagent pack carousel 704. FIG. 24 illustrates an exemplary reagent pack carousel 704 positioned in an openable compartment 702 of second module 400 in one embodiment. Compartment 702 includes a carousel frame 716 disposed on a track that enables frame 716 to slide into or out of second module 400 as a drawer. Frame 716 includes a drawer front 720 that is exposed on the front surface of second module 400 (see also FIG. 1B). The top surface of frame 716 includes a substantially circular recess that is shaped to conform to the shape of the carousel 704, and the carousel 704 is disposed in the recess of frame 716. Carousel 704 includes a number of reagent pack stations 706, each of which is adapted to receive and carry a reagent pack 760. To increase reagent pack packing density, while enabling a bar code reader access to a bar code (or other identifiable indicia) on reagent packs 760, reagent pack stations 706 on carousel 704 may be angled (e.g., between about 5-20°) with respect to a radial direction of carousel 704. Reagent pack stations 706 are configured (e.g., sized, etc.) such that user can load (and remove) reagent packs 760 into (and from) stations 706. In some embodiments, reagent pack changer 700 includes a motor to effect powered rotation of carousel 704. The motor may be mounted to frame 716 and may move in and out with frame 716. Carousel compartment 702 may also include one or more position sensors configured to detect when compartment 702 is an open or closed position and communicate that information to a system controller. Second module 400 may include a reader (e.g., a barcode reader) configured to read indicia (e.g., a barcode), provided on reagent pack 760, that provides information regarding reagent pack 760 (e.g., identity of the assay reagents carried within reagent pack 760, manufacturer, lot number, expiration date, etc.).

Once a reagent pack 760 is present on carousel 704, it is available to be utilized in a nucleic acid amplification assay, such as one that performs a PCR reaction. When particular reagents are required for an amplification reaction, carousel 704 rotates to a position where a reagent pack 760 containing the required reagents is accessible by receptacle distributor 312. Receptacle distributor 312 can then access reagent pack 760 and move it to a reagent pack loading station 640 (see FIGs. 23A and 23B) for reconstitution of one or more dried reagents contained in reagent pack 760. When reagent pack 760 is empty, or when the reagents of one or more wells on reagent pack 760 have been reconstituted and removed, distributor 312 may move reagent pack 760 to trash chute 428 or back to reagent pack input carousel 704 for subsequent use. U.S. Patent No. 9,732,374 describes exemplary embodiments of MRU storage stations, magnetic slots, reagent pack loading stations, and reagent pack changers in more detail.

In some embodiments, second module 400 may also include an electrostatic generator to impart an electrostatic charge to reagent 768 present in a reagent pack 760. The electrostatic charge may assist in positioning and holding reagent 768 at the bottom of mixing well 762 of reagent pack 760 (see FIG. 13C). Though reagent 768 may be held at the bottom of mixing well 762 with a previously-imparted electrostatic charge, the inclusion of an electrostatic generator in module 400 to actively pull reagent 768 down to the bottom of mixing well 762 at the time of reconstitution may assist in positioning reagent 768 at the correct spot during reconstitution. In some embodiments, the electrostatic generator may be positioned below reagent pack loading station 700 or in carousel 704.

### Storage/Expansion Module

With reference to FIG. 1B, second module 400 may include a compartment 590 for storing accessories or to accommodate expansion of second module 400 (for example, to add additional reagent compartments for storage of reagents, add analytical capabilities to system 1000, etc.). In one exemplary embodiment, compartment 590 can house a standard well plate or a storage tray 452 sized to accommodate cap/vial assemblies 480. The well plate or tray 452 may be located such that at least one of front arm 408 (that includes pipettor 410) and back arm 416 (that includes vial transfer arm 418) of fluid transfer and handling system 402 (see FIG. 14) can access the location of the well plate or tray 452. As shown in FIG. 24, compartment 590 may be accessed from the front of module 400 via a drawer mechanism 450 so that the user can load and unload the well plate or storage tray 452. In some embodiments, storage tray 452 may be utilized to collect cap/vial assemblies 480 that have undergone an amplification reaction to provide for the ability to perform additional assays or reactions (e.g., thermal melt analyses, sequencing reactions, etc.) on the samples contained in the cap/vial assemblies 480. The cap/vial assemblies 480 for storage in compartment 590 may be referred to as storage receptacles (or capped storage receptacles when closed). An exemplary procedure for performing a thermal melt analysis is described in U.S. Pat. No. 8,343,754, and U.S. Patent No. 9,588,069 describes an exemplary structure for performing a thermal melt analysis. Storage tray 452 can also be used to store cap/vial assemblies 480 containing eluate that has not been subjected to a nucleic acid amplification reaction. To access the contents of a cap/vial assembly 480 stored in compartment 590, the cap 476 and vial 464 may be separated using, for example, the cap removal tray of U.S. Patent No. 9,248,449. In this embodiment, vial transfer arm 418 (with or without a pipetting capability) may transfer the cap/vial assembly 480 from storage tray 452 to the cap removal tray, which may be located in one of the cap/vial compartments 440. In some embodiments, compartment 590 may also be accessed from the side of module 400. In some embodiments, compartment 590 may be configured to position containers containing reagents therein. In some embodiments, compartment 590 may include a drive system including, for example, a motor-driven belt, to translate the well plate or reagent containing container (or another component stored in compartment 590) into or out of second module 400.

### IVD+ASR Embodiments

System 1000 is also adapted to perform existing IVD assays supplemented with additional reagents, such as one or more ASRs (e.g., oligonucleotides), that can expand or improve the capabilities of the assay. Exemplary situations in which such supplementation may be appropriate include detection of a new or different target, which may be a new or different form (e.g., variant, subspecies, genotype, allele, strain, polymorphism, haplotype, mutant, and the like) of a target in the same general class of targets already detected by the IVD assay but for which an IVD is not commercially available on system 1000.

For example, in the context of an IVD for methicillin-resistant *S. aureus* (MRSA), the new or different target could be an additional type of MRSA, such as MRSA comprising a type of *mec* right extremity junction (MREJ) not already detected by the IVD. Depending on the differences between the new or different target and existing targets relative to the target sequences of oligonucleotides in the existing IVD, one or two supplemental amplification oligonucleotides and/or a supplemental detection probe may be provided as ASRs. As another example in the context of an IVD for MRSA, the IVD could be designed to detect *mecA* and *mecC,* but the user might also have an interest in detecting *mecB.* The IVD could be supplemented with an ASR having oligonucleotides that are capable of amplifying and detecting the *mecB* gene.

Alternatively, the new or different target could also be a sequence other than a new or different variant or mutant, e.g., a sequence from a different organism, such as a species of bacterium or virus not detected by the original IVD, or a control sequence. For example, an IVD for detecting a panel of viruses could be expanded by including a set of oligonucleotides (e.g., one or two amplification oligonucleotides and one or two detection probes, depending on the assay format and whether any IVD oligonucleotides may play a role in detection of the new or different target) for an additional virus. As an example, an IVD for detecting a set of respiratory viruses such as adenovirus, rhinovirus, and human metapneumovirus could be supplemented with oligonucleotides for detecting coronavirus. With respect to control sequences, the addition of a control may be used to test for inhibition or other problems with the assay. When ASRs are provided for amplifying a control, the template sequence for generating the control amplicon may also be provided.

In some cases, the ASR comprises an amplification oligonucleotide. One additional amplification oligonucleotide may be sufficient, e.g., where the new or different target comprises a sequence that adversely impacts the performance of an existing IVD amplification oligonucleotide, e.g., by lowering the melting temperature of a hybridized complex of the IVD amplification oligonucleotide to the new or different target (which may result, e.g., from a polymorphism such as a mutation that arose, was discovered, or increased in prevalence or importance after the IVD reagents were designed), which will generally reduce or eliminate the degree of amplification of the new or different target (without a supplemental ASR) relative to an original target. The ASR amplification oligonucleotide may, together with an oppositely oriented IVD amplification oligonucleotide, amplify the new or different target for detection by one or more IVD detection probes.

In some cases, the ASR comprises a pair of amplification oligonucleotides. This approach may be used when the new or different target is a sequence to which the IVD amplification oligonucleotides do not hybridize efficiently, e.g., a sequence in a new or different target organism or a variant of a target organism that lacks sufficient homology over the target region to permit efficient hybridization.

In some cases, the ASR comprises a detection probe. One additional detection probe may be sufficient, e.g., where the new or different target comprises a sequence that adversely impacts the performance of an existing IVD detection probe, e.g., by altering the structure and/or lowering the melting temperature of a hybridized complex of the IVD detection probe to the new or different target (which may result, e.g., from a polymorphism such as a mutation that arose, was discovered, or increased in prevalence or importance after the IVD reagents were designed), which will generally reduce or eliminate the degree of detection of the new or different target (without a supplemental ASR) relative to an original target. The ASR detection probe is designed to detect an amplicon generated from the new or different target by the IVD amplification oligonucleotides.

Alternatively, where the new or different target is detected using ASR oligonucleotides that amplify a sequence dissimilar to sequences detected by the IVD oligonucleotides and/or where distinguishable detection is desired (e.g., as discussed below), an ASR detection probe may be provided in combination with ASR amplification oligonucleotides.

In assay formats using primary and secondary detection probes such as Invader Plus^{®} assays, the ASR detection probe may be the invasive probe or the signal (primary) probe of an Invader Plus assay, which interacts directly with the amplicon of the new or different target. It may comprise a non-target hybridizing sequence that interacts with an IVD oligonucleotide that is a secondary, labeled detection probe (e.g., a FRET cassette of an Invader Plus^{®} assay). Chemistries for performing Invader Plus assays are described in U.S. Patent Application Publication No. 2005/0186588 and U.S. Patent No. 9,096,893. In assay formats using a detection probe that both binds the amplicon and comprises a label, such as TaqMan, the ASR detection probe may comprise the same label as an IVD detection probe. Chemistries for performing TaqMan assays are described in PCT Application No. PCT/US2018/024021, filed March 23, 2018, and U.S. Patent No. 5,723,591. As such, the new or different target may be detected using a channel already used for detecting an original target of the IVD assay. This approach is particularly appropriate where the significance of the new or different target being present is similar to or indistinguishable from the presence of an original IVD target, e.g., where the purpose of the assay is to determine whether or not a target pathogen such as MRSA was in a sample and the ASR serves to facilitate detection of an additional type, variant, or mutant of the target pathogen.

Alternatively, to distinguishably detect a new or different target, a detection probe may be provided that is distinguishably labeled relative to the IVD detection probes. This can be, e.g., a distinguishably labeled detection probe that is configured to bind the target amplicon directly (e.g., for a TaqMan assay), or a distinguishably labeled secondary detection probe that is configured to bind a cleaved, non-complementary 5' flap of a primary detection probe also provided as an ASR (e.g., for an Invader Plus assay). This approach is particularly appropriate where the significance of the new or different target being present is not similar to the presence of an original IVD target, e.g., where the new or different target is a different organism or is a control.

The one or more ASRs for supplementing the IVD assay can be provided in a separate receptacle or cartridge from the standard IVD oligonucleotides. This facilitates augmenting the capabilities of the assay without necessitating a reformulation of the reagent containing the IVD oligonucleotides. The reagent or cartridge containing the supplemental ASR or ASRs can further comprise additional materials for use in the assay, such as one or more lyophilized enzymes, dNTPs, buffer, one or more salts, or a combination thereof.

Accordingly, in some embodiments, methods disclosed herein comprise providing a reagent pack 760 having mixing wells 762 comprising oligonucleotides (and possibly other amplification reagents) for performing an IVD assay and a receptacle(s) 1940 containing one or more ASRs. The contents of mixing wells 762 may be reconstituted (e.g., if provided in dry form, such as a lyophilizate). The contents of mixing wells 762 can be combined with samples in vials 464 and subjected to reaction conditions, such as the reaction conditions of the IVD assay, which may comprise thermocycling. Detection may be performed in the same manner as the unmodified IVD assay or may comprise the same steps as the IVD assay plus detecting an ASR detection probe, if present, which may or may not be distinguishably labeled as discussed above.

The one or more ASRs can be provided by an end user, which essentially converts the IVD into an LDT. Alternatively, one or more ASRs may be provided by the source of the original IVD in combination with original IVD reagents following validation, such that the original IVD in conjunction with the one or more ASRs may remain an IVD.

### Example

MRSA is a notoriously polymorphic group of pathogens, with much of the polymorphism occurring at the right extremity junction of the mobile genetic element (SCC*mec*) carrying the methicillin resistance gene and the insertion site in the *orfX* gene of the bacterial chromosome. *See* U.S. Patent Application No. 62/544,491 and U.S. Patent No. 7,838,221 for further discussion of MRSA and exemplary reagents and methods for detecting MRSA.

A MRSA isolate designated CI5683 was found to comprise a polymorphism that interferes with the structure and therefore the cleavage of an Invader Plus primary probe of an existing MRSA assay reagent set when hybridized to an *orfX*/SCC*mec* amplicon of MRSA CI5683. The original primary probe generated some signal but did not do so sufficiently to exceed the Ct threshold for positive results, meaning that performing the assay on a sample comprising MRSA CI5683 gave a false negative result.

The oligonucleotides for the standard assay were provided in a reagent pack. A receptacle contained either MgCl₂ alone (control) or MgCl₂ with an additional primary probe as an ASR (test). Samples (n=3) prepared from CI5683 at 10⁴ CFU/ml were subjected to Invader Plus assays on a Panther Fusion^{®} system (Hologic, Inc.; Marlborough, MA) with the following results.

**Table 1. CI5683 Detection**

| Reagents | *orfX*/SCC*mec* average Ct | Standard Deviation |
|---|---|---|
| Test | 29.7 | 0.05 |
| Control | 42.7 | 0.58 |

The *mecA*/*C* and GAPDH genes were also detected in multiplex, along with an internal control. The positivity of each of these was unaffected by the presence of the ASR primary probe (data not shown).

A MRSA isolate designated CI5685 contains a type xvii MREJ. The existing MRSA assay reagent set does not contain an amplification oligonucleotide that efficiently hybridizes to and primes synthesis on the type xvii MREJ sequence.

As above, the oligonucleotides for the standard assay were provided in a first reagent pack. A second reagent pack contained either MgCl₂ alone (control) or MgCl₂ with an additional amplification oligomer complementary to type xvii MREJ sequence as an ASR (test). Samples (n=3) prepared from CI5685 at 10⁴ CFU/ml were subjected to Invader Plus^{®} assays on a Panther Fusion^{®} system with the following results.

**Table 2. CI5685 Detection**

| Reagents | *orfX*/SCC*mec* average Ct | Standard Deviation |
|---|---|---|
| Test | 30.7 | 0.12 |
| Control | - | - |

The *mecA*/*C* and GAPDH genes were also detected in multiplex, along with an internal control. The positivity of each of these was unaffected by the presence of the ASR amplification oligonucleotide (data not shown).

Thus, additional amplification oligonucleotides and/or detection probes can be provided in separate receptacles from existing assay oligonucleotides and used in combination therewith to augment the capabilities of the assay.

### Exemplary Method of Operation

In system 1000, first module 100 may be used for the sample preparation portion of a molecular assay (e.g., steps for isolating and purifying a target nucleic acid that may be present in a sample). Samples and a target capture reagent (TCR), which may include a magnetically-responsive solid support, are loaded onto first module 100. These samples may include samples on which different types of molecular assays (IVD assays, LDTs, etc.) are desired to be performed. TCR may include capture probes designed to specifically bind to targeted nucleic acids or to non-specifically bind all (or most) nucleic acids in a sample. In other words, non-specific capture probes do not discriminate between targeted and non-targeted nucleic acids. Exemplary approaches for specific and non-specific immobilization of targeted nucleic acids are described in U.S. Patent Nos. 6,534,273 and 9,051,601. Non-specific capture techniques that do not require a capture probe are well known to the skilled person and include, for example, techniques described in U.S. Patent No. 5,234,809. Reagent containers 1520 are loaded on first reagent container-carrier 1500 in reagent container compartment 500 of second module 400 (see FIG. 6B). Reagent container transport 1700 then moves first reagent container-carrier 1500 from reagent container compartment 500 to a location within first module 100 (see FIG. 8) where it can be accessed by a fluid transfer device of first module 100.

An exemplary fluid transfer device 805 of first module 100 is illustrated in FIG. 25. In the embodiment illustrated in FIG. 25, fluid transfer device 805 includes a reagent pipettor 810 and a sample pipettor 820 mounted on a gantry system. In some embodiments, one or both pipettors 810, 820 may be adapted to move in multiple orthogonal directions (x, y, z, etc.) on the rails of the gantry system. Through information provided to first module 100 (e.g., by a user via a user interface, or through machine-readable information (e.g., a bar code) on the sample container), first module 100 recognizes the type of assay to be performed. To process samples, receptacle distributor 150 of first module 100 retrieves a fresh MRU 160 (see FIG. 19) and places it into a sample dispense position within first module 100. TCR and sample are transferred from a reagent container and sample tube, respectively, to a receptacle 162 of MRU 160 by the fluid transfer device 805 of first module 100. In some embodiments, reagent pipettor 810 of fluid transfer device 805 may be used to transfer the reagent and the sample pipettor 820 may be used to transfer the sample into MRU 160. The contents of MRU 160 are then incubated (in incubator 112, see FIGs. 2A, 2B) for a prescribed period at a prescribed temperature before MRU 160 is transferred to a magnetic wash station 118, 120 for a magnetic wash procedure. Exemplary target capture procedures using magnetically-responsive particles or beads are described in U.S. Patent Nos. 6,110,678 and 9,051,601, and target capture procedures using silica beads are described in U.S. Patent No. 5,234,809.

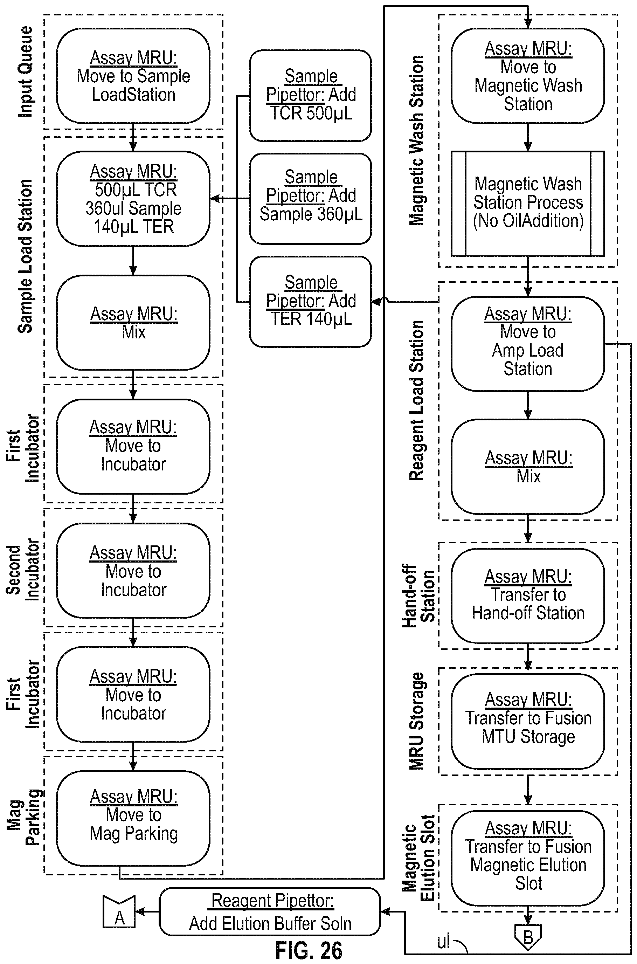

FIG. 26 illustrates and describes an exemplary extraction process employing a target capture process using magnetic particle target capture. In a receptacle 162 of an MRU 160 (see FIG. 19), the sample is combined with a target capture reagent (TCR) containing magnetic particles and a lysing reagent. The contents of MRU 160 are mixed using orbital rotation at a defined speed and then exposed to a series of heating steps (on incubators 112 and 114, see FIGs. 2A, 2B) designed to lyse the cells and immobilize sample nucleic onto the magnetic particles using a specific or non-specific capture probe. After the sample is combined with TCR in MRU 160, MRU 160 may first be transferred to a first incubator (e.g., transition incubator 112 maintained at a temperature of, for example, 43.7°C) to elevate the temperature of the contents of MRU 160 closer to the temperature of the second incubator (e.g., the high temperature incubator 114 which may be maintained at a temperature of, for example, 54°C) to which MRU 160 is transferred from the first incubator 112. While in the second incubator 114, the capture probe may bind to any target analyte which may be present in the sample. However, in some embodiments, the capture probe may not bind to the solid support while in the second incubator 114 (due to, for example, the high temperature of the second incubator 114). MRU 160 is then transferred back to the first incubator 112 to bind the capture probe to the solid support. After incubation, MRU 160 is exposed to a magnetic field to isolate the particles within receptacle 162. While immobilized within receptacle 162, proteins and cellular debris (potential amplification inhibitors) are removed using a series of aspiration and wash steps in a magnetic wash station 118, 120 (see FIG. 2A). MRU 160 is then moved to an amplification load station 104, 106 (see FIG. 2A) where 50 µL of elution buffer (e.g., from one of reagent containers 1520) is added to receptacle 162 of MRU 160 using reagent pipettor 810 (see FIG. 25). The contents of MRU 160 are then agitated (e.g., in a load station, such as, for example, amplification mix load station 104) to re-suspend the particles before receptacle handoff device 602 transfers MRU 160 to second module 400 for PCR reaction setup. In second module 400, MRU 160 may be placed in an available slot 614 of one of MRU storage stations 608, 610, 612 (see FIG. 5D). When signaled by the system controller, second module 400 may then move MRU 160 to a magnetic slot 620 to separate the eluted nucleic acids from the magnetic particles.

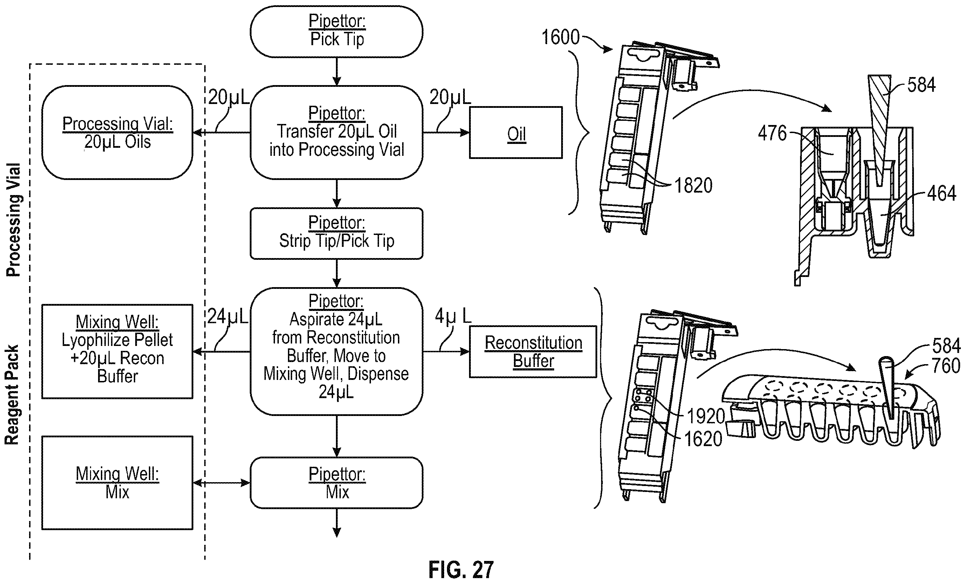

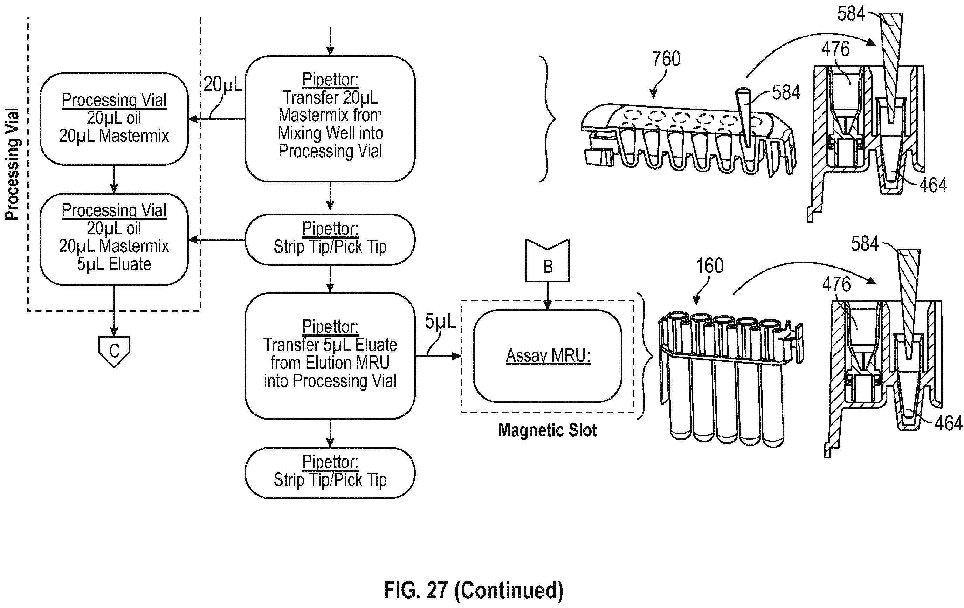

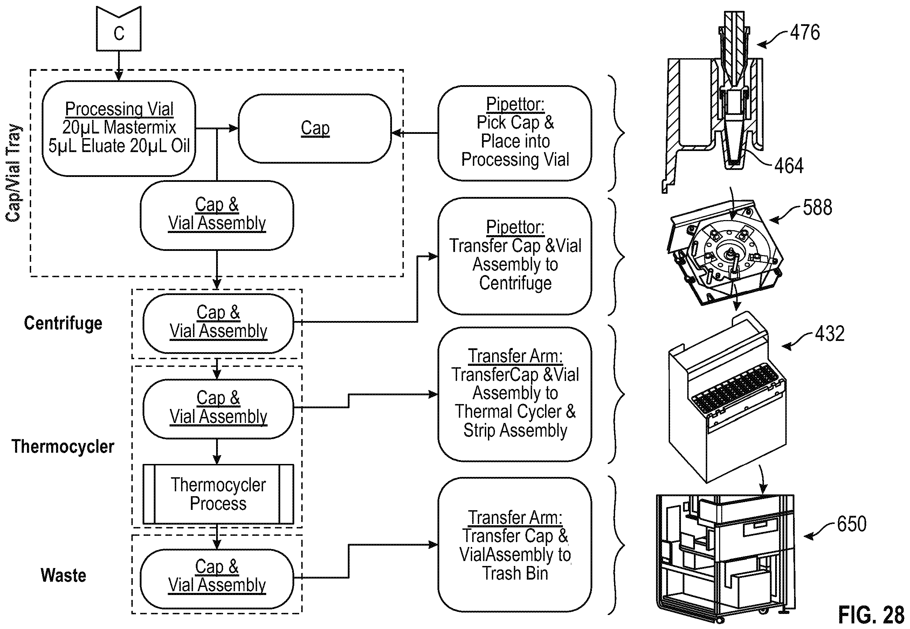

A fluid transfer device, such as robotic pipettor 410, then initiates the amplification process. FIG. 27 schematically illustrates and describes an exemplary amplification process. Pipettor 410 first attaches a disposable tip 584 (from a disposable tip tray 582 carried in one of tip compartments 580, see FIG. 5A) to mounting end 425 of its aspirator probe 415. Pipettor then aspirates oil (e.g., from the oil containers 1820 located in the reagent container compartment 500) and dispenses about 20 µL of oil into each processing vial 464 queued for testing. Pipettor 410 then separately aspirates the eluate/sample from receptacle 162 and a solvent from a solvent container (e.g., container 1620 or 1920), and dispenses them into a mixing well 762 of a reagent pack 760 containing a desired unit-dose reagent 768 (see FIGs. 13C, 13D) (e.g., a lyophilizate). As explained previously, if an IVD assay is to be performed on the sample, the solvent used in this step is reconstitution buffer 1670 from one of solvent containers 1620 (see FIG. 6B) stored in second reagent container-carrier 1600. And if an LDT is to be performed, the solvent used is a reconstitution fluid (1970A, 1970B, etc.) from one of solvent containers 1920 (see FIG. 6B) stored in reagent container compartment 500 or in another compartment (e.g., chilled/heated compartment). In some cases, the fluid in mixing well 762 may be drawn into and released from pipettor 410 multiple times to promote rapid reconstitution and mixing of the solvent and reagent 768. The reconstituted amplification reagent is then aspirated and dispensed into processing vial 464. Vial 464 is then capped with cap 476 using pipettor 410 to form cap/vial assembly 480 (see FIGs. 15A and 15B). Pipettor 410 then transfers cap/vial assembly 480 to centrifuge 588, where cap/vial assembly 480 is centrifuged at a sufficient speed and for a sufficient period of time to concentrate the contents of vial 464 and to remove air bubbles. After centrifuging, vial transfer arm 418 engages cap 476 of the centrifuged cap/vial assembly 480 and transports it to a receptacle holders 4010 of thermal cycler 432. The contents of cap/vial assembly 480 are thermally cycled in thermal cycler 432 in accordance with an amplification procedure (e.g., PCR amplification). In some embodiments, amplification and detection may simultaneously occur in thermal cycler 432. FIG. 28 schematically illustrates an exemplary method of transferring cap/vial assembly 480 to thermal cycler 432. The results of the assay may be displayed on an instrument monitor or a user interface 50 and may also be printed or communicated to the LIS.

In some embodiments, first module 100 may perform a nucleic acid amplification reaction (e.g., isothermal amplification reaction) on the contents of receptacle 162 before transporting MRU 160 to second module 400. Additionally, before or after the contents of MRU 160 are processed in second module 400, an amount of eluate/sample may be transferred from receptacle 162 to one or more vials 464 for performing another reaction (e.g., PCR or other process), and/or MRU 160 may be transported back to first module 100 to perform an a nucleic acid amplification reaction on the remaining contents of receptacle 162.

Exemplary processes embodying aspects of the present disclosure will now be described. It should be noted that these processes are only exemplary and other processes (e.g., by omitting and/or reordering some of the described steps) may be performed by system 1000. In some embodiments, a described process may include a number of additional or alternative steps, and in some embodiments, one or more of the described steps may be omitted. Any described step may be omitted or modified, or other steps added, in an analysis. Although a certain order of steps is described or implied in the described processes, in general, these steps need not be performed in the illustrated and described order. Further, parts of (or all of) a described process may be incorporated in another process.

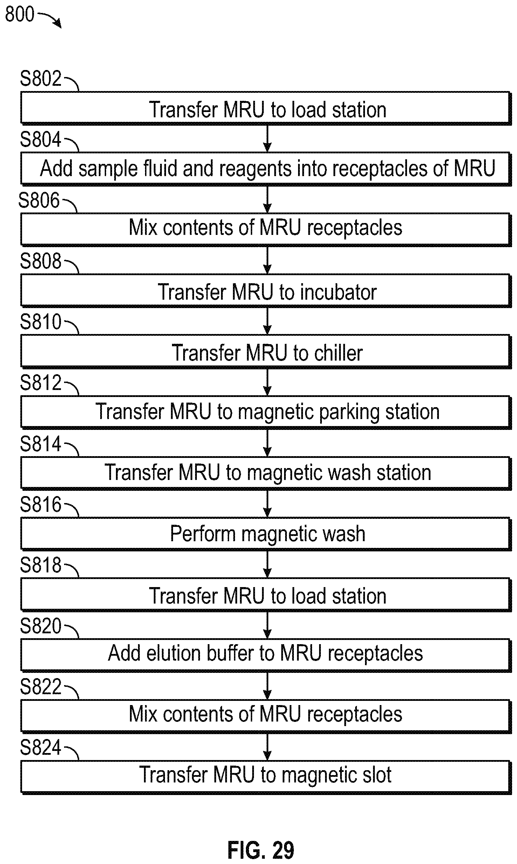

An exemplary sample eluate preparation process 800 is illustrated in FIG. 29. As explained previously, in some embodiments, sample preparation may be conducted primarily in first module 100 of system 1000. In step S802, receptacle distributor 150 of first module 100 moves an MRU 160 from receptacle compartment 102 to one of load stations 104, 106 or 108 (or to another location at which reaction materials can be added to receptacles 162). In step S804, a robotic pipettor 810 of first module 100 transfers sufficient quantity of TCR (target capture reagent), sample fluid, and target enhancer reagent (TER) into each receptacle 162 of MRU 160. Exemplary target enhancer reagents are described in U.S. Patent No. 8,420,317. In an exemplary process, about 500 µL of TCR, about 360 µL of the sample fluid, and about 140 µL of TER may be transferred to each receptacle 162. In step S806, the TCR, sample fluid, and TER in receptacles 162 are mixed by, for example, oscillating MRU 160 at a high frequency (e.g., for about 60 seconds at about 16 Hz). In step S808, MRU 160 is moved into an environment that will promote the desired reaction. For example, in some embodiments, receptacle distributor 150 removes MRU 160 from load station 104 and transfers MRU 160 to, for example, incubator 114 to incubate the contents of MRU 160 at a prescribed temperature for a prescribed period of time (e.g., about 1800 seconds at about 64°C or another suitable temperature and time). In some embodiments, to minimize temperature fluctuations within the incubator, before moving MRU 160 to the incubator, MRU 160 may first be placed in a heated station (e.g., one of heated loading stations 104, 106, 108 (e.g., for about 300 seconds at about 64°C) to heat the contents of MRU 160 to a temperature closer to that of incubator 114. In some embodiments, the desired reaction may require multiple incubations at different temperatures. In such embodiments, receptacle distributor 150 may transfer MRU 160 from the first incubator to another incubator (e.g., maintained at a different temperature) to continue the incubation process. In some embodiments, after the incubation steps, in S810, receptacle distributor 150 may transfer MRU 160 from the incubator to a chiller module 122 (e.g., maintained at a predetermined temperature) to terminate any incubation reactions occurring in receptacles 162. Chiller 122 may aid in oligo hybridization and cools MRU 160 before luminescence measurements.

If an assay includes a step for immobilizing targeted nucleic acid on a magnetically-responsive solid support, then a magnetic separation procedure is performed on the contents of receptacles 162. In such embodiments, in step S812, receptacle distributor 150 transfers MRU 160 from chiller module 122 (after a predetermined period of time, e.g., about 830 seconds) to a magnetic parking station 110 that includes magnets for attracting magnetically-responsive solid supports to the inner walls of receptacles 162, thereby pulling the solid supports out of suspension. An exemplary parking station is described in U.S. Patent No. 8,276,762. In step S814, after a prescribed period of time in the magnetic parking station (e.g., about 300 seconds), receptacle distributor 150 transfers MRU 160 to one of magnetic wash stations 118, 120. In step S816, a magnetic wash procedure is performed on the contents of MRU 160 placed in magnetic wash station 118, 120 (see FIG. 2F). Exemplary magnetic wash station is described in U.S. Patent Nos. 6,335,166 and 9,011,771. The magnetic separation procedure may involve multiple magnetic dwells, during which the contents of the receptacles 162 are exposed to magnetic forces for predetermined periods of time. During each magnetic dwell, the fluid contents of receptacles 162 are aspirated, while the magnetic particles largely remain isolated within receptacles 162. In one exemplary embodiment, three magnetic dwells of about 120 seconds each are performed. At the conclusion of each magnetic dwell, the magnetic force is removed from the contents of the receptacle. In some embodiments, after each magnetic dwell (except the last magnetic dwell), a predetermined amount of wash fluid (e.g., about 1000 µL of a wash buffer) is added to each receptacle 162 to re-suspend the magnetic particles before beginning the next magnetic dwell.

After the magnetic wash process is complete (e.g., after the last magnetic dwell followed by an aspiration of the fluid contents of receptacles 162), in step S818, receptacle distributor 150 transfers MRU 160 from magnetic wash station 118, 120 to one of load stations 104, 106, 108. While positioned in the load station, in step S820, a predetermined amount of elution buffer (e.g., about 50-110 µL) from one of reagent containers 1520 (transferred into first module 100 by reagent container transport 1700) is added to each receptacle 162 of MRU 160. The elution buffer is added to elute nucleic acids from the solid supports, which could otherwise interfere with detection during real-time amplification. In some embodiments, the contents of receptacles 162 may be heated (e.g., by transferring MRU 160 to incubators 112 or 114) to improve the efficiency of the nucleic acid elution. In step S822, following the addition of the elution buffer, the contents of receptacles 162 are mixed by agitating MRU 160 (e.g., in amplification mix load station 104). In step S824, MRU 160 is transferred from first module 100 to a magnetic slot 620 in second module 400. To transfer MRU 160 from first module 100 to second module 400, distribution head 152 of receptacle distributor 150 first places MRU 160 in receptacle handoff device 602. Handoff device 602 is then rotated to present manipulation structure 166 of MRU 160 to receptacle distributor 312. A manipulating hook 318 of receptacle distributor 312 engages with manipulation structure 166 and transfers MRU 160 to magnetic slot 620 or, optionally, to MRU storage 608.

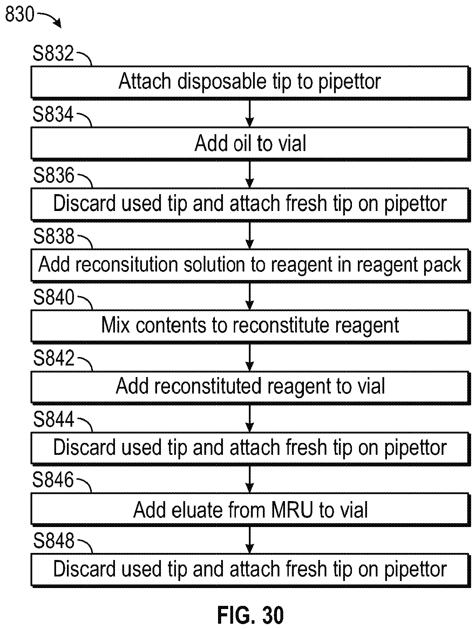

FIG. 30 illustrates an exemplary reaction mixture preparation process 830. As would be recognized by persons skilled in the art, one or more of the steps of process 830 may proceed in parallel with one or more of the steps of process 800 shown in FIG. 29. In step S832, pipettor 410 of second module 400 picks up a disposable tip 584 from a disposable tip tray 582 carried in one of tip compartments 580. In step S834, pipettor 410 aspirates and transfers an amount of oil (e.g., about 15 µL) from one of oil containers 1820 carried in reagent container compartment 500 to one or more processing vials 464 held in cap and vial trays 460 of cap/vial compartment 440. In some embodiments, the oil and reaction mixture may be biphasic, where the oil floats on top of the reaction mixture. During some exemplary nucleic acid amplification reactions, such as PCR, the oil may aid in preventing the formation of a condensate in the vial during thermal cycling. In step S836, pipettor 410 discards the used pipette tip 584 into the trash chute 428 and picks up a fresh disposable pipette tip 584 from disposable tip tray 582. In step S838, pipettor 410 transfers an amount of reconstitution reagent (e.g., about 20 µL) from a solvent container to a mixing well 762 of reagent pack 760 that was previously transferred by receptacle distributor 312 from reagent pack carousel 704 to a reagent pack loading station 640.

In embodiments where a known IVD assay is being performed on a sample, in step S838, pipettor 410 transfers a desired amount of reconstitution buffer 1670 from a solvent container 1620 (e.g., carried in second reagent container-carrier 1600 of reagent container compartment 500) to a mixing well 762 that contains a unit-dose reagent 768 that includes constituents for performing a nucleic acid amplification reaction, such as amplification oligomers, probes, a polymerase, nucleoside triphosphates (dNTPs), etc. And in embodiments where an LDT is being performed on a sample, in step S838 pipettor 410 may transfer a desired amount of a reconstitution fluid 1970A, 1970B (that, for example, includes third party or customer-developed constituents for the amplification reaction, such as amplification oligomers, probes, etc.) from a solvent container 1920 to a mixing well 762 having a reagent 768 that does not include such constituents. As explained previously, in some embodiments, solvent container 1920 (containing the reconstitution fluid 1970A, 1970B) may be provided in the same second reagent container-carrier 1600 that also supports solvent container 1620 (containing reconstitution buffer 1670). That is, one of multiple pockets 1610 of container-carrier 1600 may support solvent container 1920 and another pocket of the same container-carrier 1600 may support solvent container 1620. However, in some embodiments, solvent container 1920 with reconstitution fluids 1970A, 1970B may be supported in a different container-carrier and/or a different reagent container compartment (e.g., a heated or a cooled compartment) than solvent container 1620. In embodiments, where an IVD assay is performed on some samples and an LDT is performed on other samples, in step S838, pipettor 410 delivers both a reconstitution buffer 1670 to a first mixing well 762 that includes a suitable amplification reagent 768 (that includes constituents such as, for example, amplification oligomers, probes, a polymerase, dNTPs, etc.) and a reconstitution fluid 1970A or 1970B to a second mixing well 762 that includes a suitable amplification reagent 768 (that does not include constituents such as, for example, amplification oligomers, probes, polymerase, etc.), where the first and second mixing wells may be part of the same or different reagent packs 760.

In step S840, the contents of mixing well 762 are mixed to fully dissolve reagent 768 (e.g., lyophilized reagent). In one example, pipettor 410 mixes the fluid within mixing well 762 by alternately aspirating the fluid into pipette tip 584 and dispensing the fluid back in well 762 one or more times to dissolve reagent 768. In step S842, pipettor 410 transfers an amount (e.g., about 20 µL) of the reconstituted reagent from mixing well 762 of amplification reagent pack 760 into a vial 464. In some embodiments, the reconstituted reagent may include all components necessary for performing a nucleic acid amplification reaction (e.g., a polymerase (e.g., Taq DNA polymerase), dNTPs, magnesium chloride (MgCl₂), etc.) in a premixed and optimized format. In some embodiments, amplification oligomers may not be included in the reconstituted reagent. In step S844, pipettor 410 disposes of the used tip 584 (into the trash chute 428) and picks up a fresh pipette tip 584 from tip tray 582. In step S846, pipettor 410 transfers an amount of eluate (e.g., about 5 µL) from receptacle 162 of MRU 160 (of step S824 of process 800 of FIG. 29) to processing vial 464 (to which oil and reagent were added in steps S834 and S842), thereby forming a reaction mixture. In step S848, pipettor 410 again disposes of the used pipette tip 584.

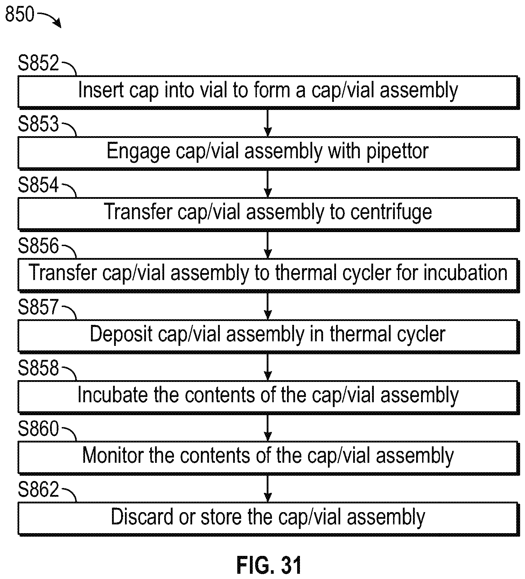

FIG. 31 illustrates an exemplary process 850 for performing an automated process, such as a PCR reaction. In step S852, pipettor 410 picks up a cap 476 from cap well of cap and vial tray 460, such as by inserting the pipettor probe 422 into cap 476 and forming a frictional engagement therewith. In step S853, pipettor 410 then inserts cap 476 into processing vial 464 (from step S846 of process 830) held in processing vial well 474 until cap 476 locks with vial 464 to form cap/vial assembly 480 (see, for example, FIGs. 15A and 15B). In step S854, pipettor 410 transfers cap/vial assembly 480 to centrifuge 588, where cap/vial assembly 480 is centrifuged for a period of time sufficient to concentrate the reaction mixture within vial 464 (e.g., centrifuging the vial for 30 seconds at 3000 RPM). In step S856, following a predetermined period of time in the centrifuge, vial transfer arm 418 is inserted into cap 476 of cap/vial assembly 480 held in centrifuge 588 and removes cap/vial assembly 480 from centrifuge 588. In step S857, vial transfer arm 418 then transfers cap/vial assembly 480 to thermal cycler 432 and deposits (e.g., ejects) cap/vial assembly 480 into a well 4004 of a receptacle holder 4010, where the reaction mixture is exposed to the temperature conditions of a nucleic acid amplification reaction. An exemplary method for depositing cap/vial assembly 480 into receptacle holder 4010 is described in U.S. Published Patent Application No. 2014/0038192. In step S858, an incubation process is performed on the reaction mixture of cap/vial assembly 480. The incubation process may include thermal cycling, such as the thermal cycling associated with a PCR reaction. In some embodiments, the thermal cycling may comprise multiple temperature cycles, where the temperatures may vary, for example, between (i) about 94°C to about 98°C to facilitate for denaturation or melting double-stranded DNA target molecules, (ii) about 50°C to about 65°C for primers to anneal to the resulting single-stranded DNA templates, and (iii) about 70°C to about 80°C, depending on the DNA polymerase, to all for extension of the primers and synthesis of new DNA strands complementary to the DNA templates. In step S860, the contents of vial 464 may be monitored, for example, by fluorescence monitoring. In some embodiments, fluorescence monitoring may be performed during amplification (real-time amplification), while in other embodiments, fluorescence monitoring or some other form of detection may be carried out following amplification (end-point amplification). Fluorescence monitoring may be used to detect the presence (or absence) of one or more analytes in the contents of vial 464 based on the detection of one or more associated wavelengths (e.g., colored wavelengths) of electromagnetic signals emitted by the vial 464 contents using a signal detector 4020 (see FIGs. 16I, 17A, 17B), such as a fluorometer. In embodiments where monitoring is carried out during amplification, signal detector 4020 may be coupled to thermal cycler 432. In some embodiments, during amplification, periodic fluorescence intensity measurements at different wavelengths may be made at regular intervals to generate fluorescence time series data for later processing and analysis. In step S862, after monitoring, the samples may be discarded or stored. That is, following steps S858 and S860, vial transfer arm 418 may retrieve cap/vial 480 assembly from thermal cycler 432 and dispose of it in the trash chute 428 or the transfer cap/vial assembly 480 to a storage tray 452 in compartment 590.

In some embodiments, analytical system 1000 may be used to perform two or more assays (that include nucleic acid amplification reactions) that require differently constituted reagents (e.g., different unit-dose reagents, reagents with different constituents, etc.) and/or different solvents. FIG. 32 illustrates an exemplary process 870 of using analytical system 1000 to perform different assays on samples (the same sample or different samples). At step S872, a plurality of samples are loaded into analytical system 1000. One or more of the samples (e.g., a first subset) may be designated for one assay (a first assay), and one or more of the samples (e.g., a second subset) may be designated for a different assay (a second assay). In general, the first and second subset of samples may be portions of the same sample or portions of different samples. That is, the two different assays may be performed on aliquots of the same sample (e.g., sample contained in a single receptacle 107, see FIG. 4B) or on different samples (e.g., samples contained in different receptacles 107). If the first and second subsets of samples are contained in different receptacles 107, they may be loaded into system 1000 at the same time (e.g., before beginning either the first or the second assay) or at different times. In some embodiments, the second subset of samples (e.g., configured for an LDT) may be loaded on system 1000 after the first subset (e.g., configured for an IVD assay) is loaded. For example, in some embodiments, the second subset of samples (e.g., configured for an LDT) may be loaded on system 1000 after the first assay (e.g., IVD assay) has already begun (e.g., during or after the reaction mixture preparation process (see FIG. 30)).

In general, system 1000 is configured to process samples in the order in which they are received onto the system 1000, regardless of the types of assays to be performed on the samples. This is in contrast to batch-mode systems, where samples are grouped together based on assay type, and then batch processed together. System 1000 is capable of simultaneously performing assays requiring different reagents and/or conditions, including both IVD assays and LDTs, based solely on the order in which the samples are loaded onto system 10 (samples loaded together on system 1000 can be processed in any order). In some embodiments, system 1000 may even allow subsequently loaded samples to be processed out of order and, as a consequence, more quickly than previously loaded samples. In this embodiment, the processing of a first, earlier loaded sample may be interrupted at some stage of the processing to permit processing of a second, later loaded sample to be completed before or at the same time as the first sample.

In some embodiments, system 1000 may recognize the type of assay to be performed based on indicators (e.g., barcodes) provided on the sample receptacles and/or by information entered into the system (e.g., using a user-interface 50 of system 10) by the user. In some embodiments, the first assay may include an IVD assay using a first unit-dose reagent stored in system 1000. The second assay may include an LDT using a second unit-dose reagent (different from the first unit-dose reagent) stored in system 1000. Each of the first and second assays may include a temporal workflow schedule associated with the respective assay and may be performed in accordance with the steps described with reference to FIGs. 29-31. In some embodiments, at step S874, analytical system 1000 coordinates the schedule for performing the first assay and the second assay such that use of resources is optimized. For example, the first and second assays may require use of some of the same resources (e.g., fluid transfer devices, centrifuge 588, incubators (112, 114, 116), thermal cycler 432, etc.) of system 1000. To increase efficiency (e.g., increase throughput, minimize processing time, etc.), system 1000 may manipulate (shift, rearrange, etc.) the schedules of the two assays such that both the assays can use these resources in an efficient manner.

At step S876, analytical system 1000 performs the first assay on the first sample subset. In an exemplary embodiment, the first assay may be performed using a first unit-dose reagent 768 that includes constituents such as, for example, amplification oligomers, probes, a polymerase, dNTPs, etc. And, while reconstituting this reagent 768 in step S838 (of FIG. 30), a reconstitution buffer 1670 (contained in a solvent container 1620 of reagent container compartment 500) that does not include these constituents may be used. At step S878, system 1000 performs the second assay on the second sample subset. In some exemplary embodiments, the second assay may use a second reagent 768 that does not include at least some of these constituents, such as amplification oligomers and probes. And, while reconstituting the second reagent 768 in step S838 (of FIG. 30), the second assay may use a reconstitution fluid 1970A, 1970B (contained in solvent container 1920 stored in container compartment 500 or in a different compartment) that includes these constituents. In some embodiments, first and second reagents 768 may be provided in different reagent packs 760. However, in some embodiments, both the first and the second reagents 768 may be provided in a single reagent pack 760 (for example, different mixing wells 762 of a single reagent pack 760).

Accordingly, system 1000, which stores and provides operative access to the first unit-dose reagent used in the first assay and the second unit-dose reagent used in the second assay, performs both steps S876 and S878. In some embodiments, steps S876 and S878 may be performed without additional equipment preparation (for example, wiping down the equipment of system 1000), reagent preparation (replacing reagent bottles stored in system 1000), consumable preparation (replacing empty tip trays), etc. In some embodiments, step S878 starts while step S876 is being performed. That is, analytical system 1000 simultaneously performs the first assay and the second assay. In some embodiments, during steps S876 and S878, system 1000 verifies whether reagent packs 760 containing the required reagents 768 are positioned at one of loading stations 640. If not, the distributor system replaces a reagent pack 760 located at loading station 640 with a reagent pack 760 that contains a reagent 768 needed for the requested assay. In some embodiments, step S878 starts after step S876 is completed. And in some embodiments, although step S878 starts after step S876, step S878 may be completed before step S876 is completed. In some embodiments, system 1000 may alternate between steps S876 and S878. For example, analytical system 1000 may perform the first assay on one or more samples of the first sample subset, and then perform the second assay on one or more samples of the second sample subset. System 1000 may then switch back to step S876 and perform the first assay on one or more additional samples of the first sample subset. In some embodiments, system 1000 may be configured to modify the schedule of assays. For example, the samples (e.g., aliquots of the same or different samples) for the first assay (i.e., step S876) may have been previously loaded on system 1000 and analysis initiated. To accommodate, for example, an urgent request to perform a different assay (e.g., second assay, step S878) on a sample (the same sample on which the first assay is being performed or a different sample), the schedule of the assays may be modified to prioritize the second assay over the first assay. In embodiments, where the sample for the second assay has not already been loaded into system 1000, a receptacle 107 containing the sample may be loaded into system 1000. The reprioritized schedule may include, for example, performing the second assay in a more prioritized manner than the first assay, rearranging the schedule of the assays such that the second assay is not delayed because of the first assay, etc.

### Hardware and Software

Aspects of the disclosure are implemented via control and computing hardware components, user-created software, data input components, and data output components. Hardware components include computing and control modules (e.g., system controller(s)), such as microprocessors and computers, configured to effect computational and/or control steps by receiving one or more input values, executing one or more algorithms stored on non-transitory machine-readable media (e.g., software) that provide instruction for manipulating or otherwise acting on the input values, and output one or more output values. Such outputs may be displayed or otherwise indicated to a user for providing information to the user, for example information as to the status of the instrument or a process being performed thereby, or such outputs may comprise inputs to other processes and/or control algorithms. Data input components comprise elements by which data is input for use by the control and computing hardware components. Such data inputs may comprise positions sensors, motor encoders, as well as manual input elements, such as graphic user interfaces, keyboards, touch screens, microphones, switches, manually-operated scanners, voice-activated input, etc. Data output components may comprise hard drives or other storage media, graphic user interfaces, monitors, printers, indicator lights, or audible signal elements (e.g., buzzer, horn, bell, etc.). Software comprises instructions stored on non-transitory computer-readable media which, when executed by the control and computing hardware, cause the control and computing hardware to perform one or more automated or semi-automated processes.

In some embodiments, system 1000 may include a control system including a computer-controlled controller 5000 (schematically represented in FIG. 33). Controller 5000 may be a control system or computer connected to system 1000 or may include computer components integrated with system 1000. These computer components may include one or more microprocessors, displays, keyboards (and/or other user input devices), memory components, printer(s), etc. Controller 5000 may be configured to receive inputs from a user (e.g., user-inputs), inputs (e.g., identification information from barcode readers, etc.) from samples (e.g., receptacles 107 and sample-holding racks 10, etc., see FIGs. 3B and 3C), reagent packs 760, reagent container carriers 1600, reagent containers 1620, 1920, etc., and manage the performance of the assays on system 1000. Controller 5000 may include software algorithms that enable a user to enter user-defined assay parameters related to an assay (e.g., LDT) into system 1000, schedule different assays on system 1000 (e.g., associate samples with assays and schedule the time when the different steps of the assays are to be performed, etc.), and cause control system 1000 to perform the different steps associated with the assays, monitor the performance of the assays, and output results (on display, printout, etc.) for the user. Controller 5000 may send instructions to different devices of system 1000 to perform different steps associated with the assay (e.g., the steps associated with FIGs. 26-32). For example, controller 5000 may send instructions to pipettor 410 (e.g., motors, etc. associated with pipettor 410) to pick up a disposable tip 584 from a disposable tip tray 582 from one of tip compartments 580 to perform step S832 of FIG. 30. And, to perform step S834 (of FIG. 30), controller 5000 may send instructions to pipettor 410 to transfer a sufficient amount of oil (e.g., about 15 µL) from oil container 1820 to one or more processing vials 464 held in cap and vial trays 460, etc. It should be noted that the devices of system 1000 that controller 5000 sends instructions to may include any of the previously-described devices of system 1000 or devices that are a combination or modification of the previously described devices. Since such combinations and modifications are well known to people skilled in the art, they are not expressly described herein. Controller 5000 may also be configured to reprioritize a previously determined order of assays (e.g., to perform a different assay on subsequently loaded samples before or while performing another assay on previously loaded samples).

### Assay Protocol Definition

A nucleic acid amplification assay is performed by system 1000 in accordance with different parameters that define the assay (i.e., the assay protocol). In general, these parameters comprise computer-executable instructions that, when executed by the controller 5000, results in steps performed by system 1000 during the assay (e.g., the types and quantities of reagents to be used, incubation conditions, temperature cycling parameters (e.g., cycle times, temperatures, including denaturation, annealing and extension temperatures, selection of an RNA or DNA target, etc.), etc.). These parameters also include computer-executable steps that define data processing, data reduction, and result interpretation steps for the data generated by the protocols, where such steps may be performed by the controller 5000 or in whole or in part by a computer that is remote from the controller 5000 and system 1000.

Since IVD assays are known, standardized (and regulated) assays, their parameters are typically known and/or fixed and cannot be changed by a user. In some embodiments, the parameters for IVD assays may be preinstalled/preloaded (e.g., preprogrammed) on system 1000. IVD assays may be stored as executable files in a database known as a protocol library (e.g., a list of assay protocols stored in the software tool). Certain assay parameters that are common to all assays may not be store in individual IVD assay files in a protocol library but may be "hardwired" in the controller 5000 of the system 1000.

Since LDTs are developed or established by a user or a third party, however, at least some of the parameters that define LDTs are provided by the user/third party. Instruments configured to perform IVD assays are pre-programmed by the instrument manufacturer to perform the IVD assay protocols. To enable the instrument to perform a user-defined LDT, the instrument controller must be reprogrammed to also include the LDT protocol. In various embodiments, the controller 5000 is configured to enable the user to define (or modify) and store an LDT protocol by selecting user-defined assay parameters associated with the assay. Thus, the system is configurable by the user to perform non-pre-programmed protocols, such as LDT protocols. This not only enables the user to program new, previously unused protocols, but also to modify existing protocols without requiring the instrument manufacturer or provider to reprogram the instrument.

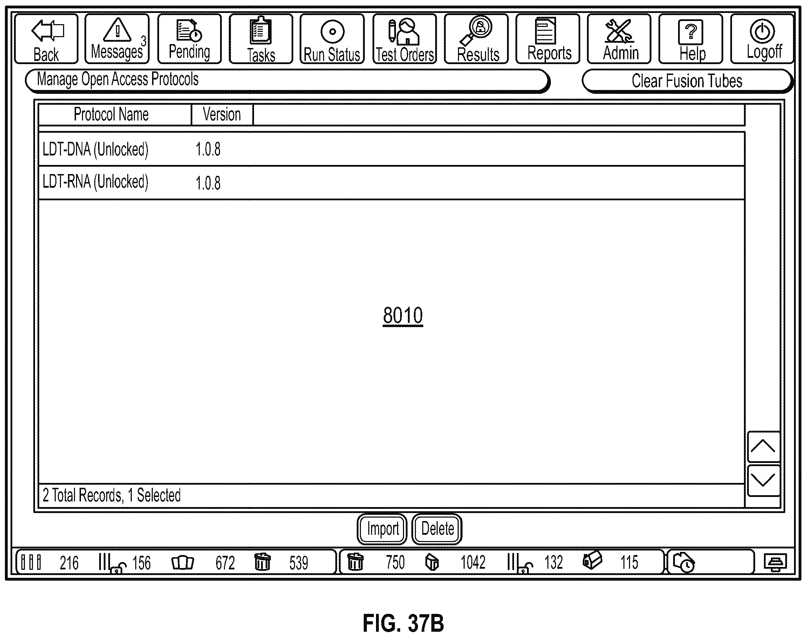

As will be described in more detail later, after an LDT is run or performed by system 1000 and a data set is obtained, controller 5000 may enable the user to process the data and review the results of the assay. Controller 5000 may also enable the user to modify at least some of the user-defined assay parameters, rerun the data set using the modified user-defined assay parameters, and re-review the results to study the effect of the selected user-defined assay parameters on the assay results. Thus, in some embodiments, controller 5000 may enable a user to determine an optimized set of user-defined assay parameters (e.g., a set of user-defined assay parameters that produces the results approved by the user) for performing the LDT. Controller 5000 may then allow a user to associate the optimized user-defined assay parameters to the created (or established) LDT protocol and finalize and lock the parameters (e.g., so that they are not inadvertently changed) for the developed LDT. In some embodiments, locking the protocol may enable system 1000 to report assay results to a laboratory information management system (or LIS). It should be noted that even if a protocol is locked, it may be unlocked and modified in the software tool described in more detail below. If a locked protocol is modified within the software tool, it will automatically be unlocked, and the user would need to select the Lock feature to relock it. System 1000 identifies all unlocked protocols as "Unlocked" and all locked protocols as "Locked" on display device 50 (see open access protocol screen 8010 of FIG. 37B).

In some embodiments, the software tool comprises software algorithms in system 1000 (e.g., loaded on controllers or other computer systems of system 1000) enable a user to define or establish an LDT protocol using user-defined assay parameters. In various embodiments, the software tool provides a system enabling a user to specify user-defined assay parameters of an assay protocol for processing a sample suspected of containing a targeted analyte, the user-defined assay parameters comprising computer-executable instructions causing the computer-controlled, automated analyzer (e.g., system 1000 controlled by controller 5000) to perform an assay in accordance with the assay protocol created by the system.

In some embodiments, these algorithms may be run on a computer system remote from system 1000 to define an LDT using user-defined assay parameters, and an output file produced by the computer system may be installed in system 1000. In some embodiments, the user developed LDTs (locked or unlocked) may be transferred to system 1000 via a wired connection or transported to system 1000 in a portable memory device (e.g., USB drive, memory stick, etc.). An exemplary software interface (hereinafter referred to as "software tool") that may be used to define or modify an LDT protocol will now be described. It should be noted that the described software tool is only exemplary and many variations are possible and are within the scope of this disclosure. As explained above, in general the software tool may be installed and run on system 1000 (e.g., via display device 50 of system 1000), or may be installed and run on a computer system remote from system 1000. For example, in some embodiments, the software tool may be installed and run on a desktop or a laptop computer to create an assay protocol with user-defined assay parameters and settings that are then installed on system 1000. In various embodiments, the assay protocol created by the software tool includes both user-defined (or user-adjustable) parameters and non-user-defined (non-user-adjustable) parameters. After running the assay on system 1000, the raw data produced by system 1000 (e.g., during the assay) may then be transferred to the computer system (e.g., the remote computer system), and the raw data processed on the computer system using data analysis parameters to produce amplification curves. The data analysis parameters used by the computer system includes both user-defined (or user-adjustable) parameters and non-user-defined (non- user-adjustable) parameters.

As described above, the software tool is capable of generating computer-executable assay protocols for system 1000. Each assay may be defined in an Assay Definition File (ADF), which may include information that describes how to process results, what process steps are executed, the order they are executed, interpretations generated, etc. The protocol for an LDT may use a series of mathematical calculations and tests that determine the emergence cycle of a signal (e.g., fluorescent signal) above the background signal from a real-time detector (e.g., fluorometer) during a polymerase chain reaction (PCR) amplification. Real-Time PCR monitors the amplification of a targeted analyte (i.e., DNA or RNA) in real-time. In some embodiments, PCR is carried out in thermal cycler 432 with fluorescence detection capability. A targeted analyte of the sample will be amplified during PCR and generate a fluorescent signal, which may be recorded in relative fluorescence unit (RFU) readings. This recorded data is processed in a series of steps (sometimes referred to as the TCycle (or Ct) Algorithm) in order to determine the targeted analyte status in the original sample (e.g., valid, invalid, positive, negative and/or concentration). An exemplary TCycle (or Ct) algorithm is described infra. A cycle refers to one round of a thermal processing reaction in a thermal cycler (e.g., thermal cycler 432). Typically, a PCR reaction goes through multiple cycles (e.g., 35-50 cycles, 35-45 cycles, 40-50 cycles, etc.). Multiple fluorescence measurements per detection channel may be taken within each cycle. Ct is the number of cycles before which the analyte specific signal has reached a preset threshold limit during the amplification (also called emergence cycle).

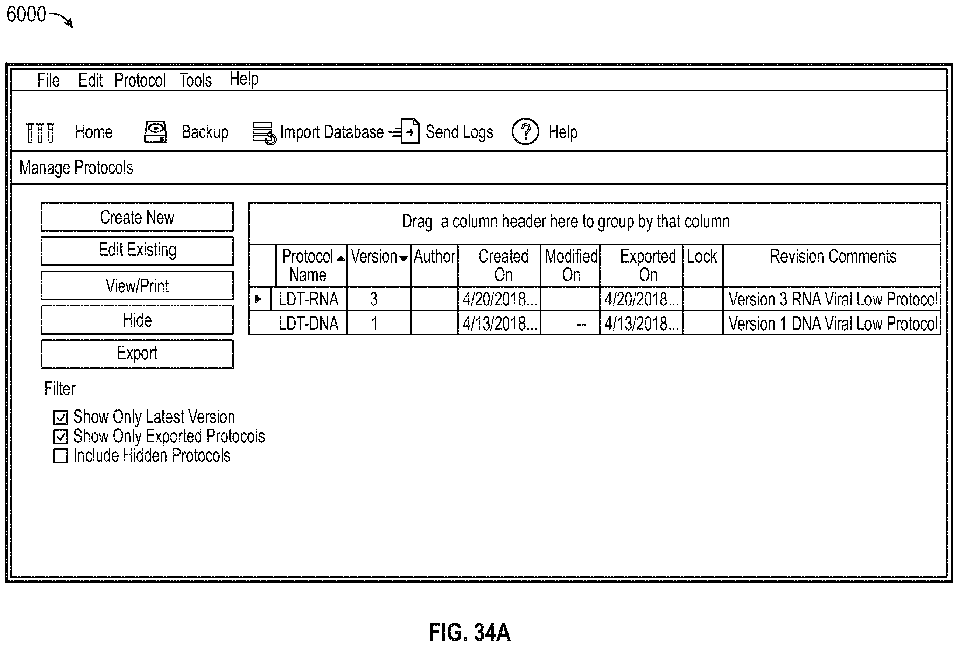

The software tool enables a user to develop and define an LDT protocol via one or more windows, screens, or GUIs that include interactive buttons, menus, and/or icons that provide access to different functions and information. When run or launched by a user, the software tool may open to a manage protocol screen which displays the protocol library. FIG. 34A illustrates an exemplary manage protocol screen (GUI) 6000 of the software tool. The manage protocol screen 6000 may enable a user to create, edit, view/print, and export assay protocols. A list of available assay protocols is displayed in the manage protocol screen 6000. By selecting various selection criteria in the "Filter," a list of protocols satisfying the chosen selection criteria is displayed on the manage protocol screen 6000. Selecting the "Edit Existing" icon, or double clicking on the protocol name, enables a user to open and edit an existing protocol. Selecting the "View/Print" icon after selecting a displayed protocol displays details of the selected protocol in readable and printable format and selecting "Export" saves the selected protocol in a file (e.g., a pdf file). The "Hide" icon hides the selected protocol to make it unavailable for edits. When "Hide" is selected, the icon may be changed to "Unhide." Selecting "Unhide" makes the hidden protocol available for edits. As will be described later, selecting "Export" exports the selected protocol (e.g., to transfer to system 1000). Selecting the "Create New" icon may display a series of screens that enable a user to define a new protocol by selecting or defining parameters of the protocol, such as the name, extraction type, targets, thermal profile, results processing parameters, result interpretation parameters, protocol status, LIS reporting, export, etc.

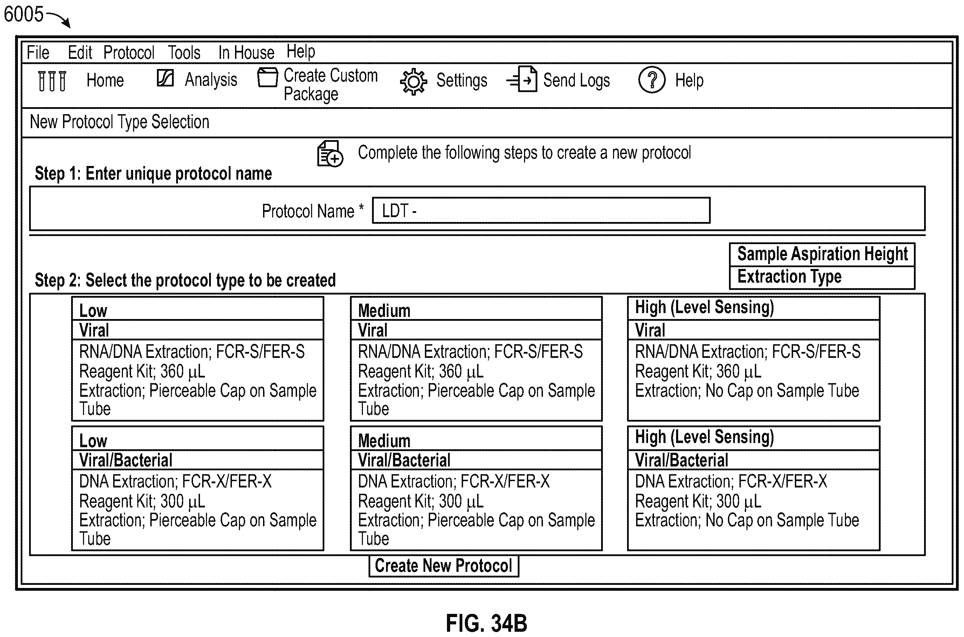

In some embodiments, selecting the "Create New" icon may display a new protocol type selection screen 6005. FIG. 34B illustrates an exemplary new protocol type selection screen (GUI) 6005 of the software tool. The new protocol type selection screen 6005 allows the user to enter a protocol name in the "Protocol Name" field. The entered name may be used to identify the defined assay in the software tool (and system 1000 after it is installed in system 1000). In some embodiments, there may be limitations (e.g., the name must be unique, number of characters in the name must be ≤ 11, etc.) that restrict the type of name that can be assigned to the assay. In some embodiments, a prefix (e.g., "LDT-") may be added to the name to identify the assay as an LDT. The new protocol type selection screen 6005 may then prompt the user to select the protocol type by selecting the appropriate extraction type (i.e., extraction process, such as, for example, the extraction process depicted in FIG. 26) and sample aspiration height from the presented options. "Viral" and "Viral/Bacterial" in the selectable extraction types are exemplary designations referring to the extraction reagent kit (i.e., the extraction reagents (e.g., target capture reagents) to be used) and pre-programmed workflow (e.g., as shown in FIG. 26) to be used in the extraction process. A selected "extraction parameter" may include one or more parameters relating to the process of extracting a targeted analyte from the sample material, including one or more of the extraction type (e.g., "Viral" or "Viral/Bacterial"), type of material to be extracted (e.g., RNA/DNA or DNA), specification of extraction reagent(s) (kit) (e.g., FCR-S/FER-S or FCR-X/FER-X), volume of sample fluid to be withdrawn from a sample container (e.g., 360 µL or 300 µL), and sample aspiration height (low, medium, or high). In an embodiment, as illustrated in FIG. 34B, the extraction parameter may comprise a selected pre-defined combination of the foregoing parameters. Typically, some or all of the following factors may be considered when selecting a desired extraction type: whether the assay is a viral or a bacterial assay; whether a sample is difficult to lyse; whether the sample is expected to include particulates; and whether the sample tube includes a penetrable cap. "Low," "Medium," and "High" in the new protocol type selection screen 6005 refer to the height of the sample to be aspirated from a sample tube. The sample aspiration height may be dependent on the sample matrix. Samples with sediment, such as stool samples, may need a "Medium" or a "High" setting, for example, to avoid clogging system 1000 with particulate matter that may accumulate near the bottom of the sample tube. Thus, the new protocol type selection screen (GUI) 6005 of the software tool enables a user to select one or more extraction parameters to control operation of the system 1000 to extract (isolate and purify) the targeted analyte from the sample material in a manner most compatible with the LDT. Selecting the "Create New Protocol" button or icon after selecting the desired extraction type, may launch a protocol identification screen 6010.

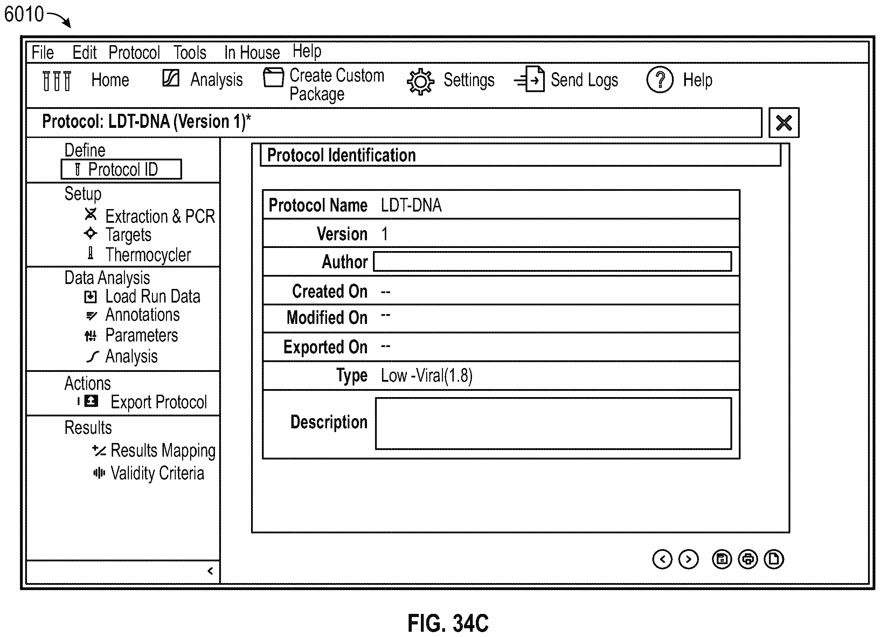

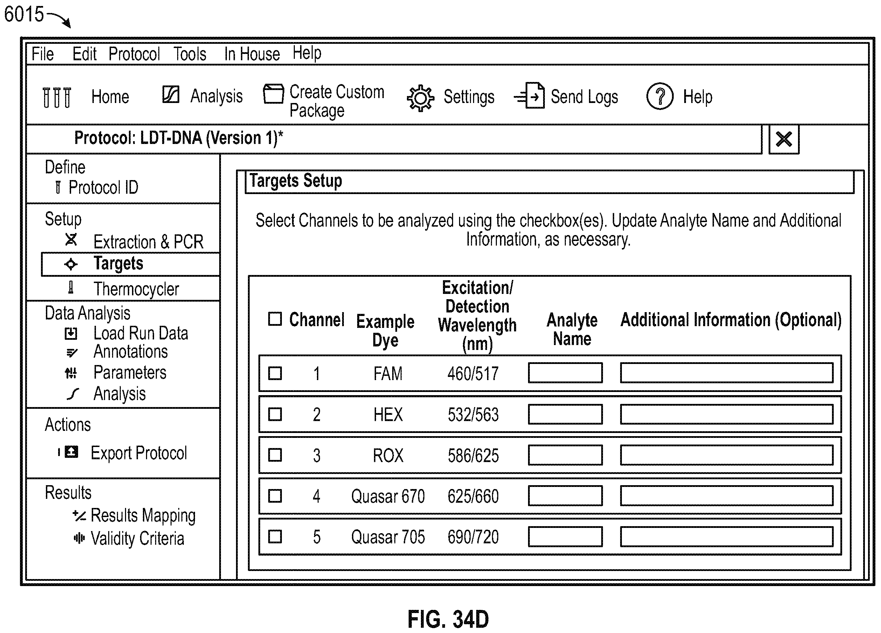

FIG. 34C illustrates an exemplary protocol identification screen (GUI) 6010 of the software tool. The protocol identification screen 6010 allows the user to enter the author name and other optional identification information. Selecting the "Extraction & PCR" button from the navigation pane under "Setup" may launch a screen (not shown) with pre-populated fields with extraction details for the extraction type selected in the new protocol type selection screen 6005. Selecting the "Targets" button from the "Setup" navigation pane may launch the target setup screen 6015 that enables the user to define a target parameter specifying one or more targeted analytes to be detected in a given channel of a multi-channel signal detector of the system 1000 during execution of the protocol. In this context "channel" refers to an element of a signal detector - or a different signal detector - that is configured to detect a unique signal that may be associated with a detection probe indicating the presence of a particular analyte. For example, for a signal detector comprising a fluorometer, such as fluorometer 4030 described above, the term "channel" refers to unique fluorescent colors that are excited and detected by each fluorometer. FIG. 34D illustrates an exemplary target setup screen (GUI) 6015 of the software tool. In an embodiment, the software tool may allow up to five channels to be selected using the target setup screen 6015. These selected channels and target names may also be edited after creating the protocol. In a system 1000 employing a multi-channel fluorometer, the user may use the target setup screen 6015 to select the fluorescence channel(s) to be used with the protocol. Exemplary detection wavelength ranges and dye names may be provided for each channel. The wavelength ranges of the selected channel for a given targeted analyte will correspond to the dye used in the detection probes of the reconstitution fluid of the LDT for which the assay protocol is being developed. Each channel may be individually selected by selecting the associated box on the left of the channel number, or all the channels may be automatically selected or deselected by selecting the check box on the top left corner of the channel window. The user may enter the analyte name for each selected channel in the "Analyte Name" field. The entered analyte names may be associated with results from these channels on exports and reports. In some embodiments, there may be restrictions (e.g., ≤ 10 characters long, start with a letter, etc.) on the names that may be entered in the "Analyte Name" field. The user may also optionally enter additional information related to each selected channel in the "Additional Information (Optional)" field. Thus, the target setup screen 6015 enables the user to control operation of the system 1000 to detect the detection probe used in the LDT, thereby customizing the protocol for detecting the targeted analyte for which the LDT was developed.

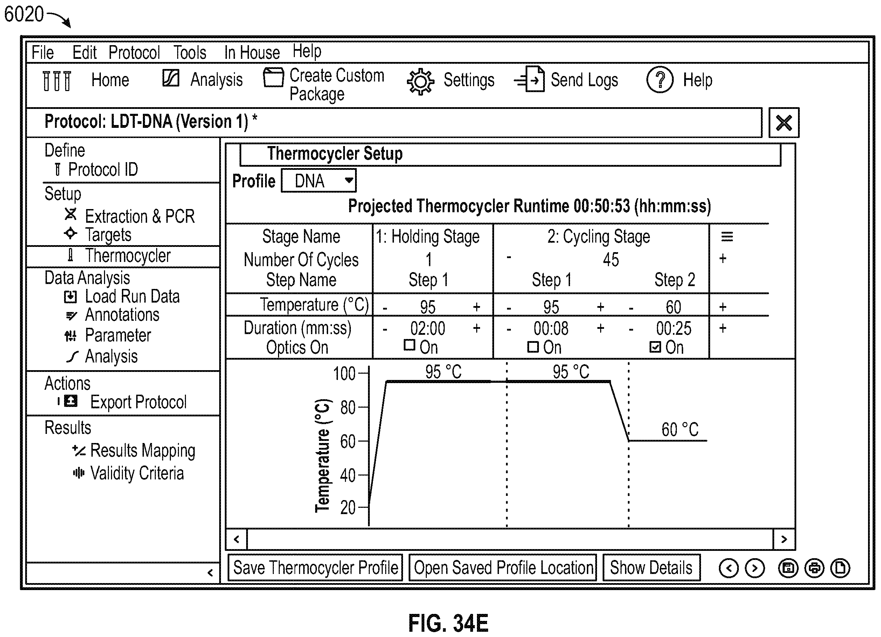

Selecting the "Thermocycler" button from the "Setup" navigation pane may launch the thermocycler setup screen 6020. FIG. 34E illustrates an exemplary thermocycler setup screen (GUI) 6020 of the software tool. Using the thermocycler setup screen 6020, the user may select a default thermal profile or create a custom thermal profile defining computer-executable instructions for controlling the thermal cycler 432 of system 1000. A default thermal profile may be selected or a custom profile entered using the "Profile" drop down menu. In some embodiments, using the "Profile" drop down menu, the user may select a default thermal profile from, for example, "DNA" and "RNA/DNA," or enter a custom profile by selecting "Custom." As illustrated in FIG. 34E, a main pane of the thermocycler setup screen 6020 includes boxes where thermal parameters, such as temperature, duration, number of cycles (i.e., the number of times the two or more temperature steps of the cycling stage are repeated), etc. can be entered (or selected) by the user to define a custom thermal profile - or combinations of such thermal parameters will be predefined if the user selects a default thermal profile. Adjusting the thermal cycle steps in a default thermal cycle profile may automatically force the thermal profile selection under "Profile" to "Custom." Selecting one of the default thermal profiles may return the selection to the selected default thermal profile. In general, any desired thermal profile may be defined by entering or selecting temperature and duration values in the boxes for "Temperature" and "Duration" in the main pane. In some embodiments, there may be limitations on the defined custom thermal profile. For example, in some embodiments, a defined custom thermal profile may need to follow some or all of the following rules: the total duration of a defined thermal profile must be less than or equal to 55 minutes; the thermal profile must have a minimum of 5 seconds for any step above 80°C; the thermal profile must not cool below 55°C after a heating step of greater than 70°C; the thermal profile must have a maximum of one step with optics on; the optics (in the step with options on) must be on for at least 13 seconds; etc. It should be noted that the above-described rules are only exemplary, and any type of rule may be implemented to optimize the use of the thermal cycler 432. In general, such rules are implemented in the software tool to achieve optimized ramp rates and preserve timing for interleaving the defined LDT protocols with IVD protocols. For example, these rules may allow samples that are subjected to different assays (IVD, LTD, etc.) to share the same zone of the thermal cycler 432 and thus maximize its use. Although the default custom thermal profile is a thermal profile having two steps ("Step 1" and "Step 2"), or a 2-step temperature profile, the user may select a different number of steps (e.g., a 3-step temperature cycle), as long as the rules (if any) of the software tool governing custom thermal profiles are satisfied. Thus, the thermocycler setup screen 6020 enables the user to control operation of the system 1000 - and, in particular, the thermocycler 432 - to implement thermal cycling within the protocol of the LDT that optimizes amplification of the targeted analyte. While the software tool has been described above as providing graphical user interfaces and associated user input functionality for defining multiple parameters of the LDT protocol, including extraction parameter(s), target parameter(s), and thermal parameter(s), it should be noted that the software tool, may, in alternate embodiments, enable user input for fewer parameters of the LDT protocol, while the other protocol parameters are pre-programmed, system-defined assay parameters. For example, a software tool may enable the user to specify only thermal parameter(s) while extraction parameter(s) and target parameter(s) are system defined.

After the parameters for defining the assay (e.g., parameters associated with "Extraction & PCR," "Targets," and/or "Thermocycler" in the "Setup" navigation pane (see FIG. 34E)) have been defined, parameters for data analysis may be defined using the software tool. In the software tool, data analysis may be performed by a data analysis computer - which may or may not be part of the remote computer executing the software tool and may or may not be remote from the controller 5000 of the system 1000 - executing a software module or algorithm that accepts as input raw data (e.g., data output by system 1000 after performing an LDT protocol defined using the software tool as described above). In an embodiment, the raw data includes fluorescence data (in RFU) recorded by the fluorometer of thermal cycler 432 versus cycle number per channel. The cycle number starts with cycle one and ends with the number of cycles defined in the thermal cycler file (e.g., 45 cycles, e.g., as defined using the thermocylcer setup screen (FIG. 34E)). The data analysis parameters define the type of data reduction and data processing that will be applied to the raw data.

In some embodiments, the raw data from system 1000 may first be validated and smoothed prior to the data analysis. That is, the raw data from system 1000 may first be validated (and, in some embodiments, the data reduced), and then smoothed to create smoothed raw data, and data analysis algorithms (using user-defined assay parameters) may then be applied to the smoothed raw data. The parameters for data analysis may be defined (or previously defined parameters reviewed) by selecting the "Parameters" tab from the "Data Analysis" pane of a displayed screen (see, e.g., protocol identification screen 6010, target setup screen 6015, thermocycler setup screen 6020, etc.) of the software tool. Selecting the "Parameters" tab may launch screens or windows (GUI) that enable the user to enter data analysis parameters to apply to the raw data (e.g., after validation and smoothing). In some embodiments, the data analysis parameters may include four sets of data analysis parameters - parameters associated with curve correction, parameters associated with positivity criteria of data, parameters associated with channel validity criteria, and parameters associated with sample validity criteria. In some embodiments, selecting the "Parameters" tab may launch a screen with four tabs for defining data analysis parameters, including, "Curve Correction," "Positivity Criteria," "Channel Validity Criteria," and "Sample Validity Criteria," that may be individually selected by the user to enter the corresponding sets of data analysis parameters.

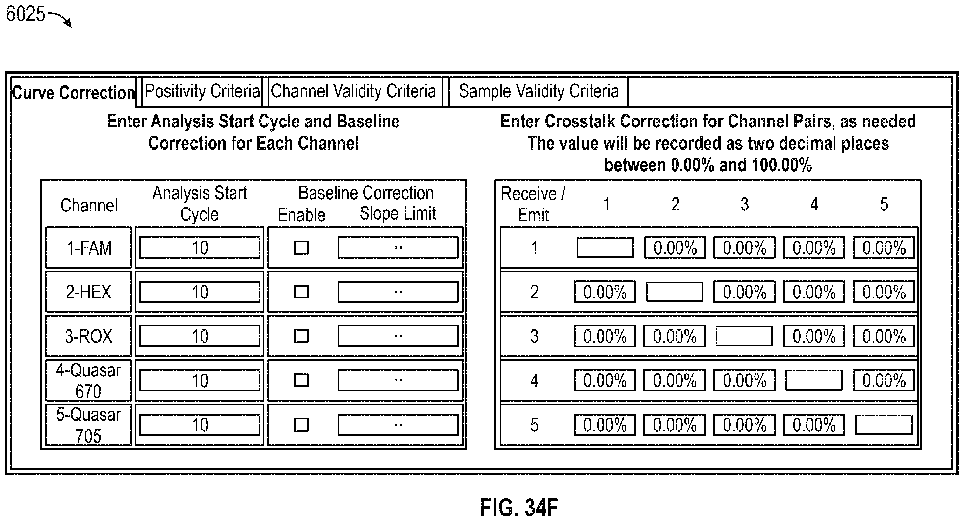

FIG. 34F illustrates an exemplary data analysis parameters screen (GUI) with the "Curve Correction" tab selected (referred to herein as the curve correction parameter screen 6025). In the illustrated embodiment, the curve correction parameter screen 6025 allows the user to define the number of cycles of each channel to remove from data analysis, to correct for ramping of baseline fluorescence, and to suppress channel to channel bleed through. Typically, data (even after smoothing) in the initial stages of an assay may include variability due to non-sample related noise or artifacts. To reduce the inaccuracies in the calculated Ct caused by this variability, it may be desirable to disregard or eliminate readings from the initial cycles of an assay. The user may select the number of cycles of each channel to disregard from the Ct calculation by entering values for "Analysis Start Cycle" for each channel of the multi-channel signal detector. In some embodiments, the user may be prompted (or provided with information) to enter a value within a predetermined range (e.g., between 8 and 12) for the "Analysis Start Cycle" for each channel. The predetermined range may indicate the number of initial cycles for each channel that are known to contain artifacts in the data (e.g., based on prior experience). Based on user input, the data analysis algorithm of the software tool may create a new data set (e.g., from the smoothed data set) by removing all data before the user-defined "Analysis Start Cycle" for each channel.

Before calculating Ct, it may be desirable to ensure that the curve (i.e., fluorescence curve of signal magnitude vs. time or cycle number defined by the data) begins from a point considered as having no fluorescence. In some amplification cases, baseline drifting (or ramping up) in the fluorescence curve is observed due to the poor quenching of fluorophores, especially at the end of the baseline cycles. Baseline drifting may have an adverse impact on the correct calculation of Ct (and/or differentiation between positive and negative results) when the drifted baseline creeps into the region of the fluorescence curve used for linear regression calculation of Ct. In such cases, correction of the drifted baseline may be required. The data analysis algorithm of the software tool may analyze the data to determine the level of general background florescence (for example as described infra) so that the determined background florescence may be subtracted from the measured data to shift the curve and thereby numerically correct for baseline florescence. The user may enable baseline correction for any channel by selecting "Enable" for the corresponding channel in the curve correction parameter screen 6025 of FIG. 34F. The user may also specify a slope limit for the baseline correction of an enabled channel by entering values corresponding to "Slope Limit." In some embodiments, the user may be prompted to enter a value within a predetermined range (e.g., between 0 and 100) for the "Slope Limit" for each channel based, for example, on prior experience. During data analysis, the algorithm will apply baseline correction to all changes in RFU or slopes (in the data) that are less than the user selected "Slope Limit" value selected for each channel. That is, if a value of 50 is selected by the user for channel 1, and the slope in the data (or a portion of the data) is 60, baseline correction is not applied. And, if the slope in the data (or a portion of the data) is 40, baseline correction is applied. If baseline correction is enabled, the algorithm may use a 4-parameter or a 5-parameter logistic regression model to calculate the baseline florescence and remove the calculated baseline value from the data.

The curve correction parameter screen 6025 also allows a user to suppress channel to channel bleed through (signal crosstalk) by selecting "Crosstalk Correction" values for each channel. These user selected values correct for any assay-specific florescence bleed-over between channels. Due to the overlap of spectra between some fluorophores, the fluorophore being excited in one channel may also be excited in a fraction of signals in an adjacent channel. Therefore, a signal bleed-through (or crosstalk) from the emitting channel to a receiving channel may be observed. That is, a probe emits florescence having a range of wavelengths (e.g., defined by a bell curve). And, some of these wavelengths may be detected by one channel and other wavelengths may be detected by another channel due to cause crosstalk. The crosstalk signal may potentially lead to false positive readings in the receiving channel. If crosstalk correction is enabled, based on the user-specified "Crosstalk correction" fraction between an emitting channel and a receiving channel, the software tool may minimize the amount of crosstalk between the channels in a numerical way. In some embodiments, the user may be prompted to enter a value within a predetermined range (e.g., between 0% and 3%) for "Crosstalk Correction" values based, for example, on prior experience. Thus, the curve correction screen 6025 enables the user to define computer-executable curve corrections to the data analysis performed by the system controller for data generated from an LDT without which corrections, data analysis of the raw LDT data (e.g., fluorescence data) may lead to inaccurate test results. Thus, the user is able to execute non-standard, non-preprogrammed assays on the system without requiring the assistance of the system manufacturer or provider to provide such data corrections.

The crosstalk correction parameters for channel pairs that are entered into the screen is 6025 can be empirically determined. For example, to determine the amount of bleed through at each of channels 2, 3, 4, and 5 while measuring a signal at channel 1, a reaction container with contents expected to give a positive result is measured with channel 1 and signals at each of channels 2, 3, 4, and 5 are measures. The signals measured at each of channels 2, 3, 4, and 5, which should be zero, indicate the amount of crosstalk correction that may be necessary or desired. Thus, for example, if the signal measured at channel 1 were 1000 RFU, and the signals measured at channels 2, 3, 4, and 5 (the bleed through or crosstalk signals) were 200 RFU, 100 RFU, 50 RFU, 10 RFU, and 0 RFU, respectively, the corresponding crosstalk correction parameters would be 20% (0.20), 10% (0.10), 5% (0.05), 1% (0.01), and 0% (0.00).

The parameters may be entered as percentages by which the signal measured at each channel is reduced when a different channel is being interrogated. In FIG. 34F, each of the percentages is 0.00, but one or more of the entries can be changed to non-zero percentages based on empirically-determined blead through. For example, in the first row, when an emission is being measured at channel 1, signals received (measured) at each of channels 2, 3, 4, and 5 can reduced by the empirically-determined bleed through or crosstalk percentages, while the signal at channel 1 is not reduced. In the second row, when an emission is being measured at channel 2, signals received (measured) at each of channels 1, 3, 4, and 5 can reduced by the empirically-determined bleed through or crosstalk percentages, while the signal at channel 3 is not reduced. In the third row, when an emission is being measured at channel 3, signals received (measured) at each of channels 1, 2, 4, and 5 can reduced by the empirically-determined bleed through or crosstalk percentages, while the signal at channel 3 is not reduced. In the fourth row, when an emission is being measured at channel 4, signals received (measured) at each of channels 1, 2, 3, and 5 can reduced by the empirically-determined bleed through or crosstalk percentages, while the signal at channel 4 is not reduced. And, in the fifth row, when an emission is being measured at channel 5, signals received (measured) at each of channels 1, 2, 3, and 4 can be reduced by the empirically-determined bleed through or crosstalk percentages, while the signal at channel 1 is not reduced.

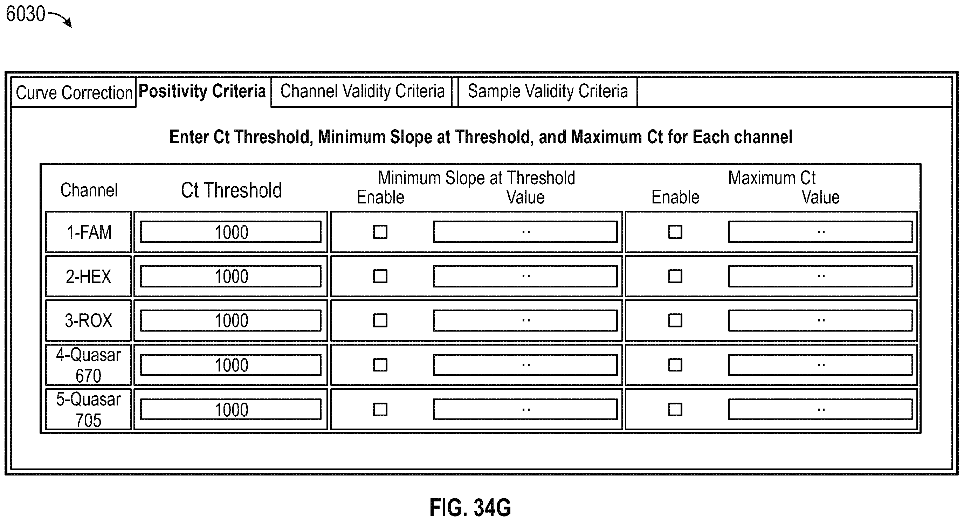

After selecting the user-defined assay parameters associated with curve correction in the curve correction parameter screen 6025, the user may select the "Positivity Criteria" tab to access positivity criteria parameter screen 6030. FIG. 34G illustrates an exemplary positivity criteria parameter screen (GUI) 6030 of the software tool. In the positivity criteria parameter screen 6030, the user may select a Ct threshold for each fluorescence channel by entering a value for "Ct Threshold" for each channel. Software determines Ct (or TCycle) as the cycle number at which the measured fluorescence signal in a channel intersects the Ct threshold value. If the detected fluorescence in a channel is greater than the user-defined Ct threshold value, a positive result may be indicated, and if the detected fluorescence is less than the Ct threshold value, a negative result may be indicated. A positive result indicates that an analyte is present in the sample and a negative result indicates that the analyte is not present in the sample. Typically, Ct threshold values are channel and assay specific (i.e., Ct threshold values vary with assay and channel). In general, a Ct threshold may have any value. Typical Ct threshold values for various assays may be between 100 and 1000 RFUs. In some embodiments, the software tool may prompt the user to enter a value for "Ct Threshold" within this range. In some embodiments, the suggested range for Ct threshold for each channel may be provided in another manner (e.g., help window, user manuals, publications, etc.).

In addition to "Ct Threshold" for each channel, the positivity criteria parameter screen 6030 also lets the user input parameters related to evaluation criteria used to determine if an observed positive result is a truly a positive result or an artifact. These result evaluation parameters include "Minimum Slope at Threshold," and "Maximum Ct." The user may enable either or both of these evaluation criteria by selecting "Enable" associated with the respective criteria. "Minimum Slope at Threshold" defines the minimum slope (of the curve) required at the user-defined "Ct Threshold" for a positive result. That is, even if the measured data indicates that the "Ct Threshold" for a channel has been exceeded, if the slope of the curve at the Ct threshold is not greater than or equal to the user-defined "Minimum Slope at Threshold," a negative result is indicated. "Maximum Ct" defines the maximum allowable Ct for a positive result. That is, if the observed Ct (i.e., number of cycles before the RFU curve reaches the Threshold) is greater than or equal to the user-defined "Maximum Ct" value, a negative result is indicated because the observed result may be an artifact due to contamination and/or other reasons (e.g., nonspecific activity of primer /probes with other regions or organisms present in the sample), etc. Suitable values for "Minimum Slope at Threshold" and "Maximum Ct" may be specific to the assay. In some embodiments, suitable values for the "Minimum Slope at Threshold" may be between 0 and 200. In some embodiments, the software tool may prompt the user with suggested values for these parameters based on other parameters. In some embodiments, the suggested values for each channel may be provided in another manner (e.g., help window, user manuals, advice from support personnel, etc.) or may be derived by the user, for example, using previously reported data (e.g., previously reported slope at S_{threshold}). Thus, the positivity criteria parameter screen 6030 enables the user to define computer-executable criteria for determining a positive result applied by the system controller - e.g., a data interpretation computer, which may or may not be part of the remote computer executing the software tool and may or may not be remote from the controller 5000 of the system 1000 - for data generated from an LDT. Thus, the user is able to execute non-standard, non-preprogrammed assays on the system without requiring the assistance of the system manufacturer or provider to program criteria into the system for determining positive and negative results for the non-standard assay.

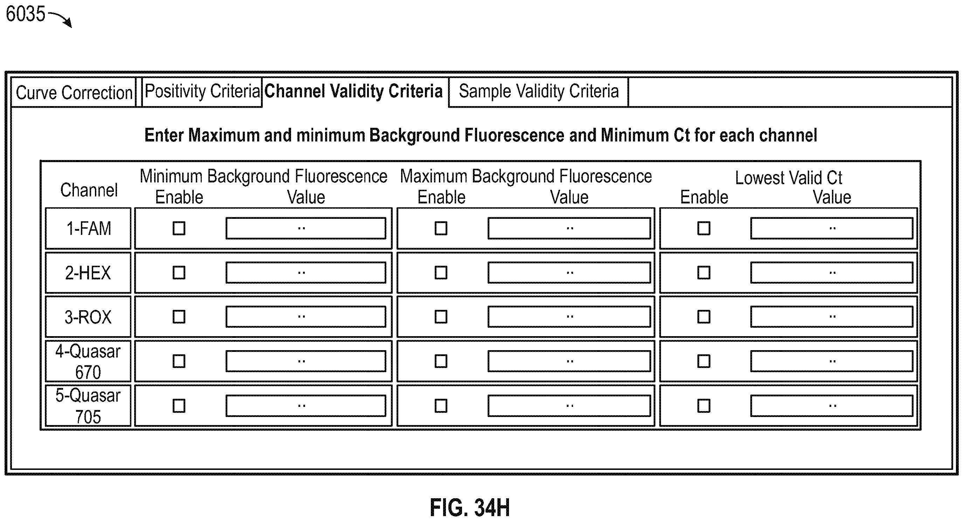

The user may select the "Channel Validity Criteria" tab to access the channel validity criteria parameter screen 6035. FIG. 34H illustrates an exemplary channel validity criteria parameter screen (GUI) 6035 of the software tool. In the channel validity criteria parameter screen 6035, the user can enable different validity tests that may be used to flag errors related to assay specific components (primer, probes, reagents, etc.). These validity tests may be used by a sample validity evaluation computer - which may or may not be part of the data analysis computer or the remote computer executing the software tool and may or may not be remote from the controller 5000 of the system 1000 - executing the data analysis algorithm to determine if the observed fluorescence values are within an expected range. The user may use these tests to confirm proper formulation of the user provided reagent (e.g., probe/primer reagent) and of the PCR reaction. In some embodiments, as illustrated in FIG. 34H, the channel validity criteria parameter screen 6035 allows the user to enable tests for "Minimum Background Fluorescence," "Maximum Background Fluorescence," and "Lowest Valid Ct" by selecting the "Enable" button corresponding to each test. The user may enable "Minimum Background Fluorescence" and enter a desired minimum value for the fluorescence. The user may also enable "Maximum Background Fluorescence," and enter the desired maximum value for the observed fluorescence. These parameters enable the software tool to check for proper formulation of the user provided reagent, proper master mix addition to the PCR vial, and proper functioning of fluorescence detection. Background Fluorescence is channel and assay-specific. Typical "Minimum Background Fluorescence" values are between 500 and 15,000 RFUs, and typical "Maximum Background Fluorescence" values are between 1000 and 30,000 RFUs with the maximum allowable value being 50,000 RFUs. The user may also enable "Lowest Valid Ct" per analyte and specify a Ct value. If enabled, the software tool will invalidate the PCR curve if an analyte has a Ct value less than or equal to the user-specified "Lowest Valid Ct" value. Thus, the channel validity criteria parameter screen 6035 enables the user to define computer-executable criteria for determining if signals measured by the multi-channel signal detector are within expected ranges. Thus, the user is able to program the system to assess the validity of signals measured while executing non-standard, non-preprogrammed assays on the system and without requiring the assistance of the system manufacturer or provider to program criteria into the system for assessing the validity of measured signals.

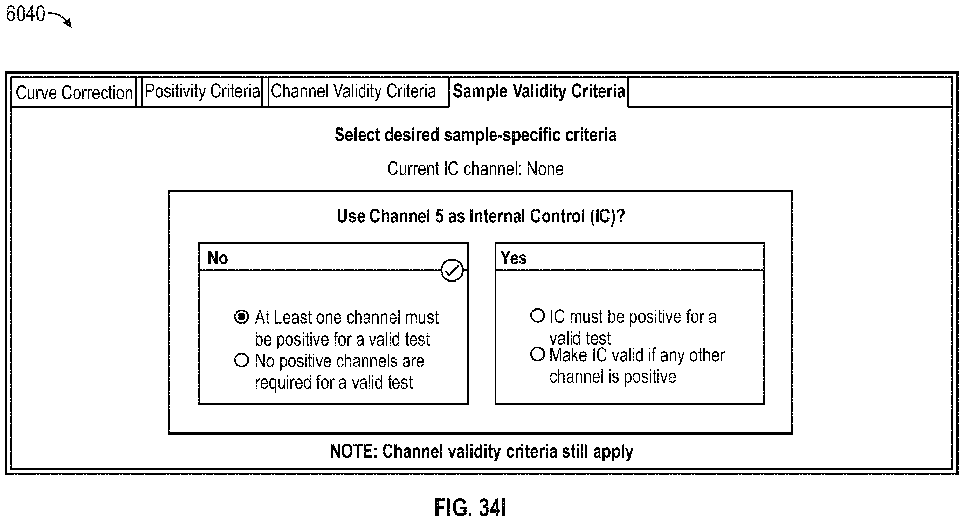

Selecting the "Sample Validity Criteria" tab may launch the sample validity criteria parameter screen 6040 of the software tool. FIG. 34I illustrates an exemplary sample validity criteria parameter screen (GUI) 6040 of the software tool. In the illustrated embodiment, the sample validity criteria parameter screen 6040 allows the user to denote that channel 5 of system 1000 is an internal control (IC). An internal control is an agent that is included in a reaction mixture to confirm the presence or absence of an analyte. Detection of the internal control typically serves to validate assay process steps. In the context of a nucleic acid amplification assay, an internal control is a nucleic acid template that should be co-amplified and detected with the nucleic acid analyte, provided the analyte is present in the sample. Detection of internal control amplification products at an appropriate level confirms success of the extraction and amplification process steps. If channel 5 includes an internal control, the user may select the "Yes" box in the sample validity criteria parameter screen 6040 and specify whether a valid result requires the internal control to be positive or if the internal control should be reported valid if any analyte is positive, even if the internal control was not detected. If the internal control is not in channel 5, the user may select the "No" box and specify whether a valid result requires at least one analyte to be positive or not. It should be noted that the use of channel 5 for an internal control is only exemplary. In general, any channel may be used for an internal control. Thus, the sample validity criteria parameter screen (GUI) 6040 enables the user to specify whether an internal control will be used with the user defined assay, and to define different computer-executable criteria for determining if a test is valid depending on whether or not an internal control is being used. Thus, the user is able to program the system to assess the validity of tests while executing non-standard, non-preprogrammed assays on the system and without requiring the assistance of the system manufacturer or provider to program criteria into the system for assessing the test validity.

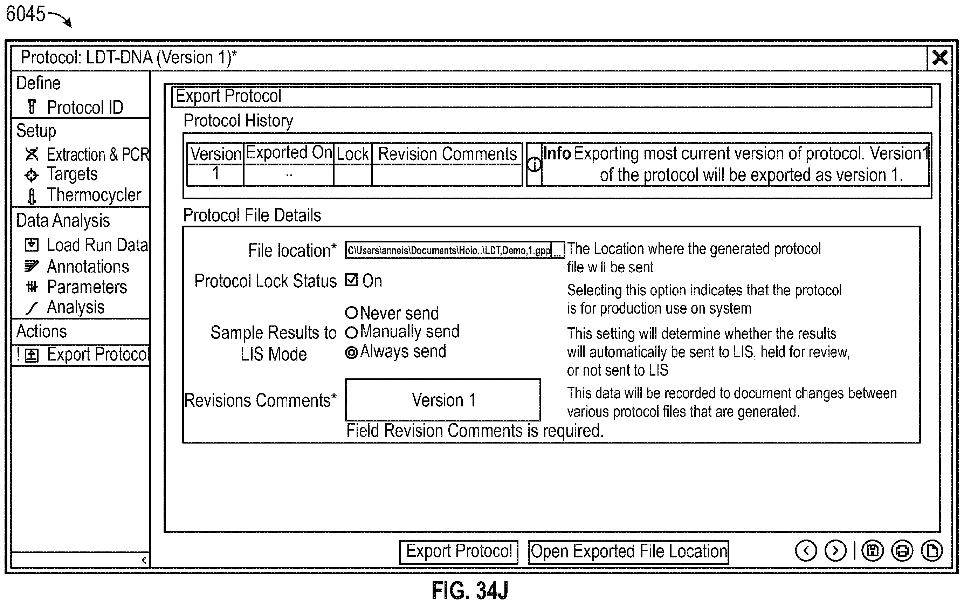

After parameters defining the assay have been selected or edited, a new or edited protocol may be exported from the software tool for installation on system 1000. The protocol may be exported by selecting "Export Protocol" under the "Actions" navigation pane of a screen (see, e.g., FIGS. 34C-34E) to open an export protocol screen 6045. FIG. 34J illustrates an exemplary export protocol screen (GUI) 6045 of the software tool. In some embodiments, before exporting a protocol, the file must be defined as locked or unlocked. Typically, a protocol under optimization (e.g., parameters have not been finalized) is denoted as an unlocked protocol. In some embodiments, a protocol is indicated as unlocked by default. The protocol can be indicated as being locked by selecting "On" under "Protocol Lock Status." A locked protocol may be unlocked by deselecting the "On" button. In some embodiments, making changes to a locked protocol will automatically change the file back to unlocked by default. Typically, a protocol is locked after protocol optimization is complete and all user-defined assay parameters have been finalized. In some embodiments, when a protocol is unlocked, results reporting to an LIS is disabled, and when a protocol is locked, results reporting to an LIS is enabled. "Sample Results to LIS Mode" options provide additional flexibility for reporting results to an LIS for a locked protocol. By selecting the appropriate option, a protocol can be locked with automatic, manual or no results reporting to an LIS.

Modification of the protocol under optimization may be tracked through version number and version comments during each export. In some embodiments, the user may be prompted to enter mandatory revision comments to both new and edited protocols. The revision comments may be displayed on the manage protocol screen 6000 (see FIG. 34A) along with a listing of the protocol revisions. After all the required fields in the export protocol screen 6045 have been filled, the "Export Protocol" button may be enabled. The "Export Protocol" button may be selected to export the protocol. In some embodiments, the exported file may have ".gpp" extension. As previously explained, in general, the exported protocol may be transferred to system 1000 wirelessly, via a wired connection, or via a portable memory. In some embodiments, a copy of the file may be saved to a portable memory device (e.g., memory stick, USB device, etc.) to install on system 1000. The software tool may also enable the user to backup the entire protocol library by selecting the Backup icon. Once selected, the tool may prompt the user to enter a file location for the Backup file. The backup file may be saved with a GSF file extension. After the file exported from the software tool (e.g., the ".gpp" file) is installed in system 1000, the assay may be run on system 1000 using the defined protocol and data (e.g., fluorescence vs. cycle number data) is saved. The saved data may then be exported from system 1000 to the software tool to visualize the data (e.g., process the raw data and visualize results).

In some embodiments, both raw data (data without applying the previously described curve correction, positivity criteria, channel validity criteria, sample validity criteria, etc.) and processed data (e.g., data processed by applying the user-defined assay parameters) may be exported by system 1000. In some embodiments, the raw data may be exported as a ".gpr" file and may be used to visualize amplification curves using the software tool. In some embodiments (e.g., when the protocol is being developed), the software tool may also be used to view the amplification curves and optimize the user-defined assay parameters. For example, some or all of the previously described user-defined assay parameters (parameters related to curve correction, positivity criteria, channel validity criteria, sample validity criteria, etc.) may be modified, the raw data processed using the modified user-defined assay parameters, and the results reviewed again. In some embodiments, in addition to raw data (i.e., the ".gpr" file), system 1000 may also export processed data and interpreted results (e.g., as a ".csv" file). This file may include information related to the analysis run in addition to processed data and interpreted results. The ".csv" file may be viewed in another program (e.g., Microsoft Excel^{®}). The processed data may be suitable for viewing processed results and trouble-shooting data related to locked protocols.

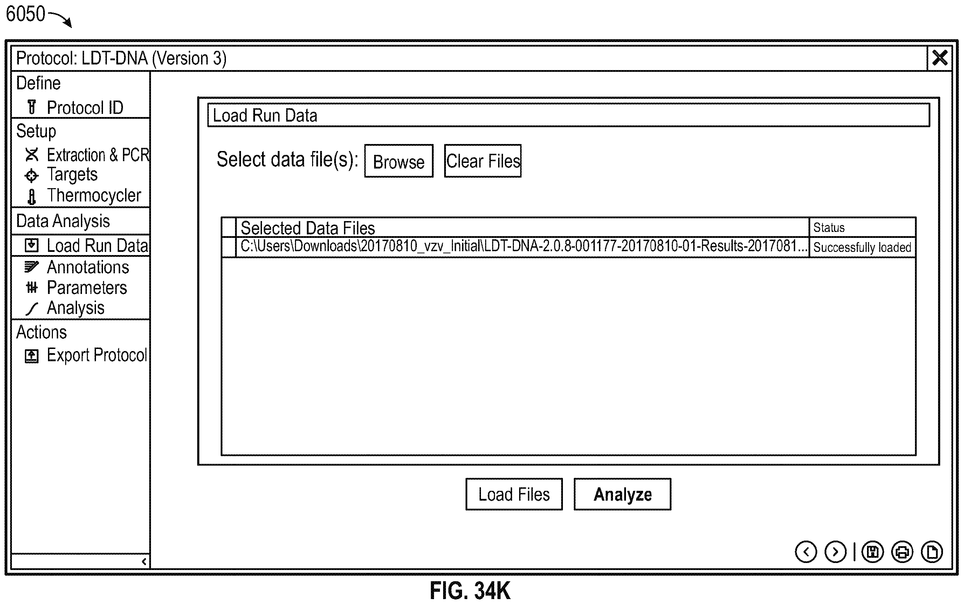

The data set from system 1000 for an assay may be transferred to the software tool wirelessly, via a wired connection, or via a portable memory device. The data set may include information and parameters related to the assay (e.g., the user-defined assay parameters for the protocol) and amplification curve data. The transferred assay data set from system 1000 is included in the list of available assay protocols displayed in the manage protocol screen 6000 (see FIG. 34A) of the software tool. To review the data, a desired protocol is selected and opened (e.g., by double clicking) from the list of presented options. The data associated with the selected protocol may be selected by clicking on the "Load Run Data" under "Data Analysis" in the navigation pane (see FIG. 34C). Clicking on this button may open a run data screen 6050. FIG. 34K illustrates an exemplary run data screen 6050 of the software tool. The desired data files (e.g., the ".gpr" file) may be selected by clicking on "Browse" and navigating to the file location and opening it. In some embodiments, the text identifying the file (e.g., file name) may turn color (e.g., to green) to indicate that the file is loaded. In some embodiments, the file name may turn to a different color (e.g., red) to indicate that the file has not loaded (e.g., indicate an error). After the desired data file is selected, the "Annotations" button (in the navigation pane) may be selected to annotate the data, and the "Analysis" button (or the "Analyze" button at the bottom of the screen) may be selected to view amplification curves.

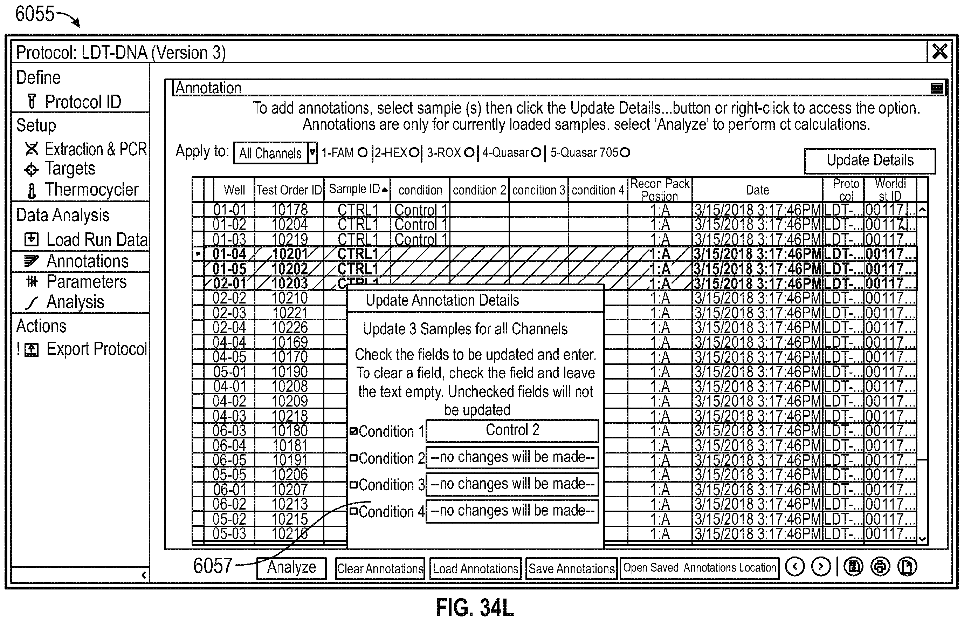

Selecting the "Annotations" button may open an annotations screen 6055 of the software tool. FIG. 34L illustrates an exemplary annotations screen (GUI) 6055. To annotate data, the desired samples are first selected (see three samples selected in FIG. 34L), and the "Update Details" button selected to open the update annotations details window 6057. See FIG. 34L. The desired annotations are then entered in the condition fields of window 6057. Selecting "Update" applies the entered annotations to the selected data. The applied annotations may be deleted or changed by editing the condition fields. The annotations can be used to associate details regarding the samples and/or run conditions to the test results.

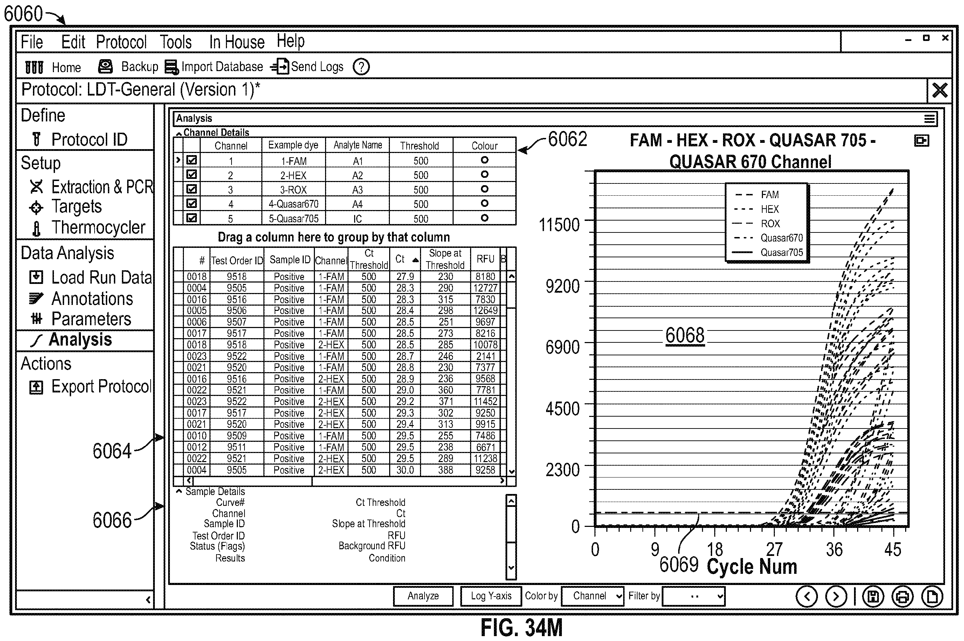

Clicking on the "Analysis" button may open an analysis screen 6060 of the software tool. FIG. 34M illustrates an exemplary analysis screen (GUI) 6060 of the software tool. Analysis screen 6060 includes, among others, a channel details table 6062, a sample analysis table 6064, a sample details portion 6066, and an analysis plot 6068. Channel details table 6062 lists the channels of the multi-channel signal detector of the system 1000 and the targeted analyte associated with each channel (e.g., as defined by the user using the target setup screen (GUI) 6015 (FIG. 34D) of the software tool). Channel details table 6062 allows a user to choose which channels (1-5) to include in sample analysis table 6064 and analysis plot 6068. The desired channels may be selected by clicking on the associated selection box for each channel in channel details table 6062. The color (or another distinguishable characteristic) associated with the data for each channel may also be selected in channel details table 6062. For example, a color dot in the "Color" cell of channel details table 6062 may be selected to change the color associated with the data for each selected channel. Data in the "Threshold" cell of channel details table 6062 may be modified to dynamically change the user-defined "Ct Threshold" value (recall that the "Ct Threshold" value for each channel and which need not be the same for each channel was selected by the user using positivity criteria parameter screen 6030 of FIG. 34G). After changing this data, clicking the "Analyze" button will update sample analysis table 6064. Ct threshold may be changed by changing the value of "Ct Threshold" in positivity criteria parameter screen 6030, by changing the value in "Threshold" cell of channel details table 6062, or by clicking and sliding a threshold indicator 6069 up or down in analysis plot 6068. After changing the Ct threshold, clicking the "Analyze" button will reprocess the data.

Sample analysis table 6064 includes the analysis output, settings, and run details for the loaded data. For example, as illustrated in FIG. 34M, data in sample analysis table 6064 indicates whether the analysis result for a sample is "Positive" (or negative) and related details (e.g., recorded "Ct," "Slope at Threshold," fluorescence ("RFU"), etc.). The configuration of the presented data in sample analysis table 6064 may be changed by the user. For example, the columns may be moved from side to side, samples may be grouped in any desired order, columns may be sorted (ascending, descending, etc.), etc. If a sample is selected in sample analysis table 6064 (e.g., by clicking on a row in the table), sample details portion 6066 will display details of the selected sample. Note that since none of the samples are selected in sample analysis table 6064 illustrated in FIG. 34M, no data is displayed in sample details portion 6066. Analysis plot 6068 displays the amplification curves for the samples selected in sample analysis table 6064. Typically, amplification curves of all samples are shown in analysis plot 6068 unless a subset of samples are selected in sample analysis table 6064. In some embodiments, analysis plot 6068 may include several options to change the way in which the plot is presented. For example, in addition to the options accessible through analysis screen 6060 of FIG. 34M, additional options may be accessed via context menus and/or other menus (e.g., accessible by icons, etc.) that present options tailored for different regions of analysis screen 6060. For example, in some embodiments, right clicking on a region of analysis screen 6060 (e.g., channel details table 6062, sample analysis table 6064, sample details portion 6066, or analysis plot 6068) may open a context menu that presents user selectable options relevant to that area. For example, using the options presented in context menus of analysis plot 6068, the title, axis settings, labeling, etc. of analysis plot 6068 may be changed. Context menus may also include features, such as, for example, copy, save, print preview, zoom/unzoom, etc. Other features of the plot 6068, for example, legends and other indicators (e.g., threshold indicator 6069) may be displayed or hidden, the analysis plot may be moved to a new window, analysis view and format may be changed, etc., using menu icons on the screen.

During development of an LDT, the user may use the results of the analysis to determine the appropriate parameter settings for the assay. For example, data in sample analysis table 6064 may indicate that the analysis result for a sample or a set of samples is positive. However, the user may suspect the validity or accuracy of the result, for example, based on other information (e.g., information in sample details portion 6066, prior information, etc.). The user may then change any desired data analysis parameter (e.g., "Analysis Start Cycle," "Ct Threshold," "Crosstalk Correction" parameters, etc.), reanalyze the data set from system 1000, and review the results again until the user is satisfied with the results (e.g., amplification curves in analysis plot 6068). The user may also use the results of the analysis to find the optimal chemistry of the reagents (e.g., formulation of fluids 1970A and 1970B, etc. in fluid-containing receptacles 1940 (see FIG. 11B) used in the LDT, etc.) and/or processing conditions (e.g., thermal cycling condition, etc.) for the LDT. For example, using the results as a guide, the user may reformulate a desired reagent and/or fine-tune the processing conditions to optimize the LDT. Thus, the user may use the software tool to optimize the values of the user-defined assay parameters for an LDT. After these parameters have been optimized and finalized, the LDT may be locked.

### Data Analysis Algorithm

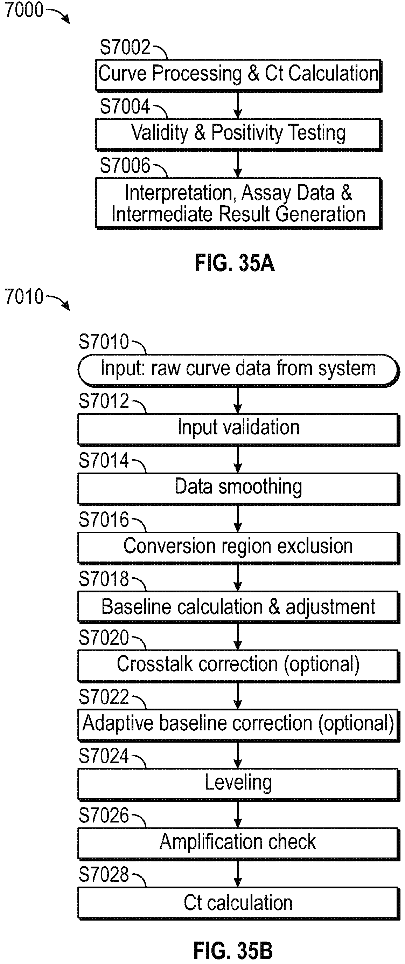

The software tool includes algorithms (one or more), installed on the computer system, that perform assay protocol definition and data analysis. For example, these algorithms analyze the data from system 1000 and present the analysis results in analysis screen 6060 (of FIG. 34M). An exemplary data analysis algorithm will be briefly described below. It should be noted that the described algorithm is only exemplary and many variations are possible and are within the scope of the current disclosure. FIG. 35A is a flow chart that illustrates an exemplary method 7000 used by the algorithms of the software tool to process and analyze data from system 1000. As illustrated in FIG. 35A, data from system 1000 is first processed by an algorithm that performs curve processing and Ct calculation (step S7002). In this step, the algorithm uses the user-defined assay parameters for curve correction (described previously with reference to FIG. 34F) to process the data and determine Ct for each channel. Throughout this discussion, the term "curve" is used to refer to a set of fluorescence measurements or adjusted versions thereof from a probe during a plurality of cycles of a cycled amplification reaction present as ordered pairs with the cycle or time at which they were acquired. The output of step S7002 may then be processed by one or more algorithms that perform validity and positivity testing (step S7004). During this step, the algorithm uses the user-defined assay parameters for positivity, channel, and sample validity criteria (previously described with reference to FIGS. 34G-34I) to determine if the computed Ct (in step S7002) is a valid result. The output of step S7004 is then processed to generate the intermediate results presented in the analysis screen 6060 of the software tool (step S7006). During parameter optimization, the user may modify any of the user-specified data analysis parameters (e.g., "Analysis Start Cycle," "Ct Threshold," "Baseline Correction," "Crosstalk Correction" parameters, etc.) and repeat some or all of the above described steps (i.e., steps S7002-S7006).

FIG. 35B is a flow chart that illustrates an exemplary method 7010 used by software tool during curve processing and Ct calculation (i.e., step S7002 of FIG. 35A). Raw data from system 1000 may be input into the software tool. In some embodiments, this raw data may also include additional data (e.g., for troubleshooting). Input validation may first be performed on the input data (step S7012). During input validation, all input parameters and curves are checked to verify their validity. During input validation, tests may be run to determine if data is missing from any cycle (e.g., if there is at least one measurement per cycle, if any invalid input parameter is present, etc.). In some embodiments, data reduction may also be performed during this step. For example, the input data may be averaged for each cycle for input validation. If the input data does not pass the input validation step (step S7012), a fatal error may be issued and data analysis stopped. Data smoothing may then be performed on the validated data (step S7014) to reduce raw data fluctuations. Data smoothing may be performed to ensure that the analysis is not affected by minor fluctuations in the measurement process by averaging a set number of points for a given cycle. Any type of smoothing algorithm (e.g., n-point moving average smoothing algorithm, polynomial fitting (Savitzky-Golay), etc.) may be applied to the raw data. In some smoothing algorithms, data may be averaged across, for example, 3, 4, 5, 6, 7, 8, 9, 10, or 11 cycles. In some embodiments, data may be averaged over five cycles. In some embodiments, no averaging may be performed on the first and last few cycles, e.g., cycles 1 to M/2 (rounded down) and N - M/2 (rounded up) to N, where M is the number of cycles used for smoothing an individual measurement (e.g., the moving average window size) and N is the number of cycles in the reaction, such as the first two and last two cycles (e.g., when M is 5). Typically, validation and smoothing of the raw data (i.e., steps S7012 and S7014) may be applied to the raw data without input from the user. That is, in some embodiments, the user may not be able to disable or change the preset parameters used by the algorithm in these steps. However, it is also contemplated that in some embodiments, the software tool may enable the user to make decisions (e.g., select whether to apply the validation and/or smoothing, the type of validation and/or smoothing algorithm to apply, define parameters related to the validation and/or smoothing algorithm, etc.) regarding the validation and/or smoothing step (steps S7012, S7014). In the conversion region exclusion step (step S7016), readings at the initial time period (e.g., the cycles before the user-defined "Analysis Start Cycle") are eliminated from the data used for subsequent Ct calculations.

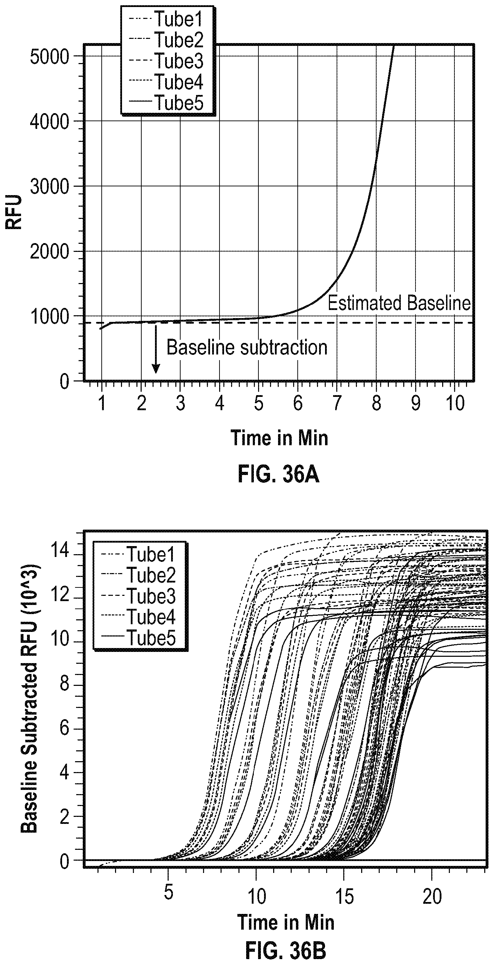

After the data has been smoothed in step S7014, and the unreliable variable points have been removed in step S7016, the data is adjusted based on a determined baseline level of fluorescence in step S7018. PCR curves typically have non-zero baseline measurements, which is due, at least in part, to assay chemistries and fluorometer optics. Each channel of a fluorometer corresponds to a different dye and, therefore, each channel may have a different level of background fluorescence affecting it. Thus, in some embodiments, step 7018 is performed for each channel of a fluorometer. In some embodiments, the baseline adjustment may involve both additive and multiplicative components. Baseline subtraction may be applied to the data to correct for additive components, and measurement scaling may be applied to the data to correct for multiplicative components. To reduce or eliminate multiplicative components, a scaling factor may be determined for a curve based on a commonly expected baseline, and the determined scaling factor applied to the curve. In some embodiments, the baseline measurements may be empirically decomposed into multiplicative and additive components. Examples of multiplicative components are variances in gain factors for a detector, and an example of an additive component is the inherent fluorescence of a reaction vessel. One technique to determine the multiplicative component of baseline fluorescence is to perform replicate reactions across multiple fluorometers. The difference in final RFU detected by different fluorometers may be indicative of the multiplicative component. One technique to determine the additive component of baseline fluorescence is to determine the fluorescence of the empty reaction vessel, which would be indicative of the additive component. Any type of baseline estimation algorithm (e.g., 4-parameter logistic regression model, 5-parameter logistic regression model, etc.) may be used to estimate the baseline in this step. In some embodiments, if the applied baseline estimation algorithm fails, data points bounded between two cycles (e.g., cycles 10 and 15) may be used to estimate the baseline. In some embodiments, the baseline calculation and subtraction step (step S7018) may be performed without input from the user. FIGS. 36A schematically illustrates estimating and subtracting the baseline from the data curve corresponding to one channel in an exemplary embodiment, and FIG. 36B illustrates the curves for all five channels after baseline subtraction has been applied. As can be seen from FIG. 36B, after baseline subtraction (step S7018), all the curves have the same baseline.

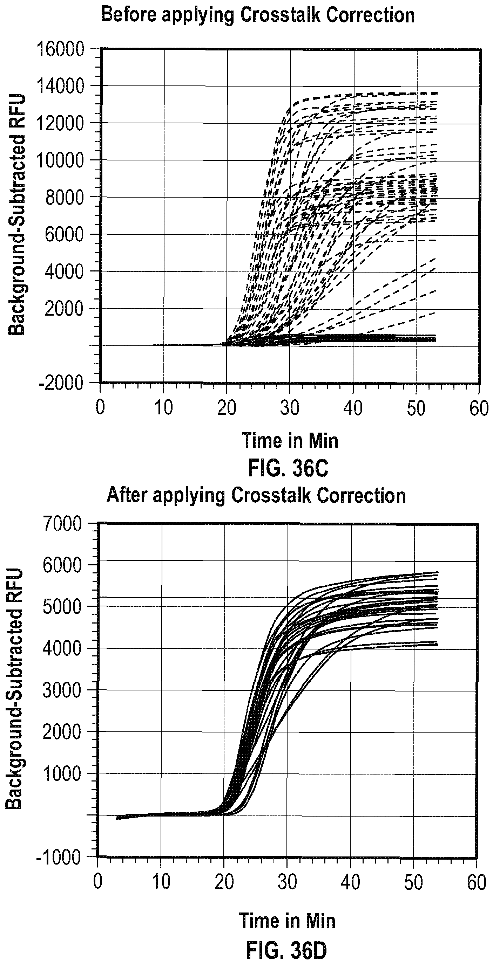

Crosstalk correction (step S7020) may then be applied to the data if enabled by the user. For example, if the user has not selected values for "Crosstalk Correction" parameters (or selected a value of 0%) in the curve correction parameter screen 6025 (see FIG. 34F), then this step is eliminated. Due to the overlap of spectra between some fluorophores, the fluorophore being excited in one channel may also be excited in a fraction of signals in an adjacent channel. So, in some embodiments, a signal bleed-through (or crosstalk) from Channel i (emitting channel) to Channel j (receiving channel) may be observed. This crosstalk signal may potentially lead to the false positive readings in the receiving channel. Based on the user-defined crosstalk correction fraction between Channel i and Channel j, in this step, the amount of crosstalk signals may be minimized numerically. Crosstalk correction is performed on baseline subtracted data and may require baseline subtracted data to be generated for all channels for a given curve. For example, crosstalk correction of a curve on Channel i may require the curve data from all other channels other than channel i. In some embodiments, the crosstalk correction step may be implemented in a modular manner in the software tool so that crosstalk correction may be modified without affecting other steps. FIGs. 36C and 36D illustrate the effect of applying crosstalk correction to the curves in an exemplary embodiment. FIG. 36C illustrate the curves before crosstalk correction is applied and FIG. 36D illustrate the curves after crosstalk correction is applied. Crosstalk correction can be performed to eliminate or reduce bleed-through signal from another reaction vessel (e.g., tube) in close proximity to the vessel from which the data were acquired. For example, neighboring vessels in a holder, comprising fluorophores with overlapping spectra (e.g., fluorophores that are the same or have indistinguishable spectra), may be in sufficient proximity for bleed-through signal to occur. Crosstalk correction can also be performed to eliminate or reduce bleed-through signal from another fluorophore in the same vessel that has a partially overlapping spectrum.

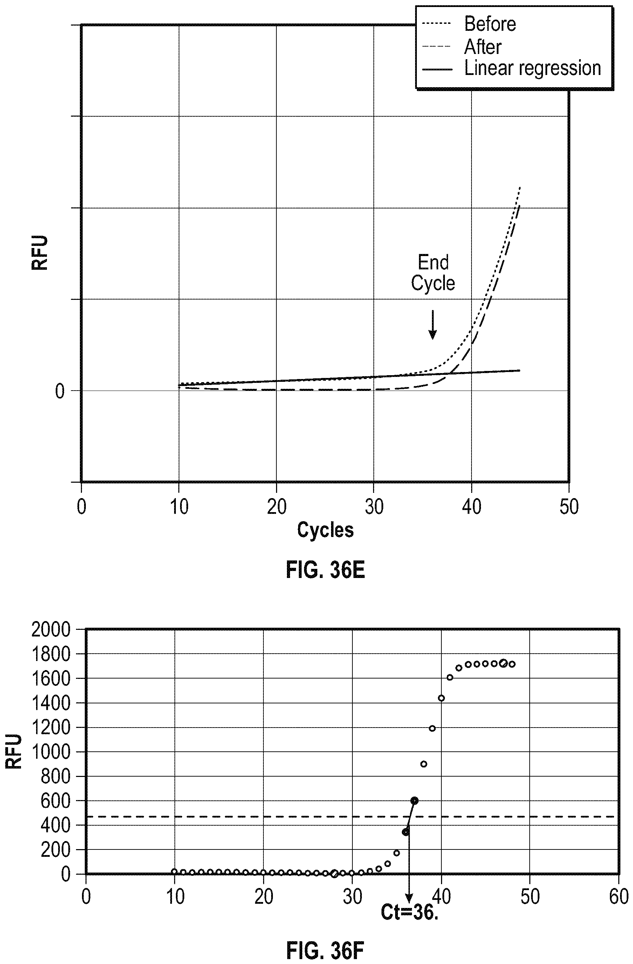

In some amplification assays, baseline drifting (e.g., baseline ramping up) is observed due to the poor quenching of fluorophores, especially towards the end of the baseline cycles. That is, due to baseline drifting, the curves ramp up prematurely. Baseline drifting may have an adverse impact on the calculation of Ct when the ramping baseline creeps into the linear regression region used for the Ct calculation. Therefore, if enabled by the user in curve correction parameter screen 6025 (see FIG. 34F), in the adaptive baseline correction step (step S7022), the algorithm corrects for the ramping baseline by, for example, (1) determining the baseline segment; and (2) subtracting a value dependent on the slope of the baseline segment and the time or cycle at which the measurements were taken. The baseline segment for purposes of this step can be identified by determining a slope between each adjacent pair of cycles of the plurality of cycles of the amplification reaction, at least until a predetermined slope is reached or exceeded for a pair of cycles, and identifying the baseline segment as consisting of fluorescence measurements from cycles earlier than the later of the pair of cycles for which the predetermined slope was reached or exceeded. In some embodiments, the slope of the baseline segment can be determined using linear regression. In some embodiments, the slope may be calculated based on the smoothed amplification curve or a regression fitted amplification curve (such as, for example, a four parameter logistic regressed curve). That is, the slope may be calculated based on smoothed observed data or fitted data. In this step, the ramping baseline of the curve is removed by subtracting the amount of ramping deviation calculated by the slope times the corresponding cycles, so that the curve is substantially flat and/or has a reduced slope relative to before the adaptive baseline correction step, such as a slope at or near 0, before true amplification begins. FIG. 36E illustrates the effect of applying adaptive baseline correction on an exemplary curve. The software tool may then apply leveling to the data (step S7024). Leveling may ensure that the lowest measurement of a curve is zero by finding the minimum value in a curve and subtracting that amount from all points in the curve. In some embodiments, leveling (i.e., step S7024) may be applied to the data without input from the user.

After baseline subtraction and noise reduction, in the amplification step (step S7026), the RFU range of the curve is calculated to distinguish negative curves from amplified curves (or positive curves). In some embodiments, RFU range may be calculated as maximum - minimum fluorescence for each channel. If the RFU range is less than or equal to a predetermined threshold, it is determined that the target nucleic acid analyte is not present in an amount equal to or greater than a predetermined limit of detection (assuming no validation errors). If the curve is positive (e.g., the RFU range is greater than a predetermined threshold), Ct is then calculated in the Ct calculation step (step S7028). Ct is calculated as the cycle number at which the measured fluorescence signal exceeds the user defined "Ct Threshold" (referred to below as the predetermined threshold) for curve emergence. FIG. 36F illustrates an exemplary method of calculating Ct. As illustrated in FIG. 36F, in some embodiments, in step S7028, Ct may be calculated using a two point Ct calculation method, e.g., using the cycle in which the earliest adjusted fluorescence measurement greater than or equal to the predetermined threshold occurred, the earliest adjusted fluorescence measurement greater than or equal to the predetermined threshold, and a fluorescence value of an adjusted fluorescence measurement from a cycle preceding the cycle in which the earliest adjusted fluorescence measurement greater than or equal to the predetermined threshold occurred. This can involve interpolation, such as linear interpolation, to provide a fractional Ct value (i.e., one which is not a whole number).

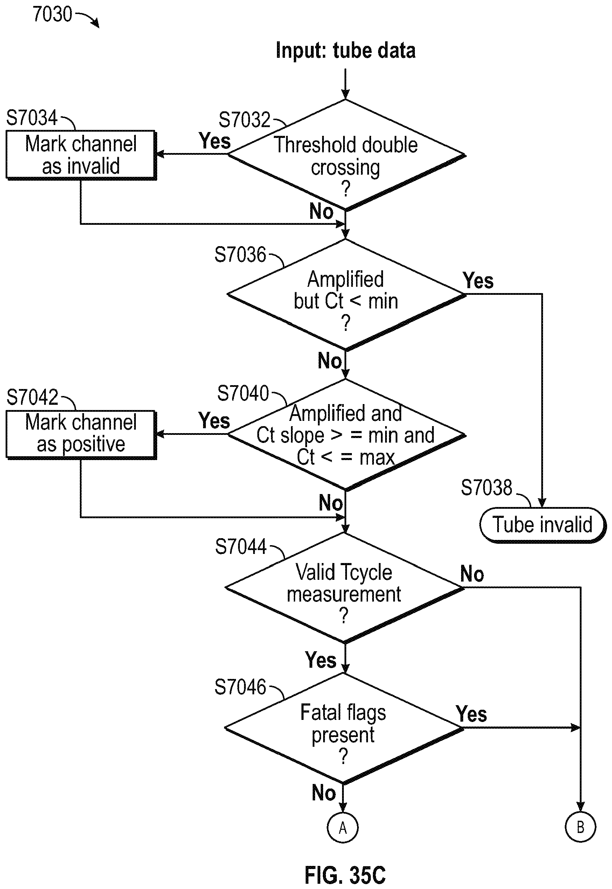

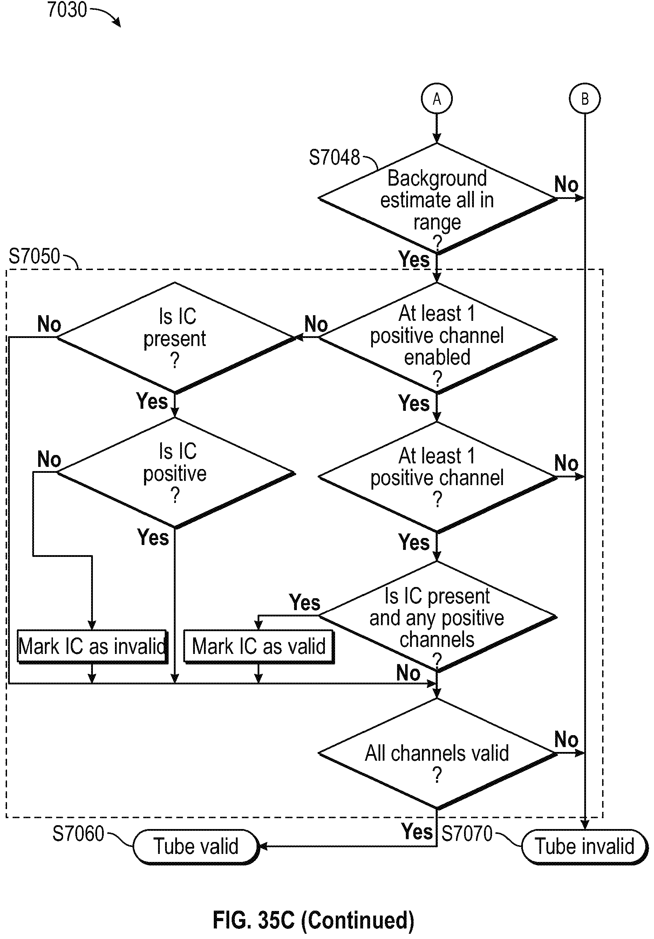

FIG. 35C is a flow chart that illustrates an exemplary method 7030 used by the algorithms of the software tool during validity and positivity testing (i.e., step S7004 of FIG. 35A). Data from each channel (or tube) is first tested for threshold double crossing (step S7032). In this step, any channel where the curve used to determine Ct is amplified but descends below the user-defined "Ct Threshold" value at a point after the calculated Ct is set as invalid (step S7034). In other words, testing for threshold double crossing comprises determining whether the adjusted fluorescence measurements comprise both (i) an adjusted fluorescence measurement greater than or equal to a predetermined threshold from a first cycle and (ii) an adjusted fluorescence measurement less than the predetermined from a second cycle later than the first cycle. The algorithm then checks to determine if the determined Ct value for any channel is less than the user-defined minimum value ("Lowest Valid Ct") (step S7036). If it is, the channel is marked invalid (step S7038). The algorithm then checks for the slope of the curve and the value of Ct for every channel (step S7040). In this step, the algorithm compares the slope of the curve at Ct with the user-defined value ("Minimum Slope at Threshold"), and the determined value of Ct with the user-defined permissible maximum value ("Maximum Ct"). If the slope is greater than or equal to (≥) the user-defined "Minimum Slope at Threshold," and Ct is less than or equal to (<_) the user-defined "Maximum Ct," the channel is marked as positive (step S7042). The algorithm then conducts a series of tests on the data from each channel. For example, the data from each channel is checked to determine if it represents a valid thermal cycler measurement (step S7044), if any fatal flags are present (step S7046), and if the background estimates are within an allowable range (step S7048). If any these tests fail, the channel is indicated to be invalid (step S7070). The algorithm then performs a series of tests on the positivity of the data (step S7050) based on the user settings in the sample validity criteria parameter screen 6040 (see FIG. 34I) and sets a channel to be valid (step S7060) or invalid (step S7070) based on the user-defined criteria.

### Installing and Running an Assay Protocol in System 1000

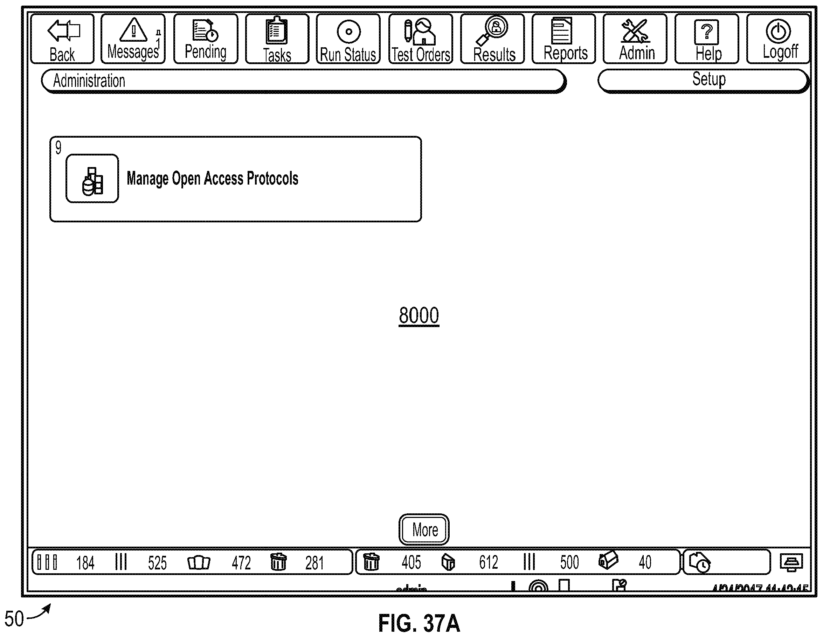

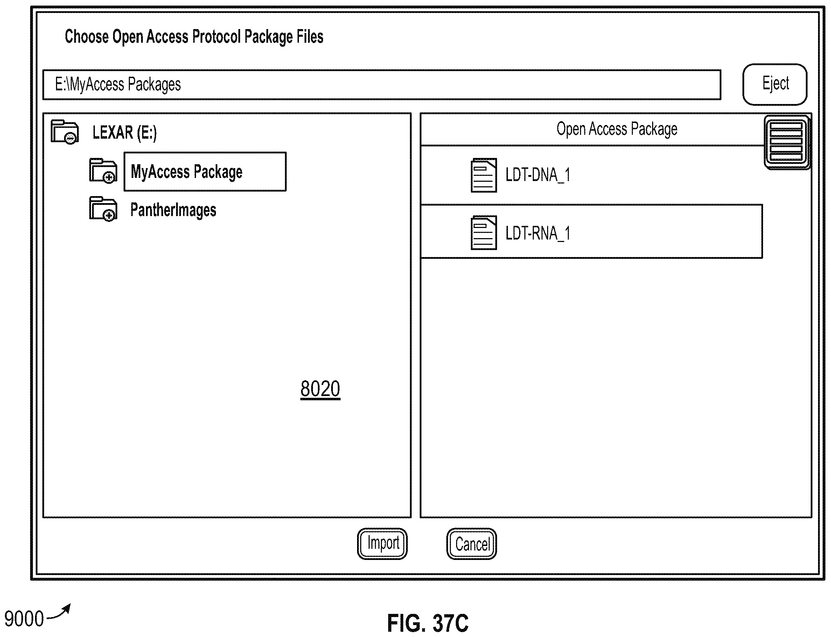

As previously explained, an assay protocol (e.g., an LDT protocol) developed using the software tool (which in some embodiments is installed in a computer system unconnected to, or separate from, system 1000) may be installed in system 1000 to perform the assay on samples. In some embodiments, the developed assay may be transferred to system 1000 in a USB device. The USB device with the assay protocol stored therein is inserted into a USB drive of system 1000, and the assay selected and installed using display device 50 (see FIG. 1) of system 1000. In some embodiments, it may be required to sign into system 1000 as an administrator to load assay protocols into system 1000. FIG. 37A illustrates display device 50 with the "Admin" option selected to open the administration screen 8000 on the display device 50. The "Manage Open Access Protocols" icon may then be selected to display the open access protocol screen 8010 on the display device 50. FIG. 37B illustrates the open access protocol screen 8010 in an exemplary embodiment. The available assay protocols (e.g., previously loaded) in system 1000 may be listed in the open access protocol screen 8010. A new assay protocol can be loaded on system 1000 by selecting "Import" from screen 8010 to open a protocol selection screen 8020. FIG. 37C illustrates the protocol selection screen 8020 in an exemplary embodiment with the available open access protocols in the USB device listed. A desired protocol is then selected and imported (e.g., by clicking in "Import"). These uploaded assays may then be added with all the other assays (IVD assays and LDTs) that have been previously loaded on system 1000. System 1000 may then run assays using the loaded assay protocol and save data which may then be exported from system 1000 to the software tool to process the data and visualize results as previously described.

The assays in system 1000 may be applied to (or associated with) samples that have been loaded in system 1000 (see FIG. 3C). During use, the user may associate the different patient samples in a sample bay to different available assays (IVD and LDTs) in system 1000. Samples may have test orders for both IVD assays and LDTs. The association of the samples with assays (or test orders) may be done on system 1000 (using display device 50) or externally (for example, using LIS) and then transferred (e.g., transmitted, uploaded, etc.) to system 1000. For example, with reference to FIGs. 3C, a user may associate different sample receptacles 107 or racks 10 of receptacles 107 (e.g., identified by reference number, barcode, etc.) with one or more assays (e.g., with one or more IVD assays and/or one or more LDTs, etc.) using LIS, and transfer a data file with this information to system 1000. And, when these sample receptacles 107 or racks 10 are inserted into sample bay 8, controller 5000 of system 1000 (see FIG. 33) may recognize the samples (e.g., based on readings from barcode reader 18, see FIG. 3B) and associate them with the user-selected assays.

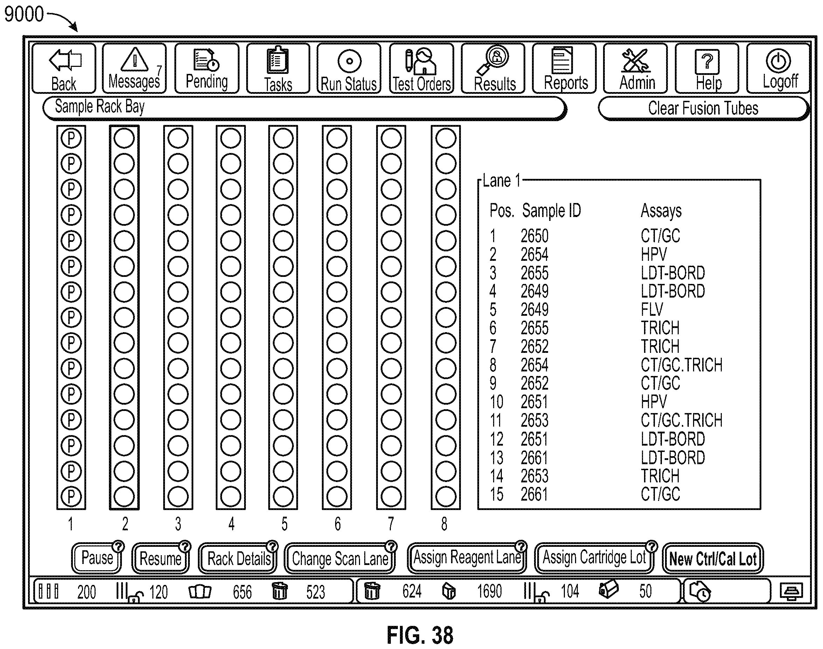

The association of samples with assays to be performed on the samples may also be done on system 1000. For example, a user may select one or more assays using display device 50, and the next rack 10 of samples (or receptacles 107) that are loaded on sample bay 8 may be associated with the user-selected assays. In some embodiments, a user may associate assays to samples after the samples have been loaded on system 1000. For example, a user reviews a list of sample receptacles 107 that are present in sample bay 8 (e.g., identified by some identifying information), and assigns/associates a desired set of assays to individual receptacles 107 or racks 10 of receptacles 107. In general, a user can assign a same set of assays to a rack 10 of receptacles 107 or to individual receptacles 107 in a rack 10. After the loaded samples are assigned an assay protocol, the specimen information for each sample rack is displayed in a sample rack screen 9000 on display device 50. FIG. 38 illustrates an exemplary sample rack screen 9000 displayed on display device 50 with corresponding sample IDs and assays listed. As can be seen in FIG. 38, multiple assays (HPV, CT/GC, etc.) have been associated to the same sample (e.g., sample ID 2654). In general, any number and type of assays (e.g., IVD and/or LDTs) may be assigned to the same sample (the number of assays will be limited only by the sample volume).

After the assays are associated with samples, controller 5000 of system 1000 schedules and performs the different assays in system 1000 in an efficient manner (e.g., to minimize throughput time, increase/improve work flow, etc.). During optimization of an LDT protocol on system 1000, it may be necessary to run a specific set of samples with fluids in specific user-provided receptacles (i.e., fluids 1970A, 1970B, etc. in fluid-containing receptacles 1940 of container 1920, see FIGs. 11A, 11B), which may be ASR receptacles. In some embodiments, to predict the sample processing order, when using multiple user-provided receptacles with the same reconstitution fluid, controller 5000 may schedule the test so that the user-provided receptacle with the lowest number of remaining tests (i.e., the tube with the lowest volume of fluid) is depleted first. If the tubes have the same number of tests, controller 5000 may use fluids from user-provided receptacles from positions A-D (i.e., from receptacle 1940 in position A of container 1920 first, then from receptacle 1940 in position B, etc., see FIG. 11A) from containers 1920 in positions 1-4 (i.e., from a container 1920 in position "Recon 1" first, then a container 1920 in position "Recon 2," etc., see FIG. 6D) of second reagent container-carrier 1600. When multiple receptacles containing user-provided reagents associated with different assay protocols are loaded on system 1000, controller 5000 may schedule tests according to which test was assigned first and may batch process loaded samples when possible. When PCR replicates are assigned for the same test, controller 5000 may run all PCR replicates from the same user-provided receptacle.

In some embodiments where an IVD assay and an LDT have been associated to the same sample, the sample eluate may be prepared jointly for both the assays (i.e., sample eluate preparation process 800 of FIG. 29 may be the common for both the assays). An aliquot of the common sample eluate may then be processed consistent with the IVD assay, and an aliquot may be processed consistent with the LDT. Although not a requirement, in some embodiments, at least some of the steps of the IVD assay and the LDT may be concurrently performed by system 1000. For example, some or all the steps of the reaction mixture preparation process 830 of FIG. 30 and/or process 850 (e.g., PCR reaction) of FIG. 31 may be performed concurrently (or simultaneously or in a parallel manner) for the IVD assay and LDT (with the reconstitution fluid from container 1920 used in step S838 for the LDT and reconstitution buffer from container 1620 used for step S838 for the IVD assay). Since thermal cycler 432 of system 1000 has multiple independently controlled thermal zones, the incubation step S858 (of process 850) for both the IVD assay and the LDTs can be concurrently performed even if both the assays have different thermal cycling conditions. However, performing the steps of the IVD assays and the LDTs in a concurrent manner is not a requirement. In some embodiments, controller 5000 may schedule some or all the steps of the IVD assay and the LDT in a serial manner.

As opposed to analytical systems that batch process IVD assays and LDTs (e.g., one of IVD assays or LDTs are performed first in one batch and then the other assays are performed in another batch), system 1000 may process IVD assays and LDTs in an interleaved and continuous manner. By "interleaved" is meant that the system 1000 can alternate between initiating and performing IVD assays and LDTs (or assays requiring ASR reagents) in a continuous and uninterrupted manner. For example, samples intended for processing in accordance with IVD assays and LDTs (or assays requiring ASR reagents) can be loaded together or consecutively on system 1000, and both types of assays can be performed seamlessly by the system without intervention (e.g., changing samples, reagents, and/or solvents) by the user. In this manner, some or all of the steps of the IVD assays and LDTs (or assays requiring ASR reagents) may be concurrently performed on the system 1000. Samples may also be loaded on system 1000 and associated with assays as the system is processing other samples. System 1000 may schedule and process the newly loaded samples along with the previously loaded samples without interruption in a continuous manner.

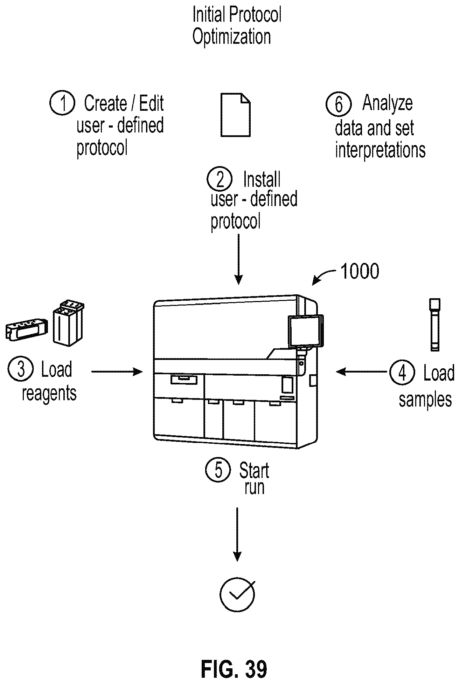

FIG. 39 is a schematic view of a workflow for protocol optimization using the software tool. In step (1) a user-defined protocol (i.e., an assay protocol that includes one or more user-defined assay parameters) is created or edited. The protocol is then exported and installed onto the system 1000 at step (2). Reagents, such as LDT reconstitution fluids 1970a, 1970b and lyophilized reagents are loaded onto the system 1000 at step (3). Samples are loaded into the system 1000 at step (4). At step (5), the system 1000 is started to run LDT assay in accordance with the user-defined protocol. In step (6), data collected during the essay is analyzed and interpreted. Based on the analysis and interpretation of the data, the user-defined protocol can be edited, thereby re-starting the process at step (1) and repeating the workflow.

While the present disclosure has been described and shown in considerable detail with reference to certain illustrative embodiments, including various combinations and sub-combinations of features, those skilled in the art will readily appreciate other embodiments and variations and modifications thereof as encompassed within the scope of the present disclosure. Moreover, the descriptions of such embodiments, combinations, and sub-combinations is not intended to convey that the disclosure requires features or combinations of features other than those expressly recited in the claims.

Although various embodiments of the present disclosure have been illustrated and described in detail, it will be readily apparent to those skilled in the art that various modifications may be made without departing from the present disclosure or from the scope of the appended claims.

## Claims

1. A method of performing a nucleic acid assay on an automated analyzer, the method comprising the steps of:
(a) presenting an interface on a computer enabling a user to use the computer to select, define, or modify one or more user-defined assay parameters of a protocol for extracting, amplifying and detecting a nucleic acid analyte on the analyzer;
(b) receiving user-defined assay parameters input to the interface by the user;
(c) assembling the protocol from the received user-defined assay parameters combined with one or more system-defined assay parameters;
(d) storing the protocol as a series of computer-executable instructions to be executed by the analyzer, wherein the user-defined assay parameters and the system-defined assay parameters of the protocol define steps executed by the analyzer to perform the nucleic acid assay; and
(e) executing the computer-executable instructions of the protocol with the analyzer to perform the nucleic acid assay, and collecting assay results data with the analyzer,
wherein, the user-defined assay parameters comprise data analysis parameters, wherein the data analysis parameters comprise computer-executable instructions to be executed by a data analysis computer for analyzing the assay results data collected by the analyzer during step (e), and wherein the method further comprises analyzing the assay results data collected during step (e) based on the data analysis parameters.

2. The method of claim 1, wherein step (e) is being executed as another nucleic acid assay is being performed on the analyzer in accordance with a protocol based solely on system-defined assay parameters.

3. The method of claim 1 or 2, wherein the computer is a personal computer that is not connected to the analyzer, and wherein step (d) comprises exporting the protocol from the personal computer and installing the protocol on the analyzer.

4. The method of any one of claims 1 to 3 wherein the user-defined assay parameters comprise one or more parameters of a thermal profile for performing a thermal cycling reaction, wherein the one or more parameters of the thermal profile comprise computer-executable instructions specifying thermal conditions to which a reaction mixture is to be exposed by the analyzer while performing the nucleic acid assay, the one or more parameters of the thermal profile including one or more of a temperature of each temperature step of the thermal cycling reaction, a duration of each temperature step, and a number of temperature cycles for the thermal cycling reaction, and wherein each cycle of the thermal cycling reaction comprises at least two discrete temperature steps.

5. The method of any one of claims 1 to 4, wherein the user-defined assay parameters comprise an analyte extraction parameter comprising computer-executable instructions to be executed by the analyzer for performing a process for extracting the nucleic acid analyte from a sample, and wherein step (e) comprises executing the computer-executable instructions of the analyte extraction parameter with the analyzer to perform the process for extracting the nucleic acid analyte from the sample, if present in the sample.

6. The method of any one of claims 1 to 5, wherein the user-defined assay parameters comprise a target parameter comprising computer-executable instructions specifying one or more channels of a multi-channel signal detector of the analyzer to be used in detecting the nucleic acid analyte, and wherein step (e) comprises executing the computer-executable instructions of the target parameter to determine the presence or absence of the nucleic acid analyte using the specified channels.

7. The method of any one of claims 1 to 6, wherein the data analysis parameters comprise curve correction parameters, wherein the curve correction parameters comprise computer-executable instructions specifying one or more modifications to be made by the data analysis computer to data collected during step (e), wherein the curve correction parameters comprise one or more of an analysis start cycle defining a cycle in the data before which any collected data is discarded, a baseline correction selectable to subtract background signal from the data, a baseline correction slope limit defining a curve slope above which baseline correction will not be applied, and a cross-talk correction parameter for suppressing channel-to-channel signal cross-talk, and wherein the method further comprises the data analysis computer modifying the collected assay results data in accordance with one or more of the analysis start cycle; the baseline correction, the baseline correction slope limit, and the cross-talk correction parameter.

8. The method of any one of claims 1 to 7, wherein the data analysis parameters comprise one or more data evaluation positivity criteria, and wherein the method further comprises the step of the data analysis computer determining a positive or negative result of the nucleic acid assay performed during step (e) based on the data evaluation positivity criteria.

9. The method of claim 8, wherein the one or more data evaluation positivity criteria comprise one or more of a signal threshold above which the presence of the nucleic acid analyte is indicated, a minimum slope at threshold defining a minimum slope of a curve crossing the signal threshold for which a positive result will be determined, and a maximum threshold cycle parameter defining a maximum number of cycles before the signal threshold is reached for which a positive result will be determined.

10. The method of any one of claims 1 to 9, wherein the data analysis parameters further comprise validity criteria parameters, and wherein the method further comprises the step of the data analysis computer determining if signals measured by a signal detector of the analyzer during step (e) are within expected ranges based on the validity criteria parameters.

11. The method of any one of claims 1 to 10, further comprising the step of presenting an interface enabling the user to specify a location within the analyzer for accessing one or more reagents for amplifying and detecting the nucleic acid analyte.

12. The method of any one of claims 1 to 11, further comprising the steps of:
computing results of the nucleic acid assay;
receiving modified user-defined assay parameters input to the interface by the user;
assembling a modified protocol from the modified user-defined inputs combined with one or more system-defined assay parameters;
storing the modified protocol as a series of computer-executable instructions to be executed by the analyzer;
executing the computer-executable instructions of the modified protocol with the analyzer to perform a modified nucleic acid assay; and
computing results of the modified nucleic acid assay.

13. The method of any one of claims 1 to 12, wherein step (d) comprises locking the protocol upon receipt of a lock command from the user to prevent further modification of the locked protocol.

14. An automated analyzer comprising a processor adapted to/configured to perform the steps of the method of any one of claims 1 to 13.

15. A computer program product comprising instructions which, when the program is executed by a computer, cause the computer to carry out the method of any one of claims 1 to 13; or
a computer-readable medium comprising instructions which, when executed by a computer, cause the computer to carry out the method of any one of claims 1 to 13.

## Patentansprüche

1. Verfahren zum Durchführen eines Nukleinsäuretests auf einem automatischen Analysegerät, wobei das Verfahren die folgenden Schritte umfasst:
(a) Darstellen einer Schnittstelle auf einem Computer, die es einem Benutzer ermöglicht, einen oder mehrere benutzerdefinierte Testparameter eines Protokolls zum Extrahieren, Verstärken und Detektieren eines Nukleinsäureanalyten auf dem Analysegerät auszuwählen, zu definieren oder zu ändern.
(b) Empfangen einer benutzerdefinierten Testparametereingabe in die Schnittstelle durch den Benutzer;
(c) Zusammenstellen des Protokolls aus den empfangenen benutzerdefinierten Testparametern kombiniert mit einem oder mehreren systemdefinierten Testparametern;
(d) Speichern des Protokolls als eine Serie von computerausführbaren Anweisungen, die von dem Analysegerät auszuführen sind, wobei die benutzerdefinierten Testparameter und die systemdefinierten Testparameter des Protokolls Schritte definieren, die von dem Analysegerät ausgeführt werden, um den Nukleinsäuretest durchzuführen; und
(e) Ausführen der computerausführbaren Anweisungen des Protokolls mit dem Analysegerät, um den Nukleinsäuretest durchzuführen, und Sammeln von Testergebnisdaten mit dem Analysegerät,
wobei die benutzerdefinierten Testparameter Datenanalyseparameter umfassen, wobei die Datenanalyseparameter computerausführbare Anweisungen umfassen, die von einem Datenanalysecomputer zum Analysieren der von dem Analysegerät im Verlauf von Schritt (e) gesammelten Testergebnisdaten auszuführen sind, und wobei das Verfahren ferner das Analysieren der im Verlauf von Schritt (e) gesammelten Testergebnisdaten auf Basis der Datenanalyseparameter umfasst.

2. Verfahren nach Anspruch 1, wobei Schritt (e) ausgeführt wird, während ein anderer Nukleinsäuretest auf dem Analysegerät gemäß einem Protokoll ausschließlich auf Basis von systemdefinierten Testparametern durchgeführt wird.

3. Verfahren nach Anspruch 1 oder 2, wobei der Computer ein Personalcomputer ist, der nicht mit dem Analysegerät verbunden ist, und wobei Schritt (d) das Exportieren des Protokolls von dem Personalcomputer und das Installieren des Protokolls auf dem Analysegerät umfasst.

4. Verfahren nach einem der Ansprüche 1 bis 3, wobei die benutzerdefinierten Testparameter einen oder mehrere Parameter eines thermischen Profils zum Durchführen einer thermischen Wechselreaktion umfassen, wobei der eine oder die mehreren Parameter des thermischen Profils computerausführbare Anweisungen umfassen, welche die thermischen Bedingungen angeben, denen ein Reaktionsgemisch mittels dem Analysegerät während der Durchführung des Nukleinsäuretests auszusetzen ist, wobei der eine oder die mehreren Parameter des thermischen Profils eines oder mehrere aus einer Temperatur jedes Temperaturschritts der thermischen Wechselreaktion, einer Dauer jedes Temperaturschritts und einer Anzahl von Temperaturzyklen für die thermische Wechselreaktion umfassen, und wobei jeder Zyklus der thermischen Wechselreaktion mindestens zwei getrennte Temperaturschritte umfasst.

5. Verfahren nach einem der Ansprüche 1 bis 4, wobei die benutzerdefinierten Testparameter einen Analytextraktionsparameter umfasst, der computerausführbare Anweisungen umfasst, die von dem Analysegerät zur Durchführung eines Prozesses zum Extrahieren des Nukleinsäureanalyten aus einer Probe ausgeführt werden, und wobei Schritt (e) das Ausführen der computerausführbaren Anweisungen des Analytextraktionsparameters mit dem Analysegerät umfasst, um den Prozess zum Extrahieren des Nukleinsäureanalyten aus der Probe durchzuführen, sofern er in der Probe vorliegt.

6. Verfahren nach einem der Ansprüche 1 bis 5, wobei die benutzerdefinierten Testparameter einen Zielparameter umfassen, der computerausführbare Anweisungen umfasst, die einen oder mehrere Kanäle eines Mehrfachkanal-Signaldetektors des Analysegeräts, das zum Detektieren des Nukleinsäureanalyten zu verwenden ist, spezifizieren, und wobei Schritt (e) das Ausführen der computerausführbaren Anweisungen des Zielparameters umfasst, um die Anwesenheit oder Abwesenheit des Nukleinsäureanalyten unter Verwendung der spezifizierten Kanäle zu bestimmen.

7. Verfahren nach einem der Ansprüche 1 bis 6, wobei die Datenanalyseparameter Kurvenkorrekturparameter umfassen, wobei die Kurvenkorrekturparameter computerausführbare Anweisungen umfassen, welche eine oder mehrere Änderungen spezifizieren, die von dem Datenanalysecomputer an den im Verlauf von Schritt (e) gesammelten Daten vorzunehmen sind, wobei die Kurvenkorrekturparameter eines oder mehrere aus Folgendem umfassen: einem Analysestartzyklus, der einen Zyklus in den Daten definiert, vor dem alle gesammelten Daten verworfen werden, eine Grundlinienkorrektur, die dazu auswählbar ist, ein Hintergrundsignal von den Daten zu subtrahieren, einer Grundlinienkorrektursteigungsgrenze, die eine Kurvensteigung definiert, oberhalb derer die Grundlinienkorrektur nicht angewendet wird, und einen Übersprechkorrekturparameter zum Unterdrücken von Signalübersprechen zwischen den Kanälen, und wobei das Verfahren ferner das Ändern der gesammelten Testergebnisdaten gemäß einem oder mehreren aus dem Analysestartzyklus, der Grundlinienkorrektur, der Grundlinienkorrektursteigungsgrenze und dem Übersprechkorrekturparameter durch den Datenanalysecomputer umfasst.

8. Verfahren nach einem der Ansprüche 1 bis 7, wobei die Datenanalyseparameter ein oder mehrere Datenbewertungs-Positivitätskriterien umfassen, und wobei das Verfahren ferner den Schritt des Bestimmens eines positiven oder negativen Ergebnisses des im Verlauf von Schritt (e) durchgeführten Nukleinsäuretests auf Basis der Datenbewertungs-Positivitätskriterien durch den Datenanalysecomputer umfasst.

9. Verfahren nach Anspruch 8, wobei das eine oder die mehreren Datenbewertungs-Positivitätskriterien eines oder mehrere aus Folgendem umfassen: einem Signalgrenzwert, oberhalb dem die Anwesenheit des Nukleinsäureanalyten erkennbar ist, eine minimale Steigung am Grenzwert, die eine minimale Steigung einer Kurve definiert, die den Signalgrenzwert, für den ein positives Ergebnis bestimmt wird, kreuzt, und einen maximalen Grenzwert-Zyklusparameter, der eine maximale Anzahl von Zyklen definiert, bevor der Signalgrenzwert, für den ein positives Ergebnis bestimmt wird, erreicht wird.

10. Verfahren nach einem der Ansprüche 1 bis 9, wobei die Datenanalyseparameter ferner Gültigkeitskriterienparameter umfassen, und wobei das Verfahren ferner den Schritt des Bestimmens durch den Datenanalysecomputer umfasst, ob von einem Signaldetektor des Analysegeräts im Verlauf von Schritt (e) gemessene Signale innerhalb erwarteter Bereiche liegen, auf Basis der Gültigkeitskriterienparameter.

11. Verfahren nach einem der Ansprüche 1 bis 10, ferner umfassend den Schritt des Darstellens einer Schnittstelle, welche es dem Benutzer ermöglicht, einen Ort innerhalb des Analysegeräts zum Zugreifen auf einen oder mehrere Reagenzien zum Verstärken und Detektieren des Nukleinsäureanalyten zu spezifizieren.

12. Verfahren nach einem der Ansprüche 1 bis 11, das ferner die folgenden Schritte umfasst:
Berechnen von Ergebnissen des Nukleinsäuretests;
Empfangen einer geänderten benutzerdefinierten Testparametereingabe in die Schnittstelle durch den Benutzer;
Zusammenstellen eines geänderten Protokolls aus den geänderten benutzerdefinierten Eingaben kombiniert mit einem oder mehreren systemdefinierten Testparametern;
Speichern des geänderten Protokolls als eine Serie von computerausführbaren Anweisungen, die vom Analysegerät auszuführen sind;
Ausführen der computerausführbaren Anweisungen des geänderten Protokolls mit dem Analysegerät, um einen geänderten Nukleinsäuretest durchzuführen; und
Berechnen von Ergebnissen des geänderten Nukleinsäuretests.

13. Verfahren nach einem der Ansprüche 1 bis 12, wobei Schritt (d) das Sperren des Protokolls nach Empfang eines Sperrbefehls von dem Benutzer umfasst, um eine weitere Änderung des gesperrten Protokolls zu verhindern.

14. Automatisches Analysegerät, umfassend einen Prozessor, der dazu geeignet/konfiguriert ist, die Schritte des Verfahrens nach einem der Ansprüche 1 bis 13 durchzuführen.

15. Computerprogrammprodukt, das Anweisungen umfasst, die, wenn das Programm von einem Computer ausgeführt wird, den Computer veranlassen, das Verfahren nach einem der Ansprüche 1 bis 13 durchzuführen, oder
computerlesbares Medium, das Anweisungen umfasst, die, wenn sie von einem Computer ausgeführt werden, den Computer veranlassen, das Verfahren nach einem der Ansprüche 1 bis 13 durchzuführen.

## Revendications

1. Procédé de réalisation d'un dosage d'acide nucléique sur un analyseur automatisé, le procédé comprenant les étapes de :
(a) présentation d'une interface sur un ordinateur permettant à un utilisateur d'utiliser l'ordinateur pour sélectionner, définir ou modifier un ou plusieurs paramètres de dosage définis par l'utilisateur d'un protocole d'extraction, d'amplification et de détection d'un analyte d'acide nucléique sur l'analyseur ;
(b) réception des paramètres de dosage définis par l'utilisateur entrés dans l'interface par l'utilisateur ;
(c) montage du protocole à partir des paramètres de dosage reçus définis par l'utilisateur, combinés avec un ou plusieurs paramètres de dosage définis par le système ;
(d) stockage du protocole sous forme d'une série d'instructions exécutables par ordinateur à exécuter par l'analyseur, dans lequel les paramètres de dosage définis par l'utilisateur et les paramètres de dosage définis par le système du protocole définissent les étapes exécutées par l'analyseur pour réaliser le dosage d'acide nucléique ; et
(e) exécution des instructions exécutables par ordinateur du protocole avec l'analyseur pour réaliser le dosage d'acide nucléique, et recueillir des données des résultats du dosage avec l'analyseur,
dans lequel les paramètres de dosage définis par l'utilisateur comprennent des paramètres d'analyse de données, dans lequel les paramètres d'analyse de données comprennent des instructions exécutables par ordinateur à exécuter par un ordinateur d'analyse de données pour analyser les données des résultats du dosage recueillies par l'analyseur pendant l'étape (e), et dans lequel le procédé comprend en outre l'analyse des données des résultats du dosage recueillies pendant l'étape (e) en fonction des paramètres d'analyse de données.

2. Procédé de la revendication 1, dans lequel l'étape (e) est exécutée alors qu'un autre dosage d'acide nucléique est réalisé sur l'analyseur conformément à un protocole basé uniquement sur des paramètres de dosage définis par le système.

3. Procédé de la revendication 1 ou 2, dans lequel l'ordinateur est un ordinateur personnel qui n'est pas connecté à l'analyseur, et dans lequel l'étape (d) comprend l'exportation du protocole depuis l'ordinateur personnel et l'installation du protocole sur l'analyseur.

4. Procédé de l'une quelconque des revendications 1 à 3, dans lequel les paramètres de dosage définis par l'utilisateur comprennent un ou plusieurs paramètres d'un profil thermique pour la réalisation d'une réaction de cyclage thermique, dans laquelle le ou les paramètres du profil thermique comprennent des instructions exécutables par ordinateur spécifiant les conditions thermiques auxquelles un mélange réactionnel doit être exposé par l'analyseur lors de la réalisation du dosage d'acide nucléique, le ou les paramètres du profil thermique incluant une ou plusieurs des températures de chaque étape de température de la réaction de cyclage thermique, une durée de chaque étape de température, et un nombre de cycles de température pour la réaction de cyclage thermique, et dans lequel chaque cycle de la réaction de cyclage thermique comprend au moins deux étapes de température distinctes.

5. Procédé de l'une quelconque des revendications 1 à 4, dans lequel les paramètres de dosage définis par l'utilisateur comprennent un paramètre d'extraction d'analyte comprenant des instructions exécutables par ordinateur à exécuter par l'analyseur pour réaliser un processus d'extraction de l'analyte d'acide nucléique à partir d'un échantillon, et dans lequel l'étape (e) comprend l'exécution des instructions exécutables par ordinateur du paramètre d'extraction d'analyte avec l'analyseur pour la réalisation d'un processus d'extraction de l'analyte d'acide nucléique à partir de l'échantillon, s'il est présent dans l'échantillon.

6. Procédé de l'une quelconque des revendications 1 à 5, dans lequel les paramètres de dosage définis par l'utilisateur comprennent un paramètre cible comprenant des instructions exécutables par ordinateur spécifiant un ou plusieurs canaux d'un détecteur de signal multi-canaux de l'analyseur à utiliser pour détecter l'analyte d'acide nucléique, et dans lequel l'étape (e) comprend l'exécution des instructions exécutables par ordinateur du paramètre cible pour déterminer la présence ou l'absence de l'analyte d'acide nucléique en utilisant les canaux spécifiés.

7. Procédé de l'une quelconque des revendications 1 à 6, dans lequel les paramètres d'analyse de données comprennent des paramètres de correction de courbe, dans lequel les paramètres de correction de courbe comprennent des instructions exécutables par ordinateur spécifiant une ou plusieurs modifications à apporter par l'ordinateur d'analyse de données aux données recueillies pendant l'étape (e), dans lequel les paramètres de correction de courbe comprennent un ou plusieurs des éléments parmi un cycle de début d'analyse définissant un cycle dans les données avant lequel toutes les données recueillies sont ignorées, une correction de ligne de base pouvant être sélectionnée pour soustraire le signal de fond des données, une limite d'inclinaison de correction de ligne de base définissant une inclinaison de courbe au-dessus de laquelle la correction de ligne de base ne sera pas appliquée, et un paramètre de correction de diaphonie pour supprimer la diaphonie entre les canaux du signal, et dans lequel le procédé comprend en outre la modification par l'ordinateur d'analyse de données des données des résultats du dosage recueillies conformément à un ou plusieurs des éléments parmi le cycle de début d'analyse ; la correction de ligne de base, la limite d'inclinaison de correction de ligne de base et le paramètre de correction de diaphonie.

8. Procédé de l'une quelconque des revendications 1 à 7, dans lequel les paramètres d'analyse de données comprennent un ou plusieurs critères de positivité d'évaluation de données, et dans lequel le procédé comprend en outre l'étape où l'ordinateur d'analyse de données détermine un résultat positif ou négatif du dosage d'acide nucléique réalisé pendant l'étape (e) en fonction des critères de positivité d'évaluation de données.

9. Procédé de la revendication 8, dans lequel un ou plusieurs critères de positivité d'évaluation de données comprennent un ou plusieurs des éléments parmi un seuil de signal au-dessus duquel la présence de l'analyte d'acide nucléique est indiquée, une inclinaison minimale au seuil définissant une inclinaison minimale d'une courbe traversant le seuil de signal pour laquelle un résultat positif sera déterminé, et un paramètre de cycle seuil maximum définissant un nombre maximum de cycles avant que le seuil de signal ne soit atteint pour lequel un résultat positif sera déterminé.

10. Procédé de l'une quelconque des revendications 1 à 9, dans lequel les paramètres d'analyse de données comprennent en outre des paramètres de critères de validité, et dans lequel le procédé comprend en outre l'étape où l'ordinateur d'analyse de données détermine si les signaux mesurés par un détecteur de signal de l'analyseur pendant l'étape (e) se situent dans les plages attendues en fonction des paramètres de critères de validité.

11. Procédé de l'une quelconque des revendications 1 à 10, comprenant en outre l'étape de présentation d'une interface permettant à l'utilisateur de spécifier un emplacement dans l'analyseur pour accéder à un ou plusieurs réactifs pour l'amplification et la détection d'analyte d'acide nucléique.

12. Procédé de l'une quelconque des revendications 1 à 11, comprenant en outre les étapes de :
calcul des résultats du dosage d'acide nucléique ;
réception des paramètres de dosage définis par l'utilisateur modifiés entrés dans l'interface par l'utilisateur ;
montage d'un protocole modifié à partir des entrées modifiées définies par l'utilisateur combinées avec un ou plusieurs paramètres de dosage définis par le système ;
stockage du protocole modifié sous forme d'une série d'instructions exécutables par ordinateur à exécuter par l'analyseur ;
exécution des instructions exécutables par ordinateur du protocole modifié avec l'analyseur pour réaliser un dosage d'acide nucléique modifié ; et
calcul des résultats du dosage d'acide nucléique modifié.

13. Procédé de l'une quelconque des revendications 1 à 12, dans lequel l'étape (d) comprend le verrouillage du protocole à la réception d'une commande de verrouillage de l'utilisateur pour empêcher toute modification ultérieure du protocole verrouillé.

14. Analyseur automatisé comprenant un processeur adapté/configuré pour réaliser les étapes du procédé de l'une quelconque des revendications 1 à 13.

15. Produit de programme informatique comprenant des instructions qui, lorsque le programme est exécuté par un ordinateur, amènent l'ordinateur à effectuer le procédé de l'une des revendications 1 à 13 ; ou
support lisible par ordinateur comprenant des instructions qui, lorsqu'elles sont exécutées par un ordinateur, amènent l'ordinateur à effectuer le procédé de l'une quelconque des revendications 1 à 13.
